# EUROPEAN PATENT SPECIFICATION

(11) **EP 1 047 418 B1**
(45) Date of publication and mention of the grant of the patent: **27.07.2005**
(21) Application number: 98965981.8
(22) Date of filing: 22.12.1998
(51) Int. Cl.: A61K 31/425, A61K 31/44, A61K 31/47, C07D 215/20, C07D 231/40, C07D 413/12, C07D 261/14, C07D 417/12, C07D 401/12, C07D 285/135, C07D 333/36, C07D 409/12

(54) **INHIBITION OF RAF KINASE USING SUBSTITUTED HETEROCYCLIC UREAS**
INHIBIERUNG DER RAF-KINASE DURCH SUBSTITUIERTE HETEROCYCLISCHE HARNSTOFFVERBINDUNGEN
INHIBITION DE RAF KINASE AU MOYEN D'UREES HETEROCYCLIQUES SUBSTITUEES

(30) Priority: 22.12.1997 US 996343
(43) Date of publication of application: 02.11.2000
(73) Proprietor: Bayer Pharmaceuticals Corp., West Haven, CT 06516 (US)
(72) Inventor: DUMAS, Jacques, Orange, CT 06477 (US); KHIRE, Uday, Hamden, CT 06518 (US); LOWINGER, Timothy, Bruno, Nishinomiya, Hyogo 662-0046 (JP); PAULSEN, Holger, D-42115 Wuppertal (DE); RIEDL, Bernd, 42329 Wuppertal (DE); SCOTT, William, J., Guilford, CT 06437 (US); SMITH, Roger, A., Madison, CT 06443 (US); WOOD, Jill, E., Hamden, CT 06517 (US); HATOUM-MOKDAD, Holia, Hamden, CT 06514 (US); JOHNSON, Jeffrey, Branford, CT 06405 (US); LEE, Wendy, Hamden, CT 06518 (US); REDMAN, Aniko, Derby, CT 06418 (US); SIBLEY, Robert, North Haven, CT 06473 (US); RENICK, Joel, San Diego, CA 92177 (US)
(74) Representative: Weiss, Wolfgang, Dipl.-Chem. Dr.
(86) International application number: PCT/US1998/026078
(87) International publication number: WO 1999/032106

(56) References cited:
- EP-A- 0 242 666
- EP-A- 0 371 876
- WO-A-93/18028
- WO-A-94/14801
- WO-A-96/40673
- WO-A-97/40028
- WO-A-98/22103
- WO-A-98/52559
- WO-A-99/24398
- WO-A-99/32111
- WO-A-99/32455
- WO-A1-96/25157
- WO-A1-97/40028
- DE-A- 2 436 179
- US-A- 3 754 887
- US-A- 3 990 879
- US-A- 4 042 372
- US-A- 4 183 854
- US-A- 5 162 360
- US-A- 5 185 358
- US-A- 5 814 646
- BOULTON, A. J. ET AL: "Heterocyclic rearrangements. X. Generalized monocyclic rearrangement" J. CHEM. SOC. C (1967), (20), 2005-7 , XP000943551
- RUSSO, F. ET AL: "Synthesis of 2,6-substituted derivatives of 5H-1,3,4-thiadiazolo[3,2-a]-s triazine-5,7-dione" FARMACO, ED. SCI. (1978), 33(12), 972-83 , XP000943466
- FOUSSARD-BLANPIN, ODETTE: "Comparative pharmacodynamic study of variously substituted carboxamides o the central nervous system" ANN. PHARM. FR. (1982), 40(4), 339-50 , XP000943598
- WHITE, ANDREW D. ET AL: "Heterocyclic Ureas: Inhibitors of Acyl-CoA:Cholesterol O-Acyltransferase as Hypocholesterolemic Agents" J. MED. CHEM. (1996), 39(22), 4382-4395 , XP002918005
- GRANT, A. M. ET AL: "Hypotensive thiadiazoles" J. MED. CHEM. (1972), 15(10), 1082-4 , XP000944187
- KUBO, HIROSHI ET AL: "Herbicidal activity of 1,3,4-thiadiazole derivatives" J. AGR. FOOD CHEM. (1970), 18(1), 60-5 , XP000943800
- FORBES, IAN T. ET AL: "N-(1-Methyl-5-indolyl)-N'-(3-methyl-5-iso thiazolyl)urea: A Novel, High-Affinity 5-HT2B Receptor Antagonist" J. MED. CHEM. (1995), 38(6), 855-7 , XP000941493
- CHEMICAL ABSTRACTS, Vol. 116, No. 21, 25 May 1992, (Columbus, Ohio, USA), pages 741-742, Abstract No. 214456r, KUJUNDZIC N., "Synthesis of 8-Methyl-1,2,3,4-Tetrahydropyrido(3,4-d)Pyr imidine-2,4-Diones", XP002938205 & CROAT. CHEM. ACTA, 1991, 64(4), 599-606, (Eng).
- WHITE et al., "Heterocyclic Ureas: Inhibitors of Acyl-CoA: Cholesterol O-Acyltransferase as Hypocholesterolemic Agents", J. MED. CHEM., 25 October 1996, Vol. 39, No. 22, pages 4382-4395, XP002918005

## Description

### Field of the Invention

This invention relates to the us eof a group of aryl ureas in treating raf mediated diseases, and pharmaceutical compositions for use in such therapy.

### Background of the Invention

The p21^{ras} oncogene is a major contributor to the development and progression of human solid cancers and is mutated in 30% of all human cancers (Bolton et al. *Ann*. *Rep. Med. Chem*. **1994,** *29,* 165-74; Bos. *Cancer Res.* **1989,** *49,* 4682-9). In its normal, unmutated form, the ras protein is a key element of the signal transduction cascade directed by growth factor receptors in almost all tissues (Avruch et al. *Trends Biochem. Sci.* **1994,** *19,* 279-83). Biochemically, ras is a guanine nucleotide binding protein, and cycling between a GTP-bound activated and a GDP-bound resting form is strictly controlled by ras' endogenous GTPase activity and other regulatory proteins. In the ras mutants in cancer cells, the endogenous GTPase activity is alleviated and, therefore, the protein delivers constitutive growth signals to downstream effectors such as the enzyme raf kinase. This leads to the cancerous growth of the cells which carry these mutants (Magnuson et al. *Semin. Cancer Biol.* **1994,** *5*, 247-53). It has been shown that inhibiting the effect of active ras by inhibiting the raf kinase signaling pathway by administration of deactivating antibodies to raf kinase or by co-expression of dominant negative raf kinase or dominant negative MEK, the substrate of raf kinase, leads to the reversion of transformed cells to the normal growth phenotype (see: Daum et al. *Trends Biochem. Sci.* **1994,** *19*, 474-80; Fridman et al. *J. Biol. Chem*. **1994**, *269*, 30105-8. Kolch et al. (*Nature* **1991,** *349,* 426-28) have further indicated that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human tumor types (Monia et al., *Nat. Med.* **1996,** *2*, 668-75).

Boulton et al. (J. Chem. Soc. 20 (1967), 20005-20007) describe heterocyclic rearrangement reactions. Boulton et aL do not disclose any medical use.

Russo et al. (Pharmaco. Sci. 33 (1978), 972-983) describe the synthesis of 2,6-substituted derivatives of 5H-1,3,4-thiadiazolo-[3,2-a]-s-triazine-5,7-dione and their anti-inflammatory activity. There is no disclosure of raf kinase inhibition.

Foussard-Blanpin et al. (An. Pharm. FR40 (1982), 339-350) disclose substituted carboxamides and their use as medicaments for disorders of the central nervous system. There is no indication of any medical use in combination with raf kinase inhibition.

White et al. (J. Med. Chem. 39 (1996), 4382-4395) discloses heterocyclic ureas as inhibitors of acyl-CoA: cholesterol O-acyl transferase. There is no indication of raf kinase inhibition.

Grant et al. (J. Med. Chem. 15 (1972), 1082-1084) describes thiadiazoles which have a hypertensive activity. There is no indication of raf kinase activity.

Kubo et al. (J. Agr. Food Chem. 18 (1970) 60-65) disclose 1,3,4-thiadiazole derivatives with herbicidal activity. There is no indication of raf kinase inhibition.

WO 97/40028 discloses urea derivatives as inhibitors of the IMPDH enzyme. There is no disclosure of raf kinase inhibition.

WO 99/24398 discloses chemical compounds which are inhibitors of the interaction between the integrin α₄β₁ and its protein ligand. There is no indication of raf kinase inhibition.

US patent 5,814,646 discloses the use of urea derivatives as inhibitors of amyloid beta-protein production. No raf kinase activity is disclosed.

WO 94/14801 discloses heterocyclic urea derivatives as 5HT_{2c} and 5HT_{2b} receptor antagonists. An inhibitory activity of raf kinase is neither disclosed not suggested.

WO 93/18028 discloses indole derivatives 5HT_{1c} antagonists. An inhibitory activity of raf kinase is neither disclosed nor suggested.

US patent 5,162,360 discloses substituted aryl-N-heterocyclic substituted urea or thioureas. The compounds are described as being suitable for the treatment of hypercholesterolemia and artherosclerosis. An inhibition of raf kinase is neither disclosed nor suggested.

EP-A-0 371 876 discloses isooxazole and isooxazoline compounds having anti-convulsivant activity. An inhibition of raf kinase is neither disclosed nor suggested.

US patent 4,042,372 discloses substituted thiadiazolo triazinediones which are active as herbicides, antimicrobial agents and antiviral agents. An inhibition of raf kinase is neither disclosed nor suggested.

US patent 3,990,879 discloses a substituted 1-thiadiazolyl-3-arylurea having herbicidal activity. An inhibition of raf kinase is neither disclosed nor suggested.

DE 2436179 discloses isooxazole derivatives having herbicidal activity. An inhibition of raf kinase is neither disclosed nor suggested.

EP-A-0 242 666 discloses a thiadiazolyl urea having herbicidal activity. An inhibition of raf kinase is neither disclosed nor suggested.

US patent 5,185,358 discloses aryl substituted urea derivatives for the treatment of hypercholesterolemia and artherosclerosis. An inhibition of raf kinase is neither disclosed nor suggested.

Forbes et al. (J. Med. Chem. 38 (1995) 855-857) disclose an indolyl isothiazolyl urea derivative which is a high affinity 5-HT_{2B} receptor antagonist. An inhibition of raf kinase is neither disclosed nor suggested.

WO 96/40673 discloses urea and thiourea compounds capable of modulating tyrosine signal transduction to prevent and treat cell proliferative disorders or cell differentiation disorders associated with particular tyrosine kinases by inhibiting one or more abnormal tyrosine kinase activities. There is no disclosure or suggestion that the claimed compounds are suitable for the treatment of tumors mediated by raf kinase. Further, there is no disclosure or suggestion of the specific substituent patterns as required by the compounds of the present invention.

WO 98/52559 discloses raf kinase inhibitors. This document, however, discloses compounds which differ in the patterns of substitutions from the compounds of the present invention.

WO 98/22103 discloses raf kinase inhibitors with a benzoamide structure which is fundamentally different from the urea structure of the compounds of the present invention.

### Summary of the Invention

The present invention provides compounds which are inhibitors of the enzyme raf kinase. Since the enzyme is a downstream effector of p21^{ras}, the instant inhibitors are useful in pharmaceutical compositions for human or veterinary use where inhibition of the raf kinase pathway is indicated, e.g., in the treatment of tumors and/or cancerous cell growth mediated by raf kinase. In particular, the compounds are useful in the treatment of human or animal, e.g., murine cancer, since the progression of these cancers is dependent upon the ras protein signal transduction cascade and therefore susceptible to treatment by interruption of the cascade, i.e., by inhibiting raf kinase. Accordingly, the compounds of the invention are useful in treating solid cancers, such as, for example, carcinomas (e.g., of the lungs, pancreas, thyroid, bladder or colon, myeloid disorders (e.g., myeloid leukemia) or adenomas (e.g., villous colon adenoma).

The present invention therefore provides compounds generally described as aryl ureas, including both aryl and heteroaryl analogues, which inhibit the raf pathway. The invention also provides the use of these compounds generally described as aryl ureas for the manufacture of medicaments for treating a raf mediated disease state in humans or mammals. Thus, the invention is directed to compounds and their use for the manufacture of a medicament for the treatment of cancerous cell growth mediated by raf kinase.

Therefore, in one aspect, the present invention concerns the use of a compound of Formula I: 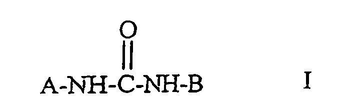 wherein the substituents A and B are defined as in claim 1.

In a further aspect, the present invention refers to a compound of the Formula 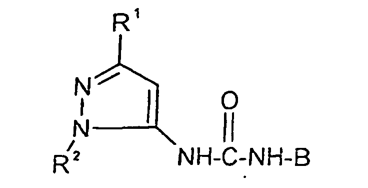 wherein the substituents R¹, R² and B are defined as in claim 31.

In yet a further aspect, the present invention also encompasses a compound of Formula 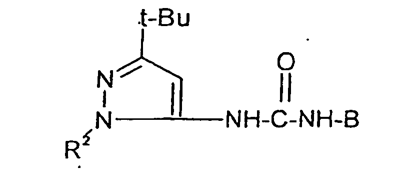 wherein the substituents R² and B are defined as in claim 35.

A still further aspect of the present invention is the compound of the Formula 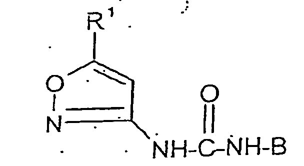 wherein the substituents R¹ and B are as defined in claim 37.

In a still further aspect the present invention refers to a compound of the Formula 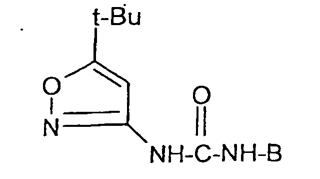 wherein the substituent B is as defined in claim 42.

In a still further aspect, the present invention encompasses a compound of the Formula 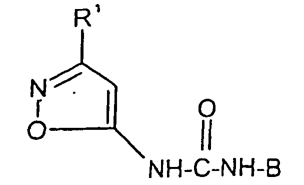 wherein R¹ and B are as defined in claim 43.

A still further aspect of the present invention is the compound of the Formula 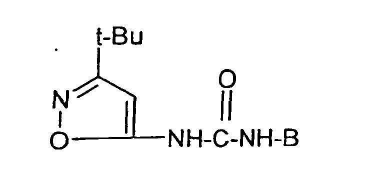 wherein the substituent B is as defined in claim 46.

In a still further aspect, the present invention encompasses a compound of the Formula 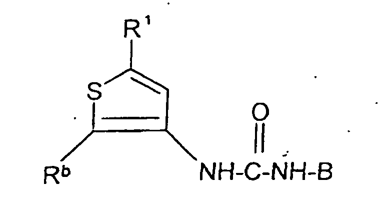 wherein the substituents R¹, R^{b} and B are in accordance with the definition in claim 49.

In a still further aspect the present invention relates to a compound of the Formula 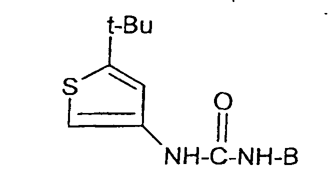 wherein the substituent B is in accordance with the definition in claim 52.

In a still further aspect, the present invention relates to a compound of the Formula 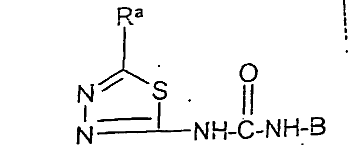 wherein the substituents R^{a} and B are as defined in claim 55.

A further aspect of the present invention concerns a compound of one of the Formulae 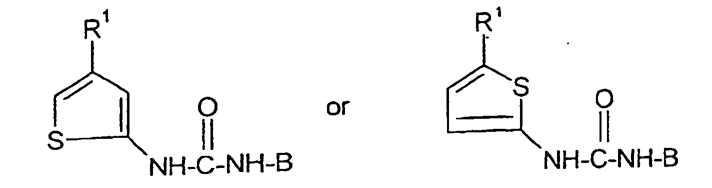 wherein the substituents R¹ and B are in accordance with the definition of claim 61.

In a still aspect, the present invention encompasses a compound of one of the Formulae 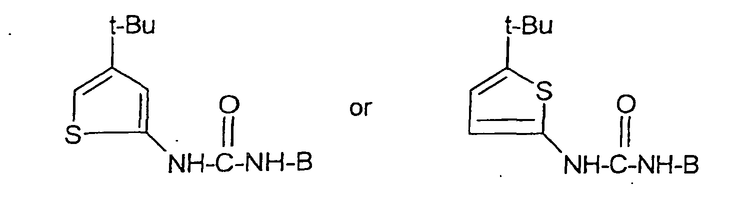 wherein the substituent B is as defined in claim 62.

In a still further aspect the present invention encompasses a compound of the Formula 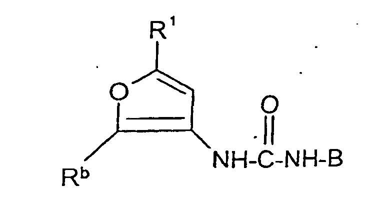 wherein the substituents R¹, R^{b} and B are as defined in claim 66.

In a still further aspect, the present invention relates to a compound of the Formula 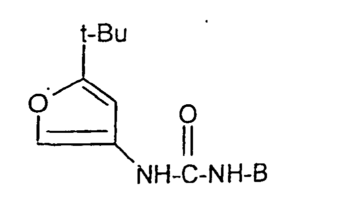 wherein B is as defined in claim 69.

Thus, in general, the invention is directed to aryl urea compounds and their use for the manufacture of a medicament for the treatment of cancerous cell growth mediated by raf kinase comprising a compound of Formula I: 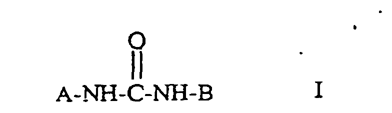 wherein B is generally an unsubstituted or substituted, up to tricyclic, aryl or heteroaryl moiety with up to 30 carbon atoms with at least one. 5 or 6 member aromatic structure containing 0-4 members of the group consisting of nitrogen, oxygen and sulfur as defined in the claims. A is a heteroaryl moiety discussed in more detail below.

The aryl and heteroaryl moiety of B may contain separate cyclic structures and can include a combination of aryl, heteroaryl and cycloalkyl structures. The substituents for these aryl and heteroaryl moieties are as defined in the claims and can vary widely and include halogen, hydrogen, hydrosulfide, cyano, nitro, amines and various carbon-based moieties, including those which contain one or more of sulfur, nitrogen, oxygen and/or halogen and are discussed more particularly below.

Suitable aryl and heteroaryl moieties for B of formula I include, but are not limited to aromatic ring structures containing 4-30 carbon atoms and 1-3 rings, at least one of which is a 5-6 member aromatic ring. One or more of these rings may have 1-4 carbon atoms replaced by oxygen, nitrogen and/or sulfur atoms.

Examples of suitable aromatic ring structures include phenyl, pyridinyl, naphthyl, pyrimidinyl, benzothiazolyl, quinoline, isoquinoline, phthalimidinyl and combinations thereof, such as, diphenyl ether (phenyloxyphenyl), diphenyl thioether (phenylthiophenyl), diphenylamine (phenylaminophenyl), phenylpyridinyl ether (pyridinyloxyphenyl), pyridinylmethylphenyl, phenylpyridinyl thioether (pyridinylthiophenyl), phenylbenzothiazolyl ether (benzothiazolyloxyphenyl), phenylbenzothiazolyl thioether (benzothiazolylthiophenyl), phenylpyrimidinyl ether, phenylquinoline thioether, phenylnaphthyl ether, pyridinylnapthyl ether, pyridinylnaphthyl thioether, and phthalimidylmethylphenyl.

Examples of suitable heteroaryl groups include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4-or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or-5-yl, 1- or 5-tetrazolyl, 1,2,3-oxadiazol-4- or -5-yl, 1,2,4-oxadiazol-3- or -5-yl, 1,3,4-thiadiazol-2-or -5-yl, 1,2,4-oxadiazol-3- or -5-yl, 1,3,4-thiadiazol-2- or -5-yl, 1,3,4-thiadiazol-3-or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl. 2-thiazolyl or 5-thiazolyl, etc. For example, B can be 4-methyl-phenyl, 5-methyl-2-thienyl. 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy, etc., throughout include methyl, ethyl, propyl, butyl, etc., including all straight-chain and branched isomers such as isopropyl, isobutyl, *sec*-butyl, *tert*-butyl, etc.

Suitable aryl groups include, for example, phenyl and 1- and 2-naphthyl.

Suitable cycloalkyl groups include cyclopropyl, cyclobutyl, cyclohexyl, etc. The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl" also includes saturated heterocyclic groups.

Suitable halogens include F, Cl, Br, and/or I, from one to persubstitution (i.e., all H atoms on the group are replaced by halogen atom), being possible, mixed substitution of halogen atom types also being possible on a given moiety.

As indicated above, these ring systems can be unsubstituted or substituted by substituents such as halogen up to per-halosubstitution. Other suitable substituents for the moieties of B include alkyl, alkoxy, carboxy, cycloalkyl, aryl, heteroaryl, cyano, hydroxy and amine. These other substituents, generally referred to as X and X' herein, include -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₂-C₁₀ alkenyl, C₁-C₁₀ alkoxy, C₁-C₁₀ cycloalkyl, C₆-C₁₄ aryl, C₇-C₂₄ alkaryl, C₃-C₁₃ heteroaryl, substituted C₁-C₁₀ alkyl, substituted C₂-C₁₀ alkenyl, substituted C₁-C₁₀ alkoxy, substituted C₃-C₁₀ cycloalkyl, : and -Y-Ar.

Where a substituent, X or X', is a substituted group, it is preferably substituted by one or more substituents independently selected from the group consisting of -CN, -CO₂R⁵, -C(O)R⁵, -C(O)NR⁵R^{5'}, -OR⁵, -SR⁵, -NR⁵R^{5'} , -NO₂, -NR⁵C(O)R^{5'}, -NR⁵C(O)OR^{5'} and halogen up to per-halo substitution.

The moieties R⁵ and R^{5'} are preferably independently selected from H. C₁-C₁₀ alkyl, C₂-C₁₀ alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂-C₁₀ alkenyl, up to per-halosubstituted C₃-C₁₀ cycloalkyl, up to per-halosubstituted C₆-C₁₄ aryl and up to per-halosubstituted C₃-C₁₃ heteroaryl.

The bridging group Y is preferably -O-, -S-, -N(R⁵)-, -(CH₂)-ₘ, -C(O)-, -CH(OH)-, -(CH₂)ₘO-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-, where m = 1-3, and X^{a} is halogen.

The moiety Ar is preferably a 5-10 member aromatic structure containing 0-4 members of the group consisting of nitrogen, oxygen and sulfur which is unsubstituted or substituted by halogen up to per-halosubstitution and optionally substituted by Zₙ₁, wherein n1 is 0 to 3.

Each Z substituent is preferably independently selected from the group consisting of -CN, -CO₂R⁵, -C(O)NR⁵R^{5'}, -C(O)- NR⁵, -NO₂, -OR⁵, - SR⁵, - NR⁵R^{5'}, -NR⁵C(O)OR^{5'}, =O, -NR⁵C(O)R^{5'}, -SO₂R⁵, -SO₂NR⁵R^{5'}, C₁-C₁₀ alkyl, C₁-C₁₀ alkoxy, C₃-C₁₀ cycloalkyl,
substituted C₁-C₁₀ alkyl, substituted C₃-C₁₀ cycloalkyl,
If Z is a substituted group, it is substituted by the one or more substituents independently selected from the group consisting of-CN, -CO₂R⁵, -C(O)NR⁵R^{5'}, -OR⁵, -SR⁵, -NO₂, -NR⁵R^{5'}, =O, -NR⁵C(O)R^{5'}, -NR⁵C(O)OR^{5'}, C₁-C₁₀ alkyl, C₁-C₁₀ alkoxy, C₃-C₁₀ cycloalkyl,

The aryl and heteroaryl moieties of B of Formula I are preferably selected from the group consisting of 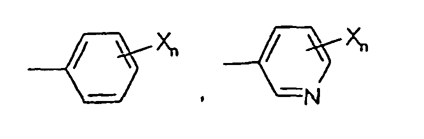 which are unsubstituted or substituted by halogen, up to per-halosubstitution. X is as defined above and n = 0-3.

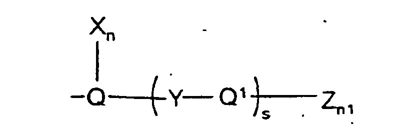

The aryl and heteroaryl moieties ofB are more preferably of the formula:
wherein Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂- and X^{a} is halogen.

Q is a six member aromatic structure containing 0-2 nitrogen, substituted or unsubstituted by halogen, up to per-halosubstitution and Q¹ is a mono- or bicyclic aromatic structure of 3 to 10 carbon atoms and 0-4 members of the group consisting of N, O and S, unsubstituted or unsubstituted by halogen up to per-halosubstitution. X, Z, n and n1 are as defined above and s = 0 or 1.

In preferred embodiments, Q is phenyl or pyridinyl, substituted or unsubstituted by halogen, up to per-halosubstitution and Q¹ is selected from the group consisting of phenyl, pyridinyl, naphthyl, pyrimidinyl, quinoline, isoquinoline, imidazole and benzothiazolyl, substituted or unsubstituted by halogen, up to per-haio substitution.

Z and X are preferably independently selected from the group consisting of -R⁶, -OR⁶, -SR⁶, and -NHR⁷, wherein R⁶ is hydrogen, C₁-C₁₀-alkyl or C₃-C₁₀-cycloalkyl and R⁷ is preferably selected from the group consisting of hydrogen, C,-C₁₀-alkyl, C₃-C₆-cycloalkyl and C₆-C₁₀-aryl, wherein R⁶ and R⁷ can be substituted by halogen or up to per-halosubstitution.

The heteroaryl moiety A of formula I is preferably selected from the group consisting of:

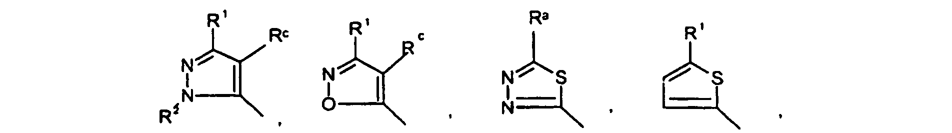

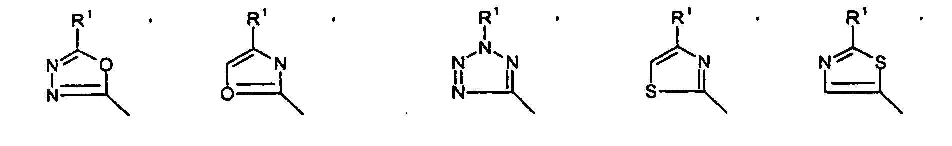

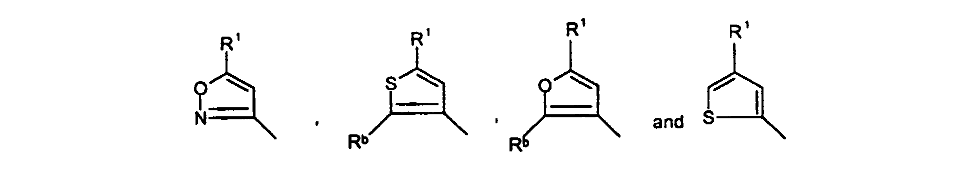

The substituent R¹ is preferably selected from the group consisting of halogen,C₃-C₁₀ alkyl, C₃-C₁₀ cycloalkyl, C₁-C₁₃ heteroaryl, C₆-C₁₃ aryl, C₁-C₂₄ alkaryl,_up to per-halosubstituted C₁-C₁₀ alkyl and up to per-halosubstituted C₃-C₁₀ cycloalkyl, up to per-halosubstituted C₁-C₁₃ heteroaryl, up to per-halosubstituted C₆-C₁₃ aryl and up to per-halosubstituted C₁-C₂₄ alkaryl.

The substituent R² is preferably selected from the group consisting of H, -C(O)R⁴, -CO₂R⁴, -C(O)NR³R^{3'}, C₁-C₁₀ alkyl, C₃-C₁₀ cycloalkyl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl. Where R² is a substituted group, it is preferably substituted by one or more substituents independently selected from the group consisting of -CN, - CO₂R⁴, -C(O)-NR³R^{3'}, -NO₂, -OR⁴, -SR⁴, and halogen up to per-halosubstitution.

R³ and R^{3'} are preferably independently selected from the group consisting of H, -OR⁴, -SR⁴, -NR⁴R^{4'}, -C(O)R⁴, -CO₂R⁴, -C(O)NR⁴R^{4'}, C₁-C₁₀ alkyl, C₃-C₁₀ cycloalkyl, C₆-C₁₄ aryl, C₃-C₁₃ heteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl.

R⁴ and R^{4'} are preferably independently selected from the group consisting of H, C₁-C₁₀ alkyl, C₃-C₁₀ cycloalkyl, C₆-C₁₄ aryl, C₃-C₁₃ heteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl.

R^{a} is preferably C₁-C₁₀ alkyl, C₃-C₁₀ cycloalkyl, up to per-halosubstituted C₁-C₁₀ alkyl and up to per-halosubstituted C₃-C₁₀ cycloalkyl.

R^{b} is preferably hydrogen or halogen.

R^{c} is hydrogen, halogen, C₁-C₁₀ alkyl, up to per-halosubstituted C₁-C₁₀ alkyl or combines with R¹ and the ring carbon atoms to which R¹ and R^{c} are bound to form a 5- or 6-membered cycloalkyl, aryl or hetaryl ring with 0-2 members selected from O, N and S;

The invention also relates to compounds of general formula I described above and includes pyrazoles, isoxazoles, thiophenes, furans and thiadiazoles. These more particularly include pyrazolyl ureas of the formula 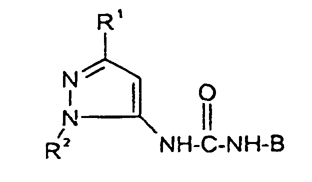 wherein R² , R¹ and B are as defined in the claims;
and both 5,3- and 3.5- isoxazolyl ureas of the formulae 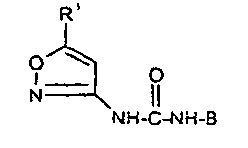 and 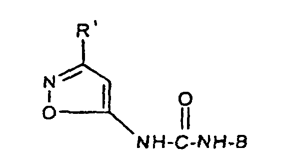 wherein R¹ and B are also as defined in the claims.

Component B for these compounds is a 1-3 ring aromatic ring structure selected from the group consisting of: 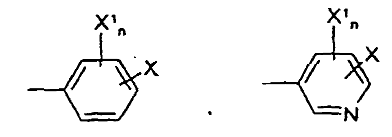 which is substituted or unsubstituted by halogen, up to per-halosubstitution. Here R⁵ and R^{5'} are as defined above, n = 0-2 and each X¹ substituent is independently selected from the group of X or from the group consisting of-CN, -CO₂R⁵, -C(O)R⁵, -C(O)NR⁵R^{5'}, -OR⁵, - NO₂, -NR⁵R^{5'}, C₁-C₁₀ alkyl, C₂₋₁₀-alkenyl, C₁₋₁₀-alkoxy, C₃-C₁₀ cycloalkyl, C₆-C₁₄ aryl and C₇-C₂₄ alkaryl.

The substituent X is selected from the group consisting of -SR⁵, -NR⁵C(O)OR^{5'}, NR⁵C(O)R^{5'}, C₃-C₁₃ heteroaryl, C₄-C₂₃ alkheteroaryl, substituted C₁-C₁₀ alkyl, substituted C₂₋₁₀-alkenyl, substituted C₁₋₁₀-alkoxy, substituted C₃-C₁₀ cycloalkyl, substituted C₆-C₁₄ aryl, substituted C₇-C₂₄ alkaryl, substituted C₃-C₁₃ heteroaryl, substituted C₄-C₂₃ alkheteroaryl, and -Y-Ar, where Y and Ar are as defined above. If X is a substituted group, as indicated previously above, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'} , NO₂, -NR⁵C(O)R^{5'}, -NR⁵C(O)OR^{5'} and halogen up to per-halosubstitution, where R⁵ and R^{5'} are as defined above.

The components of B are subject to the following provisos, where R¹ is t-butyl and R² is methyl for the pyrazolyl ureas, B is not

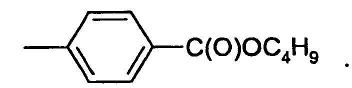

Where R¹ is t-butyl for the 5,3-isoxazolyl ureas, B is not 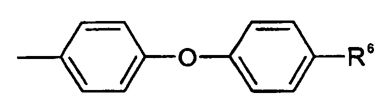 wherein R⁶ is -NHC(O)-O-t-butyl, -O-n-pentyl, -O-n-butyl, -O-propyl, -C(O)NH-(CH₃)₂, -OCH₂CH(CH₃)₂, or -O-CH₂ -phenyl. Where R¹ is t-butyl for the 3,5-isoxazole ureas, B is not 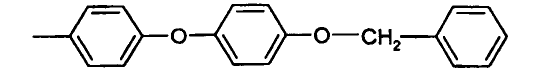 and where R¹ is -CH₂ -t-butyl for the 3,5 -isoxazolyl ureas, B is not

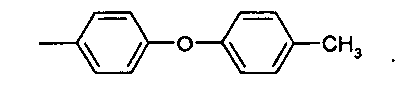

Preferred pyrazolyl ureas, 3,5-isoxazolyl ureas and 5,3-isoxazolyl ureas are those wherein B is of the formula 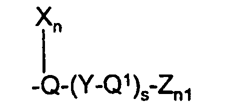 wherein Q, Q¹, X, Z, Y, n, s and n1 are as defined above.

Preferred pyrazole ureas more particularly include those wherein Q is phenyl or pyridinyl, Q¹ is pyridinyl, phenyl or benzothiazolyl, Y is -O-, -S-, -CH₂S-, -SCH₂-, -CH₂O-, -OCH₂- or-CH,-, and Z is H, -SCH₃, or -NH-C(O)-CₚH₂ₚ₋₁, wherein p is 1-4, n = 0, s = 1 and n1 = 0-1. Specific examples of preferred pyrazolyl ureas are:
*N*-(3-*tert*-Butyl-5-pyrazolyl)-*N'*-(4-phenyloxyphenyl)urea;
*N*-(3-*tert*-Butyl-5-pyrazolyl)-*N'*-(3-(3-methylaminocarbonylphenyl)-oxyphenyl)urea;
*N*-(3-*tert*-Butyl-5-pyrazolyl)-*N'*-(3-(4-pyridinyl)thiophenyl)urea;
*N*-(3-*tert*-Butyl-5-pyrazolyl)-*N'*-(4-(4-pyridinyl)thiophenyl)urea;
*N*-(3-*tert*-Butyl-5-pyrazolyl)-*N'*-(4-(4-pyridinyl)oxyphenyl)urea;
*N*-(3-*tert*-Butyl-5-pyrazolyl)-*N'*-(4-(4-pyridinyl)methylphenyl)urea;
*N*-(1-Methyl-3-*tert*-butyl-5-pyrazolyl)-*N'*-(4-phenyloxyphenyl)urea;
*N*-(1-Methyl-3-*tert*-butyl-5-pyrazolyl)-*N'*-(3-(4-pyridinyl)thiophenyl)urea;
*N*-(1-Methyl-3-*tert*-butyl-5-pyrazolyl)-*N'*-((4-(4-pyridinyl)thiomethyl)-phenyl)urea;

*N*-(1-Methyl-3-*tert*-butyl-5-pyrazolyl)-*N'*-(4-(4-pyridinyl)thiophenyl)urea;
*N*-(1-Methyl-3-*tert*-butyl-5-pyrazolyl)-*N'*-(4-(4-pyridinyl)oxyphenyl)urea;
*N*-(1-Methyl-3-*tert*-butyl-5-pyrazolyl)-*N'*-((4-(4-pyridinyl)methyloxy)phenyl)-urea;
*N*-(1-Methyl-3-*tert*-butyl-5-pyrazolyl)-*N'*-(3-(2-benzothiazolyl)oxyphenyl)-urea;
*N*-(3-*tert*-butyl-5-pyrazolyl)-*N'*-(3-(4-pyridyl)thiophenyl) urea;
*N*-(3-*tert*-butyl-5-pyrazolyl)-*N'*-(4-(4-pyridyl)thiophenyl) urea;
*N*-(3-*tert*-butyl-5-pyrazolyl)-*N'*-(3-(4-pyridyl)oxyphenyl) urea;
*N*-(3-*tert*-butyl-5-pyrazolyl)-*N'*-(4-(4-pyridyl)oxyphenyl) urea;
*N*-(1-methyl-3-*tert*-butyl-5-pyrazolyl)-*N'*-(3-(4-pyridyl)thiophenyl) urea;
*N*-(1-methyl-3-*tert*-butyl-5-pyrazolyl)-*N'*-(4-(4-pyridyl)thiophenyl) urea;
*N*-(1-methyl-3-*tert*-butyl-5-pyrazolyl)-*N'*-(3-(4-pyridyl)oxyphenyl) urea; and
*N*-(1-methyl-3-*tert*-butyl-5-pyrazolyl)-*N'*-(4-(4-pyridyl)oxyphenyl) urea.

Preferred 3,5-isoxazolyl ureas more particularly include those wherein Q is phenyl or pyridinyl, Q¹ is phenyl, benzothiazolyl or pyridinyl, Y is -O-, -S- or -CH₂-, Z is -CH₃, Cl, -OCH₃ or -C(O)-CH₃, n = 0, s = 1, and n1 = 0-1. Specific examples of preferred 3,5-isoxazolyl ureas are :
*N*-(3-Isopropyl-5-isoxazolyl)-*N'*-(4-(4-pyridinyl)thiophenyl)urea;
*N*-(3-*tert*-Butyl-5-isoxazolyl)-*N'*-(4-(4-methoxyphenyl)oxyphenyl)urea;
*N*-(3-*tert*-Butyl-5-isoxazolyl)-*N'*-(5-(2-(4-acetylphenyl)oxy)pyridinyl)urea;
*N*-(3-*tert*-Butyl-5-isoxazolyl)-*N'*-(3-(4-pyridinyl)thiophenyl)urea;
*N*-(3-*tert*-Butyl-5-isoxazolyl)-*N'*-(4-(4-pyridinyl)methylphenyl)urea;
*N*-(3-*tert*-Butyl-5-isoxazolyl)-*N'*-(4-(4-pyridinyl)thiophenyl)urea;
*N*-(3-*tert*-Butyl-5-isoxazolyl)-*N'*-(4-(4-pyridinyl)oxyphenyl)urea;
*N*-(3-*tert*-Butyl-5-isoxazolyl)-*N'*-(4-(4-methyl-3-pyridinyl)oxyphenyl)urea;
*N*-(3-*tert*-Butyl-5-isoxazolyl)-*N'*-(3-(2-benzothiazolyl)oxyphenyl)urea;
*N*-(3-(1,1-Dimethylpropyl)-5-isoxazolyl)-*N'*-(4-(4-methylphenyl)oxyphenyl)-urea;
*N*-(3-(1,1-Dimethylpropyl)-5-isoxazolyl)-*N'*-(3-(4-pyridinyl)thiophenyl)urea;
*N*-(3-(1,1-Dimethylpropyl)-5-isoxazolyl)-*N'*-(4-(4-pyridinyl)oxyphenyl)urea;
*N*-(3-(1,1-Dimethylpropyl)-5-isoxazolyl)-*N'*-(4-(4-pyridinyl)thiophenyl)urea;
*N*-(3-(1,1-Dimethylpropyl-5-isoxazolyl)-*N'*-(5-(2-(4-methoxyphenyl)oxy)-pyridinyl)urea;
*N*-(3-(1-Methyl-1-ethylpropyl)-5-isoxazolyl)-*N'*-(4-(4-pyridinyl)oxyphenyl)-urea;
*N*-(3-(1-Methyl-1-ethylpropyl)-5-isoxazolyl)-*N'*-(3-(4-pyridinyl)thiophenyl)-urea;

*N*-(3-isopropyl-5-isoxazolyl)-*N'*-(3-(4-(2-methylcarbamoyl)pyridyl)-oxyphenyl) urea;
*N*-(3-isopropyl-5-isoxazolyl)-*N'*-(4-(4-(2-methylcarbamoyl)pyridyl)-oxyphenyl) urea;
*N*-(3-*tert*-butyl-5-isoxazolyl)-*N'*-(3-(4-(2-methylcarbamoyl)-pyridyl)oxyphenyl) urea;
*N*-(3-*tert*-butyl-5-isoxazolyl)-*N'*-(4-(4-(2-methylcarbainoyl)pyridyl)-oxyphenyl) urea;
*N*-(3-*tert*-butyl-5-isoxazolyl)-*N'*-(3-(4-(2-methylcarbamoyl)pyridyl)-thiophenyl) urea;
*N*-(3-(1,1-dimethylprop-1-yl)-5-isoxazolyl)-*N'*-(3-(4-(2-methylcarbamoyl)-yridyl)oxyphenyl) urea;
*N*-(3-(1,1-dimethylprop-1-yl)-5-isoxazolyl)-*N'*-(4-(4-(2-methylcarbamoyl)-pyridyl)oxyphenyl) urea; and
*N*-(3-*tert*-butyl-5-isoxazolyl)-*N*'-(3-chloro-4-(4-(2-methylcarbamoyl)pyridyl)-thiophenyl) urea.

Preferred 5,3-isoxazolyl ureas more particularly include those wherein Q is is phenyl or pyridinyl, Q¹ is phenyl, benzothiazolyl or pyridinyl, Y is -O-, -S- or -CH₂-, X is CH, and Z is -C(O)NH-, CₚH₂ₚ₊₁, wherein p = 1-4, -C(O)CH₃, -CH₃, -OH, -OC₂H₅, -CN, phenyl, or -OCH₃, n = 0 or 1, s = 0 or 1, and n1 = 0 or 1. Specific examples of preferred 5,3-isoxazolyl ureas are:
*N*-(5-*tert*-Butyl-3-isoxazolyl)-*N'*-(4-(4-hydroxyphenyl)oxyphenyl)urea;
*N*-(5-*tert*-Butyl-3-isoxazolyl)-*N'*-(4-(3-hydroxyphenyl)oxyphenyl)urea;
*N*-(5-*tert*-Butyl-3-isoxazolyl)-*N'*-(4-(4-acetylphenyl)oxyphenyl)urea;
*N*-(5-*tert*-Butyl-3-isoxazolyl)-*N'*-(3-benzoylphenyl)urea;
*N*-(5-*tert*-Butyl-3-isoxazolyl)-*N'*-(4-phenyloxyphenyl)urea;
*N*-(5-*tert*-Butyl-3-isoxazolyl)-*N'*-(4-(3-methylaminocarbonylphenyl)-thiophenyl)urea;
*N*-(5-*tert*-Butyl-3-isoxazolyl)-*N'*-(4-(4-(1,2-methylenedioxy)phenyl)-oxyphenyl)urea;
*N*-(5-*tert*-Butyl-3-isoxazolyl)-*N'*-(4-(3-pyridinyl)oxyphenyl)urea;
*N*-(5-*tert*-Butyl-3-isoxazolyl)-*N'*-(4-(4-pyridinyl)oxyphenyl)urea;
*N*-(5-*tert*-Butyl-3-isoxazolyl)-*N'*-(4-(4-pyridyl)thiophenyl)urea;
*N*-(5-*tert*-Butyl-3-isoxazolyl)-*N'*-(4-(4-pyridinyl)methylphenyl)urea;
*N*-(5-*tert*-Butyl-3-isoxazolyl)-*N'*-(3-(4-pyridinyl)oxyphenyl)urea;
*N*-(5-*tert*-Butyl-3-isoxazolyl)-*N'*-(3-(4-pyridinyl)thiophenyl)urea;
*N*-(5-*tert*-Butyl-3-isoxazolyl)-*N'*-(3-(3-methyl-4-pyridinyl)oxyphenyl)urea;
*N*-(5-*tert*-Butyl-3-isoxazolyl)-*N'*-(3-(3-methyl-4-pyridinyl)thiophenyl)urea;
*N*-(5-*tert*-Butyl-3-isoxazolyl)-*N'*-(4-(3-methyl-4-pyridinyl)thiophenyl)urea;
*N*-(5-*tert*-Butyl-3-isoxazolyl)-*N'*-(3-(4-methyl-3-pyridinyl)oxyphenyl)urea;
*N*-(5-*tert*-Butyl-3-isoxazolyl)-*N'*-(4-(3-methyl-4-pyridinyl)oxyphenyl)urea;
*N*-(5-*tert*-Butyl-3-isoxazolyl)-*N'*-(3-(2-benzothiazolyl)oxyphenyl)urea;
*N*-(5-*tert*-butyl-3-isoxazolyl)-*N'*-(3-chloro-4-(4-(2-methylcarbamoyl)pyridyl)-oxyphenyl) urea;
*N*-(5-*tert*-butyl-3-isoxazolyl)-*N'*-(4-(4-(2-methylcarbamoyl)pyridyl)-oxyphenyl) urea;
*N*-(5-*tert*-butyl-3-isoxazolyl)-*N'*-(3-(4-(2-methylcarbamoyl)pyridyl)-thiophenyl) urea;
*N*-(5-*tert*-butyl-3-isoxazolyl)-*N'*-(2-methyl-4-(4-(2-methylcarbamoyl)pyridyl)-oxyphenyl) urea;
*N*-(5-*tert*-butyl-3-isoxazolyl)-*N'*-(4-(4-(2-carbamoyl)pyridyl)oxyphenyl) urea;
*N*-(5-*tert*-butyl-3-isoxazolyl)-*N'*-(3-(4-(2-carbamoyl)pyridyl)oxyphenyl) urea;
*N*-(5-*tert*-butyl-3-isoxazolyl)-*N'*-(3-(4-(2-methylcarbamoyl)pyridyl)-oxyphenyl) urea;
*N*-(5-*tert*-butyl-3-isoxazolyl)-*N'*-(4-(4-(2-methylcarbamoyl)pyridyl)-thiophenyl) urea;
*N*-(5-*tert*-butyl-3-isoxazolyl)-*N'*-(3-chloro-4-(4-(2-methylcarbamoyl)pyridyl)-oxyphenyl) urea; and
*N*-(5-*tert*-butyl-3-isoxazolyl)-*N'*-(4-(3-methylcarbamoyl)phenyl)oxyphenyl) urea.

Additionally included are thienyl ureas of the formulae 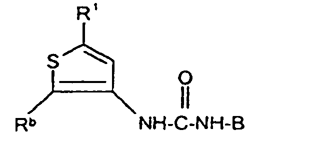 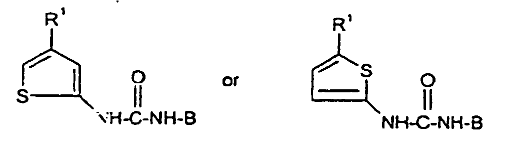 wherein R¹, R^{b} and B are as defined in the claims. Preferred B components for the thienyl ureas of this invention have aromatic ring structures selected from the group consisting of:

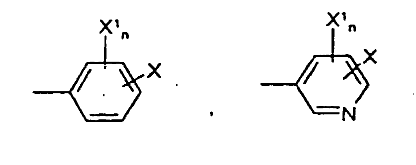

These aromatic ring structures can be substituted or unsubstituted by halogen, up to per-halosubstitution. The X¹ substituents are independently selected from the group consisting of X or from the group consisting of , -CN, -OR⁵, -NR⁵R^{5'}, C₁-C₁₀ alkyl. The X substituents are independently selected from the group consisting of -CO₂R⁵, -C(O)NR⁵R^{5'}, -C(O)R⁵, -NO₂, -SR⁵, -NR⁵C(O)OR^{5'}, -NR⁵C(O)R^{5'}, C₃-C₁₀ cycloalkyl, C₆-C₁₄ aryl, C₇-C₂₄ alkaryl, C₃-C₁₃ heteroaryl, C₄-C₂₃ alkheteroaryl, and substituted C₁-C₁₀ alkyl, substituted C₂₋₁₀-alkenyl, substituted C₁₋₁₀-alkoxy, substituted C₃-C₁₀ cycloalkyl, substituted C₆-C₁₄ aryl, substituted C₇-C₂₄ alkaryl, substituted C₃-C₁₃ heteroaryl, substituted C₄-C₂₃ alkheteroaryl, and -Y-Ar. Where X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} and halogen up to per-halo substitution. The moieties R⁵, R^{5'}, Y and Ar are as defined above and n = 0-2.

The components for B are subject to the proviso that where R¹ is t-butyl and R^{b} is H for the 3-thienyl ureas, B is not of the formula

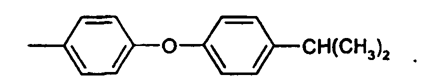

Preferred thienyl ureas include those wherein B is of the formula 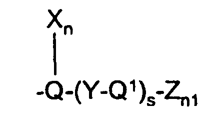 and Q, Q¹, Y, X, Z, n, s and n 1 are as defined above. The preferred thienyl ureas more particularly include those wherein Q is phenyl, Q¹ is phenyl or pyridinyl, Y is -0- or -S-, Z is -Cl, -CH₃, -OH or -OCH₃, n = 0, s = 0 or 1, and n1 = 0-2. Specific examples of preferred thienyl ureas are:
*N*-(3-Isopropyl-5-isoxazolyl)-*N'*-(4-(4-pyridinyl)thiophenyl)urea;
*N*-(3-*tert*-Butyl-5-isoxazolyl)-*N'*-(4-(4-methoxyphenyl)oxyphenyl)urea;
*N*-(3-*tert*-Butyl-5-isoxazolyl)-*N'*-(5-(2-(4-acetylphenyl)oxy)pyridinyl)urea;
*N*-(3-*tert*-Butyl-5-isoxazolyl)-*N'*-(3-(4-pyridinyl)thiophenyl)urea;
*N*-(3-*tert*-Butyl-5-isoxazolyl)-*N'*-(4-(4-pyridinyl)methylphenyl)urea;
*N*-(3-*tert*-Butyl-5-isoxazolyl)-*N'*-(4-(4-pyridinyl)thiophenyl)urea;
*N*-(3-*tert*-Butyl-5-isoxazolyl)-*N'*-(4-(4-pyridinyl)oxyphenyl)urea;
*N*-(3-*tert*-Butyl-5-isoxazolyl)-*N'*-(4-(4-methyl-3-pyridinyl)oxyphenyl)urea;
*N*-(3-*tert*-Butyl-5-isoxazolyl)-*N'*-(3-(2-benzothiazolyl)oxyphenyl)urea;
*N*-(3-(1,1-Dimethylpropyl)-5-isoxazolyl)-*N'*-(4-(4-methylphenyl)-oxyphenyl)urea;
*N*-(3-(1,1-Dimethylpropyl)-5-isoxazolyl)-*N'*-(3-(4-pyridinyl)thiophenyl)urea;
*N*-(3-(1,1-Dimethylpropyl)-5-isoxazolyl)-*N'*-(4-(4-pyridinyl)oxyphenyl)urea;
*N*-(3-(1,1-Dimethylpropyl)-5-isoxazolyl)-*N'*-(4-(4-pyridinyl)thiophenyl)urea;
*N*-(3-(1,1-Dimethylpropyl-5-isoxazolyl)-*N'*-(5-(2-(4-methoxyphenyl)-oxy)pyridinyl)urea;
*N*-(3-(1-Methyl-1-ethylpropyl)-5-isoxazolyl)-*N'*-(4-(4-pyridinyl)-oxyphenyl)urea; and
*N*-(3-(1-Methyl-1-ethylpropyl)-5-isoxazolyl)-*N'*-(3-(4-pyridinyl)thiophenyl)urea.

Preferred thiophenes include:
*N*-(5-*tert*-butyl-3-thienyl)-*N'*-(4-(4-methoxyphenyl)oxyphenyl) urea;
*N*-(5-*tert*-butyl-3-thienyl)-*N'*-(4-(4-hydroxyphenyl)oxyphenyl) urea;
*N*-(5-*tert*-butyl-3-thienyl)-*N'*-(4-(3-methylphenyl)oxyphenyl) urea; and
*N*-(5-*tert*-butyl-3-thienyl)-*N'*-(4-(4-pyridyl)thiophenyl) urea; and

Also included are the thiadiazolyl and furyl ureas of the formulae: 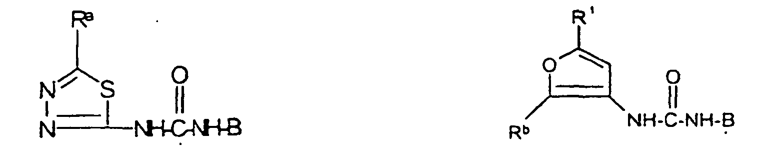 wherein R^{a}, R^{b}, R¹ and B are as defined in the claims. The thiadiazolyl and furyl ureas have preferred aromatic ring structures for B identical to those for the pyrazolyl, thienyl and isoxazolyl ureas shown above. Such ring structures can be unsubstituted or substituted by halogen, up to per-halosubstitution, and each X¹ substituent is independently selected from the group consisting of X or from the group consisting of -CN, -NO₂ ,-OR⁵ and C₁-C₁₀ alkyl. The X substituents are selected from the group consisting of -SR⁵, -CO₂R⁵, -C(O)R⁵, -C(O)NR⁵R^{5'}, -NR⁵R^{5'}, -NR⁵C(O)OR^{5'}, -NR⁵C(O)R^{5'}, substituted C₂₋₁₀-alkenyl, substituted C₁₋₁₀-alkoxy, -C₃-C₁₀ cycloalkyl, -C₆-C₁₄ aryl, -C₇-C₂₄, alkaryl, C₃-C₁₃ heteroaryl, C₄-C₂₃ alkheteroaryl, and substituted C₁-C₁₀ alkyl, substituted C₃-C₁₀ cycloalkyl, substituted aryl, substituted alkaryl, substituted heteroaryl, substituted C₄-C₂₃ alkheteroaryl and -Y-Ar. Each of R⁵, R^{5'} and Ar are as defined above, n = 0-2, and the substituents on X where X is a substituted group are as defined for the pyrazolyl, isoxazolyl and thienyl ureas.

This invention also includes pharmaceutical compositions that include compounds described above and a physiologically acceptable carrier.

Preferred furyl ureas and thiadiazole ureas include those wherein B is of the formula 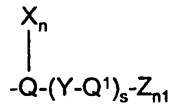 and Q, Q¹, X, Y, Z, n, s, and n1 are as defined above. The preferred thiadaizolyl ureas more particularly include those wherein Q is phenyl, Q¹ is phenyl or pyridinyl, Y is -O- or -S-, n = 0, s = 1 and n1 = 0. Specific examples of preferred thiadiazolyl ureas are:
*N*-(5-*tert*-Butyl-2-(1-thia-3,4-diazolyl))-*N'*-(3-(4-pyridinyl)thiophenyl)urea;
*N*-(5-*tert*-Butyl-2-(1-thia-3,4-diazolyl))-*N'*-(4-(4-pyridinyl)oxyphenyl)urea;
*N*-(5-*tert*-butyl-2-(1-thia-3,4-diazolyl))-*N'*-(3-(4-(2-methylcarbamoyl)pyridyl)-oxyphenyl) urea;
*N*-(5-*tert*-butyl-2-(1-thia-3,4-diazolyl))-*N'*-(4-(4-(2-methylcarbamoyl)pyridyl)-oxyphenyl) urea;
*N*-(5-*tert*-butyl-2-(1-thia-3,4-diazolyl))-*N'*-(3-chloro-4-(4-(2-methylcarbamoyl)pyridyl)oxyphenyl) urea;
*N*-(5-*tert*-butyl-2-(1-thia-3,4-diazolyl))-*N'*-(2-chloro-4-(4-(2-methylcarbamoyl)pyridyl)oxyphenyl) urea;
*N*-(5-*tert*-butyl-2-(1-thia-3,4-diazolyl))-*N'*-(3-(4-pyridyl)thiophenyl) urea;
*N*-(5-*tert*-butyl-2-(1-thia-3,4-diazolyl))-*N'*-(2-methyl-4-(4-(2-methylcarbamoyl)pyridyl)oxyphenyl) urea; and
*N*-(5-(1,1-dimethylprop-1-yl)-2-(1-thia-3,4-diazolyl))-*N'*-(4-(3-carbamoylphenyl)oxyphenyl) urea.

The preferred furyl ureas more particularly include those wherein Q is phenyl, Q¹ is phenyl or pyridinyl, Y is -O- or -S-, Z is -Cl or -OCH₃, s = 0 or 1, n = 0 and n1 = 0-2.

The present invention is also directed to pharmaceutically acceptable salts of formula I. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, sulphonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁻ Na⁺ or K⁺), alkaline earth cations (e.g., Mg⁺² , Ca⁺² or Ba⁺²), the ammonium cation, as well as acid salts of organic bases, including aliphatic and aromatic substituted ammonium, and quaternary ammonium cations such as those arising from protonation or peralkylation of triethylamine, *N,N*-diethylamine, *N,N*-dicyclohexylamine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula I possess asymmetric carbons and can therefore exist in racemic and optically active forms. Methods of separation of enantiomeric and diastereomeric mixtures are well known to one skilled in the art. The present invention encompasses any isolated racemic or optically active form of compounds described in Formula I which possess Raf kinase inhibitory activity.

### General Preparative Methods

The compounds of Formula I may be prepared by use of known chemical reactions and procedures, some of which are commercially available. Nevertheless, the following general preparative methods are presented to aid one of skill in the art in synthesizing the inhibitors, with more detailed examples being presented in the experimental section describing the working examples.

Heterocyclic amines may be synthesized utilizing known methodology (Katritzky, et al. *Comprehensive Heterocyclic Chemistry;* Permagon Press: Oxford, UK (1984). March. *Advanced Organic Chemistry*, 3^{rd} Ed.; John Wiley: New York (1985)). For example, 3-substituted-5-aminoisoxazoles (**3**) are available by the reaction of hydroxylamine with an α-cyanoketone (**2**), as shown in Scheme I. Cyanoketone **2**, in turn, is available from the reaction of acetamidate ion with an appropriate acyl derivative, such as an ester, an acid halide, or an acid anhydride. Reaction of an - cyanoketone with hydrazine (R²=H) or a monosubstituted hydrazine affords the 3-substituted- or 1,3-disubstituted-5-aminopyrazole (**5**). Pyrazoles unsubstituted at *N*-1 (R²=H) may be acylated at *N*-1, for example using di-*tert*-butyl dicarbonate, to give pyrazole 7. Similarly, reaction of nitrile **8** with an -thioacetate ester gives the 5-substituted-3-amino-2-thiophenecarboxylate (**9**, Ishizaki et al. JP 6025221). Decarboxylation of ester **9** may be achieved by protection of the amine, for example as the *tert*-butoxy (BOC) carbamate (**10**), followed by saponification and treatment with acid. When BOC protection is used, decarboxylation may be accompanied by deprotection giving the substituted 3-thiopheneammonium salt **11.** Alternatively, ammonium salt **11** may be directly generated through saponification of ester **9** followed by treatment with acid.

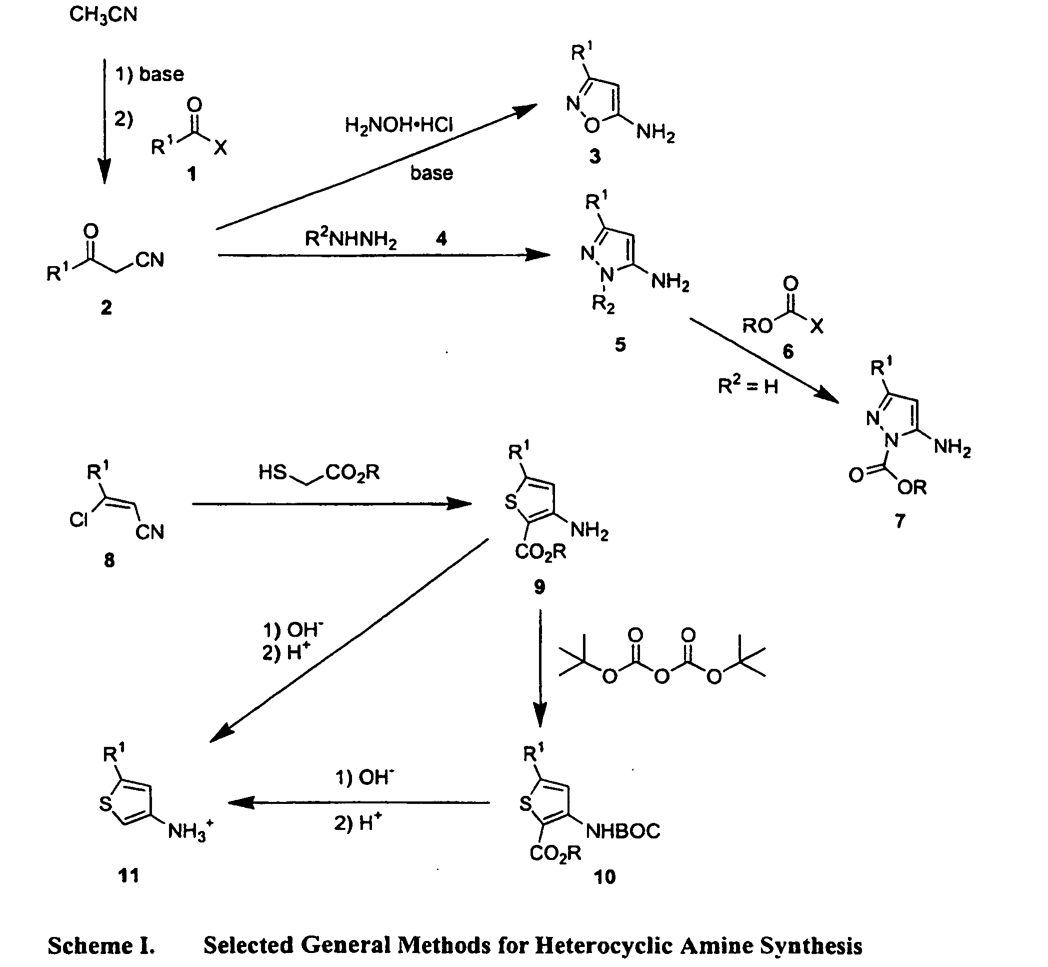

Substituted anilines may be generated using standard methods (March. *Advanced Organic Chemistry,* 3^{rd} Ed.; John Wiley: New York (1985); Larock. *Comprehensive Organic Transformations*; VCH Publishers: New York (1989)). As shown in Scheme II, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. *Hydrogenation Methods*; Academic Press: London, UK (1985)). Nitroaryls may also be directly reduced using a strong hydride source, such as LiAlH₄ (Seyden-Penne. *Reductions by the Alumino- and Borohydrides in Organic Synthesis*; VCH Publishers: New York (1991)), or using a zero valent metal, such as Fe, Sn or Ca, often in acidic media. Many methods exist for the synthesis of nitroaryls (March. *Advanced Organic Chemistry*, 3^{rd} Ed.; John Wiley: New York (1985). Larock. *Comprehensive Organic Transformations*; VCH Publishers: New York (1989)).

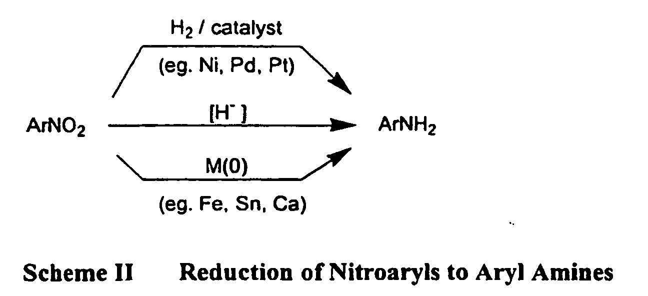

Nitroaryls are commonly formed by electrophilic aromatic nitration using HNO₃, or an alternative NO₂⁺ source. Nitroaryls may be further elaborated prior to reduction. Thus, nitroaryls substituted with 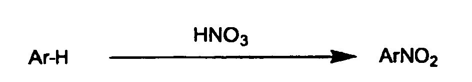 potential leaving groups (eg. F, Cl, Br, etc.) may undergo substitution reactions on treatment with nucleophiles, such as thiolate (exemplified in Scheme III) or phenoxide. Nitroaryls may also undergo Ullman-type coupling reactions (Scheme III).

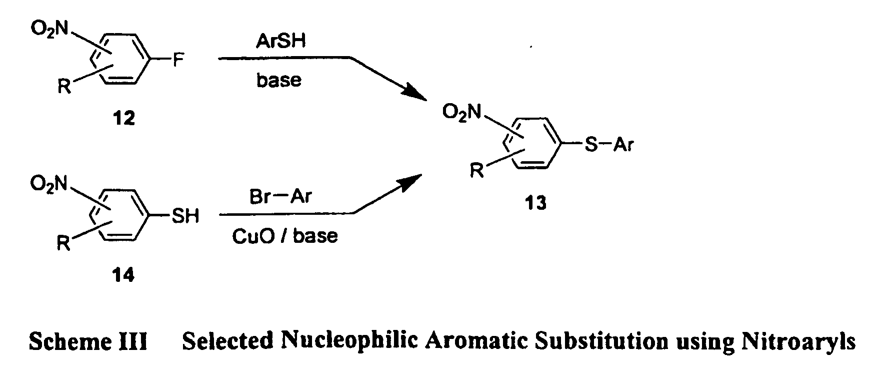

As shown in Scheme IV, urea formation may involve reaction of a heteroaryl isocyanate (17) with an aryl amine (16). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N*'-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **21** with an azide source, followed by rearrangement affords the isocyanate. The corresponding carboxylic acid (**22**) may also be subjected to Curtius-type rearrangements using diphenylphosphoryl azide (DPPA) or a similar reagent. A urea may also be generated from the reaction of an aryl isocyanate (**20**) with a heterocyclic amine.

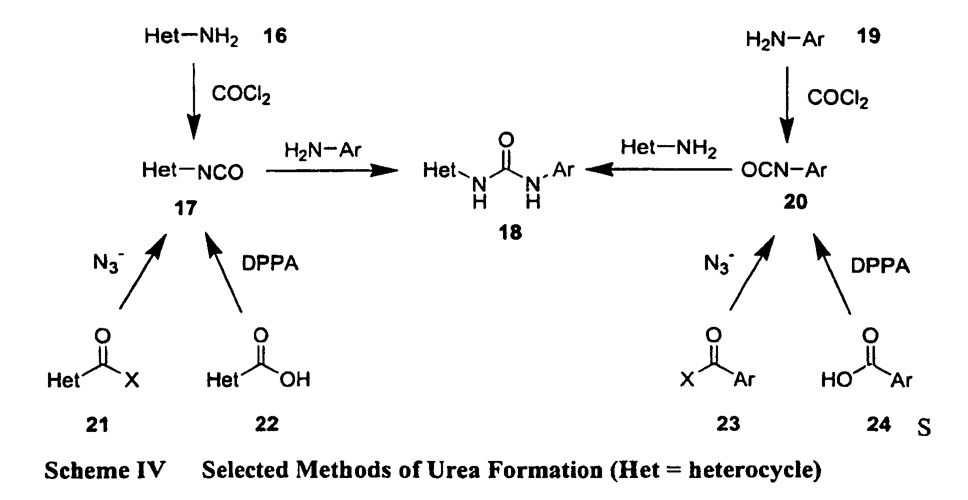

1-Amino-2-heterocyclic carboxylic esters (exemplified with thiophene **9**, Scheme V) may be converted into an isatoic-like anhydride (**25**) through saponification, followed by treatment with phosgene or a phosgene equivalent. Reaction of anhydride **25** with an aryl amine can generate acid **26** which may spontaneously decarboxylate, or may be isolated. If isolated, decarboxylation of acid **26** may be induced upon heating.

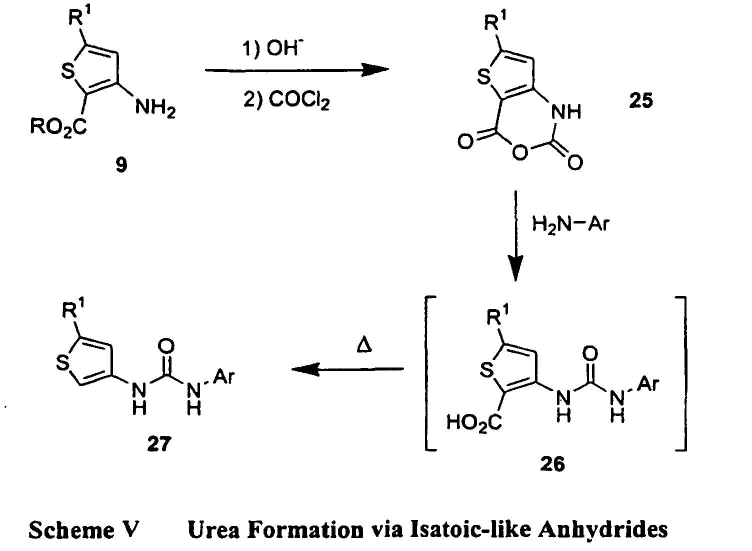

Finally, ureas may be further manipulated using methods familiar to those skilled in the art.

The invention also includes pharmaceutical compositions including a compound of Formula I or a pharmaceutically acceptable salt thereof, and a physiologically acceptable carrier.

The compounds may be administered orally, topically, parenterally, by inhalation or spray or sublingually, rectally or vaginally in dosage unit formulations. The term 'administration by injection' includes intravenous, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropyl methylcellulose, sodium alginate, polyvinylpyrrolidone, gum tragacanth and gum acacia; dispersing or wetting agents may be a naturally occurring phosphatide, for example, lecithin, or condensation products or an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl isobutyl tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene coploymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regime will preferably be from 0.01 to 200 mg/Kg of total body weight. The daily vaginal dosage regimen will preferably be from 0.01 to 200 mg/Kg of total body weight. The daily topical dosage regime will preferably be from 0.1 to 200 mg administered between one to four times daily. The transdermal concentration will preferably be that required to maintain a daily dose of from 0.01 to 200 mg/Kg. The daily inhalation dosage regime will preferably be from 0.01 to 10 mg/Kg of total body weight.

It will be appreciated by those skilled in the art that the particular method of administration will depend on a variety of factors, all of which are considered routinely when administering therapeutics.

It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy.

It will be further appreciated by one skilled in the art that the optimal course of treatment, ie., the mode of treatment and the daily number of doses of a compound of Formula I or a pharmaceutically acceptable salt thereof given for a defined number of days, can be ascertained by those skilled in the art using conventional treatment tests.

It will be understood, however, that the specific dose level for any particular patient will depend upon a variety of factors, including the activity of the specific compound employed, the age, body weight, general health, sex, diet, time of administration, route of administration, and rate of excretion, drug combination and the severity of the condition undergoing therapy.

The entire disclosure of all applications, patents and publications cited above and below are hereby incorporated by reference, including provisional application Attorney Docket BAYER 8 V1, filed on December 22, 1997, as Serial No. 08/996,343, converted on December 22, 1998.

The compounds are producible from known compounds (or from starting materials which, in turn, are producible from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construde to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg.

All temperatures are reported uncorrected in degrees Celsius (°C). Unless otherwise indicated, all parts and percentages are by weight.

Commercial grade reagents and solvents were used without further purification. Thin-layer chromatography (TLC) was performed on Whatman® pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science® silica gel.

Melting points (mp) were determined using a Thomas-Hoover melting point apparatus or a Mettler FP66 automated melting point apparatus and are uncorrected. Fourier transform infrared spectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (δ 0.00) or residual protonated solvent (CHCl₃ δ 7.26; MeOH δ 3.30; DMSO δ 2.49) as standard. Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (~1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV).

Elemental analyses were conducted by Robertson Microlit Labs, Madison NJ. All ureas displayed NMR spectra, LRMS and either elemental analysis or HRMS consistant with assigned structures.

### List of Abbreviations and Acronyms:

- AcOH: acetic acid
- anh: anhydrous
- BOC: *tert*-butoxycarbonyl
- conc: concentrated
- dec: decomposition
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EtOAc: ethyl acetate
- EtOH: ethanol (100%)
- Et₂O: diethyl ether
- Et₃N: triethylamine
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Hetrocyclic Amines

### A2. General Synthesis of 5-Amino-3-alkylisoxazoles

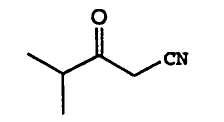

**Step 1. 3-Oxo-4-methylpentanenitrile**: A slurry of sodium hydride (60% in mineral oil; 10.3 g, 258 mmol) in benzene (52 mL) was warmed to 80 °C for 15 min., then a solution of acetonitrile (13.5 mL, 258 mmol) in benzene (52 mL) was added dropwise via addition funnel followed by a solution of ethyl isobutyrate (15 g, 129 mmol) in benzene (52 mL). The reaction mixture was heated overnight, then cooled with an ice water bath and quenched by addition of 2-propanol (50 mL) followed by water (50 mL) *via* addition funnel. The organic layer was separated and set aside. EtOAc (100 mL) was added to the aqueous layer and the resulting mixture was acidified to approximately pH 1 (conc. HCl) with stirring. The resulting aqueous layer was extracted with EtOAc (2 x 100 mL). The organic layers were combined with the original organic layer, dried (MgSO₄), and concentrated *in vacuo* to give the a-cyanoketone as a yellow oil which was used in the next step without further purification.

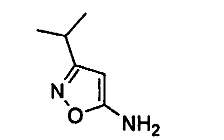

**Step 2. 5-Amino-3-isopropylisoxazole:** Hydroxylamine hydrochloride (10.3 g, 148 mmol) was slowly added to an ice cold solution of NaOH (25.9 g, 645 mmol) in water (73 mL) and the resulting solution was poured into a solution of crude 3-oxo-4-methylpentanenitrile while stirring. The resulting yellow solution was heated at 50 °C for 2.5 hours to produce a less dense yellow oil. The warm reaction mixture was immediately extracted with CHCl₃ (3 x 100 mL) without cooling. The combined organic layers were dried (MgSO₄), and concentrated *in vacuo.* The resulting oily yellow solid was filtered through a pad of silica (10% acetone/90% CH₂Cl₂) to afford the desired isoxazole as a yellow solid (11.3 g, 70%): mp 63-65 °C; TLC R_{*f*} (5% acetone/95% CH₂Cl₂) 0.19; ¹H-NMR (DMSO-d₆) d 1.12 (d, *J*=7.0 Hz, 6H), 2.72 (sept, *J*=7.0 Hz, 1H), 4.80 (s, 2H), 6.44 (s, 1H); FAB-MS *m*/*z* (rel abundance) 127 ((M+H)⁺; 67%).

### A3. General Method for the Preparation of 5-Amino-1-alkyl-3-alkylpyrazoles

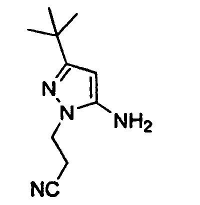

**5-Amino-3-tert-butyl-1-(2-cyanoethyl)pyrazole:** A solution of 4,4-dimethyl-3-oxopentanenitrile (5.6 g, 44.3 mmol) and 2-cyanoethyl hydrazine (4.61 g, 48.9 mmol) in EtOH (100 mL) was heated at the reflux temperature overnight after which TLC analysis showed incomplete reaction. The mixture was concentrated under reduced pressure and the residue was filtered through a pad of silica (gradient from 40% EtOAc/60% hexane to 70% EtOAc/30% hexane) and the resulting material was triturated (Et₂O/hexane) to afford the desired product (2.5 g, 30%): TLC (30% EtOAc/70% hexane) R_{*f*} 0.31; ¹H-NMR (DMSO-d₆) δ 1.13 (s, 9H), 2.82 (t, *J*=6.9 Hz, 2H), 4.04 (t, *J*=6.9 Hz, 2H), 5.12 (br s, 2H), 5.13 (s, 1H).

### A 4. Synthesis of 3-Amino-5-alkylthiophenes

### A4a. Synthesis of 3-Amino-5-alkylthiophenes by Thermal Decarboxylation of Thiophenecarboxylic Acids

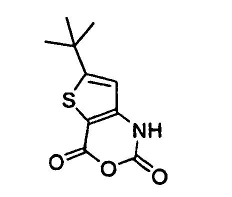

**Step 1. 7-*****tert*****-Butyl-2H-thieno[3,2-d]oxazine-2,4(1H)-dione:** A mixture of methyl 3-amino-5-*tert*-butylthiophenecarboxylate (7.5 g, 35.2 mmol) and KOH (5.92 g) in MeOH (24 mL) and water (24 mL) was stirred at 90 °C for 6 h. The reaction mixture was concentrated under reduced pressure and the residue was dissolved in water (600 mL). Phosgene (20% in toluene, 70 mL) was added dropwise over a 2 h period. The resulting mixture was stirred at room temperature overnight and the resulting precipitate was triturated (acetone) to afford the desired anhydride (5.78 g, 73%): ¹H-NMR (CDCl₃) δ 1.38 (s, 9H), 2.48 (s, 1H), 6.75 (s, 1H); FAB-MS *m*/*z* (rel abundance) 226 ((M+H)⁺, 100%).

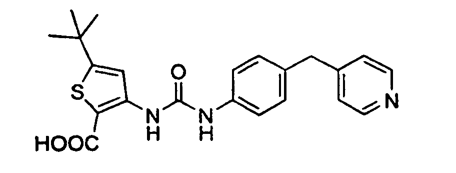

**Step 2.** ***N*****-(5-*****tert*****-Butyl-2-carboxy-3-thienyl)-*****N*****'-(4-(4-pyridinylmethyl)phenyl)-urea:** A solution of 7-*tert*-butyl-2H-thieno[3,2-d]oxazine-2,4(1H)-dione (0.176 g, 0.78 mmol) and 4-(4-pyridinylmethyl)aniline (0.144 g, 0.78 mmol) in THF (5 mL) was heated at the reflux temp. for 25 h. After cooling to room temp., the resulting solid was triturated with Et₂O to afford the desired urea (0.25 g, 78%): mp 187-189 °C; TLC (50% EtOAc/50% pet. ether) R_{*f*} 0.04; ¹H-NMR (DMSO-d₆) δ 1.34 (s, 9H), 3.90 (s, 2H), 7.15 (d, *J*=7Hz, 2H), 7.20 (d, *J*=3 Hz, 2H), 7.40 (d, *J*=7 Hz, 2H), 7.80 (s 1H), 8.45 (d, *J*=3 Hz, 2H) 9.55 (s, 1H), 9.85 (s, 1H), 12.50 (br s, 1H); FAB-MS *m*/*z* (rel abundance) 410 ((M+H)⁺; 20%).

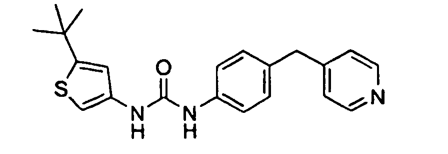

**Step 3.** ***N*****-(5-*****tert*****-Butyl-3-thienyl)-*****N'*****-(4-(4-pyridinylmethyl)phenyl)urea**: A vial containing *N*-(5-*tert*-butyl-2-carboxy-3-thienyl)-*N'*-(4-(4-pyridinylmethyl)phenyl)-urea (0.068 g, 0.15 mmol) was heated to 199 °C in an oil bath. After gas evolution ceased, the material was cooled and purified by preparative HPLC (C-18 column; gradient from 20% CH₃CN/79.9% H₂O/0.1% TFA to 99.9% H₂O/0.1% TFA) to give the desired product (0.024 g, 43%): TLC (50% EtOAc/50% pet. ether) R_{*f*} 0.18; ¹H-NMR (DMSO-d₆) δ 1.33 (s, 9H), 4.12 (s, 2H), 6.77 (s, 1H), 6.95 (s, 1H), 7.17 (d, *J*=9 Hz, 2H), 7.48 (d, *J*=9 Hz, 2H), 7.69 (d, *J*=7 Hz, 1H), 8.58 (s, 1H), 8.68 (d, *J*=7 Hz, 2H), 8.75 (s, 1H); EI-MS *m*/*z* 365 (M⁺).

### A4b. Synthesis 3-Amino-5-alkylthiophenes from 3-Amino-5-alkyl-2-thiophenecarboxylate esters

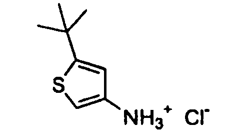

**5-*****tert*****-Butyl-3-thiopheneammonium Chloride:** To a solution of methyl 3-amino-5-*tert*-butyl-2-thiophene-carboxylate (5.07 g, 23.8 mmol, 1.0 equiv) in EtOH (150 mL) was added NaOH (2.0 g, 50 mmol, 2.1 equiv). The resulting solution was heated at the reflux temp. for 2.25 h. A conc. HCl solution (approximately 10 mL) was added dropwise with stirring and the evolution of gas was observed. Stirring was continued for 1 h, then the solution was concentrated under reduced pressure. The white residue was suspended in EtOAc (150 mL) and a saturated NaHCO₃ solution (150 mL) was added to dissolve. The organic layer was washed with water (150 mL) and a saturated NaCl solution (150 mL), dried (Na₂SO₄), and concentrated under reduced pressure to give the desired ammonium salt as a yellow oil (3.69 g, 100%). This material was used directly in urea formation without further purification.

### A4c. Synthesis 3-Amino-5-alkylthiophenes from N-BOC 3-Amino-5-alkyl-2-thiophenecarboxylate esters

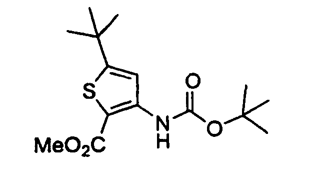

**Step 1. Methyl 3-(*****tert*****-Butoxycarbonylamino)-5-*****tert*****-butyl-2-thiophenecarboxylate:** To a solution of methyl 3-amino-5-*tert*-butyl-2-thiophenecarboxylate (150 g, 0.70 mol) in pyridine (2.8 L) at 5 °C was added di-*tert*-butyl dicarbonate (171.08 g, 0.78 mol, 1.1 equiv) and *N,N*-dimethylaminopyridine (86 g, 0.70 mol, 1.00 equiv) and the resulting mixture was stirred at room temp for 7 d. The resulting dark solution was concentrated under reduced pressure (approximately 0.4 mmHg) at approximately 20 °C. The resulting red solids were dissolved in CH₂Cl₂ (3 L) and sequentially washed with a 1 M H₃PO₄ solution (2 x 750 mL), a saturated NaHCO₃ solution (800 mL) and a saturated NaCl solution (2 x 800 mL), dried (Na₂SO₄) and concentrated under reduced pressure. The resulting orange solids were dissolved in abs. EtOH (2 L) by warming to 49 °C, then treated with water (500 mL) to afford the desired product as an off-white solid (163 g, 74%): ¹H-NMR (CDCl₃) δ 1.38 (s, 9H), 1.51 (s, 9H), 3.84 (s, 3H), 7.68 (s, 1H), 9.35 (br s, 1H); FAB-MS *m*/*z* (rel abundance) 314 ((M+H)⁺, 45%).

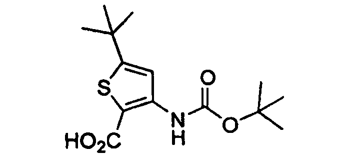

**Step 2. 3-(*****tert*****-Butoxycarbonylamino)-5-*****tert*****-butyl-2-thiophenecarboxylic Acid:** To a solution of methyl 3-(*tert*-butoxycarbonylamino)-5-*tert*-butyl-2-thiophenecarboxylate (90.0 g, 0.287 mol) in THF (630 mL) and MeOH (630 mL) was added a solution of NaOH (42.5 g, 1.06 mL) in water (630 mL). The resulting mixture was heated at 60 °C for 2 h, concentrated to approximately 700 mL under reduced pressure, and cooled to 0 °C. The pH was adjusted to approximately 7 with a 1.0 N HCl solution (approximately 1 L) while maintaining the internal temperature at approximately 0 °C. The resulting mixture was treated with EtOAc (4 L). The pH was adjusted to approximately 2 with a 1.0 N HCl solution (500 mL). The organic phase was washed with a saturated NaCl solution (4 x 1.5 L), dried (Na₂SO₄), and concentrated to approximately 200 mL under reduced pressure. The residue was treated with hexane (1 L) to form a light pink (41.6 g). Resubmission of the mother liquor to the concentration-precipitation protocol afforded additional product (38.4 g, 93% total yield): ¹H-NMR (CDCl₃) δ 1.94 (s, 9H), 1.54 (s, 9H), 7.73 (s, 1H), 9.19 (br s, 1H); FAB-MS *m*/*z* (rel abundance) 300 ((M+H)⁺, 50%).

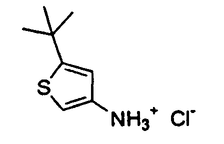

**Step 3. 5-*****tert*****-Butyl-3-thiopheneammonium Chloride:** A solution of 3-(*tert*-butoxycarbonylamino)-5-*tert*-butyl-2-thiophenecarboxylic acid (3.0 g, 0.010 mol) in dioxane (20 mL) was treated with an HCl solution (4.0 M in dioxane, 12.5 mL, 0.050 mol, 5.0 equiv), and the resulting mixture was heated at 80 °C for 2 h. The resulting cloudy solution was allowed to cool to room temp forming some precipitate. The slurry was diluted with EtOAc (50 mL) and cooled to -20 °C. The resulting solids were collected and dried overnight under reduced pressure to give the desired salt as an off-white solid (1.72 g, 90%): ¹H-NMR (DMSO-d₆) δ 1.31 (s, 9H), 6.84 (d, *J*=1.48 Hz, 1H), 7.31 (d, *J*=1.47 Hz, 1H), 10.27 (br s, 3H).

### A5. General Method for the Synthesis of BOC-Protected Pyrazoles

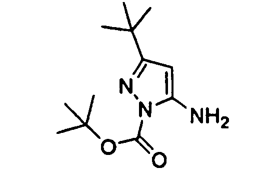

**5-Amino-3-*****tert*****-butyl-*****N***^{***1***}**-(*****tert*****-butoxycarbonyl)pyrazole:** To a solution of 5-amino-3-*tert*-butylpyrazole (3.93 g, 28.2 mmol) in CH₂Cl₂ (140 mL) was added di-*tert*-butyl dicarbonate (6.22 g, 28.5 mmol) in one portion. The resulting solution was stirred at room temp. for 13 h, then diluted with EtOAc (500 mL). The organic layer was washed with water (2 x 300 mL), dried (MgSO₄) and concentrated under reduced pressure. The solid residue was triturated (100 mL hexane) to give the desired carbamate (6.26 g, 92%): mp 63-64 °C; TLC R_{*f*} (5% acetone/95% CH₂Cl₂); ¹H-NMR (DMSO-d₆) δ 1.15 (s, 9H), 1.54 (s, 9H), 5.22 (s, 1H), 6.11 (s, 2H); FAB-MS *m*/*z* ((M+H)⁺).

### A6. General Method for the Synthesis of 2-Aminothiadiazoles

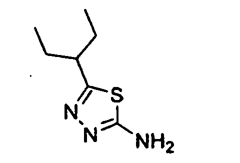

**2-Amino-5-(1-(1-ethyl)propyl)thiadiazine**: To concentrated sulfuric acid (9.1 mL) was slowly added 2-ethylbutyric acid (10.0 g, 86 mmol, 1.2 equiv). To this mixture was slowly added thiosemicarbazide (6.56 g, 72 mmol, 1 equiv). The reaction mixture was heated at 85 °C for 7 h, then cooled to room temperature, and treated with a concentrated NH₄OHsolution until basic. The resulting solids were filtered to afford 2-amino-5-(1-(1-ethyl)propyl)thiadiazine product was isolated via vacuum filtration as a beige solid (6.3 g, 51%): mp 155-158 °C; TLC (5% MeOH/ 95% CHCl₃) R_{*f*} 0.14; ¹H-NMR (DMSO-d₆) δ 0.80 (t, J=7.35 Hz, 6H), 1.42-1.60 (m, 2H), 1.59-1.71 (m, 2H), 2.65-2.74 (m, 1H), 7.00 (br s, 2H); HPLC ES-MS *m*/*z* 172 ((M+H)⁻).

### A7. GeneralMethod for the Synthesis of 2-Aminooxadiazoles

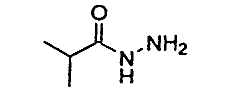

**Step 1. Isobutyric Hydrazide:** A solution of methyl isobutyrate (10.0 g) and hydrazine (2.76 g) in MeOH (500 mL) was heated at the reflux temperature over night then stirred at 60 °C for 2 weeks. The resulting mixture was cooled to room temperature and concentrated under reduced pressure to afford isobutyric hydrazide as a yellow oil (1.0 g, 10%), which was used inb the next step withour further purification.

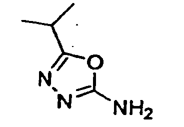

**Step 2. 2-Amino-5-isopropyl oxadiazole:** To a mixture of isobutyric hydrazide (0.093 g), KHCO₃ (0.102 g), and water (1 mL) in dioxane (1 mL) at room temperature was added cyanogen bromide (0.10 g). The resulting mixture was heated at the reflux temperature for 5 h, and stirred at room temperature for 2 d, then treated with CH₂Cl₂ (5 mL). The organic layer was washed with water (2 x 10 mL), dried (MgSO₄) and concentrated under reduced pressure to afford 2-amino-5-isopropyl oxadiazole as a white solid: HPLC ES-MS *m*/*z* 128 ((M+H)⁺).

### A8. General Method for the Synthesis of 2-Aminooxazoles

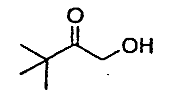

**Step 1. 3,3-Dimethyl-1-hydroxy-2-butanone:** A neat sample of 1-bromo-3,3-dimethyl-2-butanone (33.3 g) at 0 °C was treated with a 1N NaOH solution, then was stirred for 1 h. The resulting mixture was extracted with EtOAc (5 x 100 mL). The combined organics were dried (Na₂SO₄) and concentrated under reduced pressure to give 3,3-dimethyl-1-hydroxy-2-butanone (19 g, 100%), which was used in the next step withour further purification.

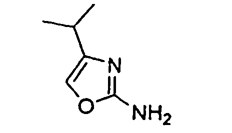

**Step 2. 2-Amino-4-isopropyl-1,3-oxazole:** To a solution of 3,3-dimethyl-I-hydroxy-2-butanone (4.0 g) and cyanimide (50% w/w, 2.86 g) in THF (10 mL) was added a 1N NaOAc solution (8 mL), followed by tetra-n-butylammonium hydroxide (0.4 M, 3.6 mL), then a 1N NaOH solution (1.45 mL). The resulting mixture was stirred at room temperature for 2 d. The resulting organic layer was separated, washed with water (3 x 25 mL), and the aqueous layer was extraced with Et₂O (3 x 25 mL). The combined organic layers were treated with a 1N NaOH solution until basic, then extracted with CH₂Cl₂ (3 x 25 mL). The combined organic layers were dried (Na₂SO₄) and concentrated under reduced pressure to afford 2-Amino-4-isopropyl-1,3-oxazole (1.94 g, 41 %): HPLC ES-MS *mlz* 141 ((M+H)⁺).

### A9. Method for the Synthesis of Substituted-5-aminotetrazoles

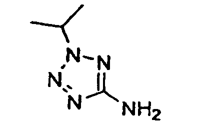

To a solution of 5-aminotetrazole (5 g), NaOH (2.04 g) and water (25 mL) in EtOH (115 mL) at the reflux temperature was added 2-bromopropane (5.9g). The resulting mixture was heated at the reflux temperature for 6 d, then cooled to room temperature, and concentrated under reduced pressure. The resulting aqueous mixture was washed with CH₂Cl₂ (3 x 25 mL), then concentrated under reduced pressure with the aid of a lyophlizer to afford a mixture of 1- and 2-isopropyl-5-aminotetrazole (50%), which was used without further purification: HPLC ES-MS *m*/*z* 128 ((M+H)⁺).

### B. General Methods for Synthesis of Substituted Anilines

### B1. General Method for Substituted Aniline Formation via Hydrogenation of a Nitroarene

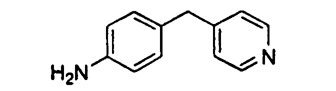

**4-(4-Pyridinylmethyl)aniline:** To a solution of 4-(4-nitrobenzyl)pyridine (7.0 g, 32.68 mmol) in EtOH (200 mL) was added 10% Pd/C (0.7 g) and the resulting slurry was shaken under a H₂ atmosphere (50 psi) using a Parr shaker. After 1 h, TLC and ¹H-NMR of an aliquot indicated complete reaction. The mixture was filtered through a short pad of Celite®. The filtrate was concentrated *in vacuo* to afford a white solid (5.4 g, 90%): ¹H-NMR (DMSO-d₆) δ 3.74 (s, 2H), 4.91 (br s, 2H), 6.48 (d, *J*=8.46 Hz, 2H), 6.86 (d, *J*=8.09 Hz, 2H), 7.16 (d, *J*=5.88 Hz, 2H), 8.40 (d, *J*=5.88 Hz, 2H); EI-MS m/z 184 (M⁻). This material was used in urea formation reactions without further purification.

### General Method for Substituted Aniline Formation via Dissolving Metal Reduction of a Nitroarene

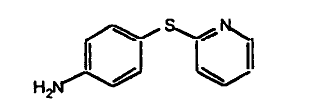

**4-(2-Pyridinylthio)aniline**: To a solution of 4-(2-pyridinylthio)-1-nitrobenzene (Menai ST 3355A; 0.220 g, 0.95 mmol) and H₂O (0.5 mL) in AcOH (5 mL) was added iron powder (0.317 g, 5.68 mmol) and the resulting slurry stirred for 16 h at room temp. The reaction mixture was diluted with EtOAc (75 mL) and H₂O (50 mL), basified to pH 10 by adding solid K₂CO₃ in portions (***Caution**:* foaming). The organic layer was washed with a saturated NaCl solution, dried (MgSO₄), concentrated in *vacuo*. The residual solid was purified by MPLC (30% EtOAc/70% hexane) to give the desired product as a thick oil (0.135 g, 70%): TLC (30% EtOAc/70% hexanes) R_{*f*} 0.20.

### B3a. General Method for Substituted Aniline Formation via Nitroarene Formation Through Nucleophilic Aromatic Substitution, Followed by Reduction

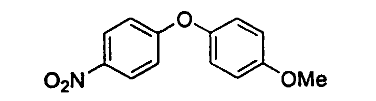

**Step 1. 1-Methoxy-4-(4-nitrophenoxy)benzene**: To a suspension of NaH (95%, 1.50 g, 59 mmol) in DMF (100 mL) at room temp. was added dropwise a solution of 4-methoxyphenol (7.39 g, 59 mmol) in DMF (50 mL). The reaction was stirred 1 h, then a solution of 1-fluoro-4-nitrobenzene (7.0 g, 49 mmol) in DMF (50 mL) was added dropwise to form a dark green solution. The reaction was heated at 95 °C overnight, then cooled to room temp., quenched with H₂O, and concentrated *in vacuo*. The residue was partitioned between EtOAc (200 mL) and H₂O (200 mL) . The organic layer was sequentially washed with H₂O (2 x 200 mL), a saturated NaHCO₃ solution (200 mL), and a saturated NaCl solution (200 mL), dried (Na₂SO₄), and concentrated *in vacuo.* The residue was triturated (Et₂O/hexane) to afford 1-methoxy-4-(4-nitrophenoxy)benzene (12.2 g, 100%): ¹H-NMR (CDCl₃) δ 3.83 (s, 3H), 6.93-7.04 (m, 6H), 8.18 (d, *J*=9.2 Hz, 2H); EI-MS *m*/*z* 245 (M⁺).

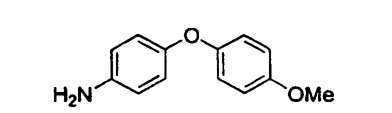

**Step 2. 4-(4-Methoxyphenoxy)aniline:** To a solution of 1-methoxy-4-(4-nitrophenoxy)benzene (12.0 g, 49 mmol) in EtOAc (250 mL) was added 5% Pt/C (1.5 g) and the resulting slurry was shaken under a H₂ atmosphere (50 psi) for 18 h. The reaction mixture was filtered through a pad of Celite® with the aid of EtOAc and concentrated *in vacuo* to give an oil which slowly solidified (10.6 g, 100%): ¹H-NMR (CDCl₃) δ 3.54 (br s, 2H), 3.78 (s, 3H), 6.65 (d, *J*=8.8 Hz, 2H), 6.79-6.92 (m, 6H); EI-MS *m*/*z* 215 (M⁺).

### B3b. General Method for Substituted Aniline Formation via Nitroarene Formation Through Nucleophilic Aromatic Substitution, Followed by Reduction

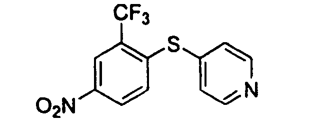

**Step 1. 3-(Trifluoromethyl)-4-(4-pyridinylthio)nitrobenzene:** A solution of 4-mercaptopyridine (2.8 g, 24 mmoles), 2-fluoro-5-nitrobenzotrifluoride (5 g, 23.5 mmoles), and potassium carbonate (6.1 g, 44.3 mmoles) in anhydrous DMF (80 mL) was stirred at room temperature and under argon overnight. TLC showed complete reaction. The mixture was diluted with Et₂O (100 mL) and water (100 mL) and the aqueous layer was back-extracted with Et₂O (2 x 100 mL). The organic layers were washed with a saturated NaCl solution (100 mL), dried (MgSO₄), and concentrated under reduced pressure. The solid residue was triturated with Et₂O to afford the desired product as a tan solid (3.8 g, 54%): TLC (30% EtOAc/70% hexane) R_{*f*} 0.06; ¹H-NMR (DMSO-d₆) δ 7.33 (dd, *J*=1.2, 4.2 Hz, 2H), 7.78 (d, *J*=8.7 Hz, 1H), 8.46 (dd, *J*=2.4, 8.7Hz, 1H), 8.54-8.56 (m, 3H).

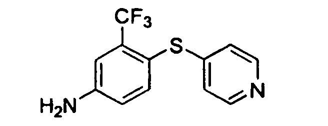

**Step 2. 3-(Trifluoromethyl)-4-(4-pyridinylthio)aniline:** A slurry of 3-trifluoromethyl-4-(4-pyridinylthio)nitrobenzene (3.8 g, 12.7 mmol), iron powder (4.0 g, 71.6 mmol), acetic acid (100 mL), and water (1 mL) were stirred at room temp. for 4 h. The mixture was diluted with Et₂O (100 mL) and water (100 mL). The aqueous phase was adjusted to pH 4 with a 4 N NaOH solution. The combined organic layers were washed with a saturated NaCl solution (100 mL), dried (MgSO₄), and concentrated under reduced pressure. The residue was filtered through a pad of silica (gradient from 50% EtOAc/50% hexane to 60% EtOAc/40% hexane) to afford the desired product (3.3 g): TLC (50% EtOAc/50% hexane) R_{*f*} 0.10; ¹H-NMR (DMSO-d₆) δ 6.21 (s, 2H), 6.84-6.87 (m, 3H), 7.10 (d, *J*=2.4 Hz, 1H), 7.39 (d, *J*=8.4 Hz, 1H), 8.29 (d, *J*=6.3 Hz, 2H).

### B3c. General Method for Substituted Aniline Formation via Nitroarene Formation Through Nucleophilic Aromatic Substitution, Followed by Reduction

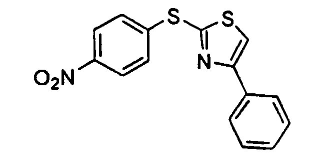

**Step 1. 4-(2-(4-Phenyl)thiazolyl)thio-1-nitrobenzene:** A solution of 2-mercapto-4-phenylthiazole (4.0 g, 20.7 mmoles) in DMF (40 mL) was treated with 1-fluoro-4-nitrobenzene (2.3 mL, 21.7 mmoles) followed by K₂CO₃ (3.18 g, 23 mmol), and the mixture was heated at approximately 65 °C overnight. The reaction mixture was then diluted with EtOAc (100 mL), sequentially washed with water (100 mL) and a saturated NaCl solution (100 mL), dried (MgSO₄) and concentrated under reduced pressure. The solid residue was triturated with a Et₂O/hexane solution to afford the desired product (6.1 g): TLC (25% EtOAc/75% hexane) R_{*f*} 0.49; ¹H-NMR (CDCl₃) δ 7.35-7.47 (m, 3H), 7.58-7.63 (m, 3H), 7.90 (d, *J*=6.9 Hz, 2H), 8.19 (d, *J*=9.0 Hz, 2H).

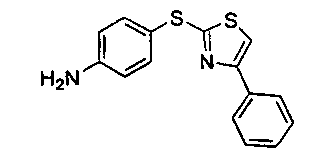

**Step 2. 4-(2-(4-Phenyl)thiazolyl)thioaniline:** 4-(2-(4-Phenyl)thiazolyl)thio-1-nitrobenzene was reduced in a manner analagous to that used in the preparation of 3-(trifluoromethyl)-4-(4-pyridinylthio)aniline: TLC (25% EtOAc/75% hexane) R_{*f*} 0.18; ¹H-NMR (CDCl₃) δ 3.89 (br s, 2H), 6.72-6.77 (m, 2H), 7.26-7.53 (m, 6H), 7.85-7.89 (m, 2H).

### B3d. General Method for Substituted Aniline Formation via Nitroarene Formation Through Nucleophilic Aromatic Substitution, Followed by Reduction

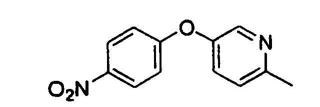

**Step 1. 4-(6-Methyl-3-pyridinyloxy)-1-nitrobenzene:** To a solution of 5-hydroxy-2-methylpyridine (5.0 g, 45.8 mmol) and 1-fluoro-4-nitrobenzene (6.5 g, 45.8 mmol) in anh DMF (50 mL) was added K₂CO₃ (13.0 g, 91.6 mmol) in one portion. The mixture was heated at the reflux temp. with stirring for 18 h and then allowed to cool to room temp. The resulting mixture was poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* to afford the desired product (8.7 g, 83%). The this material was carried to the next step without further purification.

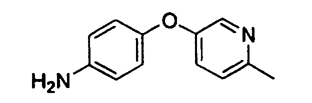

**Step 2. 4-(6-Methyl-3-pyridinyloxy)aniline:** A solution of 4-(6-methyl-3-pyridinyloxy)-1-nitrobenzene (4.0 g, 17.3 mmol) in EtOAc (150 mL) was added to 10% Pd/C (0.500 g, 0.47 mmol) and the resulting mixture was placed under a H₂ atmosphere (balloon) and was allowed to stir for 18 h at room temp. The mixture was then filtered through a pad of Celite® and concentrated *in vacuo* to afford the desired product as a tan solid (3.2 g, 92%): EI-MS *m*/*z* 200 (M⁺).

### B3e. General Method for Substituted Aniline Formation via Nitroarene Formation Through Nucleophilic Aromatic Substitution, Followed by Reduction

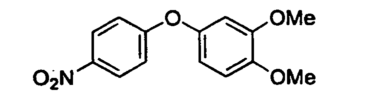

**Step 1. 4-(3,4-Dimethoxyphenoxy)-1-nitrobenzene:** To a solution of 3,4-dimethoxyphenol (1.0 g, 6.4 mmol) and 1-fluoro-4-nitrobenzene (700 µL, 6.4 mmol) in anh DMF (20 mL) was added K₂CO₃ (1.8 g, 12.9 mmol) in one portion. The mixture was heated at the reflux temp with stirring for 18 h and then allowed to cool to room temp. The mixture was then poured into water (100 mL) and extracted with EtOAc (3 x 100 mL). The combined organics were sequentially washed with water (3 x 50 mL) and a saturated NaCl solution (2 x 50 mL), dried (Na₂SO₄), and concentrated *in vacuo* to afford the desired product (0.8 g, 54%). The crude product was carried to the next step without further purification.

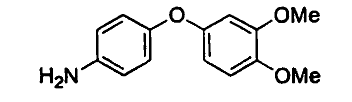

**Step 2. 4-(3,4-Dimethoxyphenoxy)aniline:** A solution of 4-(3,4-dimethoxyphenoxy)-1-nitrobenzene (0.8 g, 3.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite® and concentrated *in vacuo* to afford the desired product as a white solid (0.6 g, 75%): EI-MS *m*/*z* 245 (M⁺).

### B3f. General Method for Substituted Aniline Formation via Nitroarene Formation Through Nucleophilic Aromatic Substitution, Followed by Reduction

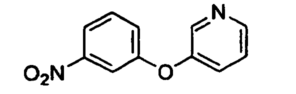

**Step 1. 3-(3-Pyridinyloxy)-1-nitrobenzene:** To a solution of 3-hydroxypyridine (2.8 g, 29.0 mmol), 1-bromo-3-nitrobenzene (5.9 g, 29.0 mmol) and copper(I) bromide (5.0 g, 34.8 mmol) in anh DMF (50 mL) was added K₂CO₃ (8.0 g, 58.1 mmol) in one portion. The resulting mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo*. The resulting oil was purified by flash chromatography (30% EtOAc/70% hexane) to afford the desired product (2.0 g, 32 %). This material was used in the next step without further purification.

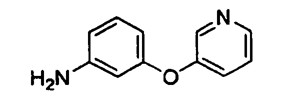

**Step 2. 3-(3-Pyridinyloxy)aniline:** A solution of 3-(3-pyridinyloxy)-1-nitrobenzene (2.0 g, 9.2 mmol) in EtOAc (100 mL) was added to 10% Pd/C (0.200 g) and the resulting mixture was placed under a H₂ atmosphere (balloon) and was allowed to stir for 18 h at room temp. The mixture was then filtered through a pad of Celite® and concentrated *in vacuo* to afford the desired product as a red oil (1.6 g, 94%): EI-MS *m*/*z* 186 (M⁺).

### B3g. General Method for Substituted Aniline Formation via Nitroarene Formation Through Nucleophilic Aromatic Substitution, Followed by Reduction

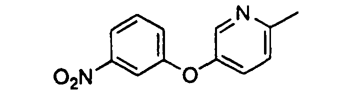

**Step 1. 3-(5-Methyl-3-pyridinyloxy)-1-nitrobenzene:** To a solution of 3-hydroxy-5-methylpyridine (5.0 g, 45.8 mmol), 1-bromo-3-nitrobenzene (12.0 g, 59.6 mmol) and copper(I) iodide (10.0 g, 73.3 mmol) in anh DMF (50 mL) was added K₂CO₃ (13.0 g, 91.6 mmol) in one portion. The mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* . The resulting oil was purified by flash chromatography (30% EtOAc/70% hexane) to afford the desired product (1.2 g, 13%).

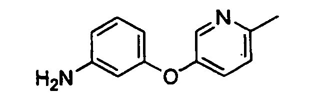

**Step 2. 3-(5-Methyl-3-pyridinyloxy)-1-nitrobenzene**: A solution of 3-(5-methyl-3-pyridinyloxy)-1-nitrobenzene (1.2 g, 5.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite® and concentrated *in vacuo* to afford the desired product as a red oil (0.9 g, 86%): CI-MS *m*/*z* 201 ((M+H)⁺).

### 2B3h. General Method for Substituted Aniline Formation via Nitroarene Formation Through Nucleophilic Aromatic Substitution, Followed by Reduction

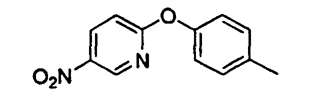

**Step 1. 5-Nitro-2-(4-methylphenoxy)pyridine**: To a solution of 2-chloro-5-nitropyridine (6.34 g, 40 mmol) in DMF (200 mL) were added of 4-methylphenol (5.4 g, 50 mmol, 1.25 equiv) and K₂CO₃ (8.28 g, 60 mmol, 1.5 equiv). The mixture was stirred overnight at room temp. The resulting mixture was treated with water (600 mL) to generate a precipitate. This mixture was stirred for 1 h, and the solids were separated and sequentially washed with a 1 N NaOH solution (25 mL), water (25 mL) and pet ether (25 mL) to give the desired product (7.05 g, 76%): mp 80-82 °C; TLC (30% EtOAc/70% pet ether) R_{*f*} 0.79; ¹H-NMR (DMSO-d₆) δ 2.31 (s, 3H), 7.08 (d, *J*=8.46 Hz, 2H), 7.19 (d, *J*=9.20 Hz, 1H), 7.24 (d, *J*=8.09 Hz, 2H), 8.58 (dd, *J*=2.94, 8.82 Hz, 1H), 8.99 (d, *J*=2.95 Hz, 1H); FAB-MS *m*/*z* (rel abundance) 231 ((M+H)⁺), 100%).

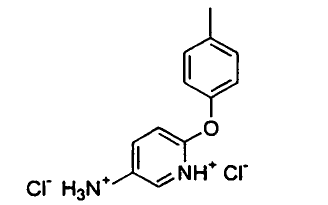

**Step 2. 5-Amino-2-(4-methylphenoxy)pyridine Dihydrochloride**: A solution 5-nitro-2-(4-methylphenoxy)pyridine (6.94 g, 30 mmol, 1 eq) and EtOH (10 mL) in EtOAc (190 mL) was purged with argon then treated with 10% Pd/C (0.60 g). The reaction mixture was then placed under a H₂ atmosphere and was vigorously stirred for 2.5 h. The reaction mixture was filtered through a pad of Celite®. A solution of HCl in Et₂O was added to the filtrate was added dropwise. The resulting precipitate was separated and washed with EtOAc to give the desired product (7.56 g, 92%): mp 208-210 °C (dec); TLC (50% EtOAc/50% pet ether) R_{*f*} 0.42; ¹H-NMR (DMSO-d₆) δ 2.25 (s, 3H), 6.98 (d, *J*=8.45 Hz, 2H), 7.04 (d, *J*=8.82 Hz, 1H), 7.19 (d, *J*=8.09 Hz, 2H), 8.46 (dd, *J*=2.57, 8.46 Hz, 1H), 8.63 (d, *J*=2.57 Hz, 1H); EI-MS *m*/*z* (rel abundance) (M⁺, 100%).

### B3i. General Method for Substituted Aniline Formation via Nitroarene Formation Through Nucleophilic Aromatic Substitution, Followed by Reduction

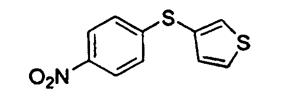

**Step 1. 4-(3-Thienylthio)-1-nitrobenzene:** To a solution of 4-nitrothiophenol (80%pure; 1.2 g, 6.1 mmol), 3-bromothiophene (1.0 g, 6.1 mmol) and copper(II) oxide (0.5 g, 3.7 mmol) in anhydrous DMF (20 mL) was added KOH (0.3 g, 6.1 mmol), and the resulting mixture was heated at 130 °C with stirring for 42 h and then allowed to cool to room temp. The reaction mixture was then poured into a mixture of ice and a 6N HCl solution (200 mL) and the resulting aqueous mixture was extracted with EtOAc (3 x 100 mL). The combined organic layers were sequentially washed with a 1M NaOH solution (2 x 100 mL) and a saturated NaCl solution (2 x 100 mL), dried (MgSO₄), and concentrated *in vacuo*. The residual oil was purified by MPLC (silica gel; gradient from 10% EtOAc/90% hexane to 5% EtOAc/95% hexane) to afford of the desired product (0.5 g, 34%). GC-MS *m*/*z* 237 (M⁺).

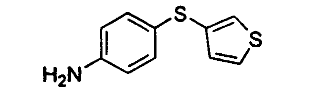

**Step 2. 4-(3-Thienylthio)aniline:** 4-(3-Thienylthio)-1-nitrobenzene was reduced to the aniline in a manner analogous to that described in Method B1.

### B3j. General Method for Substituted Aniline Formation via Nitroarene Formation Through Nucleophilic Aromatic Substitution, Followed by Reduction

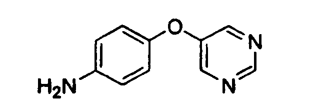

**4-(5-Pyrimininyloxy)aniline:** 4-Aminophenol (1.0 g, 9.2 mmol) was dissolved in DMF (20 mL) then 5-bromopyrimidine (1.46 g, 9.2 mmol) and K₂CO₃ (1.9 g, 13.7 mmol) were added. The mixture was heated to 100 °C for 18 h and at 130 °C for 48 h at which GC-MS analysis indicated some remaining starting material. The reaction mixture was cooled to room temp. and diluted with water (50 mL). The resulting solution was extracted with EtOAc (100 mL). The organic layer was washed with a saturated NaCl solution (2 x 50 mL), dried (MgSO₄), and concentrated *in vacuo.* The residular solids were purified by MPLC (50% EtOAc/50% hexanes) to give the desired amine (0.650 g, 38%).

### B3k. General Method for Substituted Aniline Formation via Nitroarene Formation Through Nucleophilic Aromatic Substitution, Followed by Reduction

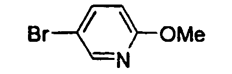

**Step 1. 5-Bromo-2-methoxypyridine:** A mixture of 2,5-dibromopyridine (5.5 g, 23.2 mmol) and NaOMe (3.76g, 69.6 mmol) in MeOH (60 mL) was heated at 70 °C in a sealed reaction vessel for 42 h, then allowed to cool to room temp. The reaction mixture was treated with water (50 mL) and extracted with EtOAc (2 x 100 mL). The combined organic layers were dried (Na₂SO₄) and concentrated under reduced pressure to give a pale yellow, volatile oil (4.1g, 95% yield): TLC (10% EtOAc / 90% hexane) R_{*f*} 0.57.

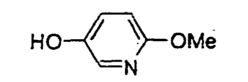

**Step 2. 5-Hydroxy-2-methoxypyridine**: To a stirred solution of 5-bromo-2-methoxypyridine (8.9 g, 47.9 mmol) in THF (175 mL) at -78 °C was added an n-butyllithium solution (2.5 M in hexane; 28.7 mL, 71.8 mmol) dropwise and the resulting mixture was allowed to stir at -78 °C for 45 min. Trimethyl borate (7.06 mL, 62.2 mmol) was added via syringe and the resulting mixture was stirred for an additional 2 h. The bright orange reaction mixture was warmed to 0 °C and was treated with a mixture of a 3 N NaOH solution (25 mL, 71.77 mmol) and a hydrogen peroxide solution (30%; approx. 50 mL). The resulting yellow and slightly turbid reaction mixture was warmed to room temp. for 30 min and then heated to the reflux temp. for 1 h. The reaction mixture was then allowed to cool to room temp. The aqueous layer was neutralized with a 1N HCl solution then extracted with Et₂O (2 x 100 mL). The combined organic layers were dried (Na₂SO₄) and concentrated under reduced pressure to give a viscous yellow oil (3.5g, 60%).

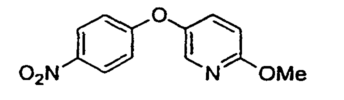

**Step 3. 4-(5-(2-Methoxy)pyridyl)oxy-1-nitrobenzene:** To a stirred slurry of NaH (97%, 1.0 g, 42 mmol) in anh DMF (100 mL) was added a solution of 5-hydroxy-2-methoxypyridine (3.5g, 28 mmol) in DMF (100 mL). The resulting mixture was allowed to stir at room temp. for 1 h, 4-fluoronitrobenzene (3 mL, 28 mmol) was added via syringe. The reaction mixture was heated to 95 °C overnight, then treated with water (25 mL) and extracted with EtOAc (2 x 75 mL). The organic layer was dried (MgSO₄) and concentrated under reduced pressure. The residual brown oil was crystalized EtOAc/hexane) to afford yellow crystals (5.23 g, 75%).

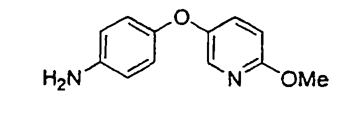

**Step 4. 4-(5-(2-Methoxy)pyridyl)oxyaniline:** 4-(5-(2-Methoxy)pyridyl)oxy-1-nitrobenzene was reduced to the aniline in a manner analogous to that described in Method B3d, Step2.

### B4a. General Method for Substituted Aniline Synthesis via Nucleophilic Aromatic Substitution using a Halopyridine

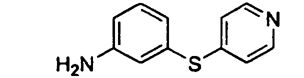

**3-(4-Pyridinylthio)aniline**: To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R_{*f*} 0.29; ¹H-NMR (DMSO-d₆) δ 5.41 (s, 2H), 6.64-6.74 (m, 3H), 7.01 (d, J=4.8, 2H), 7.14 (t, J=7.8 Hz, 1H), 8.32 (d, J=4.8, 2H).

### 2B4b. General Method for Substituted Aniline Synthesis via Nucleophilic Aromatic Substitution using a Halopyridine

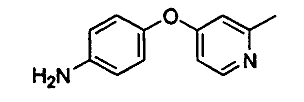

**4-(2-Methyl-4-pyridinyloxy)aniline:** To a solution of 4-aminophenol (3.6 g, 32.8 mmol) and 4-chloropicoline (5.0 g, 39.3 mmol) in anh DMPU (50 mL) was added potassium *tert*-butoxide (7.4 g, 65.6 mmol) in one portion. The reaction mixture was heated at 100 °C with stirring for 18 h, then was allowed to cool to room temp. The resulting mixture was poured into water (200 mL) and extracted with EtOAc (3 x 150 mL). The combined extracts were sequentially washed with water (3 x 100 mL) and a saturated NaCl solution (2 x 100 mL), dried (Na₂SO₄), and concentrated *in vacuo*.

The resulting oil was purified by flash chromatography (50 % EtOAc/50% hexane) to afford the desired product as a yellow oil (0.7 g, 9%): CI-MS *m*/*z* 201 ((M+H)⁻).

### B4c. General Method for Substituted Aniline Synthesis via Nucleophilic Aromatic Substitution using a Halopyridine

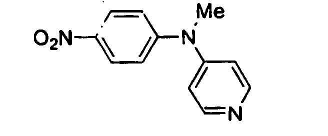

**Step 1. Methyl(4-nitrophenyl)-4-pyridylamine:** To a suspension of *N*-methyl-4-nitroaniline (2.0 g, 13.2 mmol) and K₂CO₃ (7.2 g, 52.2 mmol) in DMPU (30mL) was added 4-chloropyridine hydrochloride (2.36 g, 15.77 mmol). The reaction mixture was heated at 90 °C for 20 h, then cooled to room temperature. The resulting mixture was diluted with water (100 mL) and extracted with EtOAc (100 mL). The organic layer was washed with water (100 mL), dried (Na₂SO₄) and concentrated under reduced pressure. The residue was purified by column chromatography (silica gel, gradient from 80% EtOAc /20% hexanes to 100% EtOAc) to afford methyl(4-nitrophenyl)-4-pyridylamine (0.42 g)

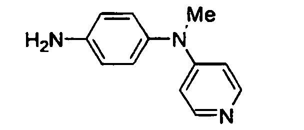

**Step 2. Methyl(4-aminophenyl)-4-pyridylamine:** Methyl(4-nitrophenyl)-4-pyridylamine was reduced in a manner analogous to that described in Method B1.

### B5. General Method of Substituted Aniline Synthesis via Phenol Alkylation Followed by Reduction of a Nitroarene

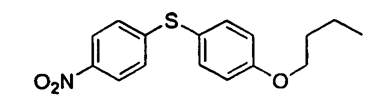

**Step 1. 4-(4-Butoxyphenyl)thio-1-nitrobenzene:** To a solution of 4-(4-nitrophenylthio)phenol (1.50 g, 6.07 mmol) in anh DMF (75 ml) at 0 °C was added NaH (60% in mineral oil, 0.267 g, 6.67 mmol). The brown suspension was stirred at 0 °C until gas evolution stopped (15 min), then a solution of iodobutane (1.12 g, .690 ml, 6.07 mmol) in anh DMF (20 mL) was added dropwise over 15 min at 0 °C. The reaction was stirred at room temp. for 18 h at which time TLC indicated the presence of unreacted phenol, and additional iodobutane (56 mg, 0.035 mL, 0.303 mmol, 0.05 equiv) and NaH (13 mg, 0.334 mmol) were added. The reaction was stirred an additional 6 h room temp., then was quenched by the addition of water (400 mL). The resulting mixture was extracted with Et₂O (2 x 500 mL). The combibed organics were washed with water (2 x 400 mL), dried (MgSO₄), and concentrated under reduced pressure to give a clear yellow oil, which was purified by silica gel chromatography (gradient from 20% EtOAc/80% hexane to 50% EtOAc/50% hexane) to give the product as a yellow solid (1.24 g, 67%): TLC (20% EtOAc/80% hexane) R_{*f*} 0.75; ¹H-NMR (DMSO-d₆) δ 0.92 (t, *J=* 7.5 Hz, 3H), 1.42 (app hex, *J*=7.5 Hz, 2H), 1.70 (m, 2H), 4.01 (t, *J=* 6.6 Hz, 2H), 7.08 (d, *J*=8.7 Hz, 2H), 7.17 (d, *J*=9 Hz, 2H), 7.51 (d, *J*= 8.7 Hz, 2H), 8.09 (d, *J*= 9 Hz, 2H).

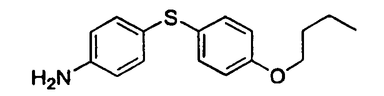

**Step 2. 4-(4-Butoxyphenyl)thioaniline:** 4-(4-Butoxyphenyl)thio-1-nitrobenzene was reduced to the aniline in a manner analagous to that used in the preparation of 3-(trifluoromethyl)-4-(4-pyridinylthio)aniline (Method B3b, Step 2): TLC (33% EtOAc/77% hexane) R_{*f*} 0.38.

### B6. General Method for Synthesis of Substituted Anilines by the Acylation of Diaminoarenes

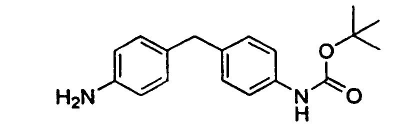

**4-(4-*****tert*****-Butoxycarbamoylbenzyl)aniline:** To a solution of 4,4'-methylenedianiline (3.00 g, 15.1 mmol) in anh THF (50 mL) at room temp was added a solution of di-*tert*-butyl dicarbonate (3.30 g, 15.1 mmol) in anh THF (10 mL). The reaction mixture was heated at the reflux temp. for 3 h, at which time TLC indicated the presence of unreacted methylenedianiline. Additional di-*tert*-butyl dicarbonate (0.664 g, 3.03 mmol, 0.02 equiv) was added and the reaction stirred at the reflux temp. for 16 h. The resulting mixture was diluted with Et₂O (200 mL), sequentially washed with a saturated NaHCO₃ solution (100 ml), water (100 mL) and a saturated NaCl solution (50 mL), dried (MgSO₄), and concentrated under reduced pressure. The resulting white solid was purified by silica gel chromatography (gradient from 33% EtOAc/67% hexane to 50% EtOAc/50% hexane) to afford the desired product as a white solid (2.09 g, 46%): TLC (50% EtOAc/50% hexane) R_{*f*} 0.45; ¹H-NMR (DMSO-d₆) δ 1.43 (s, 9H), 3.63 (s, 2H), 4.85 (br s, 2H), 6.44 (d, *J*=8.4 Hz, 2H), 6.80 (d, *J*=8.1 Hz. 2H), 7.00 (d, *J*=8.4 Hz, 2H), 7.28 (d, *J*=8.1 Hz, 2H), 9.18 (br s, 1H); FAB-MS *m*/*z* 298 (M⁺).

### General Method for the Synthesis of Aryl Amines via Electrophilic Nitration Followed by Reduction

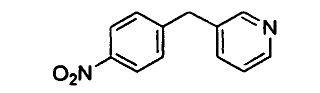

**Step 1. 3-(4-Nitrobenzyl)pyridine:** A solution of 3-benzylpyridine (4.0 g, 23.6 mmol) and 70% nitric acid (30 mL) was heated overnight at 50 °C. The resulting mixture was allowed to cool to room temp. then poured into ice water (350 mL). The aqueous mixture then made basic with a 1N NaOH solution, then extracted with Et₂O (4 x 100 mL). The combined extracts were sequentially washed with water (3 x 100 mL) and a saturated NaCl solution (2 x 100 mL), dried (Na₂SO₄), and concentrated *in vacuo.* The residual oil was purified by MPLC (silica gel; 50 % EtOAc/50% hexane) then recrystallization (EtOAc/hexane) to afford the desired product (1.0 g, 22%): GC-MS *m*/*z* 214 (M⁺).

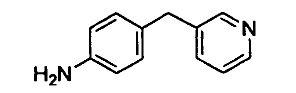

**Step 2. 3-(4-Pyridinyl)methylaniline:** 3-(4-Nitrobenzyl)pyridine was reduced to the aniline in a manner analogous to that described in Method B 1.

### General Method for Synthesis of Aryl Amines via Substitution with Nitrobenzyl Halides Followed by Reduction

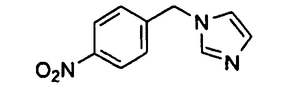

**Step 1. 4-(1-Imidazolylmethyl)-1-nitrobenzene**: To a solution of imidazole (0.5 g, 7.3 mmol) and 4-nitrobenzyl bromide (1.6 g, 7.3 mmol) in anh acetonitrile (30 mL) was added K₂CO₃ (1.0 g, 7.3 mmol). The resulting mixture was stirred at rooom temp. for 18 h and then poured into water (200 mL) and the resulting aqueous solution wasextracted with EtOAc (3 x 50 mL). The combined organic layers were sequentially washed with water (3 x 50 mL) and a saturated NaCl solution (2 x 50 mL), dried (MgSO₄), and concentrated *in vacuo.* The residual oil was purified by MPLC (silica gel; 25% EtOAc/75% hexane) to afford the desired product (1.0 g, 91 %): EI-MS *m*/*z* 203 (M⁺).

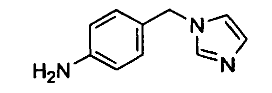

**Step 2. 4-(1-Imidazolylmethyl)aniline:** 4-(1-Imidazolylmethyl)-1-nitrobenzene was reduced to the aniline in a manner analogous to that described in Method B2.

### Formation of Substituted Hydroxymethylanilines by Oxidation of Nitrobenzyl Compounds Followed by Reduction

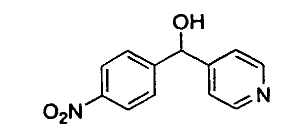

**Step 1. 4-(1-Hydroxy-1-(4-pyridyl)methyl-1-nitrobenzene:** To a stirred solution of 3-(4-nitrobenzyl)pyridine (6.0 g, 28 mmol) in CH₂Cl₂ (90 mL) was added *m*-CPBA (5.80 g, 33.6 mmol) at 10 °C, and the mixture was stirred at room temp. overnight. The reaction mixture was successively washed with a 10% NaHSO₃ solution (50 mL), a saturated K₂CO₃ solution (50 mL) and a saturated NaCl solution (50 mL), dried (MgSO₄) and concentrated under reduced pressure. The resulting yellow solid (2.68 g) was dissolved in anh acetic anhydride (30 mL) and heated at the reflux temperature overnight. The mixture was concentrated under reduced pressure. The residue was dissolved in MeOH (25 mL) and treated with a 20% aqueous NH₃ solution (30 mL). The mixture was stirred at room temp. for 1 h, then was concentrated under reduced pressure. The residue was poured into a mixture of water (50 mL) and CH₂Cl₂ (50 mL). The organic layer was dried (MgSO₄), concentrated under reduced pressure, and purified by column chromatography (80% EtOAc/ 20% hexane) to afford the desired product as a white solid. (0.53 g, 8%): mp 110-118 °C; TLC (80% EtOAc/20% hexane) R_{*f*} 0.12; FAB-MS *m*/*z* 367 ((M+H)⁻, 100%).

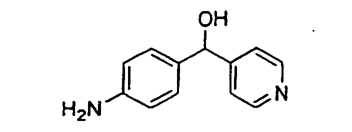

**Step 2. 4-(1-Hydroxy-1-(4-pyridyl)methylaniline:** 4-(1-Hydroxy-1-(4-pyridyl)-methyl-1-nitrobenzene was reduced to the aniline in a manner analogous to that described in Method B3d, Step2.

### B10. Formation of 2-(N-methylcarbamoyl)pyridines via the Menisci reaction

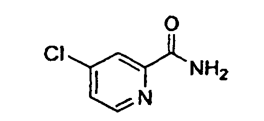

**Step 1. 2-(*****N*****-methylcarbamoyl)-4-chloropyridine. (Caution:** this is a highly hazardous, potentially explosive reaction.) To a solution of 4-chloropyridine (10.0 g) in *N*-methylformamide (250 mL) under argon at ambient temp was added conc. H₂SO₄ (3.55 mL) (exotherm). To this was added H₂O₂ (17 mL, 30% wt in H2O) followed by FeSO₄·7H2O (0.55 g) to produce an exotherm. The reaction was stirred in the dark at ambient temp for 1h then was heated slowly over 4 h at 45 °C. When bubbling subsided,the reaction was heated at 60 °C for 16 h. The opaque brown solution was diluted with H2O (700 mL) followed by a 10% NaOH solution (250 mL). The aqueous mixture was extracted with EtOAc (3 x 500 mL) and the organic layers were washed separately with a saturated NaCl solution (3 x 150 mlL. The combined organics were dried (MgSO₄) and filtered through a pad of silica gel eluting with EtOAc. The solvent was removed in vacuo and the brown residue was purified by silica gel chromatography (gradient from 50% EtOAc / 50% hexane to 80% EtOAc / 20% hexane). The resulting yellow oil crystallized at 0 °C over 72 h to give 2-(*N*-methylcarbamoyl)-4-chloropyridine in yield (0.61 g, 5.3%): TLC (50% EtOAc/50% hexane) R_{*f*} 0.50; MS; ¹H NMR (CDCl₃): d 8.44 (d, 1 H, J = 5.1 Hz, CHN), 8.21 (s, 1H, CHCCO), 7.96 (b s, 1H, NH), 7.43 (dd. 1H, J = 2.4, 5.4 Hz. ClCHCN), 3.04 (d. 3H, J = 5.1 Hz, methyl); CI-MS *m*/*z* 171 ((M+H)+).

### B11. Generalmethod for the Synthesis of ω-Sulfonylphenyl Anilines

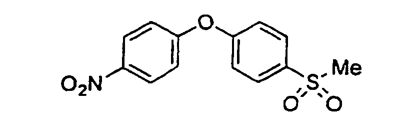

**Step 1. 4-(4-Methylsulfonylphenoxy)-1-nitrobenzene:** To a solution of 4-(4-methylthiophenoxy)-1-ntirobenzene (2 g, 7.66 mmol) in CH₂Cl₂ (75 mL) at 0 °C was slowly added *m*CPBA (57-86%, 4 g), and the reaction mixture was stirred at room temperature for 5 h. The reaction mixture was treated with a 1 N NaOH solution (25 mL). The organic layer was sequentially washed with a 1N NaOH solution (25 mL), water (25 mL) and a saturated NaCl solution (25 mL), dried (MgSO₄), and concentrated under reduced pressure to give 4-(4-methylsulfonylphenoxy)-1-nitrobenzene as a solid (2.1 g).

**Step 2. 4-(4-Methylsulfonylphenoxy)-1-aniline:** 4-(4-Methylsulfonylphenoxy)-1-nitrobenzene was reduced to the aniline in a manner anaologous to that described in Method B3d, step 2.

### B12. General Method for Synthesis of ω-Alkoxy-ω-carboxyphenyl Anilines

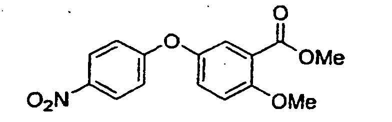

**Step 1. 4-(3-Methoxycarbonyl-4-methoxyphenoxy)-1-nitrobenzene:** To a solution of -(3-carboxy-4-hydroxyphenoxy)-1-nitrobenzene (prepared in a manner analogous to that described in Method B3a, step 1, 12 mmol) in acetone (50 mL) was added K₂CO₃ (5 g) and dimethyl sulfate (3.5 mL). The resulting mixture was heated at the reflux temperature overnight, then cooled to room temperature and filtered through a pad of Celite®. The resulting solution was concentrated under reduced pressure, absorbed onto silica gel, and purified by column chromatography (50% EtOAc / 50% hexane) to give 4-(3-methoxycarbonyl-4-methoxyphenoxy)-1-nitrobenzene as a yellow powder (3 g): mp 115 118 °C.

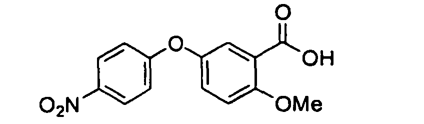

**Step 2. 4-(3-Carboxy-4-methoxyphenoxy)-1-nitrobenzene:** A mixture of 4-(3-methoxycarbonyl-4-methoxyphenoxy)-1-nitrobenzene (1.2 g), KOH (0.33 g),and water (5 mL) in MeOH (45 mL) was stirred at room temperature overnight and then heated at the reflux temperature for 4 h. The resulting mixture was cooled to room temperature and concentrated under reduced pressure. The residue was dissolved in water (50 mL), and the aqueous mixture was made acidic with a 1N HCl solution. The resulting mixture was extracted with EtOAc (50 mL). The organic layer was dried (MgSO₄) and concentrated under reduced pressure to give 4-(3-carboxy-4-methoxyphenoxy)-1-nitrobenzene (1.04 g).

### C. General Methods of Urea Formation

### C1a. Reaction of a Heterocyclic Amine with an Isocyanate

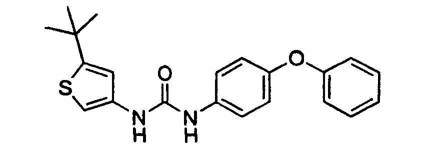

***N*****-(5-*****tert*****-Butyl-3-thienyl)-*****N'*****-(4-phenoxyphenyl)urea**: To a solution of *5-tert*-butyl-3-thiophene-ammonium chloride (prepared as described in Method A4b; 7.28 g, 46.9 mmol, 1.0 equiv) in anh DMF (80 mL) was added 4-phenoxyphenyl isocyanate (8.92 g, 42.21 mmol, 0.9 equiv) in one portion. The resulting solution was stirred at 50-60 °C overnight, then diluted with EtOAc (300 mL). The resulting solution was sequentially washed with H₂O (200 mL), a 1 N HCl solution (50 mL) and a saturated NaCl solution (50 mL), dried (Na₂SO₄), and concentrated under reduced pressure. The resulting off-white solid was recrystallized (EtOAc/hexane) to give a white solid (13.7 g, 88%), which was contaminated with approximately 5% of bis(4-phenoxyphenyl)urea. A portion of this material (4.67 g) was purified by flash chromatography (9% EtOAc/27% CH₂Cl₂/64% cyclohexane) to afforded the desired product as a white solid (3.17 g).

### C1b. Reaction of a Heterocyclic Amine with an Isocyanate

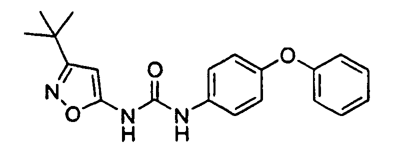

***N*****-(3-*****tert*****-Butyl-5-isoxazolyl)-*****N'*****-(4-phenoxyphenyl)urea:** To a solution of 5-amino-3-*tert*-butylisoxazole (8.93 g, 63.7 mmol, 1 eq.) in CH₂Cl₂ (60 mL) was added 4-phenyloxyphenyl isocyanate (15.47 g, 73.3 mmol, 1.15 eq.) dropwise. The mixture was heated at the reflux temp. for 2 days, eventually adding additional CH₂Cl₂ (80 mL). The resulting mixture was poured into water (500 mL) and extracted with Et₂O (3 x 200 mL). The organic layer was dried (MgSO₄) then concentrated under reduced pressure. The residue was recrystallized (EtOAc) to give the desired product (15.7 g, 70%): mp 182-184 °C; TLC (5% acetone/95% acetone) R_{*f*} 0.27; ¹H-NMR (DMSO-d₆) δ 1.23 (s, 9H), 6.02 (s, 1H), 6.97 (dd, *J*=0.2, 8.8 Hz, 2H), 6.93 (d, *J*=8.8 Hz, 2H), 7.08 (t, *J*=7.4 Hz, 1H), 7.34 (m, 2H), 7.45 (dd, *J*=2.2, 6.6 Hz, 2H), 8.80 (s, 1H), 10.04 (s, 1H); FAB-MS *m*/*z* (rel abundance) 352 ((M+H)⁺,70%).

### C1c. Reaction of a Heterocyclic Amine with an Isocyanate

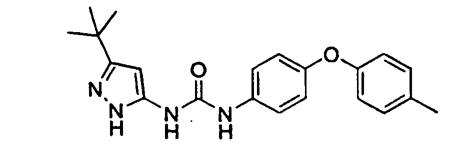

***N*****-(3-*****tert*****-Butyl-5-pyrazolyl)-*****N'*****-(4-(4-methylphenyl)oxyphenyl)urea:** A solution of 5-amino-3-*tert*-butylpyrazole (0.139 g, 1.0 mmol, 1.0 equiv) and 4-(4-methylphenoxy)phenyl isocyanate (0.225 g, 1.0 mmol 1.0 equiv) in toluene (10 mL) was heated at the reflux temp. overnight. The resulting mixture was cooled to room temp and quenched with MeOH (a few mL). After stirring for 30 min, the mixture was concentrated under reduced pressure. The residue was purified by prep. HPLC (silica, 50% EtOAc/50% hexane) to give the desired product (0.121 g, 33%): mp 204 °C; TLC (5% acetone/95% CH₂Cl₂) R_{*f*} 0.92; ¹H-NMR (DMSO-d₆) δ 1.22 (s, 9H), 2.24 (s, 3H), 5.92 (s, 1H), 6.83 (d, *J*=8.4 Hz, 2H), 6.90 (d, *J*=8.8 Hz, 2H), 7.13 (d, *J*=8.4 Hz, 2H), 7.40 (d, *J*=8.8 Hz, 2H), 8.85 (s, 1H), 9.20 (br s, 1H), 11.94 (br s, 1H); EI-MS *m*/*z* 364 (M⁺).

### C1d. Reaction of a Heterocyclic Amine with an Isocyanate

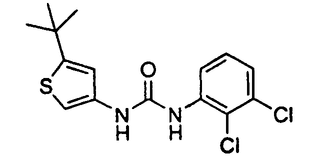

***N*****-(5-*****tert*****-Butyl-3-thienyl)-*****N'*****-(2,3-dichlorophenyl)urea:** Pyridine (0.163 mL, 2.02 mmol) was added to a slurry of 5-*tert*-butylthiopheneammonium chloride (Method A4c; 0.30 g, 1.56 mmol) and 2,3-dichlorophenyl isocyanate (0.32 mL, 2.02 mmol) in CH₂Cl₂ (10 mL) to clarify the mixture and the resulting solution was stirred at room temp. overnight. The reaction mixture was then concentrated under reduced pressure and the residue was separated between EtOAc (15 mL) and water (15 mL). The organic layer was sequentially washed with a saturated NaHCO₃ solution (15 mL), a 1N HCl solution (15 mL) and a saturated NaCl solution (15 mL), dried (Na₂SO₄), and concentrated under reduced pressure. A portion of the residue was by preparative HPLC (C-18 column; 60% acetonitrile/40% water/0.05% TFA) to give the desired urea (0.180 g, 34%): mp 169-170 °C; TLC (20% EtOAc/80% hexane) R_{*f*} 0.57; ¹H-NMR (DMSO-d₆) δ 1.31 (s, 9H), 6.79 (s, 1H), 7.03 (s, 1H), 7.24-7.33 (m, 2H), 8.16 (dd, *J*=1.84, 7.72 Hz, 1H), 8.35 (s, 1H), 9.60 (s, 1H); ¹³C-NMR (DMSO-d₆) δ 31.9 (3C), 34.0, 103.4, 116.1, 119.3, 120.0, 123.4, 128.1, 131.6, 135.6, 138.1, 151.7, 155.2; FAB-MS *m*/*z* (rel abundance) 343 ((M+H)⁺, 83%), 345 ((M+H+2)⁻, 56%), 347 ((M+H+4)⁺, 12%).

### C1e. Reaction of a Heterocyclic Amine with an Isocyanate

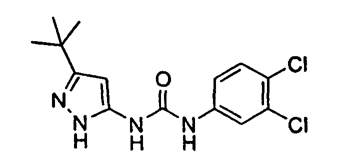

***N*****-(3-*****tert*****-Butyl-5-pyrazolyl)-*****N'*****-(3,4-dichlorophenyl)urea**: A solution of 5-amino-3-*tert*-butyl-*N*^{*1*}-(*tert*-butoxycarbonyl)pyrazole (Method A5; 0.150 g, 0.63 mmol) and 3,4-dichlorophenyl isocyanate (0.118 g, 0.63 mmol) were in toluene (3.1 mL) was stirred at 55 °C for 2 d. The toluene was removed *in vacuo* and the solid was redissolved in a mixture of CH₂Cl₂ (3 mL) and TFA (1.5 mL). After 30 min, the solvent was removed *in vacuo* and the residue was taken up in EtOAc (10 mL). The resulting mixture was sequentially washed with a saturated NaHCO₃ solution (10 mL) and a NaCl solution (5 mL), dried (Na₂SO₄), and concentrated *in vacuo.* The residue was purified by flash chromatography (gradient from 40% EtOAc/ 60% hexane to 55%EtOAc/ 5% hexane) to give the desired product (0.102 g, 48%): mp 182-184 °C; TLC (40% EtOAc/60% hexane) R_{*f*} 0.05, FAB-MS *m*/*z* 327 ((M+H)⁻).

### C2a. Reaction of a Heterocyclic Amine with Phosgene to Form an Isocyanate, then Reaction with Substituted Aniline

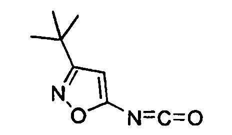

**Step 1. 3-*****tert*****-Butyl-5-isoxazolyl Isocyanate:** To a solution of phosgene (20% in toluene, 1.13 mL, 2.18 mmol) in CH₂Cl₂ (20 mL) at 0 °C was added anh. pyridine (0.176 mL, 2.18 mmol), followed by 5-amino-3-*tert*-butylisoxazole (0.305 g, 2.18 mmol). The resulting solution was allowed to warm to room temp. over 1 h, and then was concentrated under reduced pressure. The solid residue dried *in vacuo* for 0.5 h.

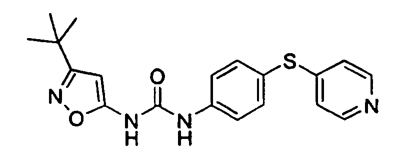

**Step 2.** ***N*****-(3-*****tert*****-Butyl-5-isoxazolyl)-*****N'*****-(4-(4-pyridinylthio)phenyl)urea**: The crude 3-*tert*-butyl-5-isoxazolyl isocyanate was suspended in anh toluene (10 mL) and 4-(4-pyridinylthio)aniline (0.200 g, 0.989 mmol) was rapidly added. The suspension was stirred at 80 °C for 2 h then cooled to room temp. and diluted with an EtOAc/CH₂Cl₂ solution (4:1, 125 mL). The organic layer was washed with water (100 mL) and a saturated NaCl solution (50 mL), dried (MgSO₄), and concentrated under reduced pressure. The resulting yellow oil was purified by column chromatography (silica gel, gradient from 2% MeOH/98% CH₂Cl₂ to 4% MeOH/6% CH₂Cl₂) to afford a foam, which was triturated (Et₂O/hexane) in combination with sonication to give the product as a white powder (0.18 g, 49%): TLC (5% MeOH/95% CH₂Cl₂) R_{*f*} 0.21; ¹H-NMR (DMSO-d₆) δ 1.23 (s, 9H), 6.06 (s, 1H), 6.95 (d, *J*=5 Hz, 2H), 7.51 (d, *J*=8 Hz, 2H), 7.62 (d, *J*=8 Hz, 2H), 8.32 (d, *J*=5 Hz. 2H), 9.13 (s, 1H), 10.19 (s, 1H); FAB-MS *m*/*z* 369 ((M+H)⁺).

### C2b. Reaction of a Heterocyclic Amine with Phosgene to Form an Isocyanate Followed by Reaction with Substituted Aniline

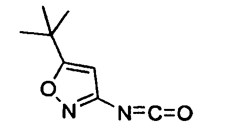

**Step 1. 5-*****tert*****-Butyl-3-isoxazolyl Isocyanate:** To a solution of phosgene (148 mL, 1.93 M in toluene, 285 mmol) in anhydrous CH₂Cl₂ (1 L) was added 3-amino-5-*tert*-butylisoxazole (10.0 g, 71 mmol) followed by pyridine (46 mL, 569 mmol). The mixture was allowed to warm to room temp and stirred overnight (ca. 16 h), then mixture was concentrated *in vacuo.* The residue was dissolved in anh. THF (350 mL) and stirred for 10 min. The orange precipitate (pyridinium hydrochloride) was removed and the isocyanate-containing filtrate (approximately 0.2 M in THF) was used as a stock solution: GC-MS (aliquot obtained prior to concentration) *m*/*z* 166 (M⁺).

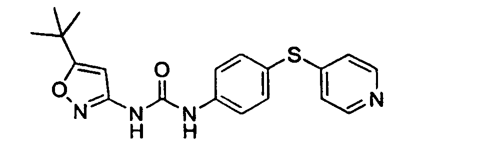

**Step 2.** ***N*****-(5-*****tert*****-Butyl-3-isoxazolyl)-*****N'*****-(4-(4-pyridinylthio)phenyl)urea**: To a solution of 5-*tert*-butyl-3-isoxazolyl isocyanate (247 mL, 0.2 M in THF, 49.4 mmol) was added 4-(4-pyridinylthio)aniline (5 g, 24.72 mmol), followed by THF (50 mL) then pyridine (4.0 mL, 49 mmol) to neutralize any residual acid. The mixture was stirred overnight (ca. 18 h) at room temp. Then diluted with EtOAc (300 mL). The organic layer was washed successively with a saturated NaCl solution (100 mL), a saturated NaHCO₃ solution (100 mL), and a saturated NaCl solution (100 mL), dried (MgSO₄), and concentrated *in vacuo*. The resulting material was purified by MPLC (2 x 300 g silica gel, 30 % EtOAc/70% hexane) to afford the desired product as a white solid (8.24 g, 90 %): mp 178-179 °C; ¹H-NMR (DMSO-d₆) δ 1.28 (s, 9H), 6.51 (s, 1H), 6.96 (d, *J*=6.25 Hz, 2H), 7.52 (d, *J*=8.82 Hz. 2H), 7.62 (d. *J*=8.83 Hz, 2H), 8.33 (d, *J*=6.25 Hz, 2H), 9.10 (s, 1H), 9.61 (s, 1H); EI-MS *m*/*z* 368 (M⁺),

### C2c. Reaction of a Heterocyclic Amine with Phosgene to Form an Isocyanate Followed by Reaction with Substituted Aniline

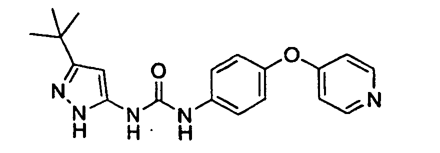

***N*****-(3-*****tert*****-Butyl-5-pyrazolyl)-*****N'*****-(4-(4-pyridinyloxy)phenyl)urea**: To a solution of phosgene (1.9M in toluene, 6.8 mL) in anhydrous CH₂Cl₂ (13 mL) at 0 °C was slowly added pyridine (0.105 mL) was added slowly over a 5 min, then 4-(4-pyridinyloxy)aniline (0.250 g, 1.3 mmol) was added in one aliquot causing a transient yellow color to appear. The solution was stirred at 0 °C for 1 h, then was allowed to warm to room temp. over 1 h. The resulting solution was concentrated *in vacuo* then the white solid was suspended in toluene (7 mL). To this slurry, 5-amino-3-*tert*-butyl-*N'*-(*tert*-butoxycarbonyl)pyrazole (0.160 g, 0.67 mmol) was added in one aliquot and the reaction mixture was heated at 70 °C for 12 h forming a white precipitate. The solids were dissolved in a 1N HCl solution and allowed to stir at room temp. for 1 h to form a new precipitate. The white solid was washed (50% Et₂O/50% pet. ether) to afford the desired urea (0.139 g, 59%): mp >228 °C dec; TLC (10% MeOH/ 90% CHCl₃) R_{*f*} 0.239; ¹H-NMR (DMSO-d₆) δ 1.24 (s, 9H), 5.97 (s, 1H), 6.88 (d, *J*=6.25 Hz, 2H), 7.10 (d, *J*=8.82 Hz, 2H), 7.53 (d, *J*=9.2 Hz, 2H), 8.43 (d, *J*=6.25 Hz, 2H), 8.92 (br s, 1H), 9.25 (br s, 1H), 12.00 (br s, 1H); EI-MS *m*/*z* rel abundance 351 (M⁺, 24%).

### C3a. Reaction of a Heterocyclic Amine with N,N'-Carbonyldiimidazole Followed by Reaction with a Substituted Aniline

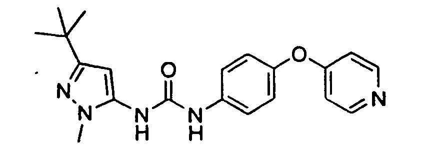

***N*****-(3-*****tert*****-Butyl-1-methyl-5-pyrazolyl)-*****N*****'-(4-(4-pyridinyloxy)phenyl)urea:** To a solution of 5-amino-3-*tert*-butyl-1-methylpyrazole (189 g, 1.24 mol) in anh. CH₂Cl₂ (2.3 L) was added *N,N*'-carbonyldiimidazole (214 g, 1.32 mol) in one portion. The mixture was allowed to stir at ambient temperature for 5 h before adding 4-(4-pyridinyloxy)aniline. The reaction mixture was heated to 36 °C for 16 h. The resulting mixture was cooled to room temp, diluted with EtOAc (2 L) and washed with H₂O (8 L) and a saturated NaCl solution (4 L). The organic layer was dried (Na₂SO₄) and concentrated *in vacuo.* The residue was purified by crystallization (44.4% EtOAc/44.4% Et₂O/11.2% hexane, 2.5 L) to afford the desired urea as a white solid (230 g, 51%): mp 149-152 °C; ¹H-NMR (DMSO-d₆) δ 1.18 (s, 9H), 3.57 (s, 3H), 6.02 (s, 1H), 6.85 (d, *J*=6.0 Hz, 2H), 7.08 (d, *J*=9.0 Hz, 2H), 7.52 (d, *J*=9.0 Hz, 2H), 8.40 (d, *J*=6.0 Hz, 2H), 8.46 (s, 1H), 8.97 (s, 1H); FAB-LSIMS *m*/*z* 366 ((M+H)⁺).

### C3b. Reaction of a Heterocyclic Amine with N,N'-Carbonyldiimidazole Followed by Reaction with a Substituted Aniline

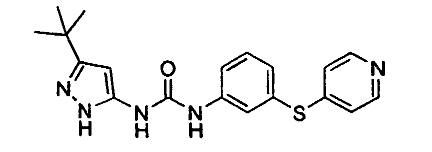

***N*****-(3-*****tert*****-Butyl-5-pyrazolyl)-*****N'*****-(3-(4-pyridinylthio)phenyl)urea**: To a solution of 5-amino-3-*tert*-butyl-*N'*-(*tert*-butoxycarbonyl)pyrazole (0.282 g, 1.18 mmol) in CH₂Cl₂ (1.2 mL) was added *N,N'*-carbonyldiimidazole (0.200 g, 1.24 mmol) and the mixture was allowed to stir at room temp. for 1 day. 3-(4-Pyridinylthio)aniline (0.239 g, 1.18 mmol) was added to the reaction solution in one aliquot and the resulting mixture was allowed to stir at room temp. for 1 day. Then resulting solution was treated with a 10% citric acid solution (2 mL) and was allowed to stir for 4 h. The organic layer was extracted with EtOAc (3 x 15 mL), dried (MgSO₄), and concentrated *in vacuo.* The residue was diluted with CH₂Cl₂ (5 mL) and trifluoroacetic acid (2 mL) and the resulting solution was allowed to stir for 4 h. The trifluoroacetic reaction mixture was made basic with a saturated NaHCO₃ solution, then extracted with CH₂Cl₂ (3 x 15 mL). The combined organic layers were dried (MgSO₄₎ and concentrated *in vacuo.* The residue was purified by flash chromatography (5% MeOH/95% CH₂Cl₂). The resulting brown solid was triturated with sonication (50% Et₂O/50% pet. ether) to give the desired urea (0.122 g, 28%): mp >224 °C dec; TLC (5% MeOH/ 95% CHCl₃) R_{*f*} 0.067; ¹H-NMR (DMSO-d₆) δ 1.23 (s, 9H), 5.98 (s, 1H), 7.04 (dm, *J*=13.24 Hz, 2H), 7.15-7.19 (m, 1H), 7.40-7.47 (m, 2H), 7.80-7.82 (m, 1H), 8.36 (dm, *J*=15.44 Hz, 2H), 8.96 (br s, 1H), 9.32 (br s, 1H), 11.97 (br s, 1H); FAB-MS m/z (rel abundance) 368 (M⁺, 100%).

### C4a. Reaction of Substituted Aniline with N,N'-Carbonyldiimidazole Followed by Reaction with a Heterocyclic Amine

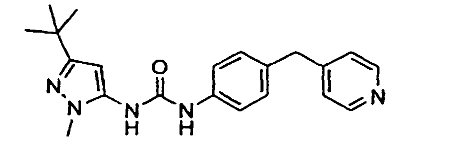

***N*****-(3-*****tert*****-Butyl-1-methyl-5-pyrazolyl)-*****N'*****-(4-(4-pyridinylmethyl)phenyl)urea:** To a solution of 4-(4-pyridinylmethyl)aniline (0.200 g, 1.08 mmol) in CH₂Cl₂ (10 mL) was added *N,N'*-carbonyldiimidazole (0.200 g, 1.23 mmol). The resulting mixture was stirred at room temp for 1 h after which TLC analysis indicated no starting aniline. The reaction mixture was then treated with 5-amino-3-*tert*-butyl-1-methylpyrazole (0.165 g, 1.08 mmol) and stirred at 40-45 °C overnight. The reaction mixture was cooled to room temp and purified by column chromatography (gradient from 20% acetone/80% CH₂Cl₂ to 60% acetone/40% CH₂Cl₂) and the resulting solids were crystallized (Et2O) to afford the desired urea (0.227 g, 58%): TLC (4% MeOH/96% CH₂Cl₂) R_{*f*} 0.15; ¹H-NMR (DMSO-d₆) δ 1.19 (s, 9H), 3.57 (s, 3H), 3.89 (s, 2H), 6.02 (s, 1H), 7.14 (d, *J*=8.4 Hz, 2H), 7.21 (d, *J*=6 Hz, 2H), 7.37 (d, *J*=8.4 Hz, 2H), 8.45-8.42 (m, 3H), 8.81 (s, 1H); FAB-MS *m*/*z* 364 (M+H)⁺).

### C4b. Reaction of Substituted Aniline with N,N'-Carbonyldiimidazole Followed by Reaction with a Heterocyclic Amine

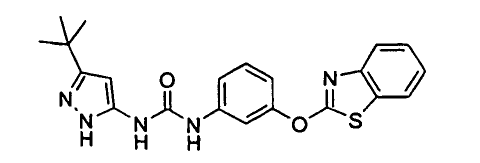

***N*****-(3-*****tert*****-Butyl-5-pyrazolyl)-*****N'*****-(3-(2-benzothiazolyloxy)phenyl)urea**: A solution of 3-(2-benzothiazolyloxy)aniline (0.24 g, 1.0 mmol, 1.0 equiv) and *N,N*'-carbonyldiimidazole (0.162 g, 1.0 mmol, 1.0 equiv) in toluene (10 mL) was stirred at room temp for 1 h. 5-Amino-3-*tert*-butylpyrazole (0.139 g, 1.0 mmol) was added and the resulting mixture was heated at the reflux temp. overnight. The resulting mixture was poured into water and extracted with CH₂Cl₂ (3 x 50 mL). The combined organic layers were concentrated under reduced pressure and dissolved in a minimal amount of CH₂Cl₂. Petroleum ether was added and resulting white precipitate was resubmitted to the crystallization protocol to afford the desired product (0.015 g, 4%): mp 110-111 °C; TLC (5% acetone/95% CH₂Cl₂) R_{*f*} 0.05; ¹H-NMR (DMSO-d₆) δ 1.24 (s, 9H), 5.97 (s, 1H), 7.00-7.04 (m, 1H), 7.21-7.44 (m, 4H), 7.68 (d, *J*=5.5 Hz, 1H), 7.92 (d, *J*=7.7 Hz, 1H), 7.70 (s, 1H), 8.95 (s, 1H), 9.34 (br s, 1H), 11.98 (br s, 1H); EI-MS *m*/*z* 408 (M⁺).

### C4c. Reaction of a Heterocyclic Amine with Phosgene to Form an Isocyanate Followed by Reaction with Substituted Aniline

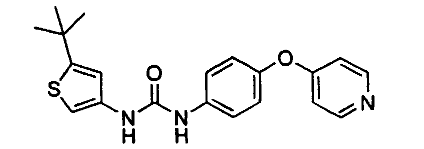

***N*****-(5-*****tert*****-Butyl-3-thienyl)-*****N'*****-(4-(4-pyridinyloxy)phenyl)urea:** To an ice cold solution phosgene (1.93M in toluene; 0.92 mL, 1.77 mmol) in CH₂Cl₂ (5 mL) was added a solution of 4-(4-pyridinyloxy)aniline (0.30 g, 1.61 mmol) and pyridine (0.255 g, 3.22 mmol) in CH₂Cl₂ (5 mL). The resulting mixture was allowed to warm to room temp. and was stirred for 1 h, then was concentrated under reduced pressure. The residue was dissolved in CH₂Cl₂ (5 mL), then treated with 5-*tert*-butylthiopheneammonium chloride (Method A4c; 0.206 g, 1.07 mmol), followed by pyridine (0.5 mL). The resulting mixture was stirred at room temp for 1 h, then treated with 2-(dimethylamino)ethylamine (1 mL), followed by stirring at room temp an additional 30 min. The reaction mixture was then diluted with EtOAc (50 mL), sequentially washed with a saturated NaHCO₃ solution (50 mL) and a saturated NaCl solution (50 mL), dried (Na₂SO₄), and concentrated under reduced pressure. The residue was purified by column chromatography (gradient from 30% EtOAc/70% hexane to 100% EtOAc) to give the desired product (0.38 g , 97%): TLC (50% EtOAc/50% hexane) R_{*f*} 0.13; ¹H-NMR (CDCl₃) δ 1.26 (s, 9H), 6.65 (d, *J*=1.48 Hz, 1H), 6.76 (dd, *J*=1.47, 4.24 Hz, 2H), 6.86 (d, *J*=1.47 Hz, 1H), 6.91 (d, *J*=8.82 Hz, 2H), 7.31 (d, *J*=8.83 Hz, 2H), 8.39 (br s, 2H), 8.41 (d, *J*=1.47 Hz, 2H); ¹³C-NMR (CDCl₃) δ 32.1 (3C), 34.4, 106.2, 112.0 (2C), 116.6, 121.3 (2C), 121.5 (2C), 134.9, 136.1, 149.0, 151.0 (2C), 154.0, 156.9, 165.2; FAB-MS *m*/*z* (rel abundance) 368 ((M+H)⁺, 100%).

### C5. General Method for the Reaction of a Substituted Aniline with Triphosgene Followed by Reaction with a Second Substituted Amine

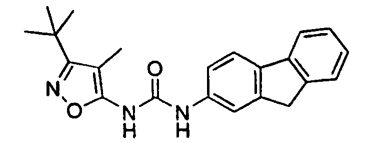

***N*****-(3-*****tert*****-Butyl-4-methyl-5-isoxazolyl)-*****N'*****-(2-fluorenyl)urea**: To a solution of triphosgene (55 mg, 0.185 mmol, 0.37eq) in 1,2-dichloroethane (1.0mL) was added a solution of 5-amino-4-methyl-3-*tert*-butylisoxazole (77.1 mg, 0.50 mmol, 1.0 eq) and diisopropylethylamine (0.104 mL, 0.60 mmol, 1.2 eq) in 1,2-dichloroethane (1.0 mL). The reaction mixture was stirred at 70 °C for 2 h, cooled to room temp., and treated with a solution of 2-aminofluorene (30.6 mg, 0.50 mmol, 1.0 eq) and diisopropylethylamine (0.087 mL, 1.0 eq) in 1,2-dichloroethane (1.0 mL). The reaction mixture was-stirred at 40 °C for 3 h and then at RT for 17 h to produce a precipitate. The solids were washed with Et₂O and hexanes to give the desired urea as a beige solid (25 mg, 14%): mp 179-181 °C; ¹H-NMR (DMSO-d₆) δ 1.28 (s, 9H), 2.47 (s, 3H), 3.86 (s, 2H), 7.22 (t, *J*=7.3 Hz, 1H), 7.34 (m, 2H), 7.51 (d, *J*=7.3 Hz, 1H), 7.76 (m, 3H), 8.89 (s, 1H), 9.03 (s, 1H); HPLC ES-MS *m*/*z* 362 ((M+H)⁺).

### C6. General Method for Urea Formation by Curtius Rearrangement and Carbamate Trapping

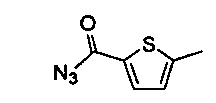

**Step 1. 5-Methyl-2-(azidocarbonyl)thiophene:** To a solution of 5-Methyl-2-thiophenecarboxylic acid (1.06 g, 7.5 mmol) and Et₃N (1.25 mL, 9.0 mmol) in acetone (50 mL) at -10 °C was slowly added ethyl chloroformate (1.07 mL, 11.2 mmol) to keep the internal temperature below 5 °C. A solution of sodium azide (0.83 g, 12.7 mmol) in water (6 mL) was added and the reaction mixture was stirred for 2 h at 0 °C. The resulting mixture was diluted with CH₂Cl₂ (10 mL) and washed with a saturated NaCl solution (10 mL). The aqueous layer was back-extracted with CH₂Cl₂ (10 mL), and the combined organic layers were dried (MgSO₄) and concentrated *in vacuo*. The residue was purified by column chromatography (10% EtOAc/ 90% hexanes) to give the azidoester (0.94 g, 75%). Azidoester (100 mg, 0.6 mmol) in anhydrous toluene (10 mL) was heated to reflux for 1 h then cooled to rt. This solution was used as a stock solution for subsequent reactions.

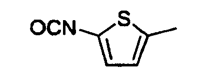

**Step 2. 5-Methyl-2-thiophene Isocyanate**: 5-Methyl-2-(azidocarbonyl)thiophene (0.100 g, 0.598 mmol) in anh toluene (10 mL) was heated at the reflux temp. for 1 h then cooled to room temp. This solution was used as a stock solution for subsequent reactions.

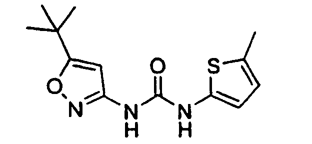

**Step 3.** ***N*****-(5-*****tert*****-Butyl-3-isoxazolyl)-*****N'*****-(5-methyl-2-thienyl)urea**: To a solution of 5-methyl-2-thiophene isocyanate (0.598 mmol) in toluene (10 mL) at room temp. was added 3-amino-5-*tert*-butylisoxazole (0.092 g, 0.658 mmol) and the resulting mixture was stirred overnight. The reaction mixture was diluted with EtOAc (50 mL) and sequentially washed with a 1 N HCl solution (2 x 25 mL) and a saturated NaCl solution (25 mL), dried (MgSO₄), and concentrated under reduced pressure. The residue was purified by MPLC (20% EtOAc/80% hexane) to give the desired urea (0.156 g, 93%): mp 200-201 °C; TLC (20% EtOAc/80% hexane) R_{*f*} 0.20; EI-MS *mlz* 368 (M⁺).

### C7. General Methods for Urea Formation by Curtius Rearrangement and Isocyanate Trapping

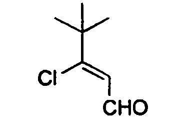

**Step 1. 3-Chloro-4,4-dimethylpent-2-enal:** POCl₃ (67.2 mL, 0.72 mol) was added to cooled (0 °C) DMF (60.6 mL, 0.78 mol) at rate to keep the internal temperature below 20 °C. The viscous slurry was heated until solids melted (approximately 40 °C), then pinacolone (37.5 mL, 0.30 mol) was added in one portion. The reaction mixture was then to 55 °C for 2h and to 75 °C for an additional 2 h. The resulting mixture was allowed to cool to room temp., then was treated with THF (200 mL) and water (200 mL), stirred vigorously for 3 h, and extracted with EtOAc (500 mL). The organic layer was washed with a saturated NaCl solution (200 mL), dried (Na₂SO₄) and concentrated under reduced pressure. The residue was filtered through a pad of silica (CH₂Cl₂) to give the desired aldehyde as an orange oil (15.5 g, 35%): TLC (5% EtOAc/95% hexane) R_{*f*} 0.54; ¹H NMR (CDCl₃) d 1.26 (s, 9H), 6.15 (d, *J*=7.0 Hz, 1H), 10.05 (d, *J*=6.6 Hz, 1H).

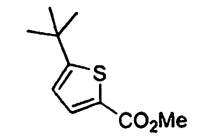

**Step 2. Methyl 5-*****tert*****-butyl-2-thiophenecarboxylate:** To a solution of 3-chloro-4,4-dimethylpent-2-enal (1.93 g, 13.2 mmol) in anh. DMF (60 mL) was added a solution of Na₂S (1.23 g, 15.8 mmol) in water (10 mL). The resulting mixture was stirred at room temp. for 15 min to generate a white precipitate, then the slurry was treated with methyl bromoacetate (2.42 g, 15.8 mmol) to slowly dissolve the solids. The reaction mixture was stirred at room temp. for 1.5 h, then treated with a 1 N HCl solution (200 mL) and stirred for 1 h. The resulting solution was extracted with EtOAc (300 mL). The organic phase was sequentially washed with a 1 N HCl solution (200 mL), water (2 x 200 mL) and a saturated NaCl solution (200 mL), dried (Na₂SO₄) and concentrated under reduced pressure. The residue was purified using column chromatography (5% EtOAc/95% hexane) to afford the desired product (0.95 g, 36%): TLC (20% EtOAc/80% hexane) R_{*f*} 0.79; ¹H NMR (CDCl₃) δ 1.39 (s, 9H), 3.85 (s, 3H), 6.84 (d, *J*=3.7 Hz, 1H), 7.62 (d, *J*=4.1 Hz, 1H); GC-MS *m*/*z* (rel abundance) 198 (M⁺, 25%).

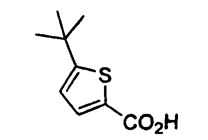

**Step 3. 5-*****tert*****-Butyl-2-thiophenecarboxylic acid:** Methyl 5-*tert*-butyl-2-thiophenecarboxylate (0.10 g, 0.51 mmol) was added to a KOH solution (0.33 M in 90% MeOH/10% water, 2.4 mL, 0.80 mmol) and the resulting mixture was heated at the reflux temperature for 3 h. EtOAc (5 mL) was added to the reaction mixture, then the pH was adjusted to approximately 3 using a 1 N HCl solution. The resulting organic phase was washed with water (5 mL), dried (Na₂SO₄), and concentrated under reduced pressure (0.4 mmHg) to give the desired carboxylic acid as a yellow solid (0.067 g, 73%): TLC (20% EtOAc/79.5% hexane/0.5% AcOH) R*f* 0.29; ¹H NMR (CDCl₃) δ 1.41 (s, 9H), 6.89 (d, *J*=3.7 Hz, 1H), 7.73 (d, *J*=3.7 Hz, 1H), 12.30 (br s, 1H); ¹³C NMR (CDCl₃) δ 32.1 (3C), 35.2, 122.9, 129.2, 135.1, 167.5, 168.2.

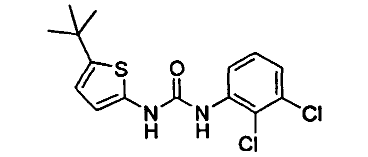

**Step 4.** ***N*****-(5-*****tert*****-Butyl-2-thienyl)-*****N'*****-(2,3-dichlorophenyl)urea**: A mixture of 5-*tert*-butyl-2-thiophenecarboxylic acid (0.066 g, 0.036 mmol), DPPA (0.109 g, 0.39 mmol) and Et₃N (0.040 g, 0.39 mmol) in toluene (4 mL) was heated to 80 °C for 2 h, 2,3-dichloroaniline (0.116 g, 0.72 mmol) was added, and the reaction mixture was heated to 80°C for an additional 2 h. The resulting mixture was allowed to cool to room temp. and treated with EtOAc (50 mL). The organic layer was washed with a 1 N HCl solution (3 x 50 mL), a saturated NaHCO₃ solution (50 mL), and a saturated NaCl solution (50 mL), dried (Na₂SO₄), and concentrated under reduced pressure. The residue was purified by column chromatography (5% EtOAc/95% hexane) to afford the desired urea as a purple solid (0.030 g, 24%): TLC (10% EtOAc/90% hexane) R*f* 0.28; ¹H NMR (CDCl₃) δ 1.34 (s, 9H), 6.59 (br s, 2H), 7.10-7.13 (m, 2H), 7.66 (br s, 1H), 8.13 (dd, *J*=2.9, 7.8 Hz, 1H); ¹³C NMR (CDCl₃) δ 32.2 (3C), 34.6, 117.4, 119.0⁷, 119.1⁵, 119.2, 121.5, 124.4, 127.6, 132.6, 135.2, 136.6, 153.4; HPLC ES-MS *m*/*z* (rel abundance) 343 ((M+H)⁺, 100%), 345 ((M+H+2)⁺, 67%), 347 ((M+H+4)⁺, 14%).

### C8. Combinatorial Method for the Synthesis of Diphenyl Ureas Using Triphosgene

One of the anilines to be coupled was dissolved in dichloroethane (0.10 M). This solution was added to a 8 mL vial (0.5 mL) containing dichloroethane (1 mL). To this was added a triphosgene solution (0.12 M in dichloroethane, 0.2 mL, 0.4 equiv.), followed by diisopropylethylamine (0.35 M in dichloroethane, 0.2 mL, 1.2 equiv.). The vial was capped and heat at 80 °C for 5 h, then allowed to cool to room temp for approximately 10 h. The second aniline was added (0.10 M in dichloroethane, 0.5 mL, 1.0 equiv.), followed by diisopropylethylamine (0.35 M in dichloroethane, 0.2 mL, 1.2 equiv.). The resulting mixture was heated at 80 °C for 4 h, cooled to room temperature and treated with MeOH (0.5 mL). The resulting mixture was concentrated under reduced pressure and the products were purified by reverse phase HPLC.

### D. Misc. Methods of Urea Synthesis

### D1. Electrophylic Halogenation

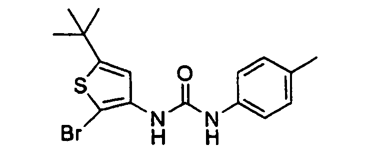

***N*****-(2-Bromo-5-*****tert*****-butyl-3-thienyl)-*****N'*****-(4-methylphenyl)urea:** To a slurry of N-(5-*tert*-butyl-3-thienyl)-*N'*-(4-methylphenyl)urea (0.50 g, 1.7 mmol) in CHCl₃ (20 mL) at room temp was slowly added a solution of Br₂ (0.09 mL, 1.7 mmol) in CHCl₃ (10 mL) via addition funnel causing the reaction mixture to become homogeneous. Stirring was continued 20 min after which TLC analysis indicated complete reaction. The reaction was concentrated under reduced pressure, and the residue triturated (2 x Et₂O/hexane) to give the brominated product as a tan powder (0.43 g, 76%): mp 161-163 °C; TLC (20% EtOAc/ 80% hexane) R_{*f*} 0.71; ¹H NMR (DMSO-d₆) δ 1.29 (s, 9H), 2.22 (s, 3H), 7.07 (d, *J*=8.46 Hz, 2H), 7.31 (d, *J*=8.46 Hz, 2H), 7.38 (s, 1H), 8.19 (s, 1H), 9.02 (s, 1H); ¹³C NMR (DMSO-d₆) δ 20.3, 31.6 (3C), 34.7, 89.6, 117.5, 118.1 (2C), 129.2 (2C), 130.8, 136.0, 136.9, 151.8, 155.2; FAB-MS *m*/*z* (rel abundance) 367 ((M+H)⁺, 98%), 369 (M+2+H)⁺, 100%).

### D2. Synthesis of ω-Alkoxy Ureas

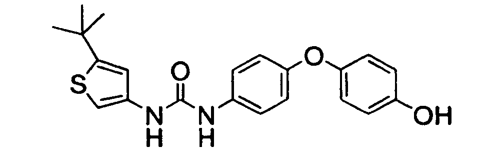

**Step 1.** ***N*****-(5-*****tert*****-Butyl-3-thienyl)-*****N*****'-(4-(4-hydroxyphenyl)oxyphenyl)urea:** A solution of *N*-(5-*tert*-butyl-3-thienyl)-*N*'-(4-(4-methoxyphenyl)oxyphenyl)urea (1.2 g, 3 mmol) in CH₂Cl₂ (50 mL) was cooled to -78 °C and treated with BBr₃ (1.0 M in CH₂Cl₂, 4.5 mL, 4.5 mmol, 1.5 equiv) dropwise via syringe. The resulting bright yellow mixture was warmed slowly to room temp and stirred overnight. The resulting mixture was concentrated under reduced pressure. The residue was dissolved in EtOAc (50 mL), then washed with a saturated NaHCO₃ solution (50 mL) and a saturated NaCl solution (50 mL), dried (Na₂SO₄), and concentrated under reduced pressure. The residue was purified via flash chromatography (gradient from 10% EtOAc/90% hexane to 25% EtOAc/75% hexane) to give the desired phenol as a tan foam (1.1 g, 92%): TLC (20% EtOAc/80% hexane) R_{*f*} 0.23; ¹H NMR (DMSO-d₆) δ 1.30 (s, 9H), 6.72-6.84 (m, 7H), 6.97 (d, *J*=1.47 Hz, 1H), 7.37 (dm, *J*=9.19 Hz, 2H), 8.49 (s, 1H), 8.69 (s, 1H), 9.25 (s, 1H); FAB-MS *m*/*z* (rel abundance) 383 ((M+H)⁺, 33%).

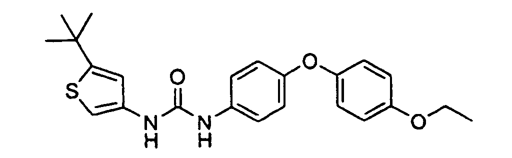

**Step 2.** ***N*****-(5-*****tert*****-Butyl-3-thienyl)-*****N'*****-(4-(4-ethoxyphenyl)oxyphenyl)urea:** To a mixture of *N*-(5-*tert*-butyl-3-thienyl)-*N'*-(4-(4-hydroxyphenyl)oxyphenyl)urea (0.20 g, 0.5 mmol) and Cs₂CO₃ (0.18 g, 0.55 mmol, 1.1 equiv) in reagent grade acetone (10 mL) was added ethyl iodide (0.08 mL, 1.0 mmol, 2 equiv) via syringe, and the resulting slurry was heated at the reflux temp. for 17 h. The reaction was cooled, filtered, and the solids were washed with EtOAc. The combined organics were concentrated under reduced pressure, and the residue was purified via preparative HPLC (60% CH₃CN/40% H₂O/0.05% TFA) to give the desired urea as a colorless powder (0.16 g, 73%): mp 155-156 °C; TLC (20% EtOAC/ 80% hexane) R_{*f*} 0.40; ¹H-NMR (DMSO-d₆) δ 1.30 (s, 9H), 1.30 (t, *J*=6.99 Hz, 3H), 3.97 (q, *J*=6.99 Hz, 2H), 6.80 (d, *J*=1.47 Hz, 1H), 6.86 (dm, *J*=8.82 Hz, 2H), 6.90 (s, 4H), 6.98 (d, *J*=1.47, 1H), 7.40 (dm, *J*=8.83 Hz, 2H), 8.54 (s, 1H), 8.73 (s, 1H); ¹³C-NMR (DMSO-d₆) δ 14.7, 32.0 (3C), 33.9, 63.3, 102.5, 115.5 (2C), 116.3, 118.4 (2C), 119.7 (2C), 119.8 (2C), 135.0, 136.3, 150.4, 152.1, 152.4, 154.4, 154.7; FAB-MS *m*/*z* (rel abundance) 411 ((M+H)⁺, 15%).

### D3. Synthesis of ω-Carbamoyl Ureas

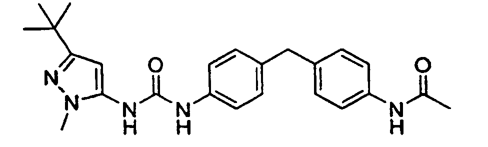

***N*****-(3-*****tert*****-Butyl-1-methyl-5-pyrazolyl)-*****N'*****-(4-(4-acetaminophenyl)methylphenyl)urea:** To a solution of *N*-(3-*tert*-butyl-1-methyl-5-pyrazolyl)-*N'*-(4-(4-aminophenyl)methylphenyl)urea (0.300 g, 0.795 mmol) in CH₂Cl₂ (15 mL) at 0 °C was added acetyl chloride (0.057 mL, 0.795 mmol), followed by anhydrous Et₃N (0.111 mL, 0.795 mmol). The solution was allowed to warm to room temp over 4 h, then was diluted with EtOAc (200 mL). The organic layer was sequentially washed with a 1M HCl solution (125 mL) then water (100 mL), dried (MgSO₄), and concentrated under reduced pressure. The resulting residue was purified by filtration through a pad of silica (EtOAc) to give the desired product as a white solid (0.160 g, 48%): TLC (EtOAc) R_{*f*} 0.33; ¹H-NMR (DMSO-d₆) δ 1.17 (s, 9H), 1.98 (s, 3H), 3.55 (s, 3H), 3.78 (s, 2H), 6.00 (s, 1H), 7.07 (d, *J*=8.5 Hz, 2H), 7.09 (d, *J*=8.5 Hz, 2H), 7.32 (d, *J*=8.5 Hz, 2H), 7.44 (d, *J*=8.5 Hz, 2H), 8.38 (s, 1H), 8.75 (s, 1H), 9.82 (s, 1H); FAB-MS *m*/*z* 420 ((M+H)⁺).

### D4. General Method for the Conversion of Ester-Containing Ureas into Alcohol-Containing Ureas

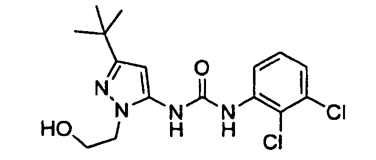

***N*****-(*****N'*****-(2-Hydroxyethyl)-3-*****tert*****-butyl-5-pyrazolyl)-*****N'*****-(2,3-dichlorophenyl)urea**: A solution of *N*-(*N'*-(2-(2,3-dichlorophenylamino)carbonyloxyethyl)-3-*tert*-butyl-5-pyrazolyl)-N '-(2,3-dichlorophenyl)urea (prepared as described in Method A3; 0.4 g, 0.72 mmoles) and NaOH (0.8 mL, 5N in water, 4.0 mmoles) in EtOH (7 mL) was heated at ~65 °C for 3 h at which time TLC indicated complete reaction. The reaction mixture was diluted with EtOAc (25 mL) and acidified with a 2N HCl solution (3 mL). The resulting organic phase was washed with a saturated NaCl solution (25 mL), dried (MgSO₄) and concentrated under reduced pressure. The residue was crystallized (Et₂O) to afford the desired product as a white solid (0.17 g, 64 %): TLC (60% EtOAc/40% hexane) R_{*f*} 0.16; ¹H-NMR (DMSO-d₆) δ 1.23 (s, 9H), 3.70 (t, *J*=5.7 Hz, 2H), 4.10 (t, *J*=5.7 Hz, 2H), 6.23 (s, 1H), 7.29-7.32 (m, 2H), 8.06-8.09 (m, 1H), 9.00 (br s, 1H), 9.70 (br s, 1H); FAB-MS *m*/*z* (rel abundance) 371 ((M+H)⁺, 100%).

### D5a. General Method for the Conversion of Ester-Containing Ureas into Amide-Containing Ureas

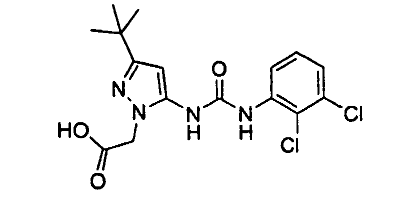

**Step 1.** ***N*****-(*****N'*****-(Carboxymethyl)-3-*****tert*****-butyl-5-pyrazolyl)-*****N'*****-(2,3-dichlorophenyl)urea**: A solution of *N*-(*N'*-(ethoxycarbonylmethyl)-3-*tert*-butyl-5-pyrazolyl)-*N'*-(2,3-dichlorophenyl)urea (prepared as described in Method A3. 0.46 g, 1.11 mmoles) and NaOH (1.2 mL, 5N in water, 6.0 mmoles) in EtOH (7 mL) was stirred at room temp. for 2 h at which time TLC indicated complete reaction. The reaction mixture was diluted with EtOAc (25 mL) and acidified with a 2N HCl solution (4 mL). The resulting organic phase was washed with a saturated NaCl solution (25 mL), dried (MgSO₄) and concentrated under reduced pressure. The residue was crystallized (Et₂O/hexane) to afford the desired product as a white solid (0.38 g, 89%): TLC (10% MeOH/90% CH₂Cl₂) R_{*f*} 0.04; ¹H-NMR (DMSO-d₆) δ 1.21 (s, 9H), 4.81 (s, 2H), 6.19 (s, 1H), 7.28-7.35 (m, 2H), 8.09-8.12 (m, 1H), 8.76 (br s, 1H), 9.52 (br s, 1H); FAB-MS *m*/*z* (rel abundance) 385 ((M+H)⁻, 100%).

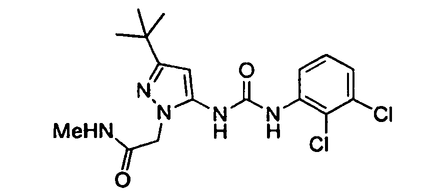

**Step 2.** ***N*****-(*****N***^{***1***}**-((Methylcarbamoyl)methyl)-3-*****tert*****-butyl-5-pyrazolyl)-*****N'*****-(2,3--dichlorophenyl)urea:** A solution of *N*-(*N*^{*1*}-(carboxymethyl)-3-*tert*-butyl-5-pyrazolyl)-*N*'-(2,3-dichlorophenyl)urea (100 mg, 0.26 mmole) and *N,N*'-carbonyldiimidazole (45 mg, 0.28 mmole) in CH₂Cl₂ (10 mL) was stirred at room temp. 4 h at which time TLC indicated formation of the corresponding anhydride (TLC (50% acetone/50% CH₂Cl₂) R_{*f*} 0.81). Dry methylamine hydrochloride (28 mg, 0.41 mmole) was then added followed by of diisopropylethylamine (0.07 mL, 0.40 mmole). The reaction mixture was stirred at room temp. overnight, then diluted with CH₂Cl₂, washed with water (30 mL), a saturated NaCl solution (30 mL), dried (MgSO₄) and concentrated under reduced pressure. The residue was purified by column chromatography (gradient from 10% acetone/90% CH₂Cl₂ to 40% acetone/60% CH₂Cl₂) and the residue was crystallized (Et₂O/hexane) to afford the desired product (47 mg, 46%): TLC (60% acetone/40% CH₂Cl₂) R_{*f*} 0.59; ¹H-NMR (DMSO-d₆) δ 1.20 (s, 9H), 2.63 (d, *J*=4.5 Hz, 3H), 4.59 (s, 2H), 6.15 (s, 1H), 7.28-7.34 (m, 2H), 8.02-8.12 (m, 2H), 8.79 (br s, 1H), 9.20 (br s, 1H); FAB-MS *m*/*z* (rel abundance) 398 ((M+H)⁺, 30%).

### D5b. General Method for the Conversion of Ester-Containing Ureas into Amide-Containing Ureas

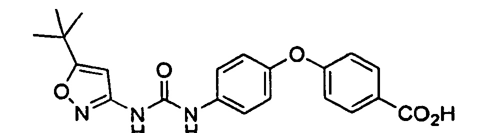

**Step 1.** ***N*****-(5-*****tert*****-Butyl-3-isoxazolyl)-*****N'*****-(4-(4-carboxyphenyl)oxyphenyl)urea:** To a solution of *N*-(5-*tert*-butyl-3-isoxazolyl)-*N'*-(4-(4-ethoxyoxycarbonylphenyl)-oxyphenyl)urea (0.524 g, 1.24 mmol) in a mixture of EtOH (4 mL) and THF (4 mL) was added a 1M NaOH solution (2 mL) and the resulting solution was allowed to stir overnight at room temp. The resulting mixture was diluted with water (20 mL) and treated with a 3M HCl solution (20 mL) to form a white precipitate. The solids were washed with water (50 mL) and hexane (50 mL), and then dried (approximately 0.4 mmHg) to afford the desired product (0.368 g, 75 %). This material was carried to the next step without further purification.

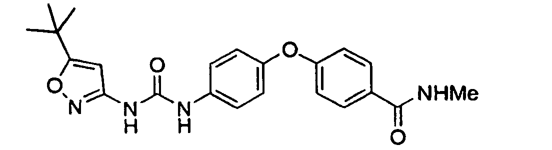

**Step 2.** ***N*****-(5-*****tert*****-Butyl-3-isoxazolyl)-*****N'*****-(4-(4-(*****N*****-methylcarbamoyl)-phenyl)oxyphenyl)urea:** A solution of *N*-(5-*tert*-butyl-3-isoxazolyl)-*N'*-(4-(4-carboxyphenyl)oxyphenyl)urea (0.100 g, 0.25 mmol), methylamine (2.0 M in THF; 0.140 mL, 0.278 mmol), 1-ethyl-3-(3-dimethylaminopropyl)carbodiimide hydrochloride (76 mg, 0.39 mmol), and *N*-methylmorpholine (0.030 mL, 0.27 mmol) in a mixture of THF (3 mL) and DMF (3mL) was allowed to stir overnight at room temp. then was poured into a 1M citric acid solution (20 mL) and extracted with EtOAc (3 x 15 mL). The combined extracts were sequentially washed with water (3 x 10 mL) and a saturated NaCl solution (2 x 10 mL), dried (Na₂SO₄), filtered, and concentrated *in vacuo*. The resulting crude oil was purified by flash chromatography (60 % EtOAc/40% hexane) to afford the desired product as a white solid (42 mg, 40%): EI-MS *m*/*z* 409 ((M+H)⁺).

### D6. General Method for the Conversion of ω-Amine-Containing Ureas into Amide-Containing Ureas

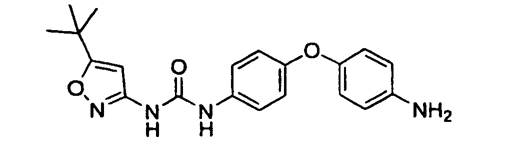

***N*****-(5-*****tert*****-Butyl-3-isoxazolyl)-*****N'*****-(4-(4-aminophenyl)oxyphenyl)urea:** To a solution of *N*-(5-*tert*-butyl-3-isoxazolyl)-*N'*-(4-(4-*tert*-butoxycarbonylaminophenyl)oxyphenyl)-urea (prepared in a manner analogous to Methods B6 then C2b; 0.050 g, 0.11 mmol) in anh 1,4-dioxane (3 mL) was added a conc HCl solution (1 mL) in one portion and the mixture was allowed to stir overnight at room temp . The mixture was then poured into water (10 mL) and EtOAc(10 mL) and made basic using a 1M NaOH solution (5 mL). The aqueous layer was extracted with EtOAc (3 x 10 mL). The combined organic layers were sequentially washed with water (3 x 100 mL) and a saturated NaCl solution (2 x 100 mL), dried (Na₂SO₄), and concentrated *in vacuo* to afford the desired product as a white solid (26 mg, 66%). EI-MS *m*/*z* 367 ((M+ H)⁺).

### D7. General Method for the Oxidation of Pyridine-Containing Ureas

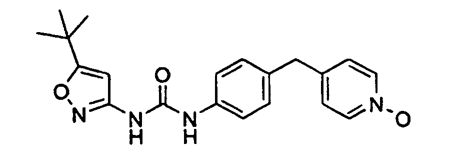

***N*****-(5-*****tert*****-Butyl-3-isoxazolyl)-*****N*****'-(4-(*****N*****-oxo-4-pyridinyl)methylphenyl)urea:** To a solution of *N*-(5-*tert*-butyl-3-isoxazolyl)-*N*'-(4-(4-pyridinyl)methylphenyl)urea (0.100 g, 0.29 mmol) in CHCl₃ (10 mL) was added *m*-CPBA (70% pure, 0.155 g, 0.63 mmol) and the resulting solution was stirred at room temp for 16 h. The reaction mixture was then treated with a saturated K₂CO₃ solution (10 mL). After 5 min, the solution was diluted with CHCl₃ (50 mL). The organic layer was washed successively with a saturated aqueous NaHSO₃ solution (25 mL), a saturated NaHCO₃ solution (25 mL) and a saturated NaCl solution (25 mL), dried (MgSO₄), and concentrated *in* *vacuo*. The residual solid was purified by MPLC (15% MeOH/85% EtOAc) to give the *N*-oxide (0.082 g, 79%).

### D8. General Method for the Acylation of a Hydroxy-Containing Urea

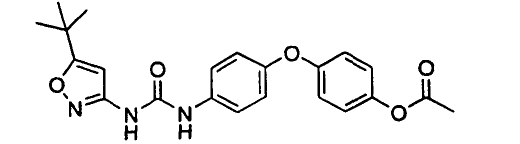

***N*****-(5-*****tert*****-Butyl-3-isoxazolyl)-*****N*****'-(4-(4-acetoxyphenyloxy)phenyl)urea**: To a solution of *N*-(5-*tert*-butyl-3-isoxazolyl)-*N'*-(4-(4-hydroxyphenyloxy)phenyl)urea (0.100 g, 0.272 mmol), *N,N*-dimethylaminopyridine (0.003 g, 0.027 mmol) and Et₃N (0.075 mL, 0.544 mmol) in anh THF (5 mL) was added acetic anhydride (0.028 mL, 0.299 mmol), and the resulting mixture was stirred at room temp. for 5 h. The resulting mixture was concentrated under reduced pressure and the residue was dissolved in EtOAc (10 mL). The resulting solution was sequentially washed with a 5% citric acid solution (10 mL), a saturated NaHCO₃ solution (10 mL) and a saturated NaCl solution (10 mL), dried (Na₂SO₄), and concentrated under reduced pressure to give an oil which slowly solidified to a glass (0.104 g, 93%) on standing under reduced pressure (approximately 0.4 mmHg): TLC (40% EtOAc/60% hexane) R_{*f*} 0.55; FAB-MS *m*/*z* 410 ((M+H)⁺).

### D9. Synthesis of ω-Alkoxypyridines

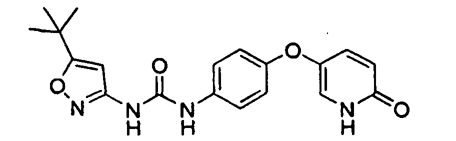

**Step 1.** ***N*****-(5-*****tert*****-Butyl-3-isoxazolyl)-*****N'*****-(4-(2(1*****H*****)-pyridinon-5-yl)oxyphenyl)-urea**: A solution of *N*-(5-*tert*-butyl-3-isoxazolyl)-*N'*-(4-(5-(2-methoxy)pyridyl)-oxyaniline (prepared in a manner analogous to that described in Methods B3k and C3b; 1.2 g, 3.14 mmol) and trimethylsilyl iodide (0.89 mL, 6.28 mmol) in CH₂Cl₂ (30 mL) was allowed to stir overnight at room temp., then was to 40 °C for 2 h. The resulting mixture was concentrated under reduced pressure and the residue was purified by column chromatography (gradient from 80% EtOAc/20% hexans to 15% MeOH/85% EtOAc) to give the desired product (0.87 g. 75%): mp 175-180 °C; TLC (80% EtOAc/20% hexane) R_{*f*} 0.05; FAB-MS *m*/*z* 369 ((M+H)⁻, 100%).

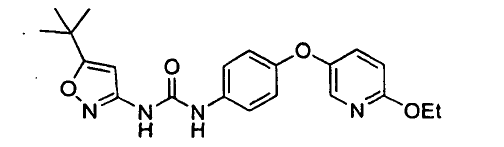

**Step 2.** ***N*****-(5-*****tert*****-Butyl-3-isoxazotyl)-*****N'*****-(4-(5-(2-Ethoxy)pyridyl)oxyphenyl)urea:** A slurry of *N*-(5-*tert*-butyl-3-isoxazolyl)-*N'*-(4-(2(1*H*)-pyridinon-5-yl)oxyphenyl)urea (0.1 g, 0.27 mmol) and Ag₂CO₃ (0.05 g, 0.18 mmol) in benzene (3 mL) was stirred at room temp. for 10 min. Iodoethane (0.023 mL, 0.285 mmol) was added and the resulting mixture was heated at the reflux temp. in dark overnight. The reaction mixture was allowed to cool to room temp., and was filtered through a plug of Celite® then concentrated under reduced pressure. The residue was purified by column chromatography (gradient from 25% EtOAc/75% hexane to 40% EtOAc/60% hexane) to afford the desired product (0.041 g, 38%): mp 146 °C; TLC (40% EtOAc/60% hexane) R_{*f*} 0.49; FAB-MS *m*/*z* 397 ((M+H)⁺, 100%).

### D10. Reduction of an Aldehyde- or Ketone-Containing Urea to a Hydroxide-Containing Urea

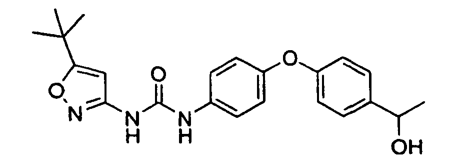

***N*****-(5-*****tert*****-Butyl-3-isoxazolyl)-*****N'*****-(4-(4-(1-hydroxyethyl)phenyl)oxyphenyl)urea:** To a solution of *N*-(5-*tert*-butyl-3-isoxazolyl)-*N*'-(4-(4-(1-acetylphenyl)oxyphenyl)urea (prepared in a manner analogous to that described in Methods B1 and C2b; 0.060 g, 0.15 mmol) in MeOH (10 mL) was added NaBH₄ (0.008 g, 0.21 mmol) in one portion. The mixture was allowed to stir for 2 h at room temp., then was concentrated *in vacuo.* Water (20 mL) and a 3M HCl solution (2 mL) were added and the resulting mixture was extracted with EtOAc (3 x 20 mL). The combined organic layers were washed with water (3 x 10 mL) and a saturated NaCl solution (2 x 10 mL), dried (MgSO₄), and concentrated *in vacuo* . The resulting white solid was purified by trituration (Et₂O/hexane) to afford the desired product (0.021 g, 32 %): mp 80-85 °C; ¹H NMR (DMSO-d₆) δ 1.26 (s, 9H), 2.50 (s, 3H), 4.67 (m. 1H), 5.10 (br s, 1H), 6.45 (s, 1H), 6.90 (m, 4H), 7.29 (d, *J*=9.0 Hz, 2H), 7.42 (d, *J*=9.0 Hz, 2H), 8.76 (s, 1H), 9.44 (s, 1H); HPLC ES-MS *m*/*z* 396 ((M+H)⁻).

### D11. Synthesis of Nitrogen-Substituted Ureas by Curtius Rearrangement of Carboxy-Substituted Ureas

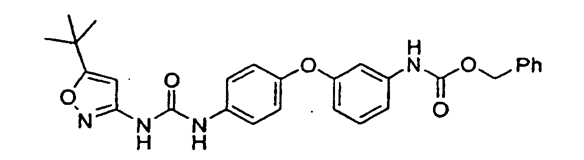

***N*****-(5-*****tert*****-Butyl-3-isoxazolyl)-*****N'*****-(4-(3-(benzyloxycarbonylamino)phenyl)-oxyphenyl)urea**: To a solution of the *N*-(5-*tert*-butyl-3-isoxazolyl)-*N'*-(4-(3-carboxyphenyl)oxyphenyl)urea (prepared in a manner analogous to that described in Methods B3a, Step 2 and C2b; 1.0 g, 2.5 mmol) in anh toluene (20 mL) was added Et₃N (0.395 mL, 2.8 mmol) and DPPA (0.610 mL, 2.8 mmol). The mixture was heated at 80 °C with stirring for 1.5 h then allowed to cool to room temp. Benzyl alcohol (0.370 mL, 3.5 mmol) was added and the mixture was heated at 80 °C with stirring for 3 h then allowed to cool to room temp. The resulting mixture was poured into a 10% HCl solution (50 mL) and the resulting solution extracted with EtOAc (3 x 50 mL). The combined organic layers were washed with water (3 x 50 mL) and a saturated NaCl (2 x 50 mL), dried (Na₂SO₄), and concentrated *in vacuo*. The crude oil was purified by column chromatography (30% EtOAc/70% hexane) to afford the desired product as a white solid (0.7 g, 60 %): mp 73-75 °C; ¹H NMR (DMSO-d₆) δ 1.26 (s, 9H), 5.10 (s, 2H), 6.46 (s, 1H), 6.55 (d, *J*=7.0 Hz, 1H), 6.94 (d, *J*=7.0 Hz, 2H), 7.70 (m, 7H), 8.78 (s, 1H), 9.46 (s, 1H), 9.81 (s, 1H); HPLC ES-MS *m*/*z* 501 ((M+H)⁺).

The following compounds have been synthesized according to the General Methods listed above:

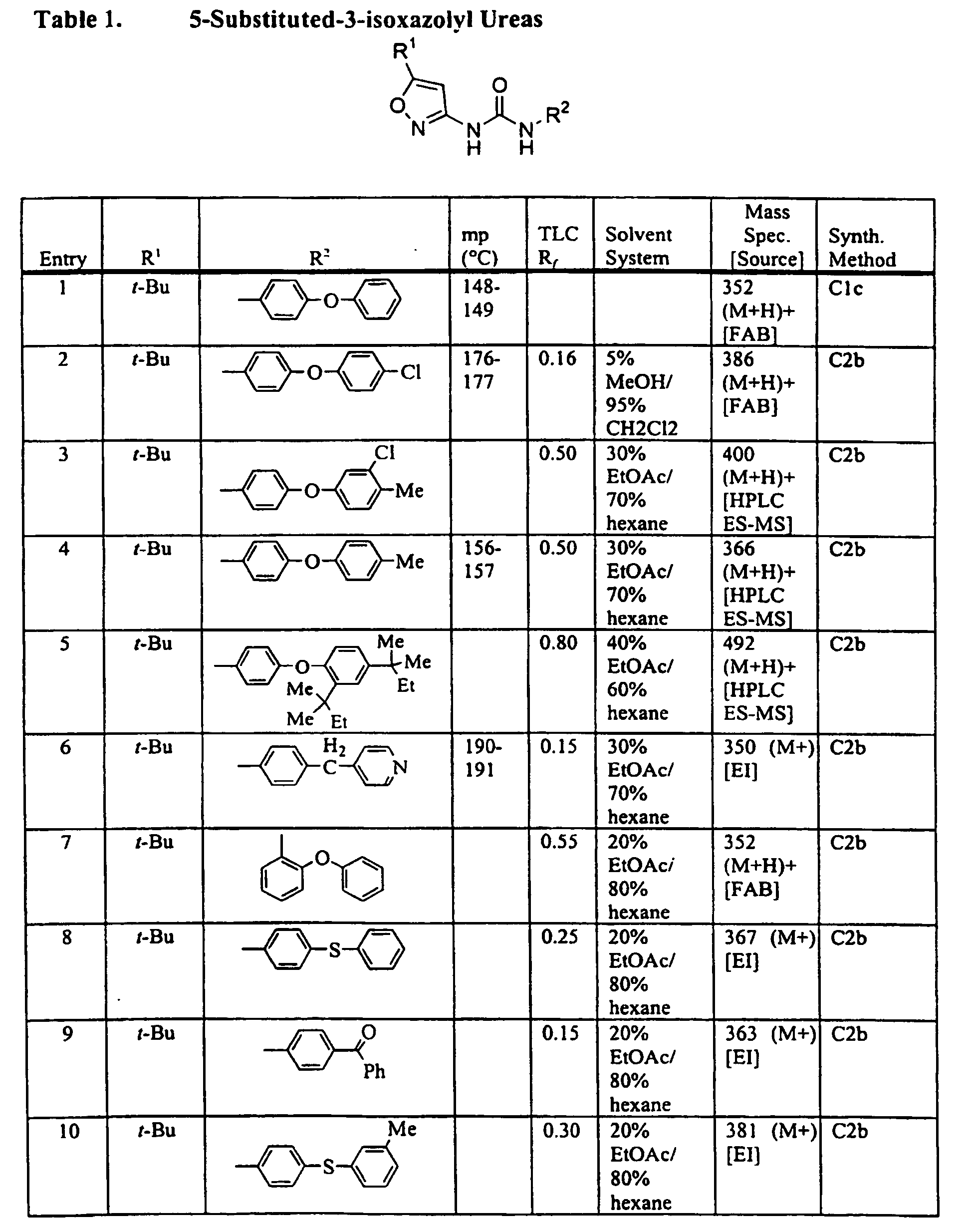

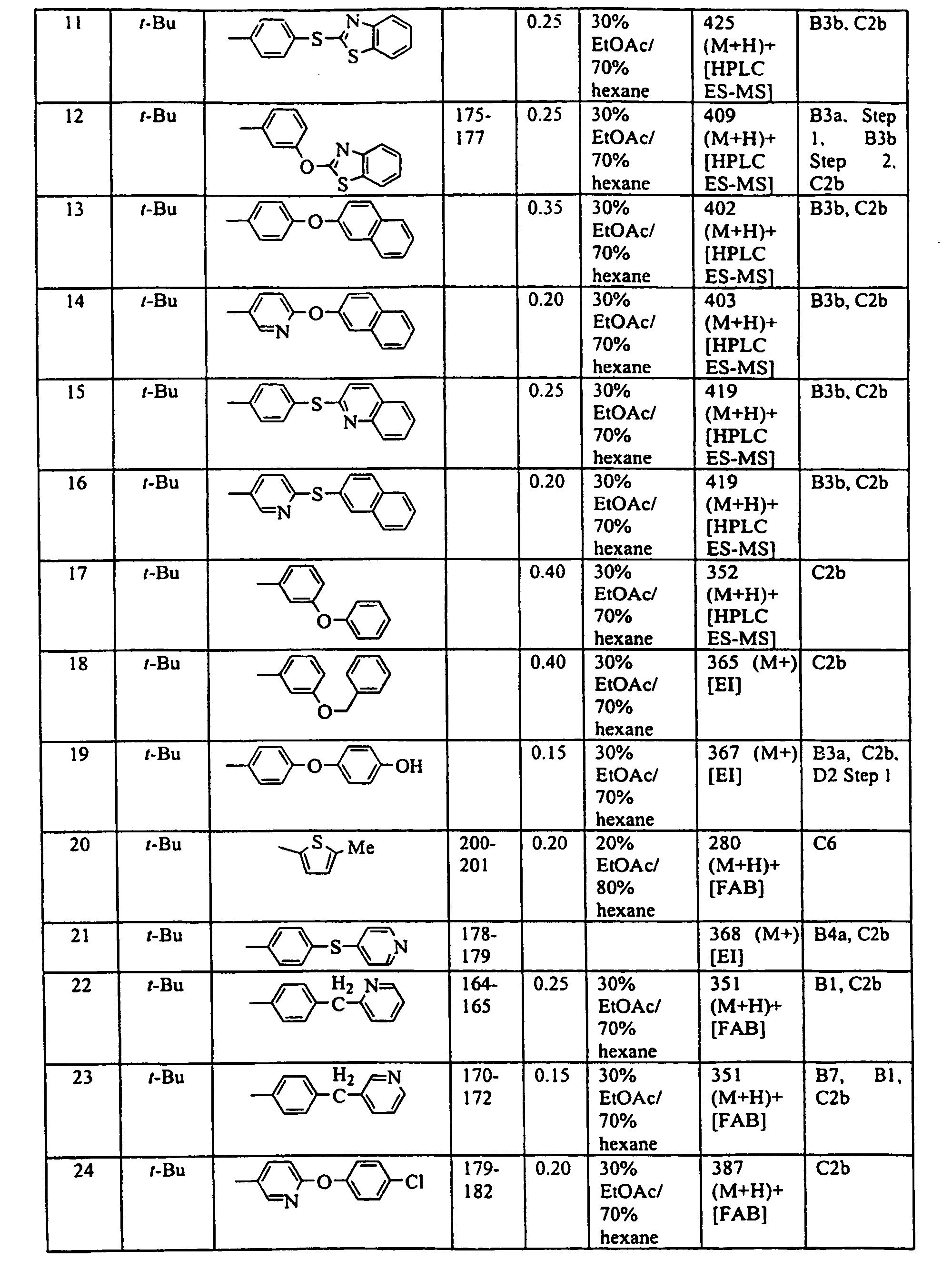

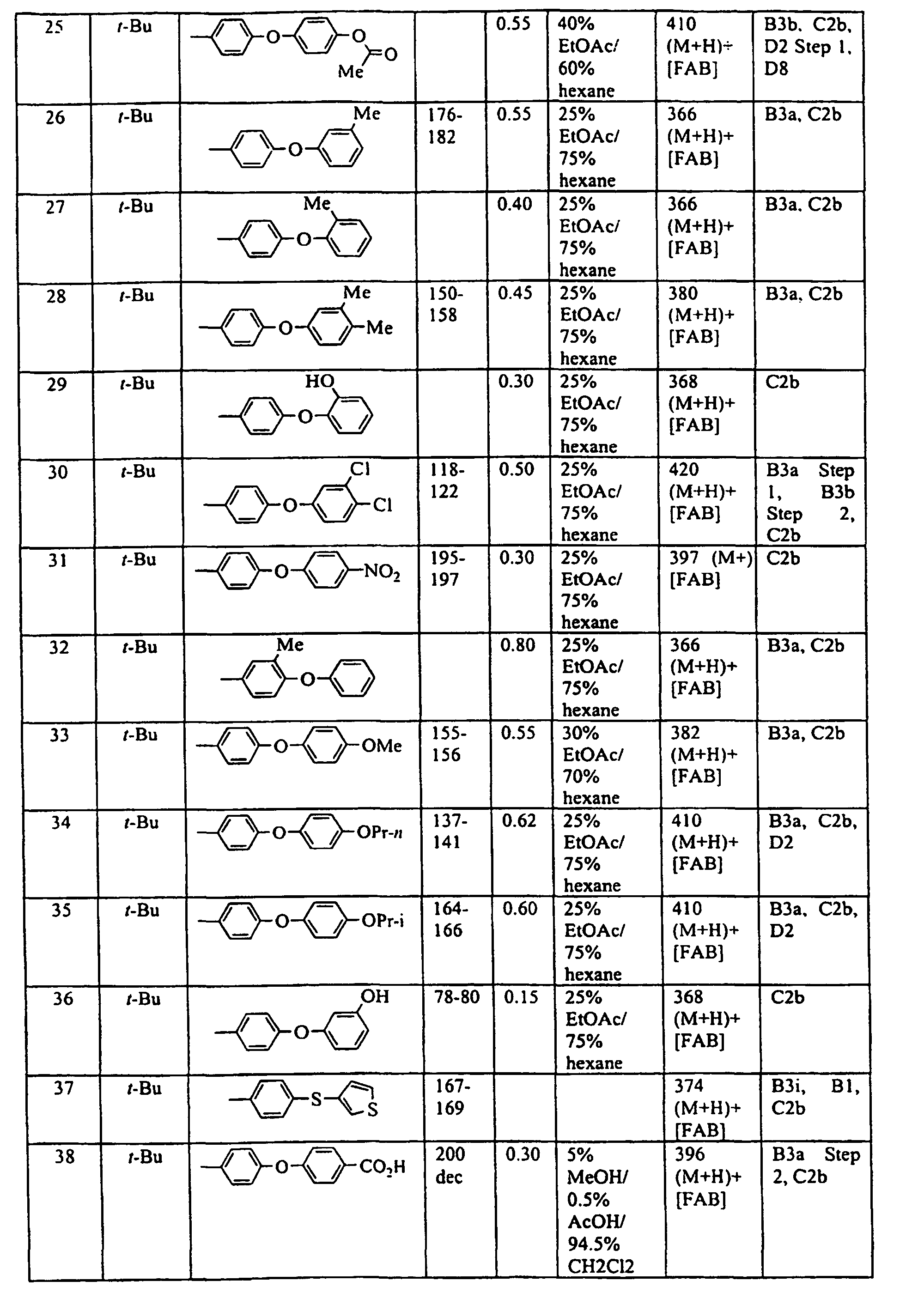

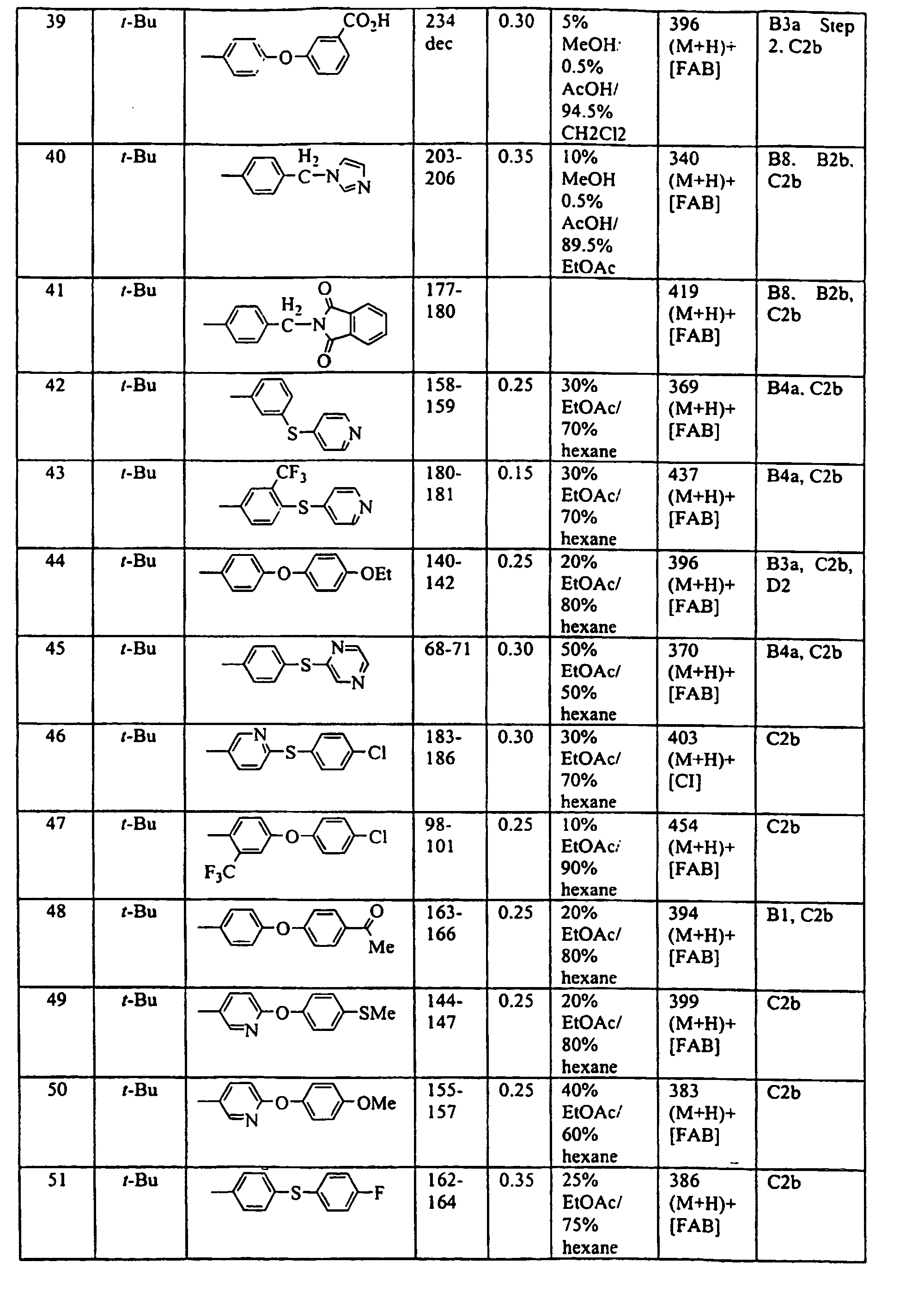

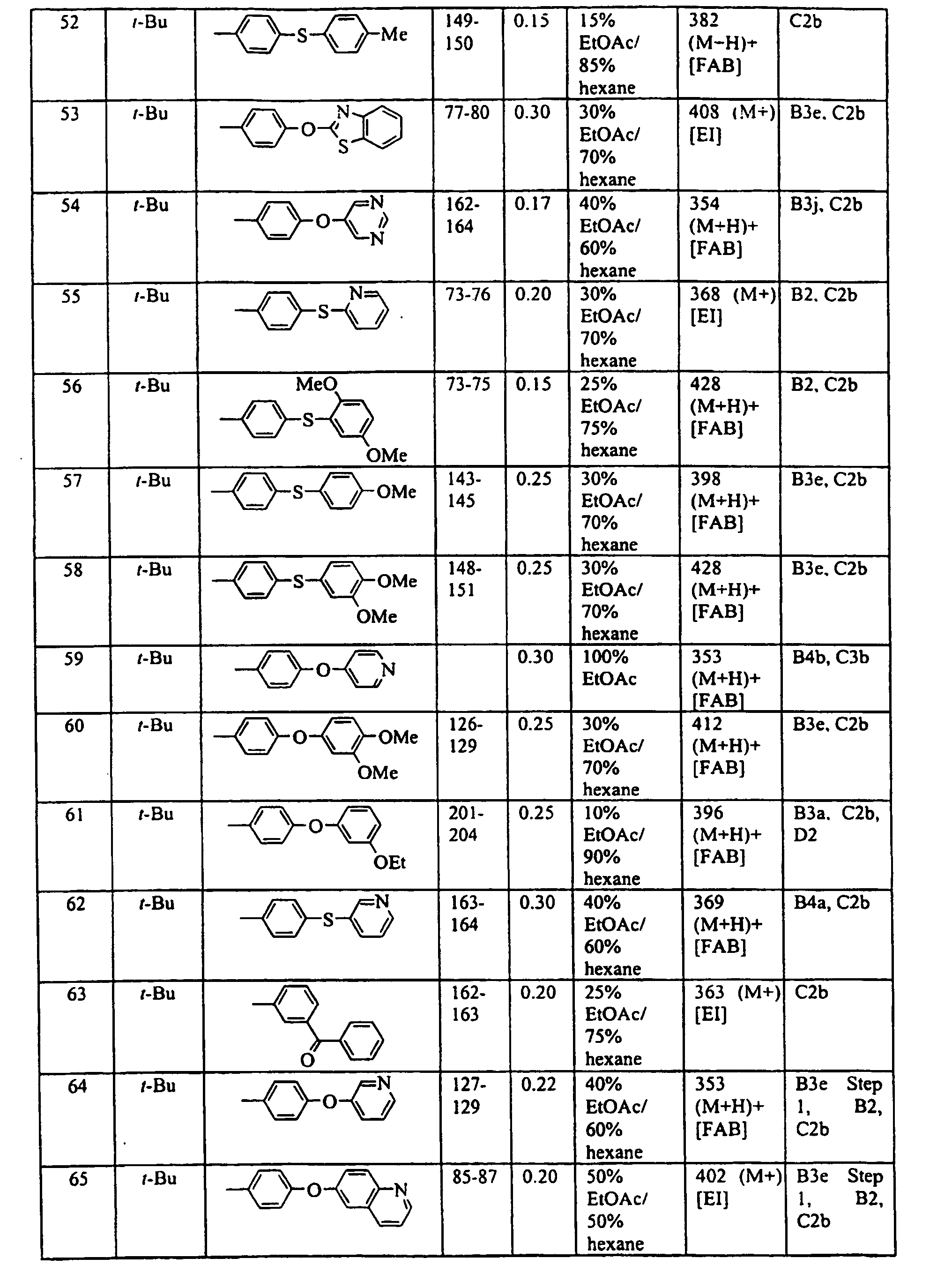

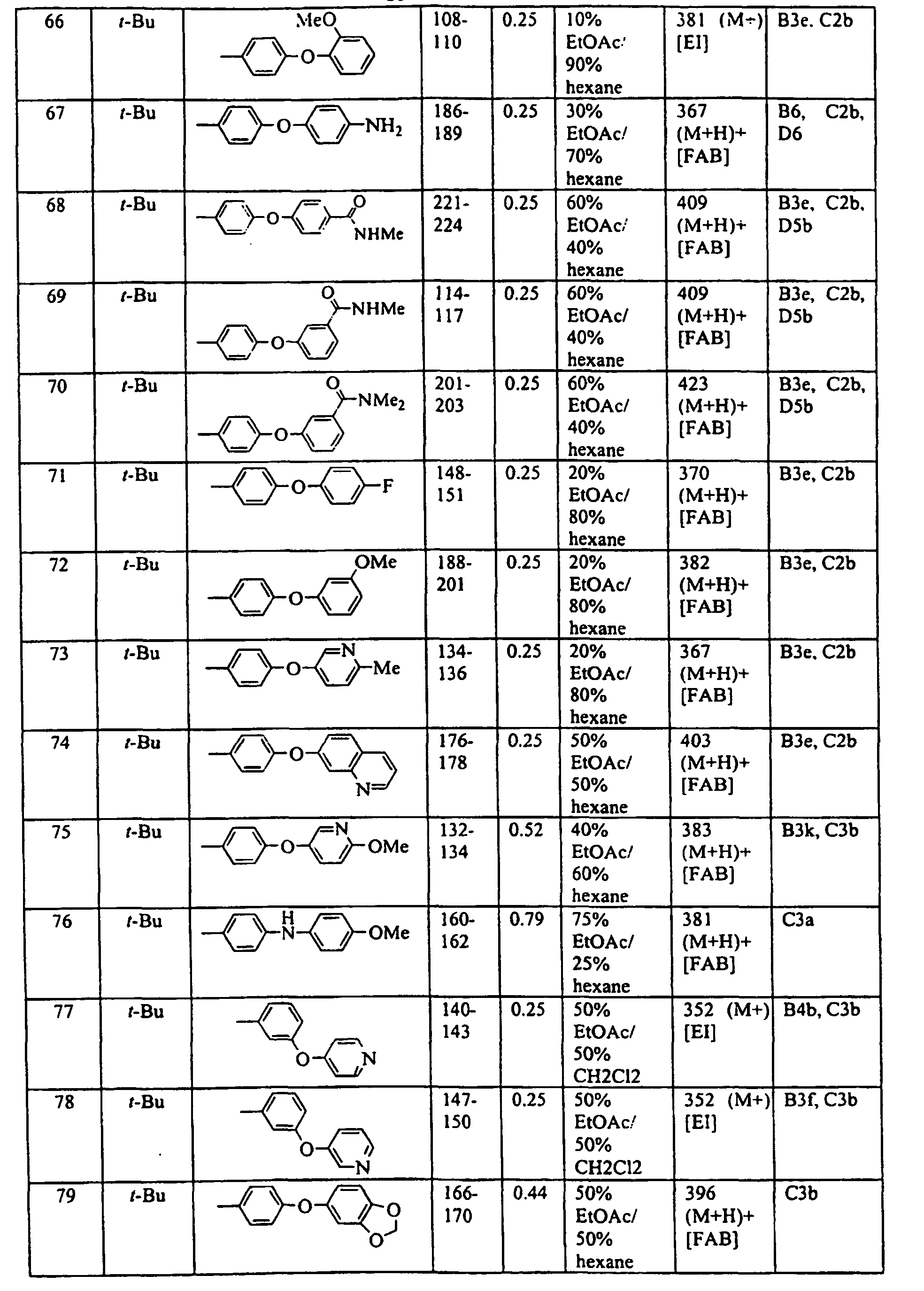

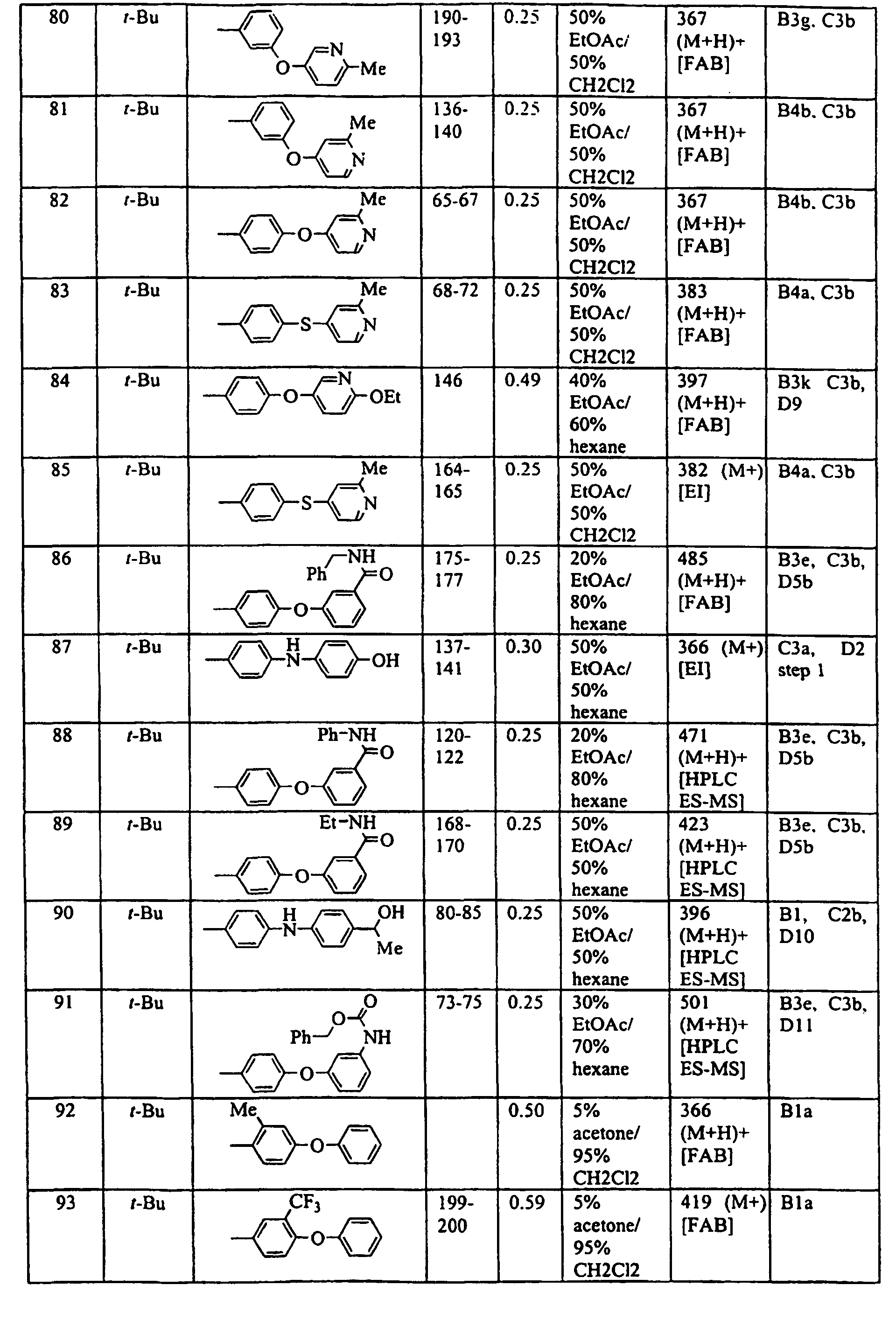

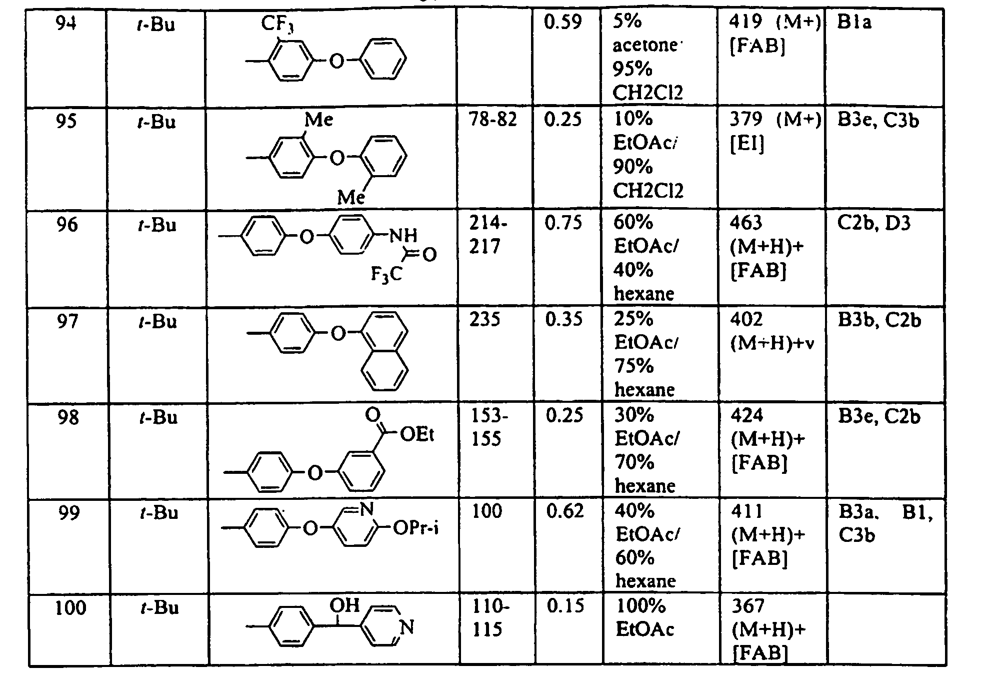

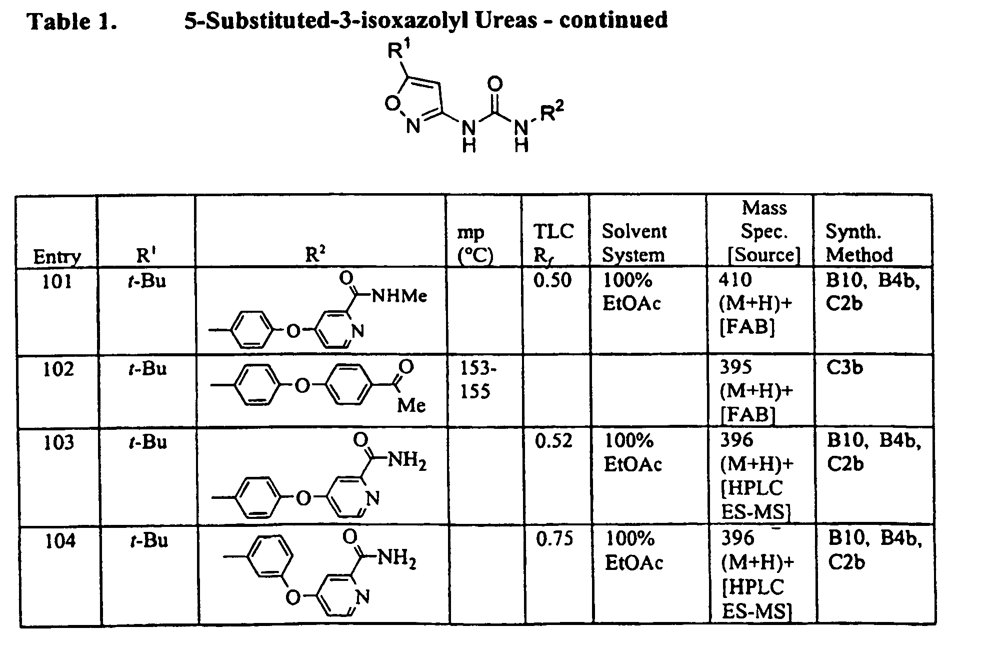

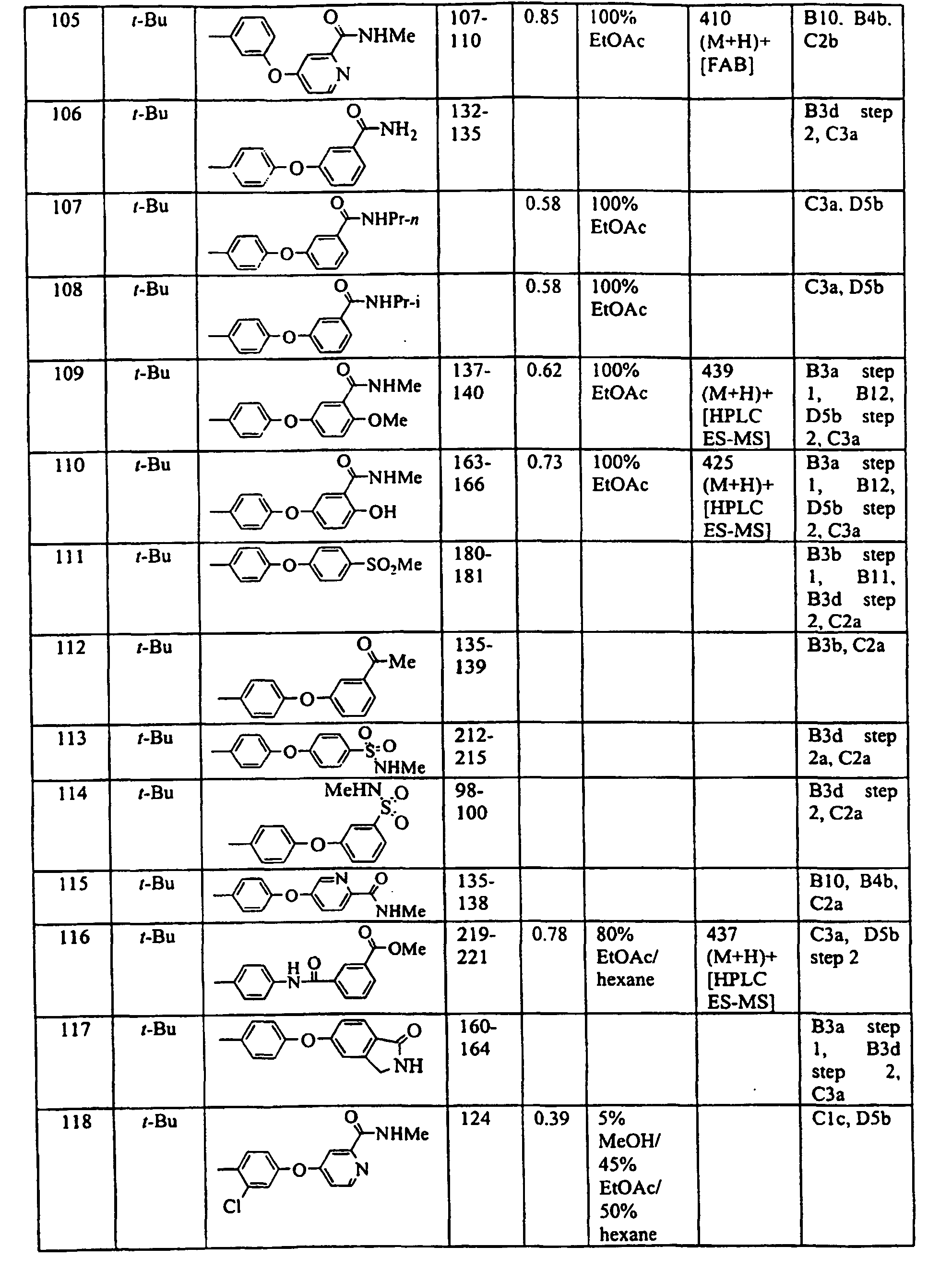

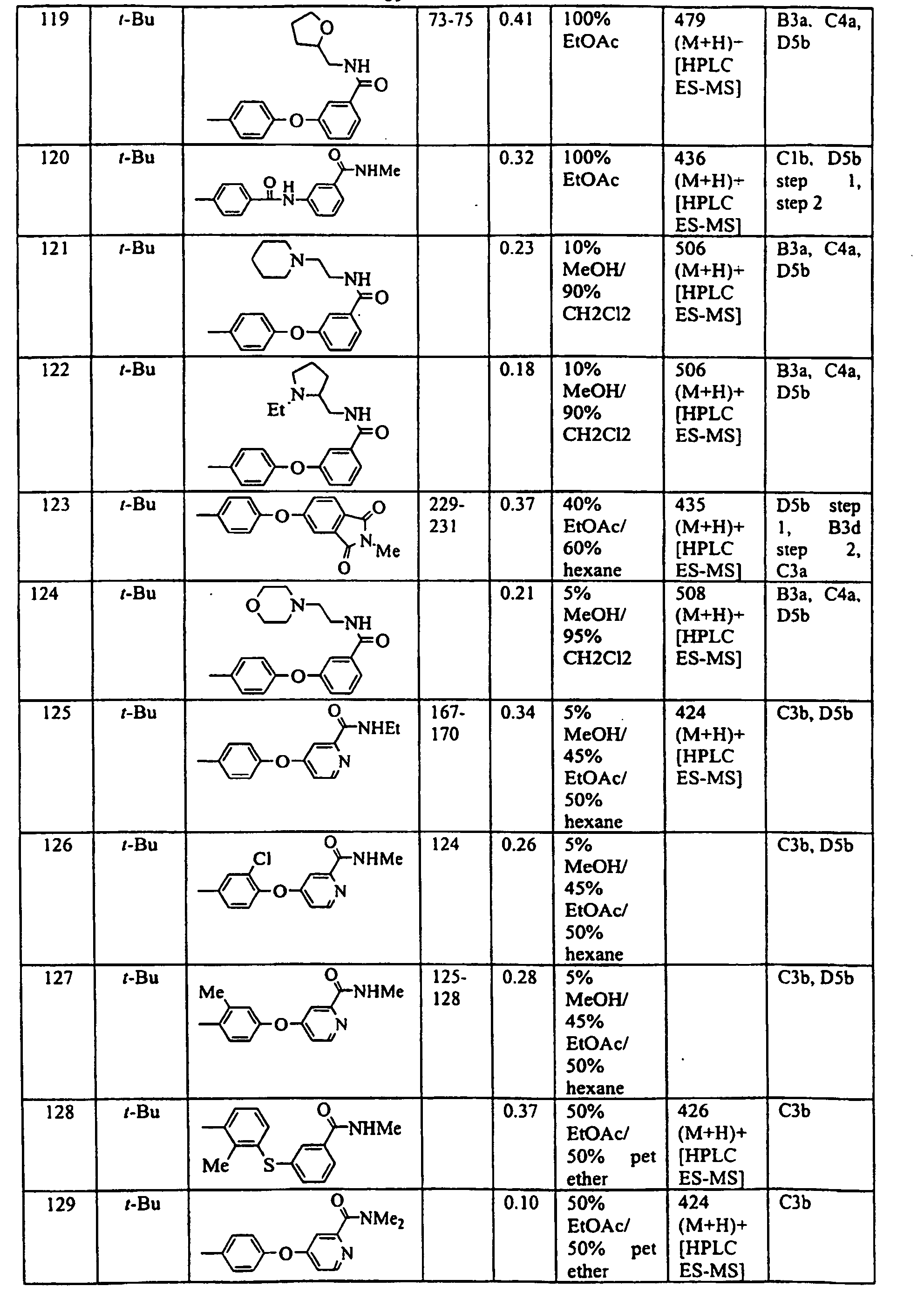

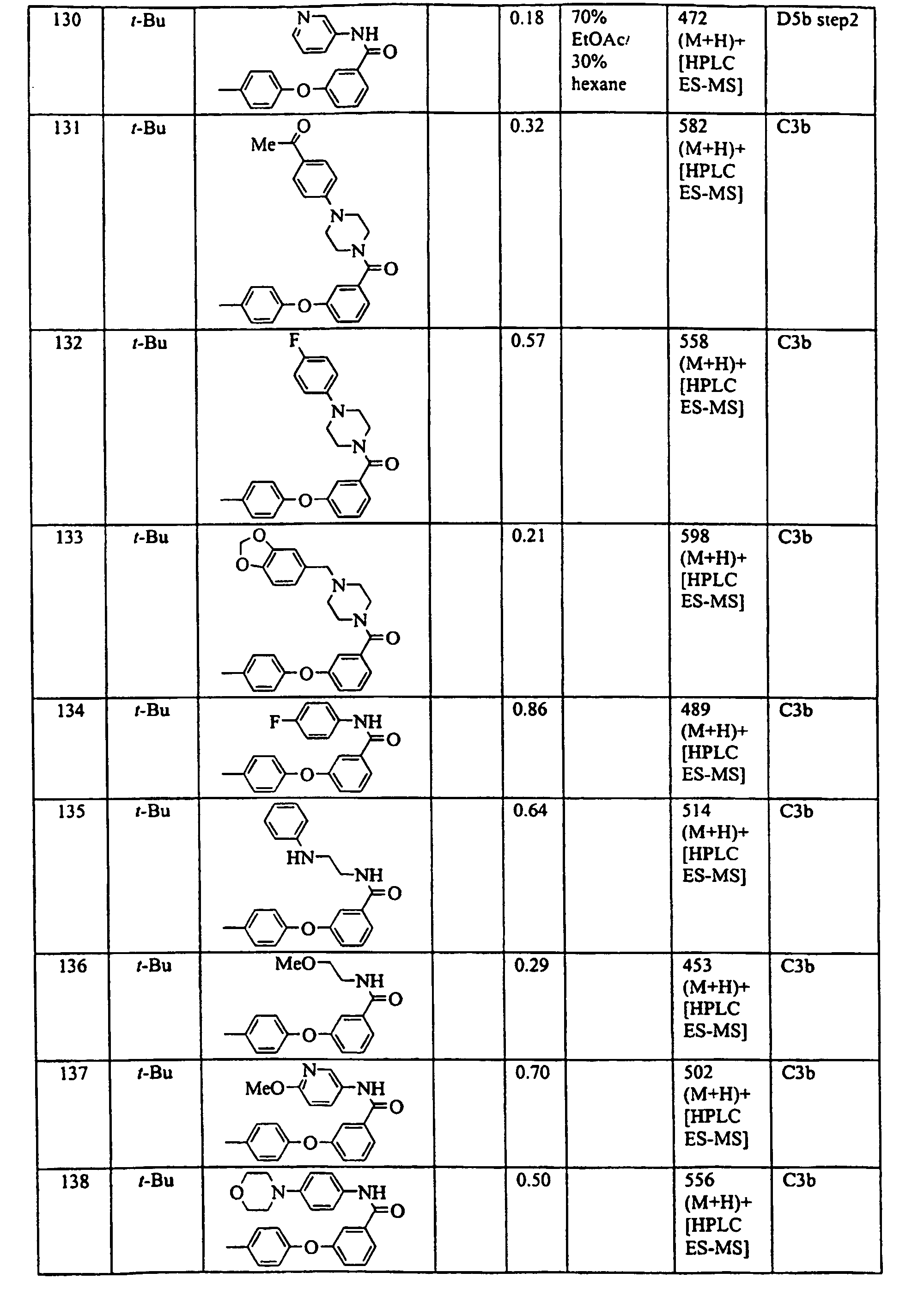

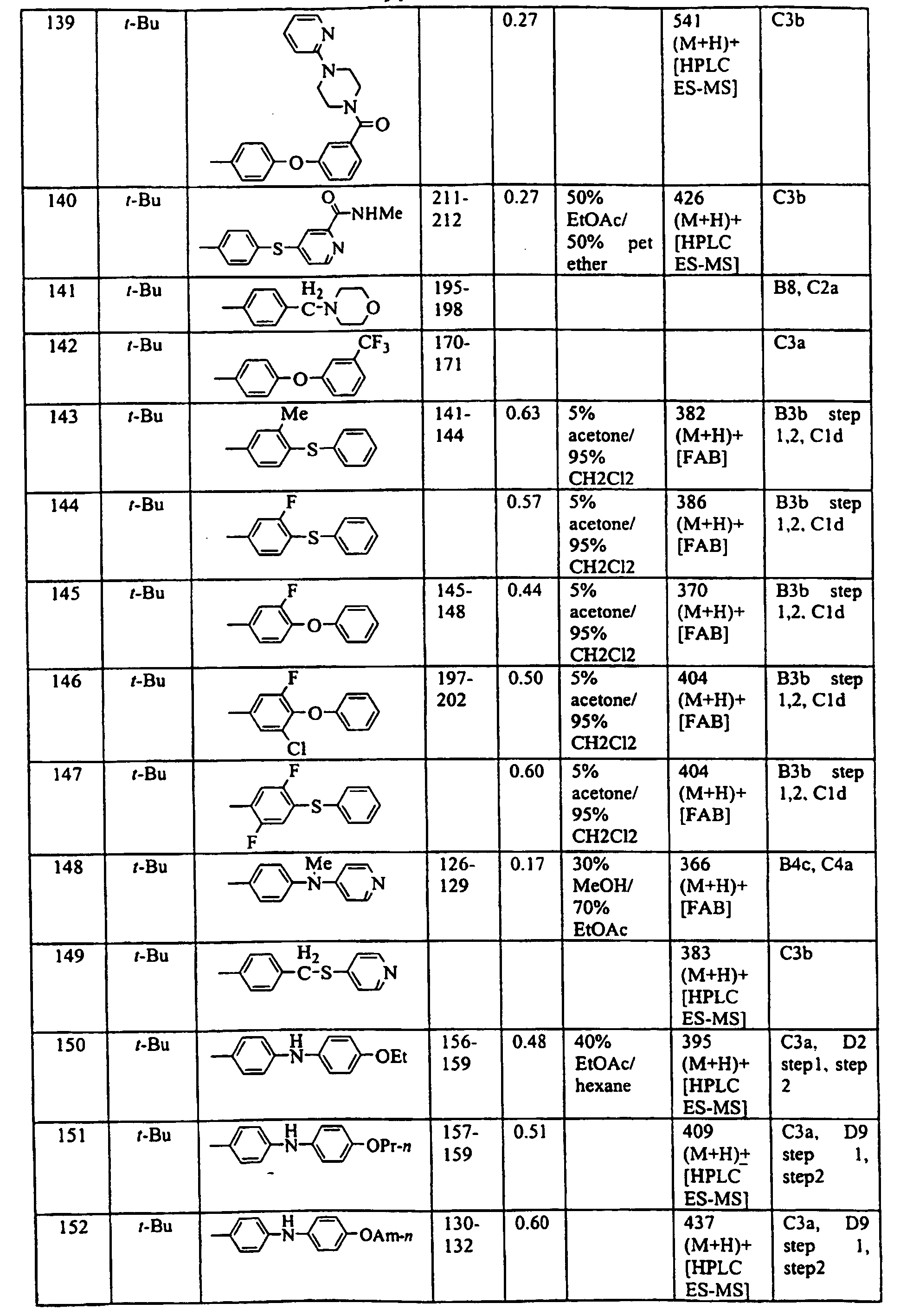

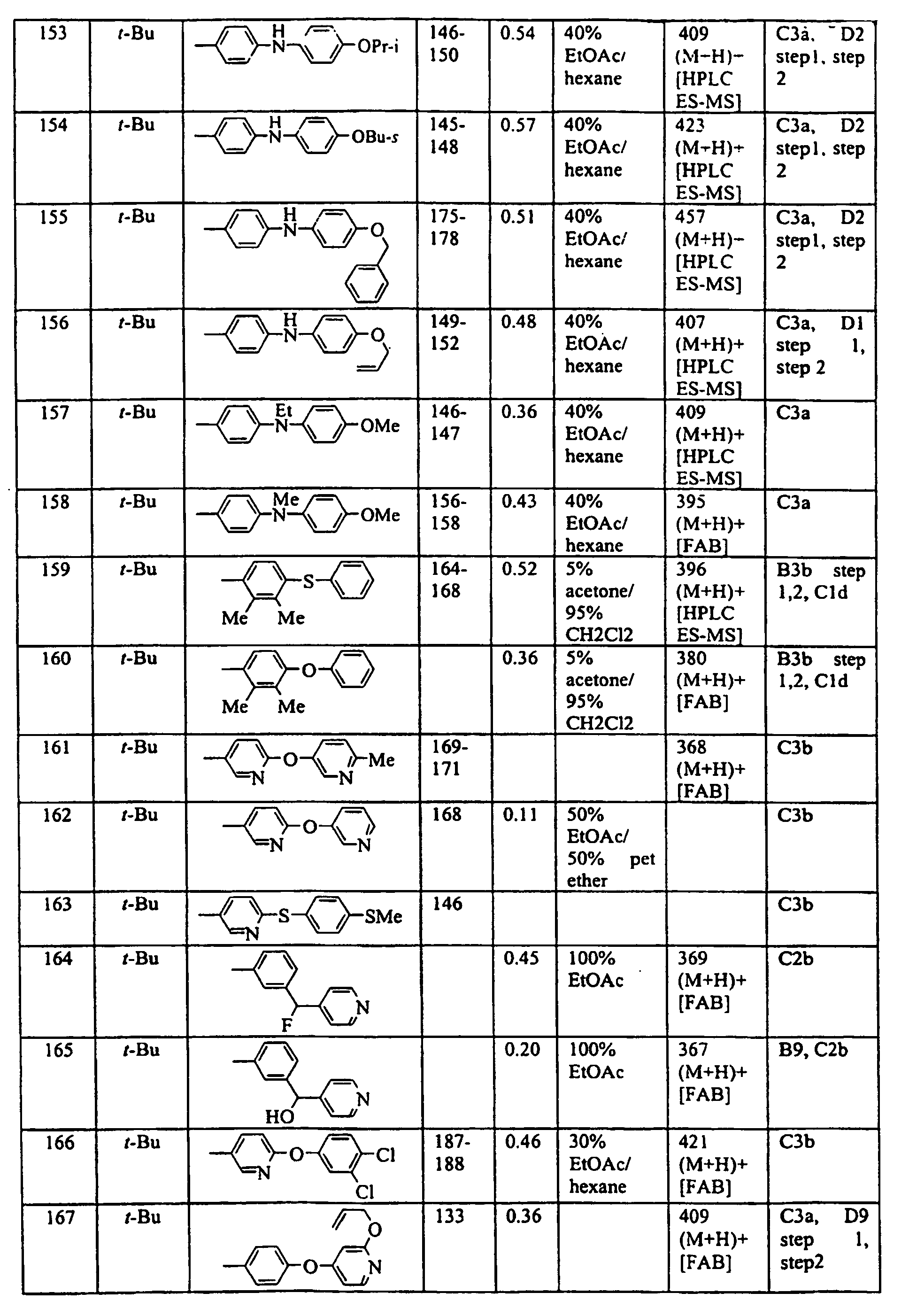

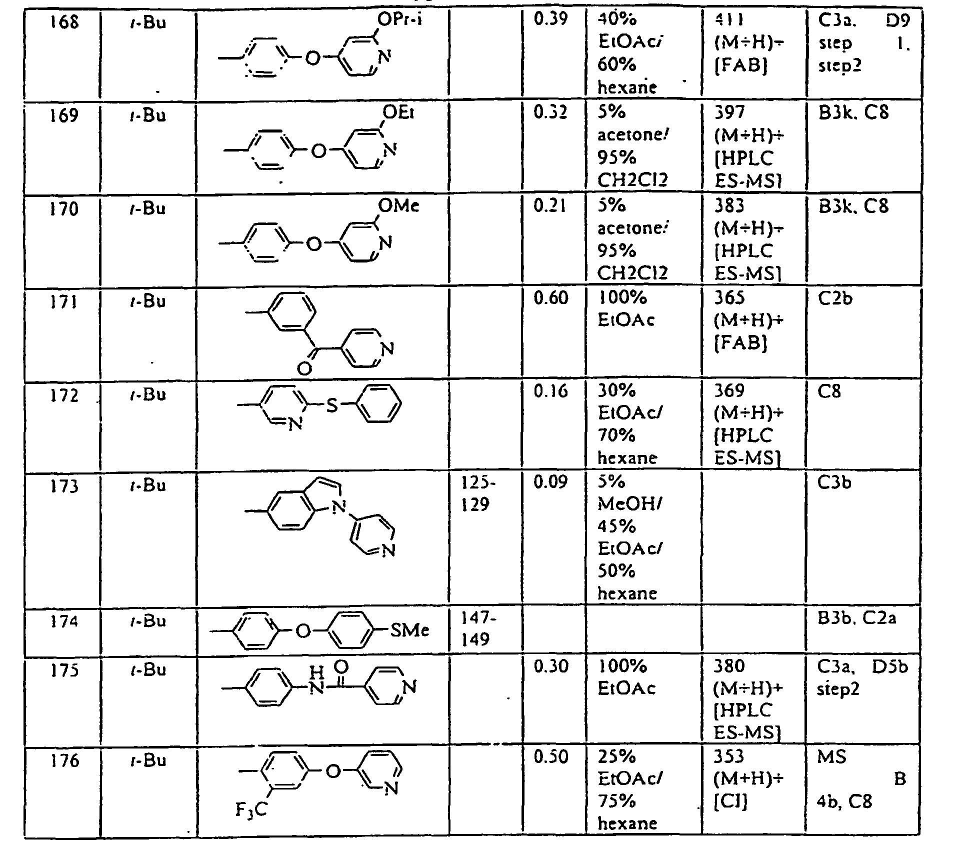

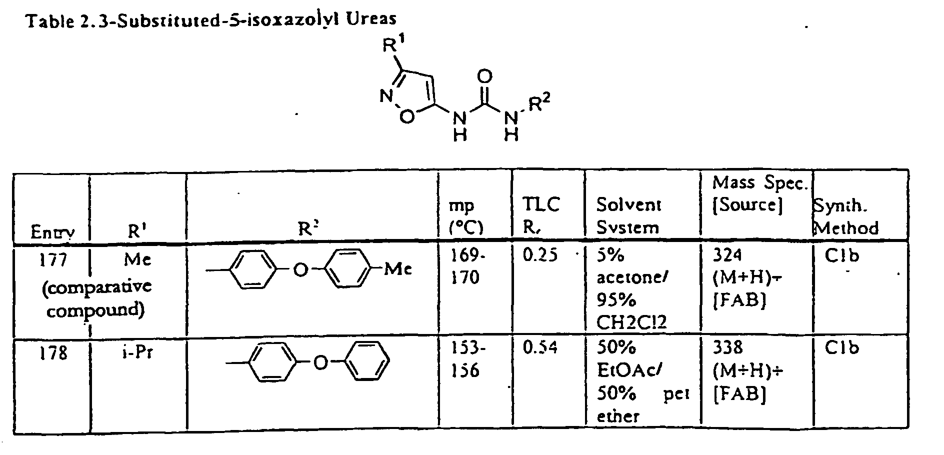

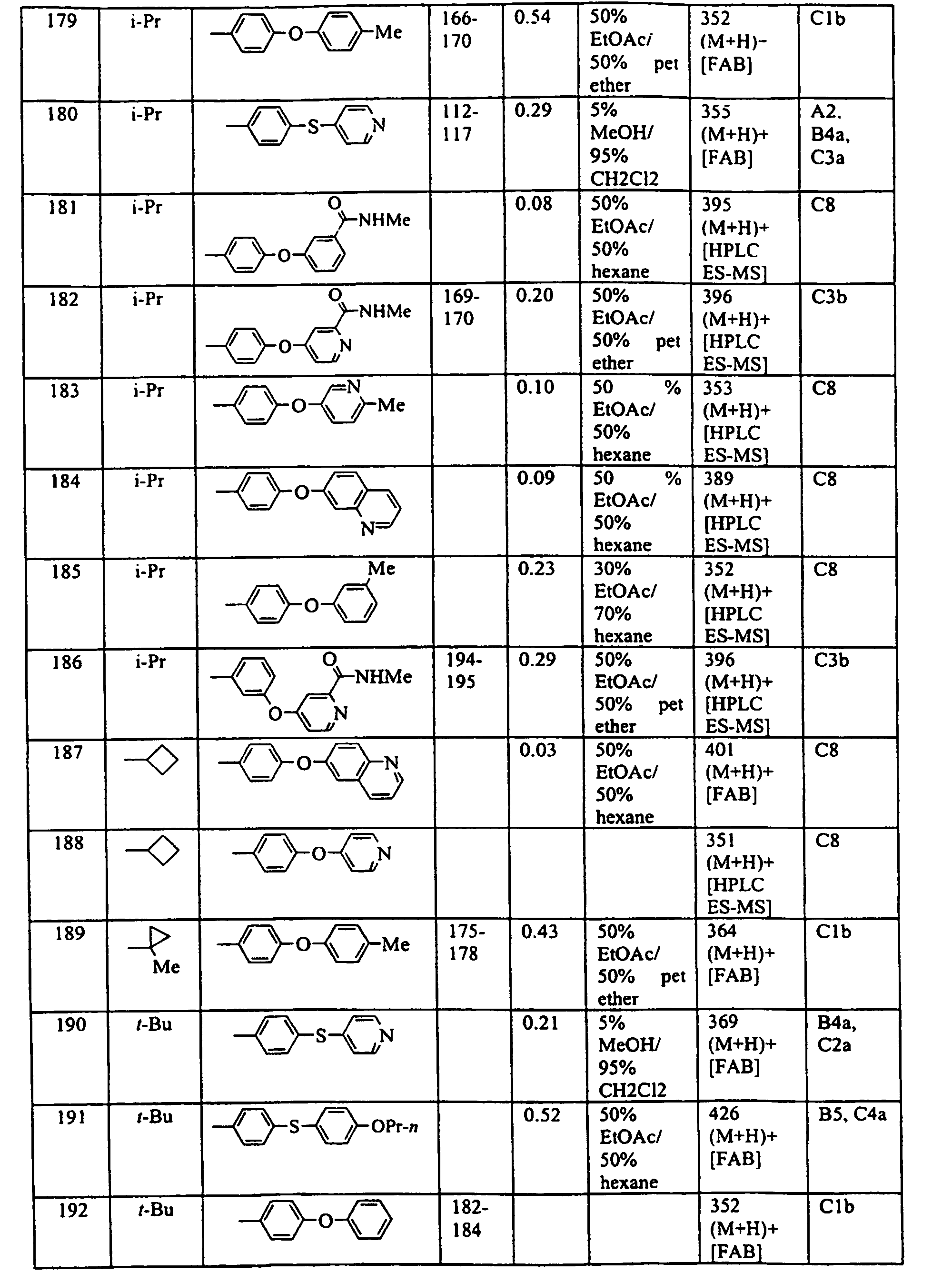

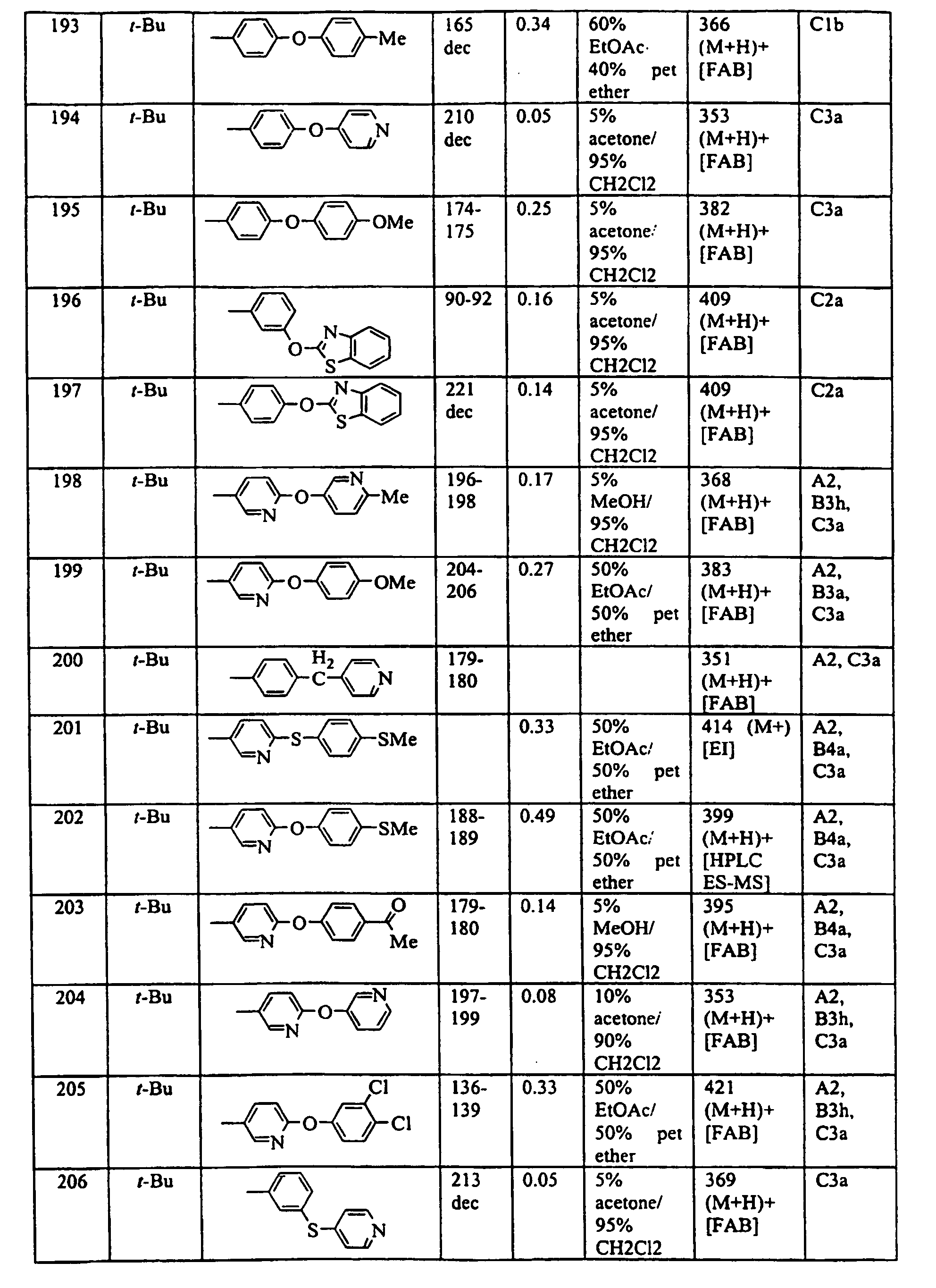

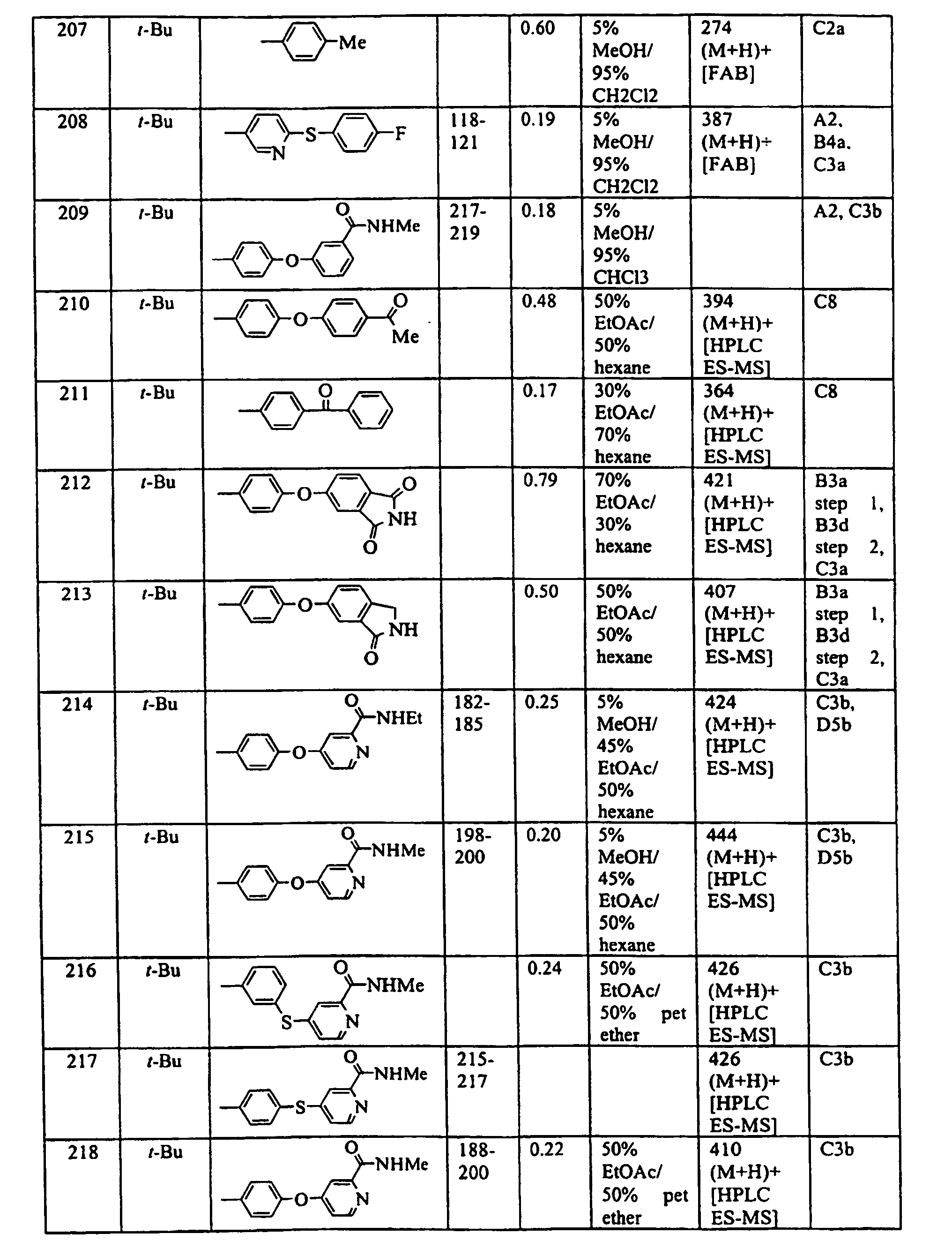

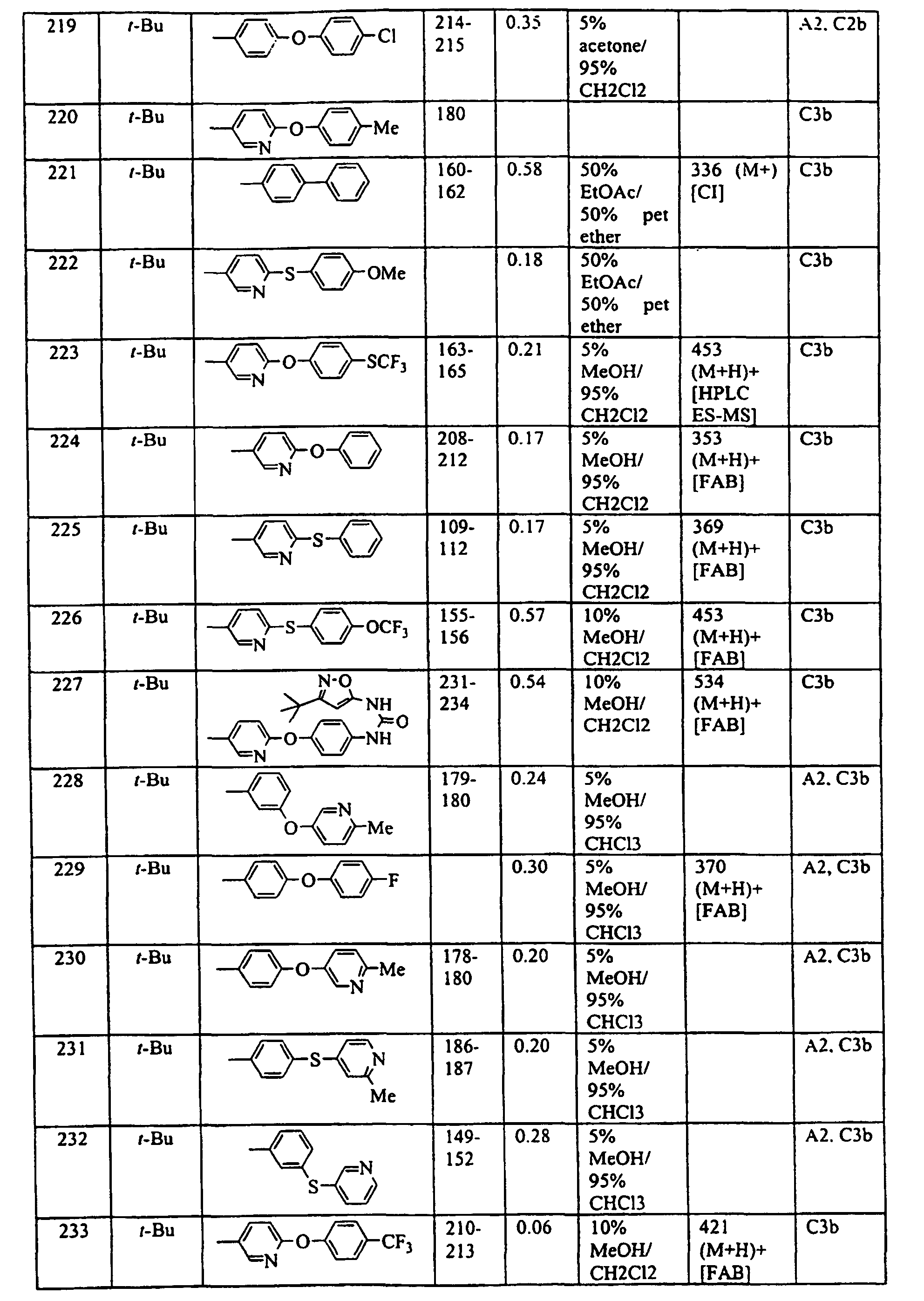

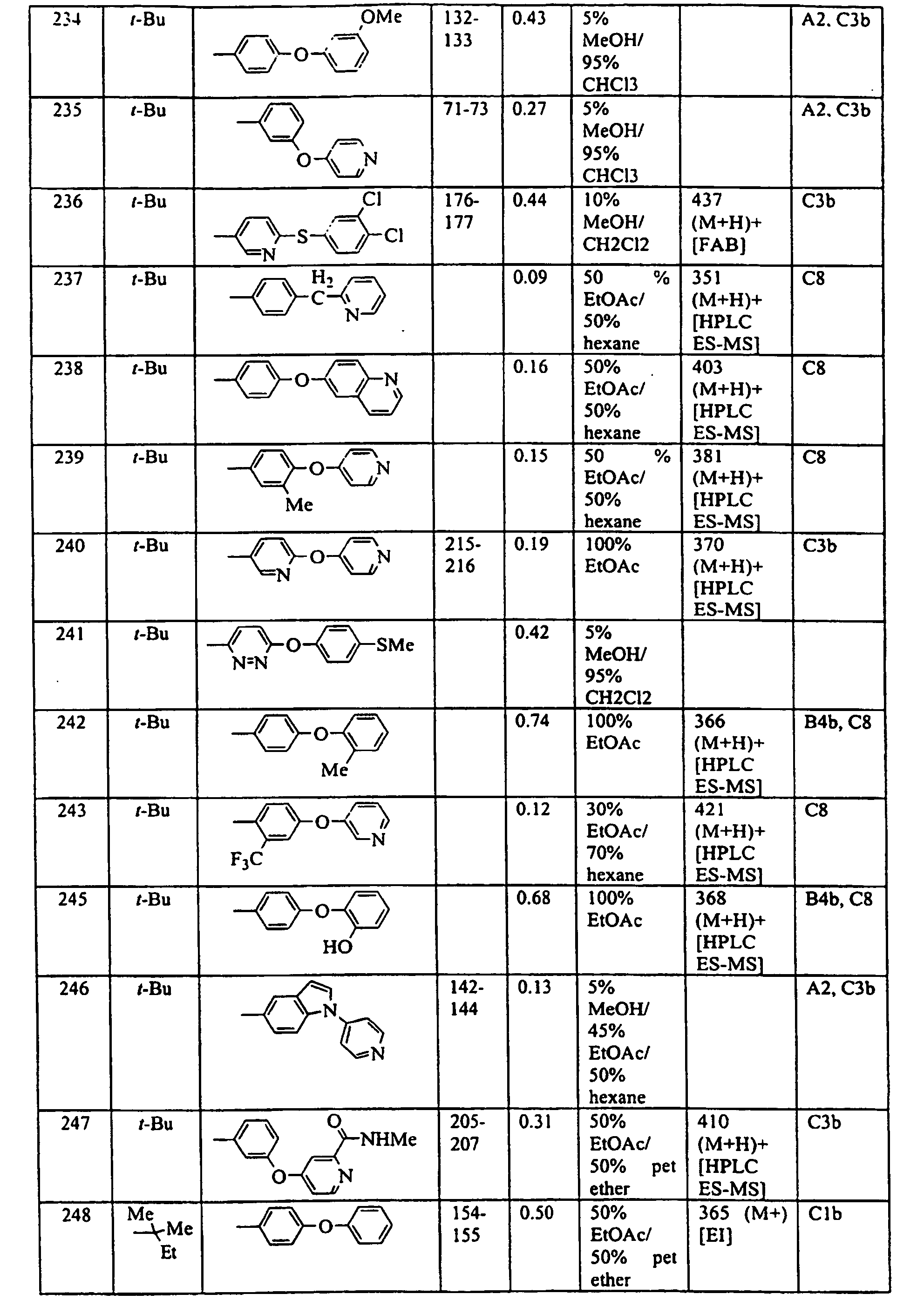

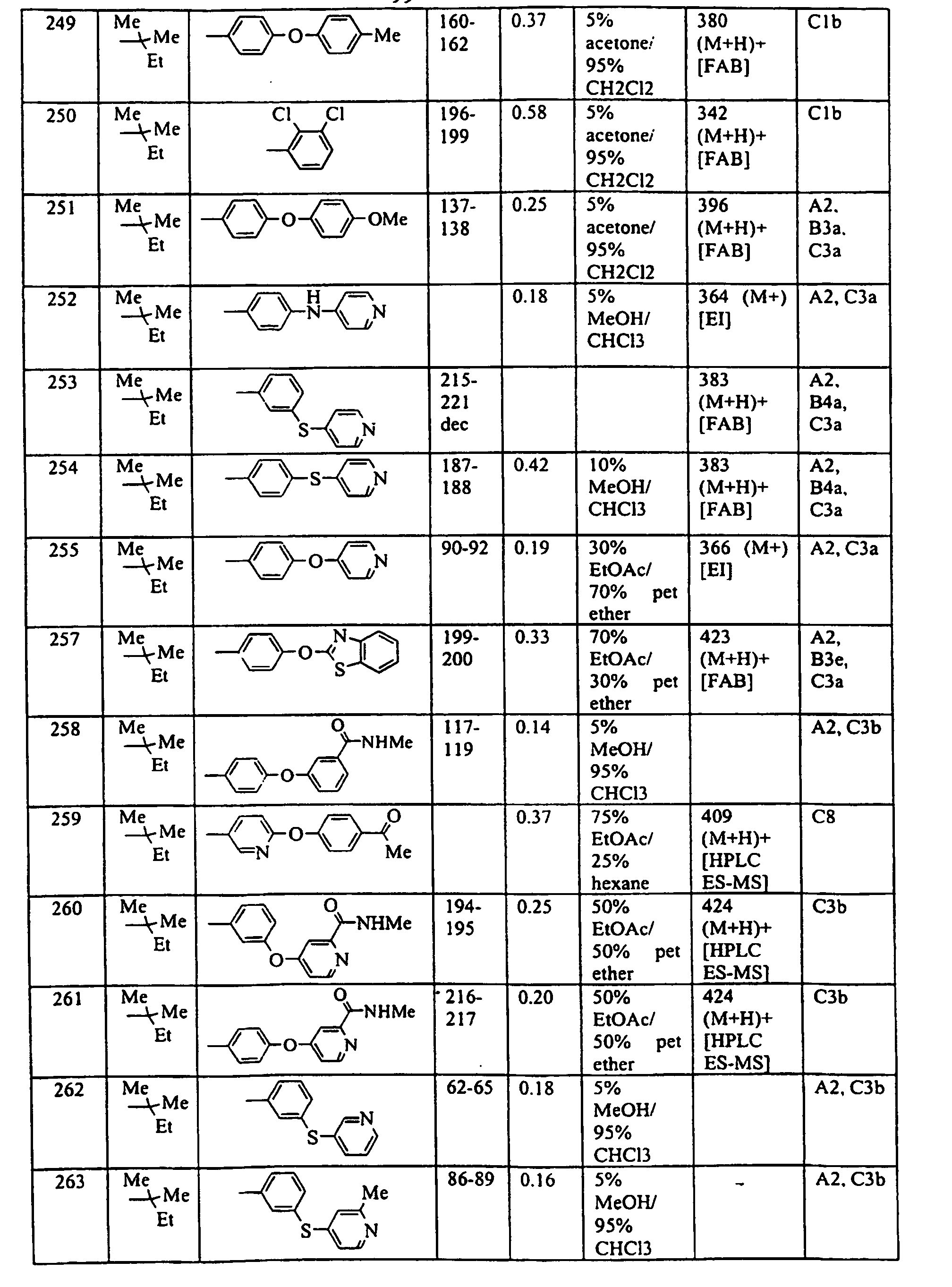

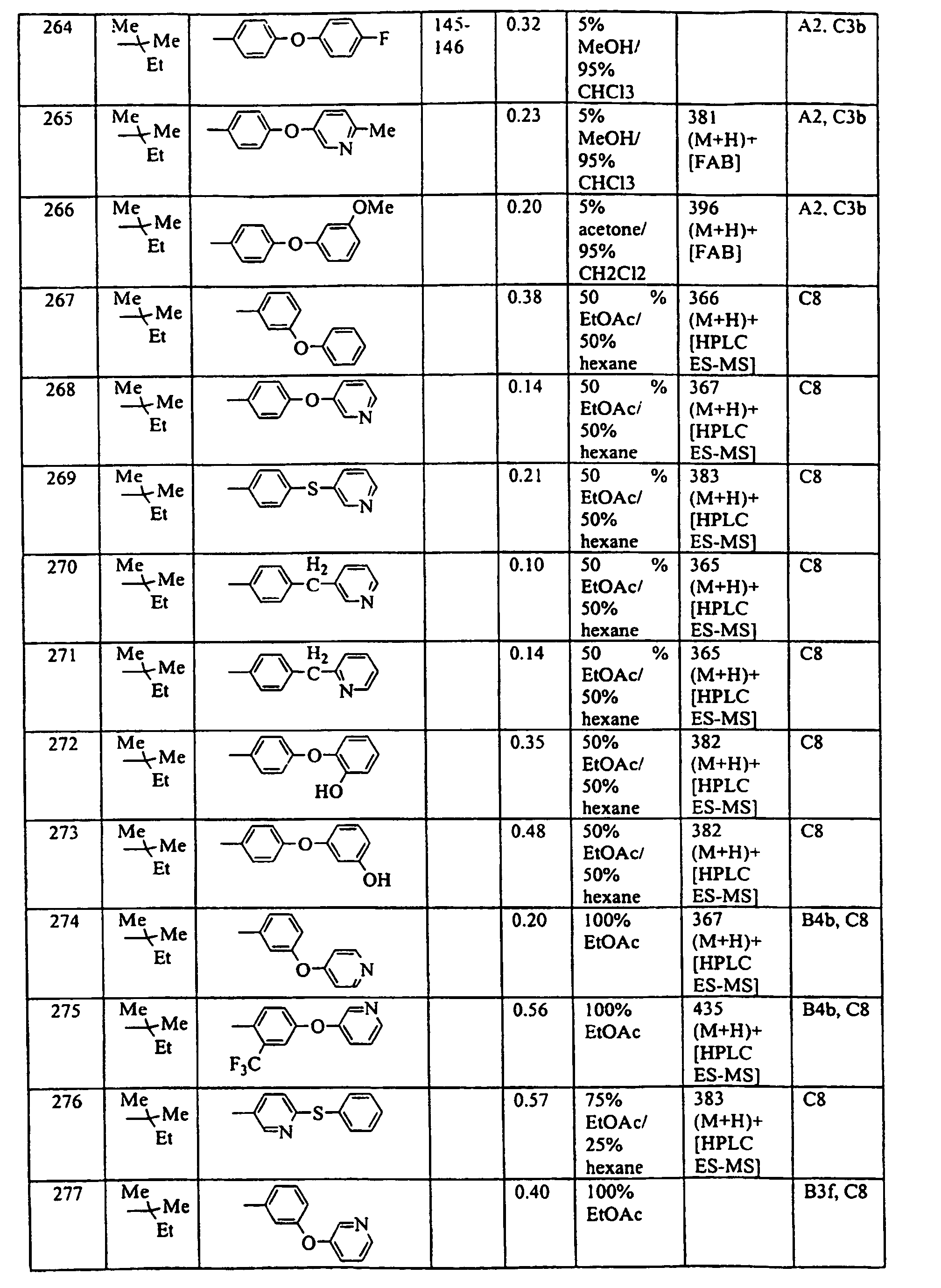

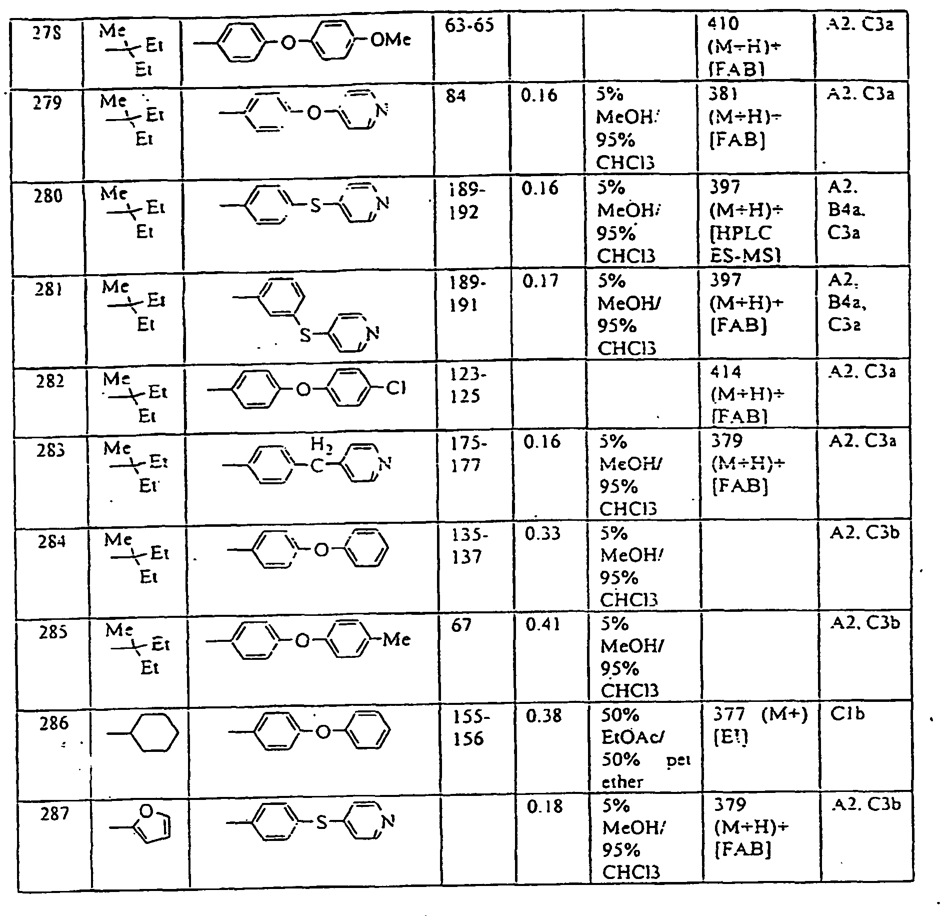

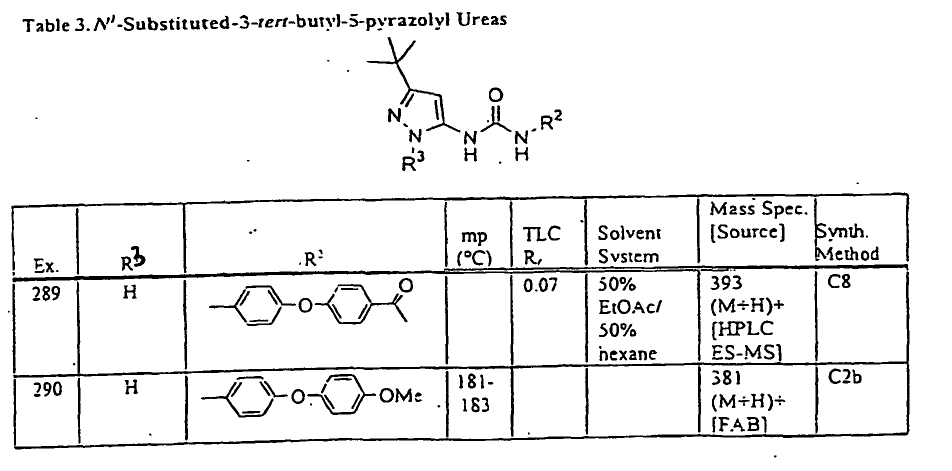

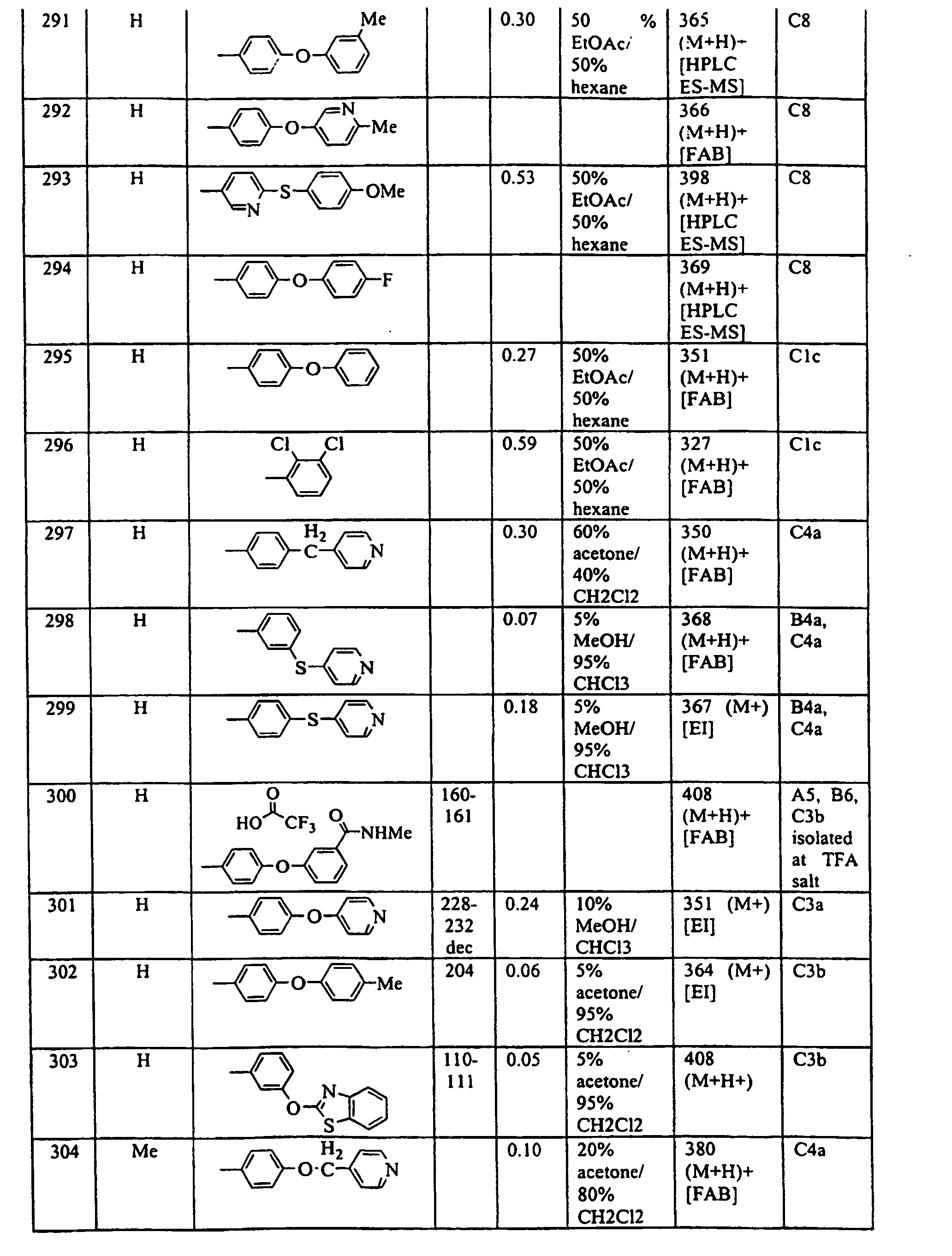

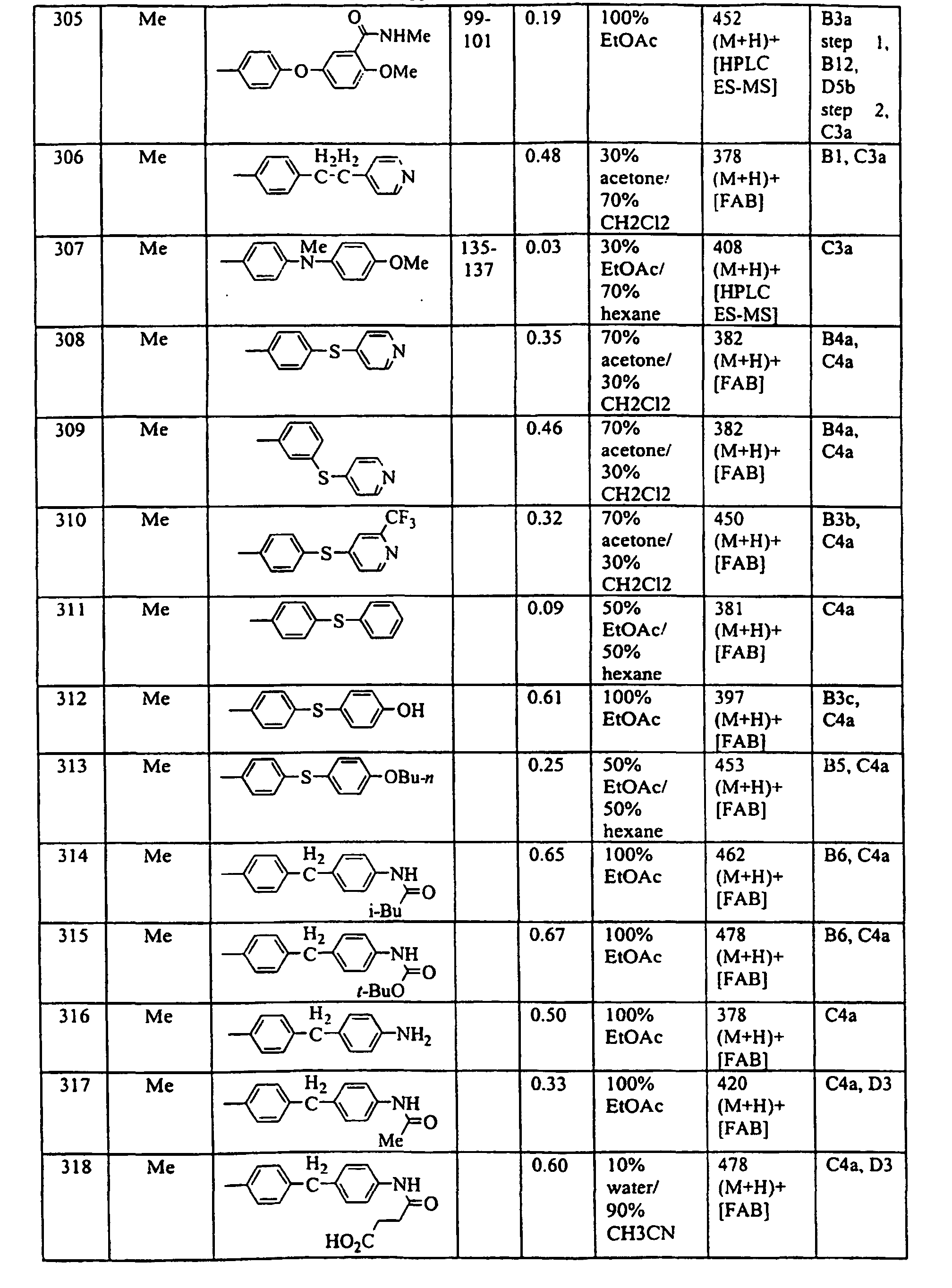

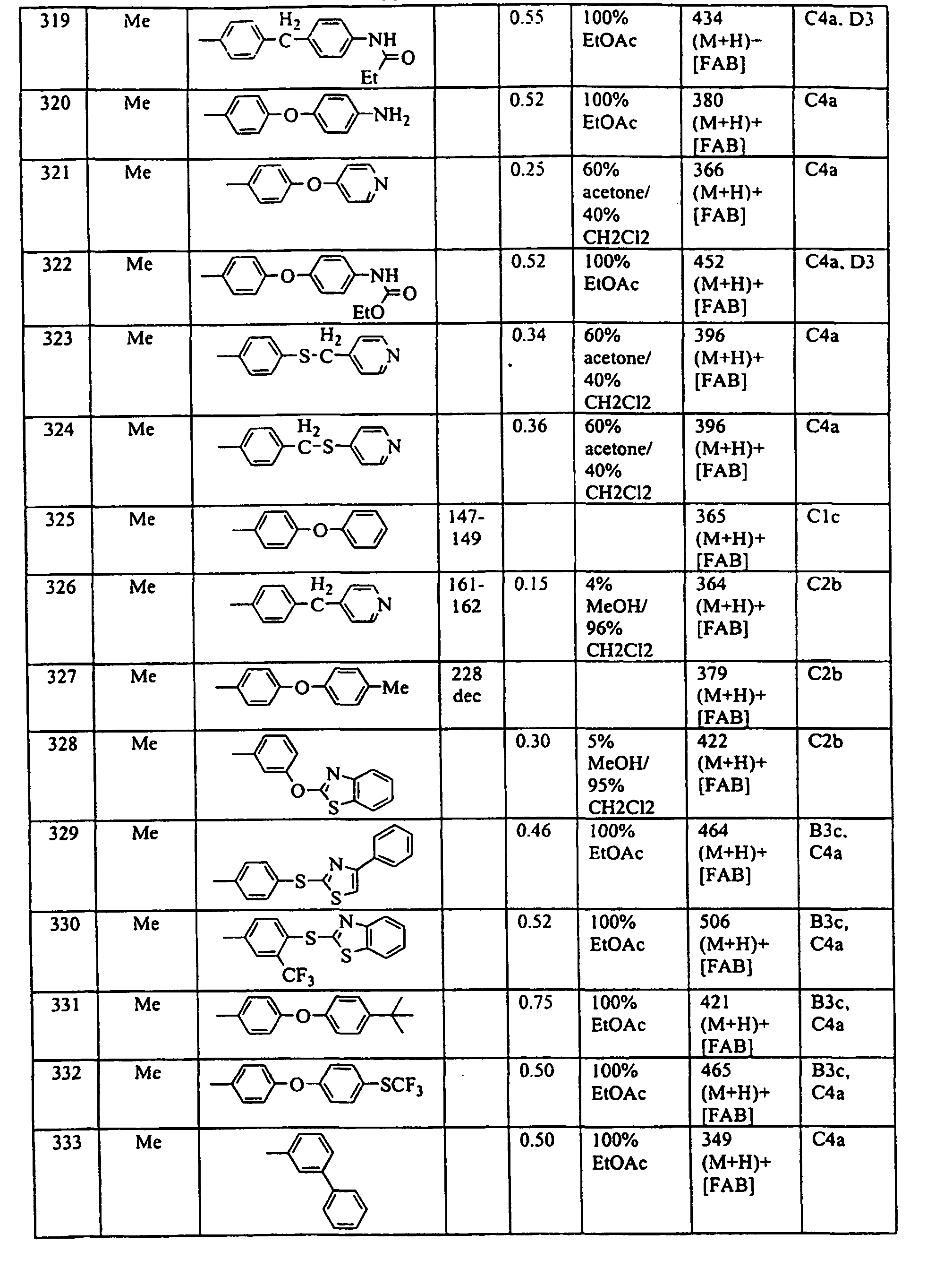

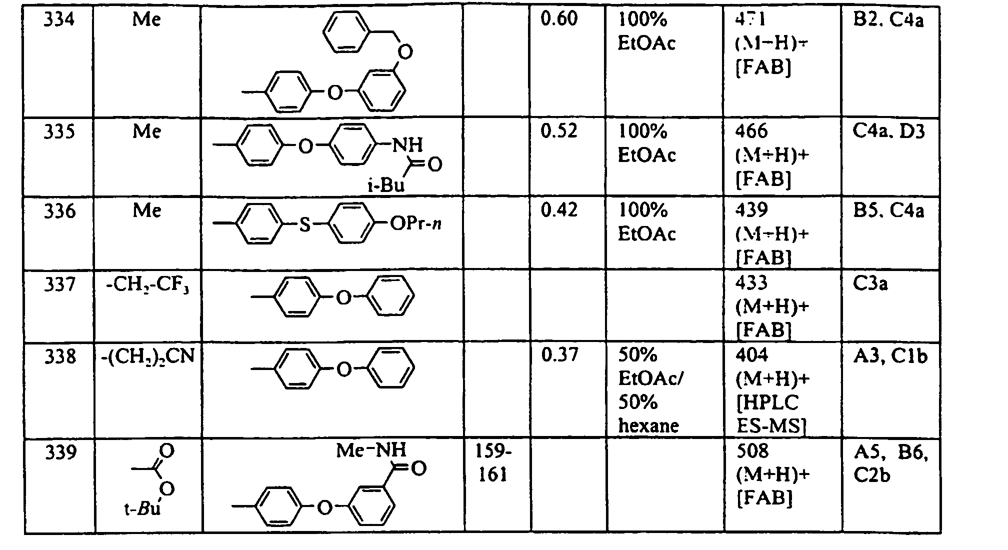

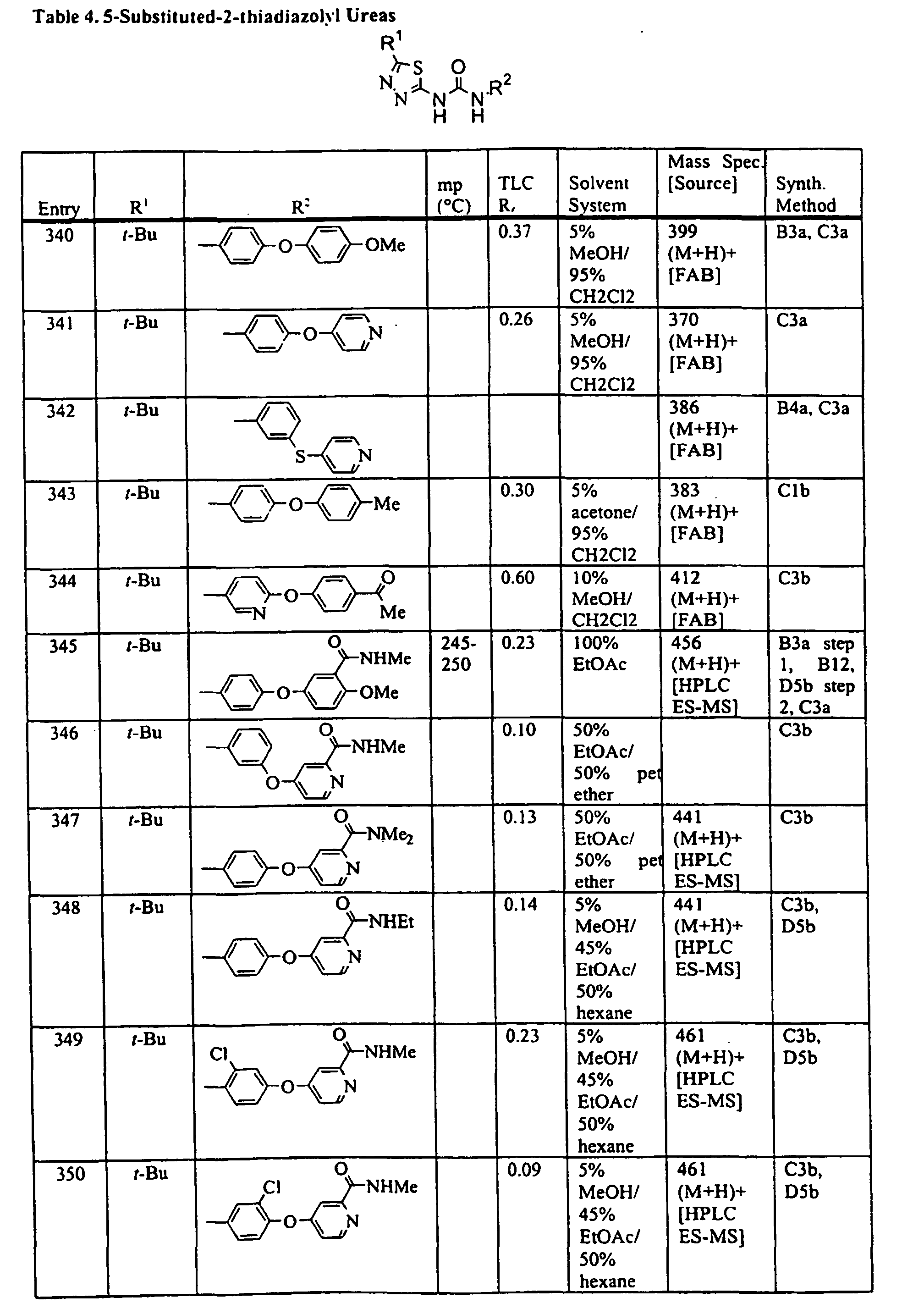

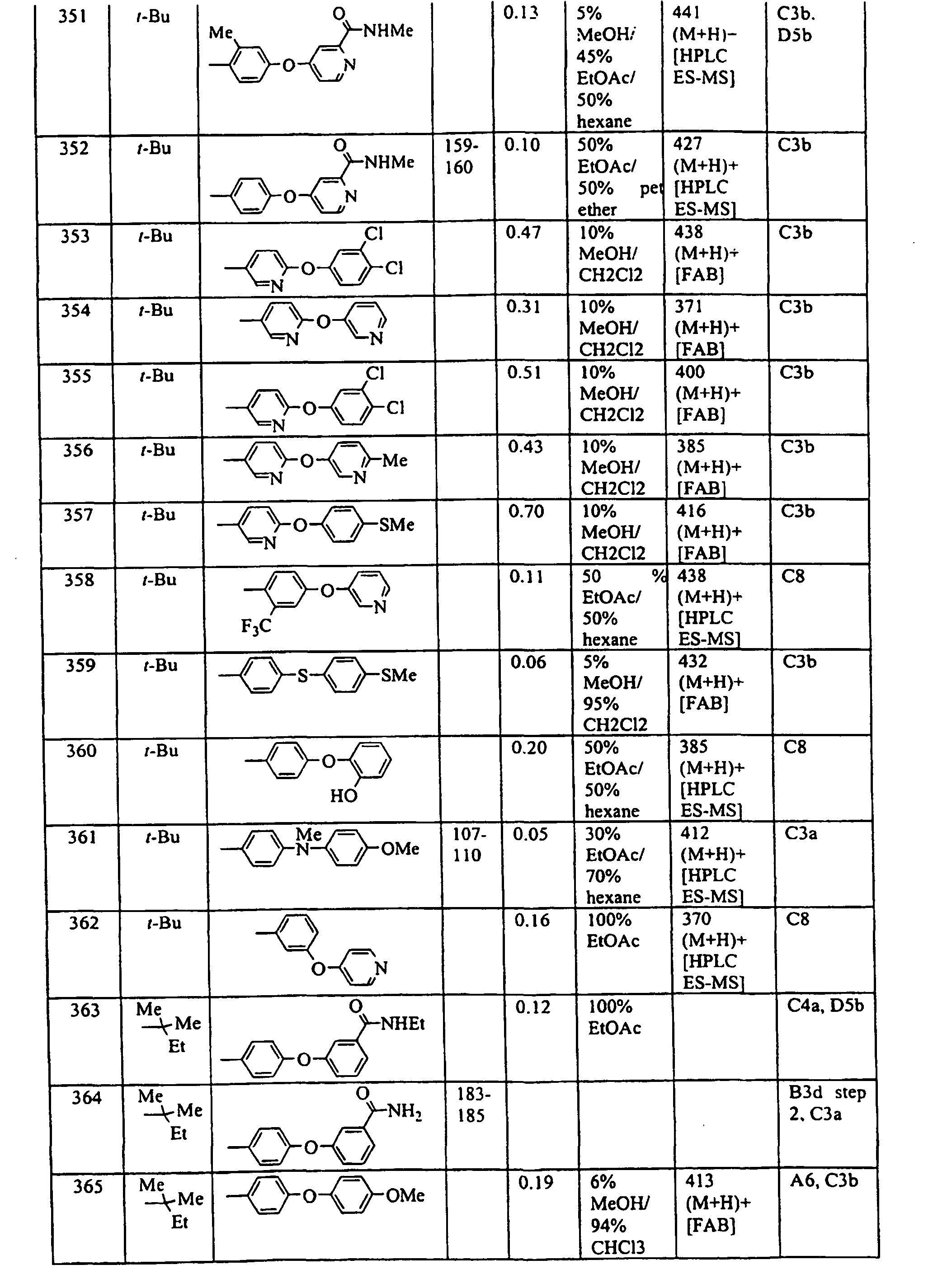

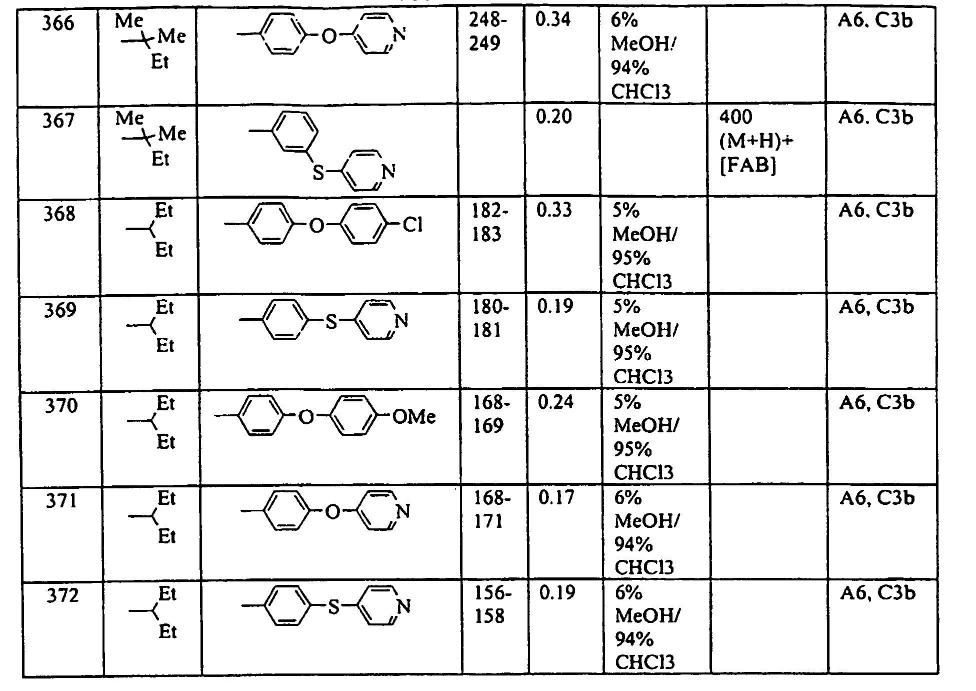

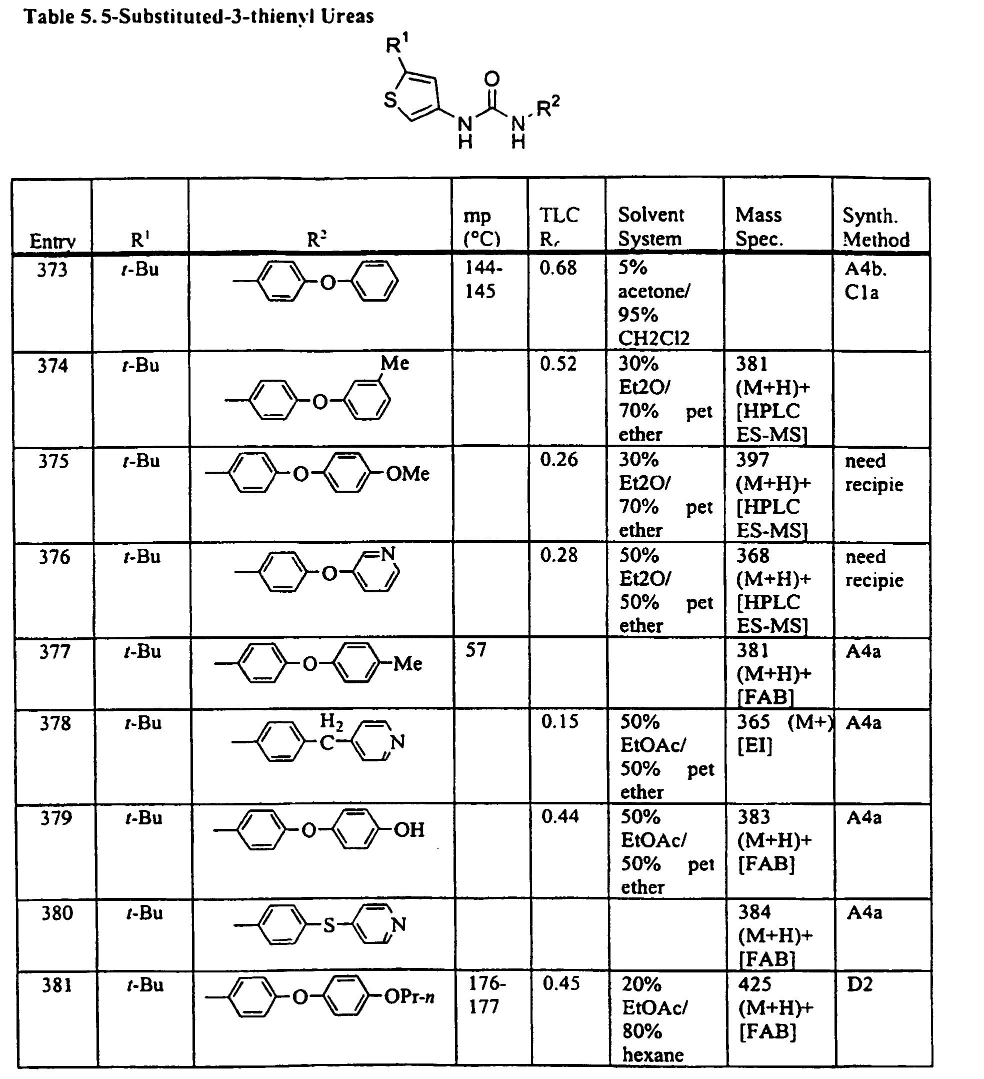

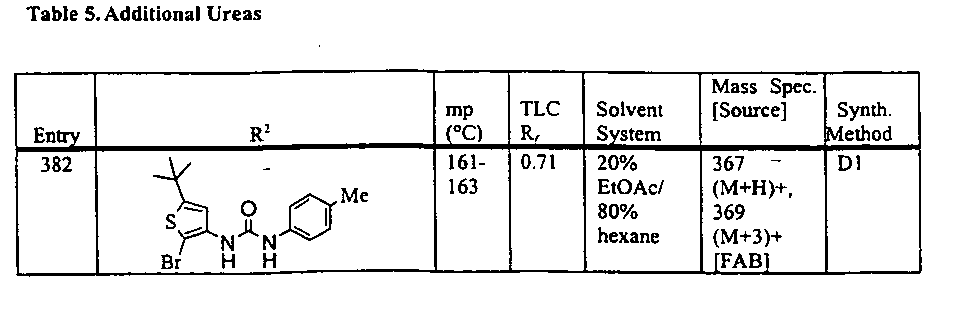

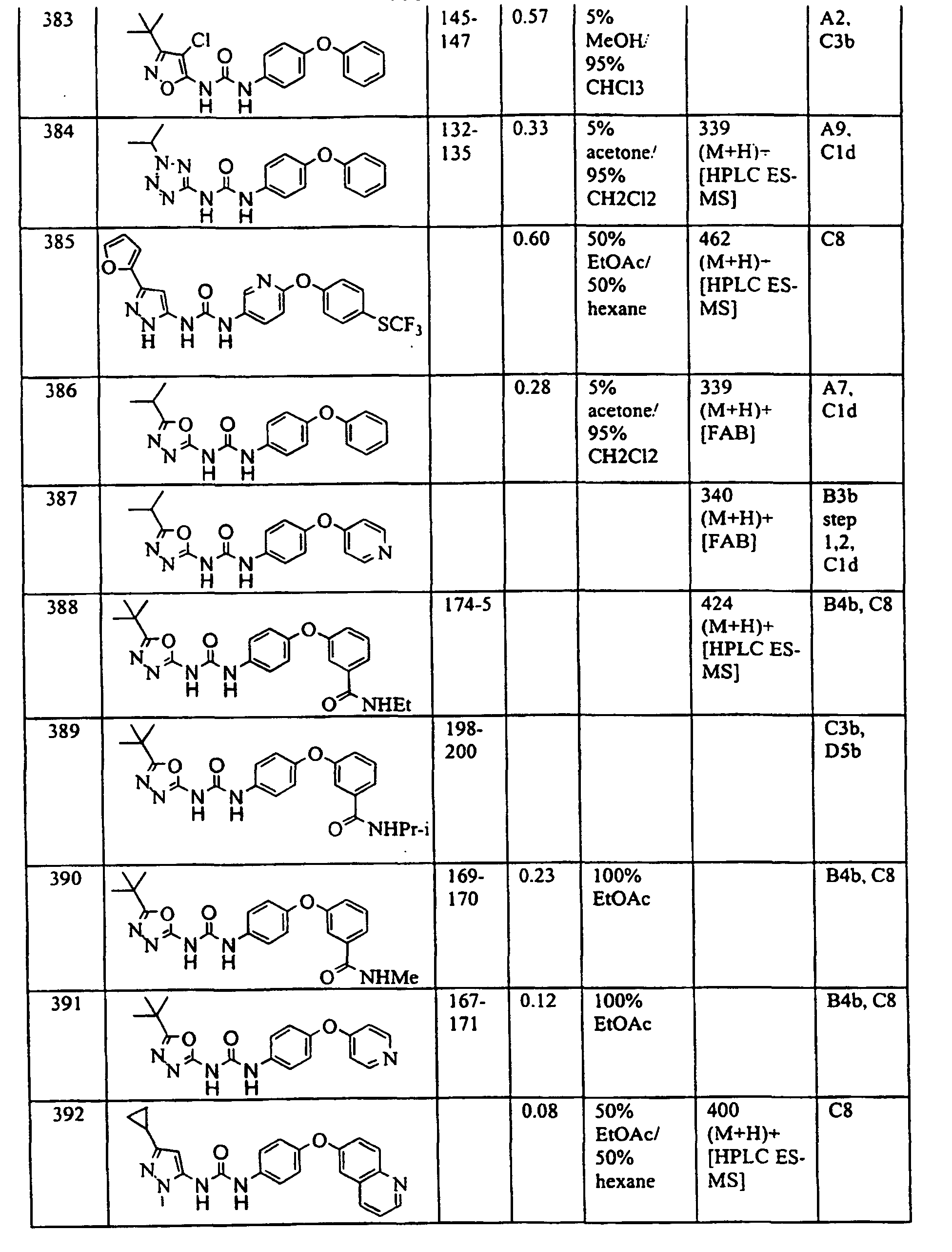

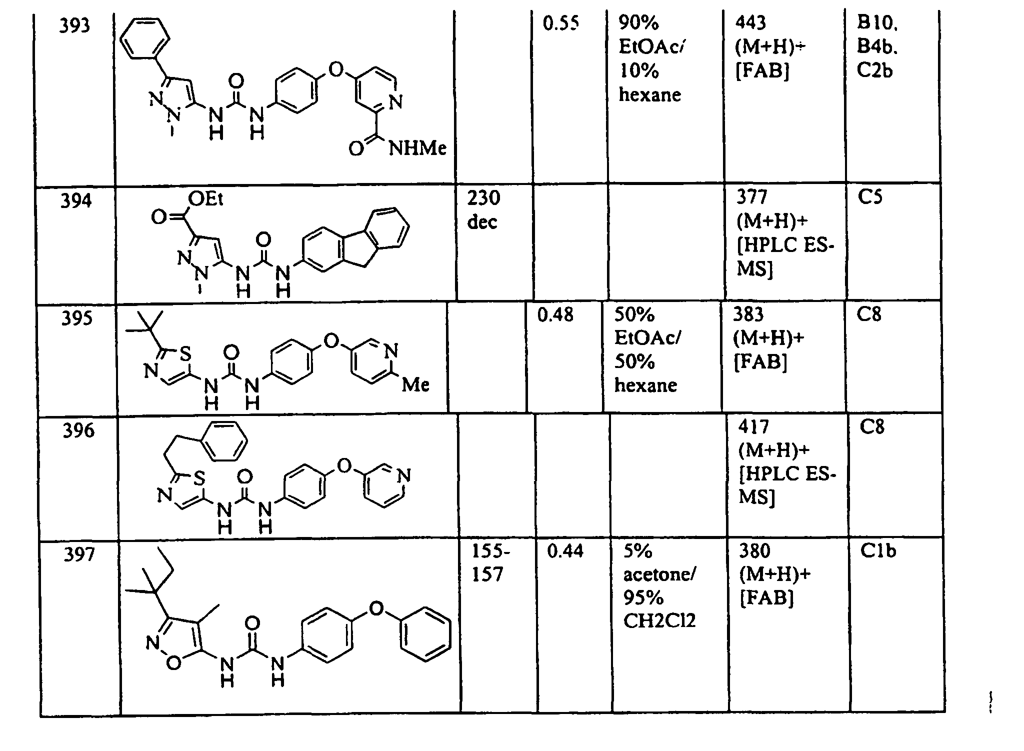

### BIOLOGICAL EXAMPLES

### In Vitro raf Kinase Assay:

In an in vitro kinase assay, raf is incubated with MEK in 20 mM Tris-HCl, pH 8.2 containing 2 mM 2-mercaptoethanol and 100 mM NaCl. This protein solution (20 µL) is mixed with water (5 µL) or with compounds diluted with distilled water from 10 mM stock solutions of compounds dissolved in DMSO. The kinase reaction is initiated by adding 25 µL [γ-³³P]ATP (1000-3000 dpm/pmol) in 80 mM Tris-HCl, pH 7.5, 120 mM NaCl, 1.6 mM DTT, 16 mM MgCl₂. The reaction mixtures are incubated at 32 °C, usually for 22 min. Incorporation of ³³P into protein is assayed by harvesting the reaction onto phosphocellulose mats, washing away free counts with a 1% phosphoric acid solution and quantitating phosphorylation by liquid scintillation counting. For high throughput screening, 10 µM ATP and 0.4 µM MEK are used. In some experiments, the kinase reaction is stopped by adding an equal amount of Laemmli sample buffer. Samples are boiled 3 min and the proteins resolved by electrophoresis on 7.5% Laemmli gels. Gels are fixed, dried and exposed to an imaging plate (Fuji). Phosphorylation is analyzed using a Fujix Bio-Imaging Analyzer System.

All compounds exemplified displayed IC₅₀s of between 1 nM and 10 µM.

### Cellular Assay:

For in vitro growth assay, human tumor cell lines, including but not limited to HCT116 and DLD-1, containing mutated K-ras genes are used in standard proliferation assays for anchorage dependent growth on plastic or anchorage independent growth in soft agar. Human tumor cell lines were obtained from ATCC (Rockville MD) and maintained in RPMI with 10% heat inactivated fetal bovine serum and 200 mM glutamine. Cell culture media and additives are obtained from Gibco/BRL (Gaithersburg, MD) except for fetal bovine serum (JRH Biosciences, Lenexa, KS). In a standard proliferation assay for anchorage dependent growth, 3 X 10³ cells are seeded into 96-well tissue culture plates and allowed to attach overnight at 37 °C in a 5% CO₂ incubator. Compounds are titrated in media in dilution series and added to 96 well cell cultures. Cells are allowed to grow 5 days typically with a feeding of fresh compound containing media on day three. Proliferation is monitored by measuring metabolic activity with standard XTT colorimetric assay (Boehringer Mannheim) measured by standard ELISA plate reader at OD 490/560, or by measuring ³H-thymidine incorporation into DNA following an 8 h culture with 1 µCu ³H-thymidine, harvesting the cells onto glass fiber mats using a cell harvester and measuring ³H-thymidine incorporation by liquid scintillant counting.

For anchorage independent cell growth, cells are plated at 1 x 10³ to 3 x 10³ in 0.4% Seaplaque agarose in RPMI complete media, overlaying a bottom layer containing only 0.64% agar in RPMI complete media in 24-well tissue culture plates. Complete media plus dilution series of compounds are added to wells and incubated at 37 °C in a 5% CO₂ incubator for 10-14 days with repeated feedings of fresh media containing compound at 3-4 day intervals. Colony formation is monitored and total cell mass, average colony size and number of colonies are quantitated using image capture technology and image analysis software (Image Pro Plus, media Cybernetics).

These assays establish that the compounds of Formula I are active to inhibit raf kinase activity and to inhibit oncogenic cell growth.

### In Vivo Assay:

An in vivo assay of the inhibitory effect of the compounds on tumors (e.g., solid cancers) mediated by raf kinase can be performed as follows:

CDI nu/nu mice (6-8 weeks old) are injected subcutaneously into the flank at 1 x 10⁶ cells with human colon adenocarcinoma cell line. The mice are dosed i.p., i.v. or p.o. at 10, 30, 100, or 300 mg/Kg beginning on approximately day 10, when tumor size is between 50-100 mg. Animals are dosed for 14 consecutive days once a day; tumor size was monitored with calipers twice a week.

The inhibitory effect of the compounds on raf kinase and therefore on tumors (e.g., solid cancers) mediated by raf kinase can further be demonstrated in vivo according to the technique of Monia et al. (*Nat. Med.* **1996,** *2*, 668-75).

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. Use of a compound of formula I 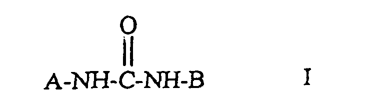 wherein B is phenyl, pyridinyl, pyrimidinyl, pyrazinyl, pyridazinyl, naphthyl, quinolinyl, isoquinolinyl, phthalimidinyl, furyl, pyrrolyl, imidazolyl, pyrazolyl, oxazolyl, isoxazolyl, thiazolyl, isothiazolyl, benzofuryl, benzothienyl, indolyl, benzopyrazolyl, benzoxazolyl, benzisoxazolyl, benzothiazolyl or benzisothiazolyl, substituted by one or more substituents independently selected from the group consisting of halogen, up to per-halosubstitution, and Xₙ, wherein n is 0-3 and each X is independently selected from the group consisting of -CN, -CO₂R⁵, -C(O)NR⁵R^{5'}, -C(O)R⁵, -NO₂, -OR⁵, - SR⁵, -NR⁵R^{5'}, -NR⁵C(O)OR^{5'}, -NR⁵C(O)R^{5'}, C₁-C₁₀ alkyl, C₂-C₁₀ alkenyl, C₁-C₁₀ alkoxy, C₃-C₁₀ cycloalkyl, C₆-C₁₄ aryl, C₃-C₁₃ heteroaryl, up to per halo-substituted C₁-C₁₀ alkyl, up to per halo-substituted C₂-C₁₀ alkenyl, up to per halo-substituted C₁-C₁₀ alkoxy, up to per halo-substituted C₃-C₁₀ cycloalkyl and -Y-Ar;
wherein R⁵ and R^{5'} are independently selected from H, C₁-C₁₀ alkyl, C₂-C₁₀ alkenyl, C₃-C₁₀ cycloalkyl up to per-halosubstituted C₁-C₁₀ alkyl, up to per-halosubstituted C₂-C₁₀ alkenyl and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵ NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur optionally substituted by halogen up to per-halosubstitution and optionally substituted by Zₙ₁, wherein n1 is 0 to 3 and each Z is independently selected from the group consisting of -CN, =O, -CO₂R⁵, -C(O)NR⁵R^{5'}, -C(O)- NR⁵, -NO₂, -OR⁵, - SR⁵, -NR⁵R^{5'}, -NR⁵C(O)OR^{5'}, -C(O)R⁵, -NR⁵C(O)R^{5'}, -SO₂R⁵, SO₂NR⁵R^{5'}, C₁-C₁₀ alkyl, C₁-C₁₀ alkoxyl, C₃-C₁₀ cycloalkyl, up to per halo-substituted C₁-C₁₀ alkyl; and up to per halo substituted C₃-C₁₀ cycloalkyl, and
A is a heteroaryl moiety selected from the group consisting of 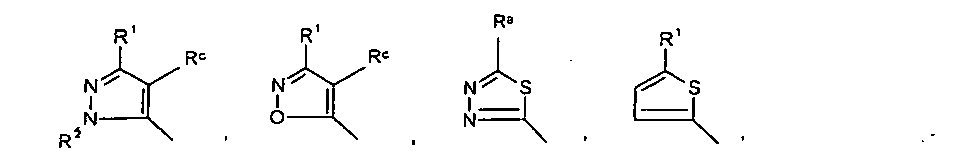 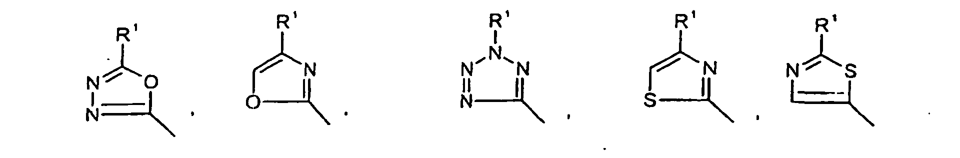 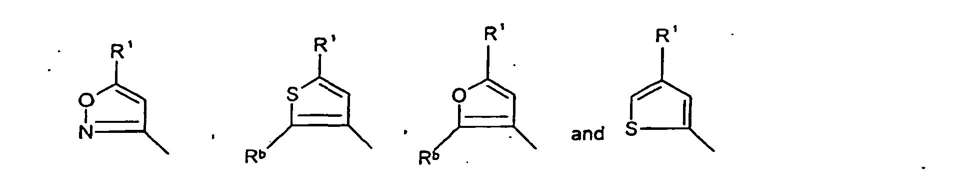 wherein
R¹ is selected from the group consisting of halogen, C₃-C₁₀ alkyl, C₃-C₁₀ cycloalkyl, C₁₋C₁₃ heteroaryl, C₆-₁₄ aryl, C₇-₂₄ alkaryl, up to per-halosubstituted C₁-C₁₀ alkyl, up to per-halosubstituted C₃-C₁₀ cycloalkyl, up to per-halosubstituted C₁₋C₁₃ heteroaryl, up to per-halosubstituted C₆-₁₄ aryl, and up to per-halosubstituted C₇-₂₄ alkaryl;
R² is selected from the group consisting of H, -C(O)R⁴, -CO₂R⁴, -C(O)NR³R^{3'}, C₁-C₁₀ alkyl, C₃-C₁₀ cycloalkyl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl,
where R² is a substituted group, it is substituted by one or more substituents independently selected from the group consisting of -CN, -CO₂R⁴, -C(O)-NR³R^{3'}, -NO₂, -OR⁴, -SR⁴, and halogen up to per-halosubstitution,
wherein R³ and R^{3'} are independently selected from the group consisting of H, -OR⁴, -SR⁴, -NR⁴R^{4'}, -C(O)R⁴, -CO₂R⁴, -C(O)NR⁴R^{4'}, C₁-C₁₀ alkyl, C₃-C₁₀ cycloalkyl, C₆-C₁₄ aryl, C₃-C₁₃ heteroaryl up to per-halosubstituted C₁-C₁₀ alkyl, up to per-halosubstituted C₃-C₁₀ cycloalkyl, and up to per-halosubstituted, C₆-C₁₄ aryl or C₃-C₁₃ heteroaryl; and
wherein R⁴ and R^{4'} are independently selected from the group consisting of H, C₁-C₁₀ alkyl, C₃-C₁₀ cycloalkyl, C₆-C₁₄ aryl, C₃-C₁₃ heteroaryl, up to per-halosubstituted C₁-C₁₀ alkyl, up to per-halosubstituted C₃-C₁₀ cycloalkyl, and up to per-halosubstituted, C₆-C₁₄ aryl or C₃-C₁₃ heteroaryl,
R^{a} is C₁-C₁₀ alkyl, C₃-C₁₀ cycloalkyl, up to per-halosubstituted C₁-C₁₀ alkyl and up to per-halosubstituted C₃-C₁₀ cycloalkyl; and
R^{b} is hydrogen or halogen,
R^{c} is hydrogen, halogen, C₁-C₁₀ alkyl, up to per-halosubstituted C₁-C₁₀ alkyl or combines with R¹ and the ring carbon atoms to which R¹ and R^{c} are bound to form a 5- or 6-membered cycloalkyl, aryl or hetaryl ring with 0-2 members selected from O, N and S; subject to the proviso that where A is 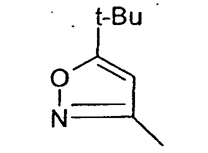 B is not 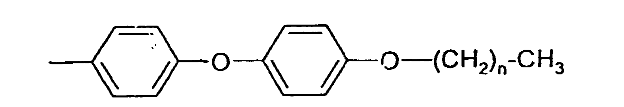 wherein n = 2-4,
or 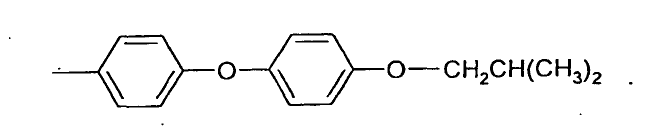 for the manufacture of a medicament for the treatment of cancerous cell growth mediated by raf kinase.

2. The use of claim 1, wherein B is 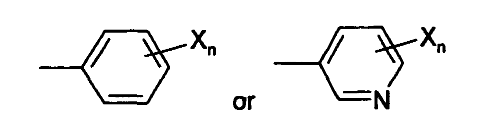 which is substituted or unsubstituted by halogen, up to per-halosubstitution, and wherein
n = 1-3 and
each X is independently selected from the group consisting of C₁₋₁₀ alkyl, up to per-halosubstituted C₁₋₁₀ alkyl and -Y-Ar;
wherein Y is - O-, -S-, -N(R⁵)-, -(CH₂)-ₘ, -C(O)-, -CH(OH)-, -(CH₂)ₘO-, -NR⁵C(O)NR⁵ NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur optionally substituted by halogen up to per-halosubstitution and optionally substituted by Zₙ₁, wherein n1 is 0 to 3 and each Z is independently selected from the group consisting of -CN, =O,
-CO₂R⁵, -C(O)NR⁵R^{5'}, -C(O)R⁵, -NO₂, -OR⁵, - SR⁵, - NR⁵R^{5'}, -NR⁵C(O)OR^{5'}, -C(O)R⁵, -NR⁵C(O)R^{5'}, -SO₂R⁵, -SO₂R⁵R^{5'}, C₁-C₁₀ alkyl, C₁-C₁₀ alkoxy, C₃-C₁₀ cycloalkyl, up to per halo-substituted C₁-C₁₀ alkyl, and up to per halo-substituted C₃-C₁₀ cycloalkyl,
wherein R⁵ and R^{5'} are independently selected from H, C₁-C₁₀ alkyl, C₂-C₁₀ alkenyl, C₃-C₁₀ cycloalkyl, up to per-halosubstituted C₁-C₁₀ alkyl, up to per-halosubstituted C₂-C₁₀ alkenyl and up to per-halosubstituted C₃-C₁₀ cycloalkyl.

3. The use of claim 1, wherein B is 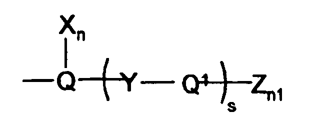 wherein
Y is selected from the group consisting of -O-, -S-, -CH₂-, -SCH₂-, -CH₂S-,
-CH(OH)-, -C(O)-, -CX^{a}₂, -CX^{a}H-, -CH₂O- and -OCH₂-,
X^{a} is halogen,
Q is phenyl or pyridinyl, substituted or unsubstituted by halogen, up to per-halosubstitution;
Q¹ is a mono- or bicyclic aromatic structure of 3 to 10 carbon atoms and 0-4 members of the group consisting of N, O and S, optionally substituted by halogen up to per-halosubstitution,
X, Z, n and n1 are as defined in claim 1, and s = 0 or 1.

4. The use of claim 3, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution, and
each X is independently selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl, wherein R⁶ and R⁷ can be substituted by halogen or up to per-halosubstitution.

5. The use of claim 1, wherein the compound has the formula 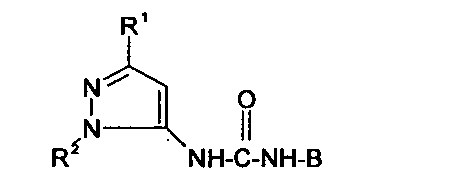 wherein R¹ and R² and B are as defined in claim 1.

6. The use of claim 5, wherein B is of the formula 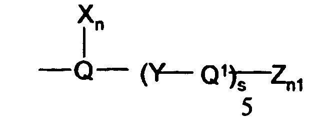 wherein Q is phenyl or pyridinyl, optionally substituted by halogen up to per-halosubstitution, Q¹ is pyridinyl, phenyl or benzothiazolyl optionally substituted by halogen up to per-halosubstitution, Y is -0-, -S-, -CH₂S-, -SCH₂-, -CH₂O-, -OCH₂- or -CH₂-, X is C₁-C₄ alkyl or up to per-halosubstituted C₁-C₄ alkyl and Z is as defined in claim 1, n = 0 or 1, s = 1 and n1 = 0-1.

7. The use of claim 1, wherein the compound is selected from the group consisting of
*N*-(3-*tert*-Butyl-5-pyrazolyl)-*N*'-(4-phenyloxyphenyl)urea;
*N*-(3-*tert*-Butyl-5-pyrazolyl)-*N*'-(3-(3-methylaminocarbonylphenyl)oxyphenyl)urea;
*N*-(3-*tert*-Butyl-5-pyrazolyl)-*N*'-(3-(4-pyridinyl)thiophenyl)urea;
*N*-(3-*tert*-Butyl-5-pyrazolyl)-*N*'-(4-(4-pyridinyl)thiophenyl)urea;
*N*-(3-*tert*-Butyl-5-pyrazolyl)-*N*'-(4-(4-pyridinyl)oxyphenyl)urea;
*N*-(3-*tert*-Butyl-5-pyrazolyl)-*N*'-(4-(4-pyridinyl)methylphenyl)urea;
*N*-(1-Methyl-3-*tert*-butyl-5-pyrazolyl)-*N*'-(4-phenyloxyphenyl)urea;
*N*-(1-Methyl-3-*tert*-butyl-5-pyrazolyl)-*N*'-(3-(4-pyridinyl)thiophenyl)urea;
*N*-(1-Methyl-3-*tert*-butyl-5-pyrazolyl)-*N*'-((4-(4-pyridinyl)thiomethyl)phenyl)urea;
*N*-(1-Methyl-3-*tert*-butyl-5-pyrazolyl)-*N*'-(4-(4-pyridinyl)thiophenyl)urea;
*N*-(1-Methyl-3-*tert*-butyl-5-pyrazolyl)-*N*'-(4-(4-pyridinyl)oxyphenyl)urea;
*N*-(1-Methyl-3-*tert*-butyl-5-pyrazolyl)-*N*'-((4-(4-pyridinyl)methyloxy)phenyl)urea;
*N*-(1-Methyl-3-*tert*-butyl-5-pyrazolyl)-*N*'-(3-(2-benzothiazolyl)oxyphenyl)urea;
*N*-(3-*tert*-butyl-5-pyrazolyl)-*N*'-(3-(4-pyridyl)thiophenyl) urea;
*N*-(3-*tert*-butyl-5-pyrazolyl)-*N*'-(4-(4-pyridyl)thiophenyl) urea;
*N*-(3-*tert*-butyl-5-pyrazolyl)-*N*'-(3-(4-pyridyl)oxyphenyl) urea;
*N*-(3-*tert*-butyl-5-pyrazolyl)-*N*'-(4-(4-pyridyl)oxyphenyl) urea;
*N*-(1-methyl-3-*tert*-butyl-5-pyrazolyl)-*N*'-(3-(4-pyridyl)thiophenyl) urea;
*N*-(1-methyl-3-*tert*-butyl-5-pyrazolyl)-*N*'-(4-(4-pyridyl)thiophenyl) urea;
*N*-(1-methyl-3-*tert*-butyl-5-pyrazolyl)-*N*'-(3-(4-pyridyl)oxyphenyl) urea;
*N*-(1-methyl-3-*tert*-butyl-5-pyrazolyl)-*N*'-(4-(4-pyridyl)oxyphenyl) urea;
and pharamceutically acceptable salts thereof.

8. The use of claim 5, wherein R¹ is t-butyl.

9. The use of claim 1, wherein the compound has the formula 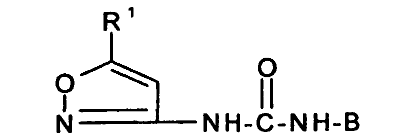 wherein R¹ and B are as defined in claim 1.

10. The use of claim 9, wherein B is of the formula 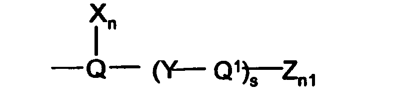 Q is phenyl or pyridinyl, optionally substituted by halogen up to per-halosubstitution, Q¹ is pyridinyl, phenyl or benzothiazolyl optionally substituted by halogen up to per-halosubstitution, Y is -O-, -S-, -C(O)- or -CH₂-, X is C₁-C₄ alkyl or up to per-halosubstituted C₁-C₄ alkyl and Z is as defined in claim 1, n = 0 or 1, s = 0 or 1 and n1 = 0 or 1.

11. The use of claim 1, wherein the compound is selected from the group consisting of:
*N*-(5-*tert*-Butyl-3-isoxazolyl)-*N'*-(4-(4-hydroxyphenyl)oxyphenyl)urea;
*N*-(5-*tert*-Butyl-3-isoxazolyl)-*N'*-(4-(3-hydroxyphenyl)oxyphenyl)urea;
*N*-(5-*tert*-Butyl-3-isoxazolyl)-*N'*-(4-(4-acetylphenyl)oxyphenyl)urea;
*N*-(5-*tert*-Butyl-3-isoxazrolyl)-*N'*-(3-benzoylphenyl)urea;
*N*-(5-*tert*-Butyl-3-isoxazolyl)-*N'*-(4-phenyloxyphenyl)urea;
*N*-(5-*tert*-Butyl-3-isoxazolyl)-*N'*-(4-(3-methylaminocarbonylphenyl)-thiophenyl)urea;
*N*-(5-*tert*-Butyl-3-isoxazolyl)-*N'*-(4-(4-(1,2-methylenedioxy)phenyl)-oxyphenyl)urea;
*N*-(5-*tert*-Butyl-3-isoxazolyl)-*N'*-(4-(3-pyridinyl)oxyphenyl)urea;
*N*-(5-*tert*-Butyl-3-isoxazolyl)-*N'*-(4-(4-pyridinyl)oxyphenyl)urea;
*N*-(5-*tert*-Butyl-3-isoxazolyl)-*N'*-(4-(4-pyridyl)thiophenyl)urea;
*N*-(5-*tert*-Butyl-3-isoxazolyl)-*N'*-(4-(4-pyridinyl)methylphenyl)urea;
*N*-(5-*tert*-Butyl-3-isoxazolyl)-*N'*-(3-(4-pyridinyl)oxyphenyl)urea;
*N*-(5-*tert*-Butyl-3-isoxazolyl)-*N'*-(3-(4-pyridinyl)thiophenyl)urea;
*N*-(5-*tert*-Butyl-3-isoxazolyl)-*N'*-(3-(3-methyl-4-pyridinyl)oxyphenyl)urea;
*N*-(5-*tert*-Butyl-3-isoxazolyl)-*N'*-(3-(3-methyl-4-pyridinyl)thiophenyl)urea;
*N*-(5-*tert*-Butyl-3-isoxazolyl)-*N'*-(4-(3-methyl-4-pyridinyl)thiophenyl)urea;
*N*-(5-*tert*-Butyl-3-isoxazolyl)-*N'*-(3-(4-methyl-3-pyridinyl)oxyphenyl)urea;
*N*-(5-*tert*-Butyl-3-isoxazolyl)-*N'*-(4-(3-methyl-4-pyridinyl)oxyphenyl)urea;
*N*-(5-*tert*-Butyl-3-isoxazolyl)-*N'*-(3-(2-benzothiazolyl)oxyphenyl)urea;
*N*-(5-*tert*-butyl-3-isoxazolyl)-*N'*-(3-chloro-4-(4-(2-methylcarbamoyl)pyridyl)-oxyphenyl) urea;
*N*-(5-*tert*-butyl-3-isoxazolyl)-*N'*-(4-(4-(2-methylcarbamoyl)pyridyl)-oxyphenyl) urea;
N-(5-*tert*-butyl-3-isoxazolyl)-*N'*-(3-(4-(2-methylcarbamoyl)pyridyl)-thiophenyl) urea;
*N*-(5-*tert*-butyl-3-isoxazolyl)-*N'*-(2-methyl-4-(4-(2-methylcarbamoyl)pyridyl)-oxyphenyl) urea;
*N*-(5-*tert*-butyl-3-isoxazolyl)-*N'*-(4-(4-(2-carbamoyl)pyridyl)oxyphenyl) urea;
*N*-(5-*tert*-butyl-3-isoxazolyl)-*N'*-(3-(4-(2-carbamoyl)pyridyl)oxyphenyl) urea;
*N*-(5-*tert*-butyl-3-isoxazolyl)-*N'*-(3-(4-(2-methylcarbamoyl)pyridyl)-oxyphenyl) urea;
*N*-(5-*tert*-butyl-3-isoxazolyl)-*N'*-(4-(4-(2-methylcarbamoyl)pyridyl)-thiophenyl) urea;
*N*-(5-*tert*-butyl-3-isoxazolyl)-*N'*-(3-chloro-4-(4-(2-methylcarbamoyl)pyridyl)-oxyphenyl) urea;
*N*-(5-*tert*-butyl-3-isoxazolyl)-*N'*-(4-(3-methylcarbamoyl)phenyl)oxyphenyl) urea;
and pharmaceutically acceptable salts thereof.

12. The use of claim 10, wherein R¹ is t-butyl.

13. The use of claim 1, wherein the compound has the formula 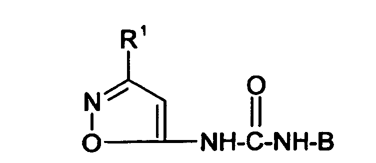 wherein R¹ and B are as defined in claim 1.

14. The use of claim 13, wherein B is of the formula 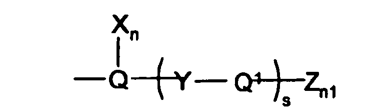 Q is is phenyl or pyridinyl optionally substituted by halogen up to per-halosubstitution, Q¹ is phenyl, benzothiazolyl or pyridinyl optionally substituted by halogen up to per-halosubstitution, Y is -O-, -S- or -CH₂-, X is C₁-C₄ alkyl or up to per-halosubstituted C₁-C₄ alkyl, Z is as defined in claim 1, n = 0 or 1, s =1, and n1 = 0 or 1.

15. The use of claim 1, wherein the compound is selected from the group consisting of
*N*-(3-Isopropyl-5-isoxazolyl)-*N'*-(4-(4-pyridinyl)thiophenyl)urea;
*N*-(3-*tert*-Butyl-5-isoxazolyl)-*N'*-(4-(4-methoxyphenyl)oxyphenyl)urea;
*N*-(3-*tert*-Butyl-5-isoxazolyl)-*N'*-(5-(2-(4-acetylphenyl)oxy)pyridinyl)urea;
*N*-(3-*tert*-Butyl-5-isoxazolyl)-*N'*-(3-(4-pyridinyl)thiophenyl)urea;
*N*-(3-*tert*-Butyl-5-isoxazolyl)-*N'*-(4-(4-pyridinyl)methylphenyl)urea;
*N*-(3-*tert*-Butyl-5-isoxazolyl)-*N'*-(4-(4-pyridinyl)thiophenyl)urea;
*N*-(3-*tert*-Butyl-5-isoxazolyl)-*N'*-(4-(4-pyridinyl)oxyphenyl)urea;
*N*-(3-*tert*-Butyl-5-isoxazolyl)-*N'*-(4-(4-methyl-3-pyridinyl)oxyphenyl)urea;
*N*-(3-*tert*-Butyl-5-isoxazolyl)-*N'*-(3-(2-benzothiazolyl)oxyphenyl)urea;
*N*-(3-(1,1-Dimethylpropyl)-5-isoxazolyl)-*N'*-(4-(4-methylphenyl)oxyphenyl)urea;
*N*-(3-(1,1-Dimethylpropyl)-5-isoxazolyl)-*N'*-(3-(4-pyridinyl)thiophenyl)urea;
*N*-(3-(1,1-Dimethylpropyl)-5-isoxazolyl)-*N'*-(4-(4-pyridinyl)oxyphenyl)urea;
*N*-(3-(1,1-Dimethylpropyl)-5-isoxazolyl)-*N'*-(4-(4-pyridinyl)thiophenyl)urea;
*N*-(3-(1,1-Dimethylpropyl-5-isoxazolyl)-*N'*-(5-(2-(4-methoxyphenyl)oxy)pyridinyl)urea;
*N*-(3-(1-Methyl-1-ethylpropyl)-5-isoxazolyl)-*N'*-(4-(4-pyridinyl)oxyphenyl)urea;
*N*-(3-(1-Methyl-1-ethylpropyl)-5-isoxazolyl)-*N'*-(3-(4-pyridinyl)thiophenyl)urea;
*N*-(3-isopropyl-5-isoxazolyl)-*N'*-(3-(4-(2-methylcarbamoyl)pyridyl)-oxyphenyl) urea;
*N*-(3-isopropyl-5-isoxazolyl)-*N'*-(4-(4-(2-methylcarbamoyl)pyridyl)-oxyphenyl) urea;
*N*-(3-*tert*-butyl-5-isoxazolyl)-*N'*-(3-(4-(2-methylcarbamoyl)pyridyl)-oxyphenyl) urea;
*N*-(3-*tert*-butyl-5-isoxazolyl)-*N'*-(4-(4-(2-methylcarbamoyl)pyridyl)-oxyphenyl) urea;
*N*-(3-*tert*-butyl-5-isoxazolyl)-*N'*-(3-(4-(2-methylcarbamoyl)pyridyl)-thiophenyl) urea;
*N*-(3-(1,1-dimethylprop-1-yl)-5-isoxazolyl)-*N'*-(3-(4-(2-methylcarbamoyl)pyridyl)-oxyphenyl) urea;
*N*-(3-(1,1-dimethylprop-1-yl)-5-isoxazolyl)-*N'*-(4-(4-(2-methylcarbamoyl)pyridyl)-oxyphenyl) urea
*N*-(3-*tert*-butyl-5-isoxazolyl)-*N'*-(3-chloro-4-(4-(2-methylcarbamoyl)pyridyl)-thiophenyl) urea
and pharmaceutically acceptable salts thereof.

16. The use of claim 13, wherein R¹ is t-butyl.

17. The use of claim 1, wherein the compound has the formula 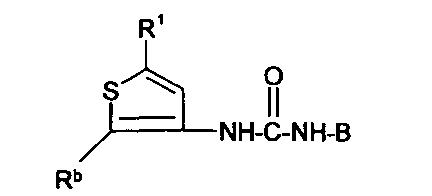 wherein R¹, R^{b} and B are as defined in claim 1.

18. The use of claim 17, wherein B is of the formula 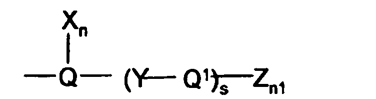 wherein Q is phenyl optionally substituted by halogen up to per-halosubstitution, Q¹ is phenyl or pyridinyl optionally substituted by halogen up to per-halosubstitution, Y is -O- or -S-, X is C₁-C₄ alkyl or up to per-halosubstituted C₁-C₄ alkyl, Z is as defined in claim 1, n = 0 or 1, s = 0 or 1 and n1 = 0-2.

19. The use of claim 1, wherein the compound is selected from the group consisting of:
*N*-(5-*tert*-Butyl-3-thienyl)-*N'*-(4-(3-methylphenyl)oxyphenyl)urea;
*N*-(5-*tert*-Butyl-3-thienyl)-*N'*-(4-(4-hydroxyphenyl)oxyphenyl)urea;
*N*-(5-*tert*-Butyl-3-thienyl)-*N'*-(4-(4-methoxyphenyl)oxyphenyl)urea;
*N*-(5-*tert*-Butyl-3-thienyl)-*N'*-(4-(4-pyridinyl)thiophenyl)urea;
and pharmaceutically acceptable salts thereof.

20. The use of claim 17, wherein R¹ is t-butyl.

21. The use of claim 1, wherein the compound has the formula 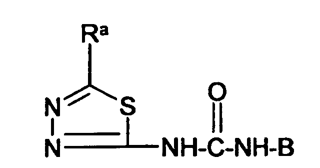 wherein R^{a} and B are as defined in claim 1.

22. The use of claim 21, wherein B is of the formula 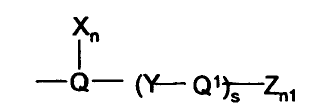 wherein Q is phenyl optionally substituted by halogen up to per-halosubstitution, Q¹ is phenyl or pyridinyl optionally substituted by halogen up to per-halosubstitution, Y is -O- or -S-, X is C₁-C₄ alkyl or up to per-halosubstituted C₁-C₄ alkyl, s = 1; Z is as defined in claim 1, n = 0 or 1 and n1 = 0 or 1.

23. The use of claim 2, wherein the compound is selected from the group consisting of:
*N*-(5-*tert*-Butyl-2-(1-thia-3,4-diazolyl))-*N*'-(3-(4-pyridinyl)thiophenyl)urea;
*N*-(5-*tert*-Butyl-2-(1-thia-3,4-diazolyl))-*N*'-(4-(4-pyridinyl)oxyphenyl)urea;
and pharmaceutically acceptable salts thereof.

24. The use of claim 21, wherein R^{a} is CF₃- or t-butyl.

25. The use of claim 1, wherein the compound is of one of the formulae 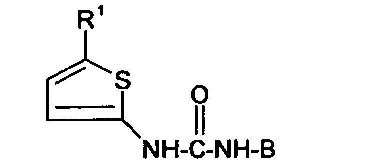 or 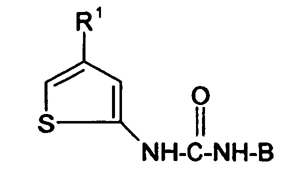 wherein R¹ and B are as defined in claim 1.

26. The use of claim 25, wherein B is up to per-halosubstituted phenyl, up to perhalosubstituted pyridinyl, or of the formula 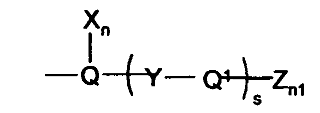 wherein Q is phenyl optionally substituted by halogen up to per-halosubstitution, Q¹ is phenyl or pyridinyl optionally substituted by halogen up to per-halosubstitution, and Y is -O- or -S-, X is C₁-C₄ alkyl or up to per-halosubstituted C₁-C₄ alkyl, Z is as defined in claim 1, n = 0 or 1, s = 0 or 1 and n1 = 0-2.

27. The use of claim 25, wherein R¹ is t-butyl.

28. The use of claim 1, wherein the a compound is of the formula 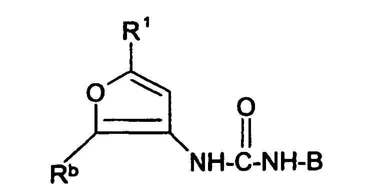 wherein R¹ and R^{b} and B are as defined in claim 1.

29. The use of claim 28, wherein B is of the formula 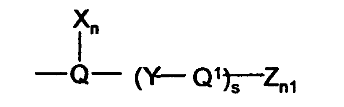 wherein Q is phenyl optionally substituted by halogen up to per-halosubstitution, Q¹ is phenyl or pyridinyl optionally substituted by halogen up to per-halosubstitution, and Y is -O- or -S-, X is C₁-C₄ alkyl or up to per-halosubstituted C₁-C₄ alkyl, Z is as defined in claim 1,n = 0 or 1, s = 0 or 1 and n1 = 0-2.

30. The use of claim 28, wherein R¹ is t-butyl.

31. A compound of the formula 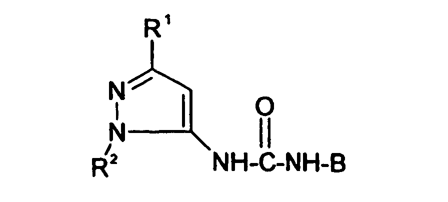 wherein R² is selected from the group consisting of H, -C(O)R⁴, -CO₂R⁴, -C(O)NR³R^{3'}, C₁-C₁₀ alkyl, C₃-C₁₀ cycloalkyl, substituted C₁-C₁₀ alkyl, substituted C₃-C₁₀ cycloalkyl, where if R² is a substituted group, it is substituted by one or more substituents independently selected from the group consisting of -CN,
-CO₂R⁴, -C(O)-NR³R^{3'}, -NO₂, -OR⁴, -SR⁴, and halogen up to per-halosubstitution,
wherein R³ and R^{3'} are independently selected from the group consisting of H, C₁-C₁₀ alkyl, C₃-C₁₀ cycloalkyl, up to per-halosubstituted C₁-C₁₀ alkyl, and up to per-halosubstituted C₃-C₁₀ cycloalkyl, and
wherein R⁴ and R^{4'} are independently selected from the group consisting of H, C₁-C₁₀ alkyl, C₃-C₁₀ cycloalkyl, up to per-halosubstituted C₁-C₁₀ alkyl, and up to per-halosubstituted C₃-C₁₀ cycloalkyl,
wherein R¹ is selected from the group consisting of C₃-C₆ alkyl, C₃-C₆ cycloalkyl, up to per-halosubstituted C₃-C₆ alkyl and up to per-halosubstituted C₃-C₆ cycloalkyl,
B is phenyl, pyridinyl, indolinyl, isoquinolinyl, quinolinyl, or napthyl;
substituted by C6-C14 aryl, C₃-C₁₃ heteroaryl, or -Y-Ar,
wherein the cyclic structures of B are optionally substituted by halogen, up to per halo, and optionally substituted by X¹ₙ
and wherein
n = 0-2; each X¹ is independently selected from the group of -CN, -CO₂R⁵, -C(O)R⁵, -C(O)NR⁵R^{5'}, -OR⁵, -NO₂, -NR⁵R^{5'}, C₁-C₁₀ alkyl, C₂₋₁₀-alkenyl, C₁₋₁₀-alkoxy, C₃-C₁₀ cycloalkyl, -SR⁵, -NR⁵C(O)OR^{5'}, NR⁵C(O)R^{5'}, substituted C₁-C₁₀ alkyl, substituted C₂₋₁₀-alkenyl, substituted C₁₋₁₀-alkoxy, substituted C₃-C₁₀ cycloalkyl,
wherein if X¹ is a substituted group, it is substituted by one or more substituents independently selected from the group consisting of -CN, -CO₂R⁵,
-C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl; up to per-halosubstituted C₂₋₁₀-alkenyl, and up to per-halosubstituted C₃-C₁₀ cycloalkyl, wherein Y is -O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur which is optionally by halogen up to per-halo and optionally substituted by Zₙ₁, wherein n1 is 0 to 3 and each Z is independently selected from the group consisting of -CN, -CO₂R⁵, -C(O)R⁵, =O,
-C(O)NR⁵R^{5'}, -C(O)R⁵, -NO₂, -OR⁵, - SR⁵, - NR⁵R^{5'}, -NR⁵C(O)OR^{5'}, NR⁵C(O)R^{5'}, -SO₂R⁵, -SO₂R⁵ R^{5'} C₁-C₁₀ alkyl, C₁-C₁₀ alkoxy, C₃-C₁₀ cycloalkyl, substituted C₁-C₁₀ alkyl, and substituted C₃-C₁₀ cycloalkyl, wherein if Z is a substituted group, it is substituted by one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀ alkyl, C₁-C₁₀ alkoxyl, and C₃-C₁₀ cycloalkyl.

32. A compound of claim 31, wherein B is 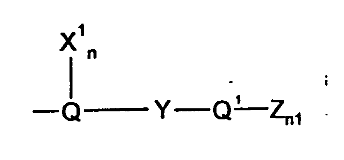 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O-, and -OCH₂-,
X^{a} is halogen,
Q is phenyl or pyridinyl substituted or unsubstituted by halogen, up to per-halosubstitution;
Q¹ is a mono- or bicyclic aromatic structure of 3 to 10 carbon atoms and 0-4 members of the group consisting of N, O and S, optionally substituted by halogen up to per-halosubstitution.
X¹ is C₁-C₄ alkyl or halosubstituted C₁-C₄ alkyl up to per halo,
Z, n and n1 are as defined in claim 31.

33. A compound of claim 32, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, optionally substituted by halogen, up to per-halo, and X¹ is as defined in claim 32, and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl, wherein R⁶ and R⁷ can be substituted by halogen or up to per-halosubstitution.

34. A compound of claim 32, wherein Q is phenyl or pyridinyl optionally substituted by halogen up to per-halosubstitution, Q¹ is pyridinyl, phenyl or benzothiazolyl optionally substituted by halogen up to per-halosubstitution, Y is -O-, -S-, -CH₂S-, -SCH₂-, -CH₂O-, -OCH₂- or -CH₂-, X¹ is as defined in claim 32, and Z is -SCH₃, or -NH-C(O)-CₚH₂ₚ₊₁, wherein p is 1-4, n = 0 or 1 and n1 = 0-1 .

35. A compound of the formula 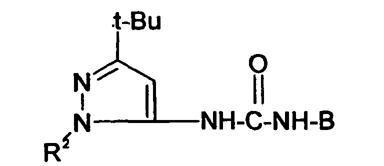 wherein R² is as defined in claim 31 and B is phenyl, pyridinyl, pyrimidinyl, pyrazinyl, pyridazinyl, naphthyl, quinolinyl, isoquinolinyl, phthalimidinyl, furyl, thienyl, pyrrolyl, imidazolyl, pyrazolyl, oxazolyl, isoxazolyl, thiazolyl, isothiazolyl, benzofuryl, benzothienyl, indolyl, benzopyrazolyl, benzoxazolyl, benzisoxazolyl, benzothiazolyl or benzisothiazolyl, substituted by one or more substituents independently selected from the group consisting of halogen, up to per-halosubstitution, and Xₙ, wherein n is 0-3 and each X is independently selected from the group consisting of -CN, -CO₂R⁵, -C(O)NR⁵R^{5'}, -C(O)R⁵, -NO₂, -OR⁵, - SR⁵, -NR⁵R^{5'}, -NR⁵C(O)OR^{5'}, -NR⁵C(O)R^{5'}, C₁-C₁₀ alkyl, C₂-C₁₀ alkenyl, C₁-C₁₀ alkoxy, C₃-C₁₀ cycloalkyl, **C**_{**6**}**-C**_{**14**} **aryl, C**_{**3**}**-C**_{**13**} **heteroaryl,** up to per halo-substituted C₁-C₁₀ alkyl, up to per halo-substituted C₂-C₁₀ alkenyl, up to per halo-substituted C₁-C₁₀ alkoxy, up to per halo-substituted C₃-C₁₀ cycloalkyl, and -Y-Ar;
wherein R⁵ and R^{5'} are independently selected from H, C₁-C₁₀ alkyl, C₂-C₁₀ alkenyl, C₃-C₁₀ cycloalkyl, C₆-C₁₄ aryl, C₃-C₁₃ heteroaryl, up to per-halosubstituted C₁-C₁₀ alkyl, up to per-halosubstituted C₂-C₁₀ alkenyl, up to per-halosubstituted C₃-C₁₀ cycloallcyl, and up to per-halosubstituted C₆-C₁₄ aryl, and C₃-C₁₃ heteroaryl,
wherein Y is - O-, -S-, -N(R⁵)-, -(CH₂)-ₘ, -C(O)-, -CH(OH)-, -(CH₂)ₘO-, -NR⁵C(O)NR⁵ NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur optionally substituted by Zₙ₁, wherein n1 is 0 to 3 and each Z is independently selected from the group consisting of -CN, =O, -CO₂R⁵, -C(O)NR⁵R^{5'}, -C(O)- NR⁵, -NO₂, -OR⁵, - SR⁵, -NR⁵R^{5'}, -NR⁵C(O)OR^{5'}, -C(O)R⁵, -NR⁵C(O)R^{5'}, -SO₂R⁵, SO₂NR⁵R^{5'}, C₁-C₁₀ alkyl, C₁-C₁₀ alkoxyl, C₃-C₁₀ cycloalkyl, up to per halo-substituted C₁-C₁₀ alkyl, and up to per halo-substituted C₃-C₁₀ cycloalkyl, subject to the proviso that where R^{a} is methyl
B is not

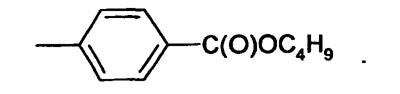

36. A compound as in claim 31 selected from the group consisting of:
*N*-(3-*tert*-Butyl-5-pyrazolyl)-*N'*-(4-phenyloxyphenyl)urea;
*N*-(3-*tert*-Butyl-5-pyrazolyl)-*N'*-(3-(3-methylaminocarbonylphenyl)oxyphenyl)urea;
*N*-(3-*tert*-Butyl-5-pyrazolyl)-*N'*-(3-(4-pyridinyl)thiophenyl)urea;
*N*-(3-*tert*-Butyl-5-pyrazolyl)-*N'*-(4-(4-pyridinyl)thiophenyl)urea;
*N*-(3-*tert*-Butyl-5-pyrazolyl)-*N'*-(4-(4-pyridinyl)oxyphenyl)urea;
*N*-(3-*tert*-Butyl-5-pyrazolyl)-*N'*-(4-(4-pyridinyl)methylphenyl)urea;
*N*-(1-Methyl-3-*tert*-butyl-5-pyrazolyl)-*N'*-(4-phenyloxyphenyl)urea;
*N*-(1-Methyl-3-*tert*-butyl-5-pyrazolyl)-*N'*-(3-(4-pyridinyl)thiophenyl)urea;
*N*-(1-Methyl-3-*tert*-butyl-5-pyrazolyl)-*N'*-((4-(4-pyridinyl)thiomethyl)phenyl)urea;
*N*-(1-Methyl-3-*tert*-butyl-5-pyrazolyl)-*N'*-(4-(4-pyridinyl)thiophenyl)urea;
*N*-(1-Methyl-3-*tert*-butyl-5-pyrazolyl)-*N'*-(4-(4-pyridinyl)oxyphenyl)urea;
*N*-(1-Methyl-3-*tert*-butyl-5-pyrazolyl)-*N'*-((4-(4-pyridinyl)methyloxy)phenyl)urea;
*N*-(1-Methyl-3-*tert*-butyl-5-pyrazolyl)-*N'*-(3-(2-benzothiazolyl)oxyphenyl)urea;
*N*-(3-*tert*-butyl-5-pyrazolyl)-*N'*-(3-(4-pyridyl)thiophenyl) urea;
*N*-(3-*tert*-butyl-5-pyrazolyl)-*N'*-(4-(4-pyridyl)thiophenyl) urea;
*N*-(3-*tert*-butyl-5-pyrazolyl)-*N'-*(3-(4-pyridyl)oxyphenyl) urea;
*N*-(3-*tert*-butyl-5-pyrazolyl)-*N'*-(4-(4-pyridyl)oxyphenyl) urea;
*N*-(1-methyl-3-*tert*-butyl-5-pyrazolyl)-*N'*-(3-(4-pyridyl)thiophenyl) urea;
*N*-(1-methyl-3-*tert*-butyl-5-pyrazolyl)-*N'*-(4-(4-pyridyl)thiophenyl) urea;
*N*-(1-methyl-3-*tert*-butyl-5-pyrazolyl)-*N'*-(3-(4-pyridyl)oxyphenyl) urea;
*N*-(1-methyl-3-*tert*-butyl-5-pyrazolyl)-*N'*-(4-(4-pyridyl)oxyphenyl) urea;
and pharmaceutically acceptable salts thereof.

37. A compound of the formula 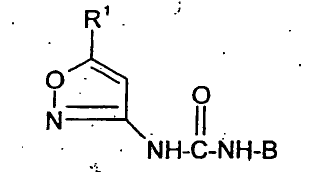 wherein R¹ is selected from the group consisting of C₃-C₁₀ alkyl, C₃-C₁₀ cycloalkyl, up to per-halosubstituted C₃-C₁₀ alkyl and up to per-halosubstituted C₃-C₁₀ cycloalkyl
B is phenyl, pyridinyl, indolinyl, isoquinolinyl, qinolinyl or napthyl
which is
substituted by phenyl, pyridinyl or Y-Ar,
wherein the cyclic structures of B are optionally substituted by halogen, up to per-halosubstitution, and
optionally substituted by X¹ₙ wherein n = 0-2;
each X¹ is independently selected from the group consisting of-CN, -CO₂R⁵, -C(O)R⁵, -C(O)NR⁵R^{5'}, -OR⁵, - NO₂, -NR⁵R^{5'}, C₁-C₁₀ alkyl, C₂₋₁₀-alkenyl, C₁₋₁₀-alkoxy, C₃-C₁₀ cycloalkyl, and C₆-C₁₄ aryl.
-SR⁵, -NR⁵C(O)OR^{5'}, NR⁵C(O)R^{5'}, C₃-C₁₃ heteroaryl, substituted C₁-C₁₀ alkyl, substituted C₂₋₁₀-alkenyl, substituted C₁₋₁₀-alkoxy, substituted C₃-C₁₀ cycloalkyl, substituted C₆-C₁₄ aryl, substituted C₃-C₁₃ heteroaryl, and
wherein if X¹ is a substituted group, it is substituted by one or more substituents independently selected from the group consisting of -CN, -CO₂R⁵,
-C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂₋₁₀-alkenyl, and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur which is unsubstituted or substituted by halogen up to per-halo and optionally substituted by Zₙ₁, wherein n1 is 0 to 3 and each Z is independently selected from the group consisting of -CN, -CO₂R⁵, -C(O)R⁵, =O, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, -SO₂R⁵, -SO₂ R⁵ R^{5'}, C₁-C₁₀ alkyl, C₁-C₁₀ alkoxy, C₃-C₁₀ cycloalkyl, substituted C₁-C₁₀ alkyl, and substituted C₃-C₁₀ cycloalkyl, wherein if Z is a substituted group, it is substituted by one or more substituents independently selected from the group consisting of -CN, -CO₂R⁵, -C(O)NR⁵R^{5'}, =O, -OR⁵, -SR⁵, -NO₂, -NR⁵R^{5'} , -NR⁵C(O)R^{5'}, -NR⁵C(O)OR^{5'} , C₁-C₁₀ alkyl, C₁-C₁₀ alkoxyl, and C₃-C₁₀ cycloalkyl, subject to the proviso that where R¹ is t-butyl,
B is not 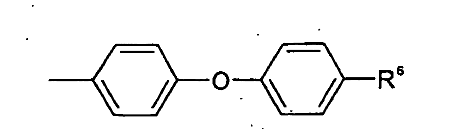 wherein R⁶ is -NHC(O)-O-t-butyl, -O-n-pentyl, -O-n-butyl, -O-n-propyl, -C(O)NH-(CH₃)₂,-OCH₂CH(CH₃)₂, or

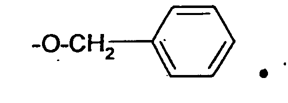

38. A compound of claim 37, wherein B is 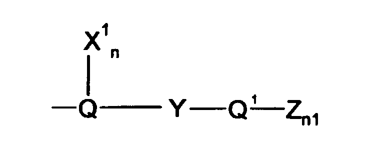 Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂-,
X^{a} is halogen,
Q is phenyl or pyridinyl substituted or unsubstituted by halogen, up to per-halosubstitution;
Q¹ is a mono- or bicyclic aromatic structure of 3 to 10 carbon atoms and 0-4 members of the group consisting of N, O and S substituted or unsubstituted by halogen up to per-halosubstitution, X¹ is C₁-C₄ alkyl or halosubstituted C₁-C₄ alkyl up to per halo, and
Z, n and n1 are as defined in claim 37.

39. A compound of claim 38, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, optionally substituted by halogen, up to per-halo, X¹ is as defined in claim 38 and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl, wherein R⁶ and R⁷ can be substituted by halogen or up to per-halosubstitution.

40. A compound of claim 38, wherein Q is phenyl or pyridinyl optionally substituted by halogen up to per-halosubstitution, Q¹ is pyridinyl, phenyl or benzothiazolyl optionally substituted by halogen up to per-halosubstitution, Y is -O-, -S-, -C(O)- or -CH₂-, X¹ is as defined in claim 38 and Z is-NH-C(O)-CₚH₂ₚ₊₁, wherein p is 1-4, -CH₃, -OH, -OCH₃, -OC₂H₅, -CN or -C(O)CH₃, n = 0 or 1, and n1 = 0 or 1.

41. A compound as in claim 37 selected from the group consisting of:
*N*-(5-*tert*-Butyl-3-isoxazolyl)-*N'*-(4-(4-hydroxyphenyl)oxyphenyl)urea;
*N*-(5-*tert*-Butyl-3-isoxazolyl)-*N'*-(4-(3-hydroxyphenyl)oxyphenyl)urea;
*N*-(5-*tert*-Butyl-3-isoxazolyl)-*N'*-(4-(4-acetylphenyl)oxyphenyl)urea;
*N*-(5-*tert*-Butyl-3-isoxazolyl)-*N'*-(3-benzoylphenyl)urea;
*N*-(5-*tert*-Butyl-3-isoxazolyl)-*N'*-(4-phenyloxyphenyl)urea;
*N*-(5-*tert*-Butyl-3-isoxazolyl)-*N'*-(4-(3-methylaminocarbonylphenyl)-thiophenyl)urea;
*N*-(5-*tert*-Butyl-3-isoxazolyl)-*N'*-(4-(4-(1,2-methylenedioxy)phenyl)-oxyphenyl)urea;
*N*-(5-*tert*-Butyl-3-isoxazolyl)-*N'*-(4-(3-pyridinyl)oxyphenyl)urea;
*N*-(5-*tert*-Butyl-3-isoxazolyl)-*N'*-(4-(4-pyridinyl)oxyphenyl)urea;
*N*-(5-*tert*-Butyl-3-isoxazolyl)-*N'*-(4-(4-pyridyl)thiophenyl)urea;
*N*-(5-*tert*-Butyl-3-isoxazolyl)-*N'*-(4-(4-pyridinyl)methylphenyl)urea;
*N*-(5-*tert*-Butyl-3-isoxazolyl)-*N'*-(3-(4-pyridinyl)oxyphenyl)urea;
*N*-(5-*tert*-Butyl-3-isoxazolyl)-*N'*-(3-(4-pyridinyl)thiophenyl)urea;
*N*-(5-*tert*-Butyl-3-isoxazolyl)-*N'*-(3-(3-methyl-4-pyridinyl)oxyphenyl)urea;
*N*-(5-*tert*-Butyl-3-isoxazolyl)-*N'*-(3-(3-methyl-4-pyridinyl)thiophenyl)urea;
*N*-(5-*tert*-Butyl-3-isoxazolyl)-*N'*-(4-(3-methyl-4-pyridinyl)thiophenyl)urea;
*N*-(5-*tert*-Butyl-3-isoxazolyl)-*N'*-(3-(4-methyl-3-pyridinyl)oxyphenyl)urea;
*N*-(5-*tert*-Butyl-3-isoxazolyl)-*N'*-(4-(3-methyl-4-pyridinyl)oxyphenyl)urea;
*N*-(5-*tert*-Butyl-3-isoxazolyl)-*N'*-(3-(2-benzothiazolyl)oxyphenyl)urea;
*N*-(5-*tert*-butyl-3-isoxazolyl)-*N'*-(3-chloro-4-(4-(2-methylcarbamoyl)pyridyl)-oxyphenyl) urea;
*N*-(5-*tert*-butyl-3-isoxazolyl)-*N'*-(4-(4-(2-methylcarbamoyl)pyridyl)-oxyphenyl) urea;
*N*-(5-*tert*-butyl-3-isoxazolyl)-*N'*-(3-(4-(2-methylcarbamoyl)pyridyl)-thiophenyl) urea;
*N*-(5-*tert*-butyl-3-isoxazolyl)-*N'*-(2-methyl-4-(4-(2-methylcarbamoyl)pyridyl)-oxyphenyl) urea;
*N*-(5-*tert*-butyl-3-isoxazolyl)-*N'*-(4-(4-(2-carbamoyl)pyridyl)oxyphenyl) urea;
*N*-(5-*tert*-butyl-3-isoxazolyl)-*N'*-(3-(4-(2-carbamoyl)pyridyl)oxyphenyl) urea;
*N*-(5-*tert*-butyl-3-isoxazolyl)-*N'*-(3-(4-(2-methylcarbamoyl)pyridyl)-oxyphenyl) urea;
*N*-(5-*tert*-butyl-3-isoxazolyl)-*N'*-(4-(4-(2-methylcarbamoyl)pyridyl)-thiophenyl) urea;
*N-*(5-*tert*-butyl-3-isoxazolyl)-*N'*-(3-chloro-4-(4-(2-methylcarbamoyl)pyridyl)-oxyphenyl) urea;
*N*-(5-*tert*-butyl-3-isoxazolyl)-*N'*-(4-(3-methylcarbamoyl)phenyl)oxyphenyl) urea;
and pharmaceutically acceptable salts thereof.

42. A compound of the formula 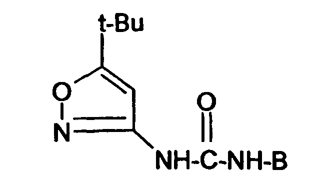 wherein B is 5-methyl-2-thienyl or
selected from the group consisting of phenyl, pyridinyl, pyrimidinyl, pyrazinyl, pyridazinyl, naphthyl, quinolinyl, isoquinolinyl, phthalimidinyl, furyl, thienyl, pyrrolyl, imidazolyl, pyrazolyl, oxazolyl, isoxazolyl, thiazolyl, isothiazolyl, benzofuryl, benzothienyl, indolyl, benzopyrazolyl, benzoxazolyl, benzisoxazolyl, benzothiazolyl or benzisothiazolyl, substituted by one or more substituents independently selected from the group consisting of halogen, up to per-halosubstitution, and Xₙ, wherein n is 0-3 and each X is independently selected from the group consisting of -CN, -CO₂R⁵, -C(O)NR⁵R^{5'}, -C(O)R⁵, -NO₂, -OR⁵, - SR⁵, -NR⁵R^{5'}, -NR⁵C(O)OR^{5'}, -NR⁵C(O)R^{5'}, C₁-C₁₀ alkyl, C₂-C₁₀ alkenyl, C₁-C₁₀ alkoxy, C₃-C₁₀ cycloalkyl, **C**_{**6**}**-C**_{**14**} **aryl, C**_{**3**}**-C**_{**13**} **heteroaryl,** up to per halo-substituted C₁-C₁₀ alkyl, up to per halo-substituted C₂-C₁₀ alkenyl, up to per halo-substituted C₁-C₁₀ alkoxy and, up to per halo-substituted C₃-C₁₀ cycloalkyl,
wherein R⁵ and R^{5'} are independently selected from H, C₁-C₁₀ alkyl, C₂-C₁₀ alkenyl, C₃-C₁₀ cycloalkyl, C₆-C₁₄ aryl, C₃-C₁₃ heteroaryl, up to per-halosubstituted C₁-C₁₀ alkyl, up to per-halosubstituted C₂-C₁₀ alkenyl, and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵ NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur optionally substituted by halogen up to per-halosubstitution and optionally substituted by Zₙ₁, wherein n1 is 0 to 3 and each Z is independently selected from the group consisting of -CN, =O, -CO₂R⁵, -C(O)NR⁵R^{5'}, -C(O)- NR⁵, -NO₂, -OR⁵, - SR⁵, -NR⁵R^{5'}, -NR⁵C(O)OR^{5'}, -C(O)R⁵, -NR⁵C(O)R^{5'}, -SO₂R⁵, SO₂NR⁵R^{5'}, C₁-C₁₀ alkyl, C₁-C₁₀ alkoxyl, C₃-C₁₀ cycloalkyl, up to per halo-substituted C₁-C₁₀ alkyl and up to per halo-substituted C₃-C₁₀ cycloalkyl; subject to the proviso that B is not 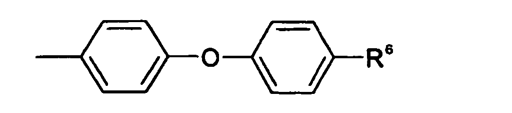 wherein R⁶ is -NHC(O)-O-t-butyl, -O-n-pentyl, -O-n-butyl, -O-n-propyl, -C(O)NH-(CH₃)₂, -OCH₂CH(CH₃)₂, or

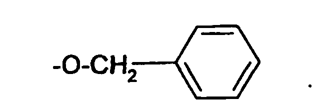

43. A compound of the formula 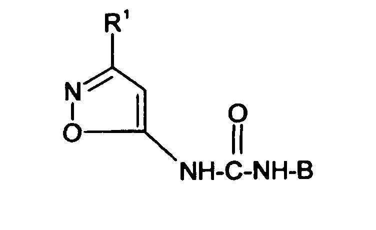 wherein R¹ is selected from the group consisting of C₃-C₁₀ alkyl, C₃-C₁₀ cycloalkyl, up to per-halosubstituted C₃-C₁₀ alkyl, and up to per-halosubstituted C₃-C₁₀ cycloalkyl, and
B is phenyl, pyridinyl, indolinyl, isoquindinyl, quinolinyl or napthyl
which is substituted by X, optionally substituted by halogen, up to per-halosubstitution, and optionally substituted by X¹ₙ, wherein n = 0-2;
each X¹ is independently selected from the group of X or from the group consisting of -CN, -CO₂R⁵, -C(O)R⁵, -C(O)NR⁵R^{5'}, -OR⁵, - NO₂, -NR⁵R^{5'}, C₁-C₁₀ alkyl, C₂₋₁₀-alkenyl, C₁₋₁₀-alkoxy, C₃-C₁₀ cycloalkyl, C₆-C₁₄ aryl and C₇-C₂₄ alkaryl, and
X is selected from the group consisting of -SR⁵, -NR⁵C(O)OR^{5'}, NR⁵C(O)R^{5'}, C₃-C₁₃ heteroaryl, substituted C₁-C₁₀ alkyl, substituted C₂₋₁₀-alkenyl, substituted C₁₋₁₀-alkoxy, substituted C₃-C₁₀ cycloalkyl, substituted C₆-C₁₄ aryl, substituted C₃-C₁₃ heteroaryl, and -Y-Ar, and wherein if X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂₋₁₀-alkenyl, and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur, which is unsubstituted or substituted by halogen up to per-halo and optionally substituted by Zₙ₁, wherein n1 is 0 to 3 and each Z is independently selected from the group consisting of -CN, -CO₂R⁵, -C(O)R⁵, =O, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, -SO₂R⁵, -SO₂ R⁵R^{5'}, C₁-C₁₀ alkyl, C₁-C₁₀ alkoxy, C₃-C₁₀ cycloalkyl, substituted C₁-C₁₀ alkyl, and substituted C₃-C₁₀ cycloalkyl, wherein if Z is a substituted group, it is substituted by one or more substituents independently selected from the group consisting of -CN, -CO₂R⁵, -C(O)NR⁵R^{5'}, =O, -OR⁵, -SR⁵, -NO₂, -NR⁵R^{5'}, NR⁵C(O)R^{5'} and -NR⁵C(O)OR^{5'}, C₁-C₁₀ alkyl, C₁-C₁₀ alkoxyl, and C₃-C₁₀ cycloalkyl,
and where R¹ is -CH₂-t-butyl,
B is not

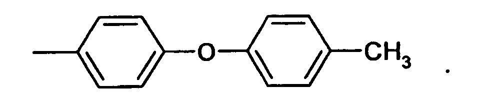

44. A compound of claim 43, wherein B is 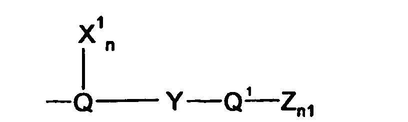 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂-,
X^{a} is halogen,
Q is phenyl or pyridinyl substituted or unsubstituted by halogen, up to per-halosubstitution;
Q¹ is a mono- or bicyclic aromatic structure of 3 to 10 carbon atoms and 0-4 members of the group consisting of N, O and S substituted or unsubstituted by halogen up to per-halosubstitution,
Z, n and n1 are as defined in claim 43 and X¹ is C₁-C₄ alkyl or halosubstituted C₁-C₄ alkyl up to per halo.

45. A compound of claim 44, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, optionally substituted by halogen, up to per-halo, X¹ is as defined in claim 44 and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl wherein R⁶ and R⁷ can be substituted by halogen or up to per-halosubstitution.

46. A compound of the formula 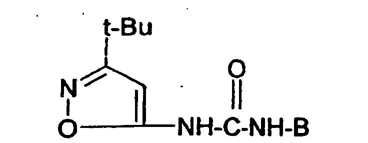 wherein B is as defined in claim 1.

47. A compound of claim 44, wherein Q is is phenyl or pyridinyl optionally substituted by halogen up to per-halosubstitution, Q¹ is phenyl, benzothiazolyl or pyridinyl optionally substituted by halogen up to per-halosubstitution, Y is -O-, -S- or -CH₂-, X¹ is as defined in claim 44, n = 0 or 1, Z is -CH₃, -Cl-, OC₂H₅ or -OCH₃, and n1 = 0 or 1.

48. A compound as in claim 43 selected from the group consisting of:
*N*-(3-Isopropyl-5-isoxazolyl)-*N'*-(4-(4-pyridinyl)thiophenyl)urea;
*N*-(3-*tert*-Butyl-5-isoxazolyl)-*N'*-(4-(4-methoxyphenyl)oxyphenyl)urea;
*N*-(3-*tert*-Butyl-5-isoxazolyl)-*N'*-(5-(2-(4-acetylphenyl)oxy)pyridinyl)urea;
*N*-(3-*tert*-Butyl-5-isoxazolyl)-*N'*-(3-(4-pyridinyl)thiophenyl)urea;
*N*-(3-*tert*-Butyl-5-isoxazolyl)-*N'*-(4-(4-pyridinyl)methylphenyl)urea;
*N*-(3-*tert*-Butyl-5-isoxazolyl)-*N'*-(4-(4-pyridinyl)thiophenyl)urea;
*N*-(3-*tert*-Butyl-5-isoxazolyl)-*N'*-(4-(4-pyridinyl)oxyphenyl)urea;
*N*-(3-*tert*-Butyl-5-isoxazolyl)-*N'*-(4-(4-methyl-3-pyridinyl)oxyphenyl)urea;
*N*-(3-*tert*-Butyl-5-isoxazolyl)-*N'*-(3-(2-benzothiazolyl)oxyphenyl)urea;
*N*-(3-(1,1-Dimethylpropyl)-5-isoxazolyl)-*N'*-(4-(4-methylphenyl)oxyphenyl)urea;
*N*-(3-(1,1-Dimethylpropyl)-5-isoxazolyl)-*N'*-(3-(4-pyridinyl)thiophenyl)urea;
*N*-(3-(1,1-Dimethylpropyl)-5-isoxazolyl)-*N'*-(4-(4-pyridinyl)oxyphenyl)urea;
*N*-(3-(1,1-Dimethylpropyl)-5-isoxazolyl)-*N'*-(4-(4-pyridinyl)thiophenyl)urea;
*N*-(3-(1,1-Dimethylpropyl-5-isoxazolyl)-*N'*-(5-(2-(4-methoxyphenyl)oxy)pyridinyl)urea;
*N*-(3-(1-Methyl-1-ethylpropyl)-5-isoxazolyl)-*N'*-(4-(4-pyridinyl)oxyphenyl)urea;
*N*-(3-(1-Methyl-1-ethylpropyl)-5-isoxazolyl)-*N'*-(3-(4-pyridinyl)thiophenyl)urea;
*N*-(3-isopropyl-5-isoxazolyl)-*N'*-(3-(4-(2-methylcarbamoyl)pyridyl)-oxyphenyl) urea;
*N*-(3-isopropyl-5-isoxazolyl)-*N'*-(4-(4-(2-methylcarbamoyl)pyridyl)-oxyphenyl) urea;
*N*-(3-*tert*-butyl-5-isoxazolyl)-*N'*-(3-(4-(2-methylcarbamoyl)pyridyl)-oxyphenyl) urea;
*N*-(3-*tert*-butyl-5-isoxazolyl)-*N'*-(4-(4-(2-methylcarbamoyl)pyridyl)-oxyphenyl) urea;
*N*-(3-*tert*-butyl-5-isoxazolyl)-*N'*-(3-(4-(2-methylcarbamoyl)pyridyl)-thiophenyl) urea;
*N*-(3-(1,1-dimethylprop-1-yl)-5-isoxazolyl)-*N'*-(3-(4-(2-methylcarbamoyl)-pyridyl)oxyphenyl) urea;
*N*-(3-(1,1-dimethylprop-1-yl)-5-isoxazolyl)-*N'*-(4-(4-(2-methylcarbamoyl)-pyridyl)oxyphenyl) urea;
*N*-(3-*tert*-butyl-5-isoxazolyl)-*N'*-(3-chloro-4-(4-(2-methylcarbamoyl)pyridyl)-thiophenyl) urea;
and pharmaceutically acceptable salts thereof.

49. A compound of the formula 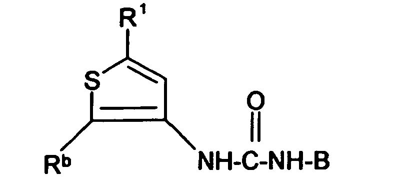 wherein R¹ is selected from the group consisting of C₃-C₁₀ alkyl, C₃-C₁₀ cycloalkyl, up to per-halosubstituted C₃-C₁₀ alkyl and up to per-halosubstituted C₃-C₁₀ cycloalkyl,
R^{b} is hydrogen or halogen and
B is phenyl, pyridinyl, indolinyl, isoquinolinyl, quinolinyl, or napthyl substituted by phenyl, pyridinyl or -Y-Ar,
wherein the cyclic structures of B are optionally substituted by halogen, up to per halo, and
optionally substituted by X¹ₙ
and
wherein n = 0-2; each X¹ is independently selected from the group consisting of CN, -OR⁵, -NR⁵R^{5'}, C₁-C₁₀ alkyl;
-CO₂R⁵, -C(O)NR⁵R^{5'}, -C(O)R⁵,
-NO₂, -SR⁵, -NR⁵C(O)OR^{5'}, -NR⁵C(O)R^{5'}, C₃-C₁₀ cycloalkyl, substituted C₁-C₁₀ alkyl, substituted C₂₋₁₀-alkenyl, substituted C₁₋₁₀-alkoxy, and substituted C₃-C₁₀ cycloalkyl,
wherein if X¹ is a substituted group, it is substituted by one or more substituents independently selected from the group consisting of -CN, -CO₂R⁵,
-C(O)R⁵, -C(O)NR⁵R^{5'}, -OR⁵, -SR⁵, -NR⁵R^{5'}, -NO₂, -NR⁵C(O)R^{5'} , -NR⁵C(O)OR^{5'} and halogen up to per-halo substitution;
wherein R⁵ and R^{5'} are independently selected from H, C₁-C₁₀ alkyl, C₂₋₁₀-alkenyl, C₃-C₁₀ cycloalkyl, up to per-halosubstituted C₁-C₁₀ alkyl, up to per-halosubstituted C₂₋₁₀-alkenyl, and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-, m = 1-3, and X^{a} is halogen; and
Ar is a 5-10 member aromatic structure containing 0-4 members of the group consisting of nitrogen, oxygen and sulfur which is unsubstituted or substituted by halogen up to per-halosubstitution and optionally substituted by Zₙ₁, wherein n1 is 0 to 3 and each Z is independently selected from the group consisting of-CN, -CO₂R⁵, -C(O)R⁵, =O, -C(O)NR⁵R^{5'}, -C(O)- NR⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀ alkyl, C₁-C₁₀ alkoxy, C₃-C₁₀ cycloalkyl, substituted C₁-C₁₀ alkyl, and substituted C₃-C₁₀ cycloalkyl, wherein if Z is a substituted group, it is substituted by the one or more substituents independently selected from the group consisting of -CN, -CO₂R⁵,
-C(O)NR⁵R^{5'}, =O, -OR⁵, -SR⁵, -NO₂, -NR⁵R^{5'}, -NR⁵C(O)R^{5'} , NR⁵C(O)OR^{5'}, C₁-C₁₀ alkyl, C₁-C₁₀ alkoxyl, and C₃-C₁₀ cycloalkyl,
subject to the proviso that where R¹ is t-butyl and R^{b} is H, B is not of the formula

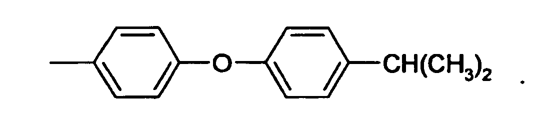

50. A compound of claim 49, wherein B is 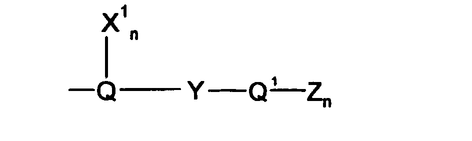 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂-,
X^{a} is halogen,
Q is phenyl or pyridinyl substituted or unsubstituted by halogen, up to per-halosubstitution;
Q¹ is a mono- or bicyclic aromatic structure of 3 to 10 carbon atoms and 0-4 members of the group consisting of N, O and S, optionally substituted by halogen up to per-halosubstitution,
X¹ is C₁-C₄ alkyl or halosubstituted C₁-C₄ alkyl up to per halo, and
Z, n and n1 are as defined in claim 49.

51. A compound of claim 50, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, substituted or unsubstituted by halogen, up to per-halo, X¹ is as defined in claim 50, and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl wherein R⁶ and R⁷ can be substituted by halogen or up to per-halosubstitution.

52. A compound of the formula 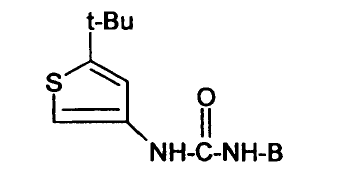 wherein B is as defined in claim 1, subject to the proviso that B is not of the formula

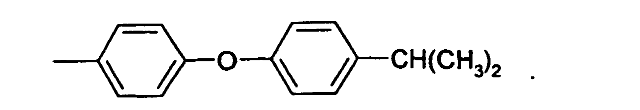

53. A compound of claim 50, wherein Q is phenyl optionally substituted by halogen up to per-halosubstitution, Q¹ is phenyl or pyridinyl optionally substituted by halogen up to per-halosubstitution, and Y is -O- or -S-, Z is -Cl, -CH₃, -OH or -OCH₃, X¹ is as defined in claim 50, n = 0 or 1 and n1 = 0-2.

54. A compound as in claim 49 selected from the group consisting of:
*N*-(5-*tert*-Butyl-3-thienyl)-*N'*-(4-(3-methylphenyl)oxyphenyl)urea;
*N*-(5-*tert*-Butyl-3-thienyl)-*N'*-(4-(4-hydroxyphenyl)oxyphenyl)urea;
*N*-(5-*tert*-Butyl-3-thienyl)-*N'*-(4-(4-methoxyphenyl)oxyphenyl)urea;
*N*-(5-*tert*-Butyl-3-thienyl)-*N'*-(4-(4-pyridinyl)thiophenyl)urea;
and pharmaceutically acceptable salts thereof.

55. A compound of the formula 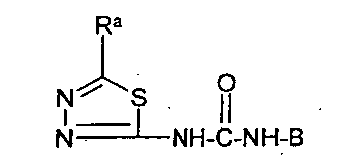 wherein R^{a} is C₁-C₁₀ alkyl, C₃-C₁₀ cycloalkyl, up to per-halosubstituted, C₁-C₁₀ alkyl and up to per-halosubstituted C₃-C₁₀ cycloalkyl;
and B is phenyl, pyridinyl, indolinyl, isoquinolinyl, quinolinyl, or napthyl; substituted by phenyl, pyridinyl or -Y-Ar,
wherein the cyclic structures of B are optionally substituted by halogen, up to per halo, and
optionally substituted by X¹ₙ
wherein n = 0-2,
each X¹ is independently selected from the group consisting of -CN, -NO₂ ,-OR⁵ and C₁-C₁₀ alkyl,
-SR⁵, -CO₂R⁵, -C(O)R⁵,
-C(O)NR⁵R^{5'}, -NR⁵R^{5'}, -NR⁵C(O)OR^{5'}, -NR⁵C(O)R^{5'}, -C₃-C₁₀ cycloalkyl,
substituted C₁-C₁₀ alkyl, substituted C₂₋₁₀-alkenyl, substituted C₁₋₁₀-alkoxy, and substituted C₃-C₁₀ Cycloalkyl,
wherein if X¹ is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, -NO₂, NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, up to per-halosubstituted C₁-C₁₀ alkyl, up to per-halosubstituted C₂₋₁₀-alkenyl, and up to per-halosubstituted C₃-C₁₀ cycloalkyl
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and N(R⁵)(CH₂)ₘ-, m = 1-3, and X^{a} is halogen; and
Ar is a 5-10 aromatic structure containing 0-4 members of the group consisting of nitrogen, oxygen and sulfur which is unsubstituted or substituted by halogen up to per-halo and optionally substituted by Zₙ₁, wherein n1 is 0 to 3 and each Z is independently selected from the group consisting of -CN, -CO₂R⁵, -C(O)R⁵, =O, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, -SO₂R⁵, -SO₂R⁵R^{5'}, C₁-C₁₀ alkyl, C₁-C₁₀ alkoxy, C₃-C₁₀ cycloalkyl, substituted C₁-C₁₀ alkyl, substituted C₃-C₁₀ cycloalkyl, wherein if Z is a substituted group, it is substituted by one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'} and -NR⁵C(O)OR^{5'}, C₁-C₁₀ alkyl, C₁-C₁₀ alkoxyl, and C₃-C₁₀ cycloalkyl.

56. A compound as in claim 55, wherein B is 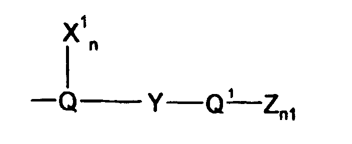 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O-, -OCH₂-,
X^{a} is halogen,
Q is phenyl or pyridinyl substituted or unsubstituted by halogen, up to per-halosubstitution;
Q¹ is phenyl, pyridinyl, naphthyl, parimidinyl, quinoline, isoquinoline, imidazolyl or benzothiazolyl, optionally substituted by halogen up to per-halosubstitution.
X¹ is C₁-C₄ alkyl or halosubstituted C₁-C₄ alkyl up to per-halo,
Z, n and n1 are as defined in claim 55, and s is 0 or 1.

57. A compound as in claim 56, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, optionally substituted by halogen, up to per-halo, X¹ is as defined in claim 56 and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, C₃-C₆-cycloalkyl and wherein R⁶ and R⁷ can be substituted by halogen or up to per-halosubstitution.

58. A compound as in claim 56,
wherein Q is phenyl optionally substituted by halogen up to per-halosubstitution, Q¹ is phenyl or pyridinyl optionally substituted by halogen up to per-halosubstitution, Y is -O- or -S-, X¹ is as defined in claim 56, n = 0 or 1 and n1 = 0.

59. A compound as in claim 55, of the formula 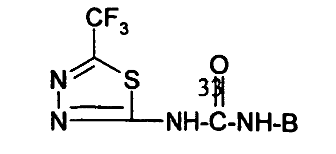 wherein B is
phenyl, pyridinyl, indolinyl, isoquinolinyl, quinolinyl, or napthyl
substituted by phenyl, pyridinyl or -Y-Ar,
optionally substituted by halogen, up to per halo, and
wherein each cyclic structure of B is optionally substituted by X¹ₙ
wherein n = 0-2; each X¹ is independently selected from the group consisting of -CN, -OR⁵, -NR⁵R^{5'}, C₁-C₁₀ alkyl;
-CO₂R⁵, -C(O)NR⁵R^{5'}, -C(O)R⁵,
-NO₂, -SR⁵, -NR⁵C(O)OR^{5'}, -NR⁵C(O)R^{5'}, C₃-C₁₀ cycloalkyl, and substituted C₁-C₁₀ alkyl, substituted C₂₋₁₀-alkenyl, substituted C₁₋₁₀-alkoxy, and substituted C₃-C₁₀ cycloalkyl,
wherein R⁵ and R^{5'} are independently selected from H, C₁-C₁₀ alkyl, C₂₋₁₀-alkenyl, C₃-C₁₀ cycloalkyl, up to per-halosubstituted C₁-C₁₀ alkyl, up to per-haiosubstituted C₂₋₁₀-alkenyl; up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-, m = 1-3, and X^{a} is halogen; and
Ar is a member aromatic structure containing 0-4 members of the group consisting of nitrogen, oxygen and sulfur which is unsubstituted or substituted by halogen up to per-halosubstitution and optionally substituted by Zₙ₁, wherein n1 is 0 to 3 and each Z is independently selected from the group consisting of-CN, -CO₂R⁵, -C(O)R⁵, =O, -C(O)NR⁵R^{5'}, -C(O)- NR⁵, -NO₂, -OR⁵, - SR⁵, -NR⁵R^{5'}, -NR⁵C(O)OR^{5'}, -NR⁵C(O)R^{5'}, -SO₂R⁵, -SO₂R⁵R^{5'}, C₁-C₁₀ alkyl, C₁-C₁₀ alkoxy, C₃-C₁₀ cycloalkyl, substituted C₁-C₁₀ alkyl, substituted C₃-C₁₀ cycloalkyl, wherein if Z is a substituted group, it is substituted by the one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'} , -NR⁵C(O)OR^{5'}, C₁-C₁₀ alkyl, C₁-C₁₀ alkoxyl, and C₃-C₁₀ cycloalkyl.

60. A compound as in claim 59 selected from the group consisting of:
*N*-(5-*tert*-Butyl-2-(1-thia-3,4-diazolyl))-*N'*-(3-(4-pyridinyl)thiophenyl)urea;
*N*-(5-*tert*-Butyl-2-(1-thia-3,4-diazolyl))-*N'*-(4-(4-pyridinyl)oxyphenyl)urea;
*N*-(5-*tert*-butyl-2-(1-thia-3,4-diazolyl))-*N'*-(3-(4-(2-methylcarbamoyl)pyridyl)-oxyphenyl) urea;
*N*-(5-*tert*-butyl-2-(1-thia-3,4-diazolyl))-*N'*-(4-(4-(2-methylcarbamoyl)pyridyl)-oxyphenyl) urea;
*N*-(5-*tert*-butyl-2-(1-thia-3,4-diazolyl))-*N'*-(3-chloro-4-(4-(2-methylcarbamoyl)pyridyl)-oxyphenyl) urea;
*N*-(5-*tert*-butyl-2-(1-thia-3,4-diazolyl))-*N'*-(2-chloro-4-(4-(2-methylcarbamoyl)pyridyl)-oxyphenyl) urea;
*N*-(5-*tert*-butyl-2-(1-thia-3,4-diazolyl))-*N'*-(3-(4-pyridyl)thiophenyl) urea;
*N*-(5-*tert*-butyl-2-(1-thia-3,4-diazolyl))-*N'*-(2-methyl-4-(4-(2-methylcarbamoyl)pyridyl)oxyphenyl) urea;
*N*-(5-(1,1-dimethylprop-1-yl)-2-(1-thia-3,4-diazolyl))-*N'*-(4-(3-carbamoylphenyl)oxyphenyl) urea;
and pharmaceutically acceptable salts thereof.

61. A compound of one of the formulae 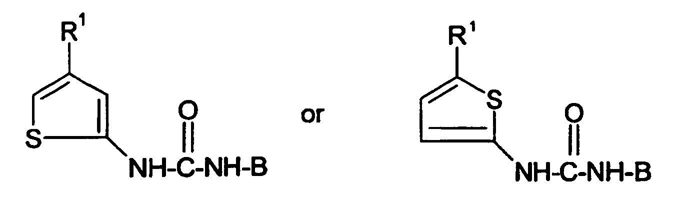 R¹ is selected from the group consisting of halogen, C₃-C₁₀ alkyl, C₃₋₁₃-heteroaryl, C₆₋₁₄-aryl, C₇₋₂₄-alkaryl, C₃-C₁₀ cycloalkyl, up to per-halosubstituted C₁-C₁₀ alkyl, up to per-halosubstituted C₃-C₁₀ cycloalkyl, up to per-halosubstituted C₁₋₁₃-heteroaryl, up to per-halosubstituted C₆₋₁₄-aryl , and up to per-halosubstituted C₇₋₂₄-alkaryl;
B is phenyl, pyridinyl, indolinyl, isoquinolinyl, quinolinyl, or napthyl
substituted by phenyl, pyridinyl or -Y-Ar,
wherein the cyclic structures of B are optionally substituted by halogen, up to per halo, and
optionally substituted by X¹ₙ
wherein n = 0-2
each X¹ is independently selected from the group consisting of-CN, -OR⁵, -NR⁵R^{5'}, C₁-C₁₀ alkyl;
-CO₂R⁵, -C(O)NR⁵R^{5'}, -C(O)R⁵, =O,
-NO₂, -SR⁵, -NR⁵C(O)OR^{5'}, -NR⁵C(O)R^{5'}, C₃-C₁₀ cycloalkyl, substituted C₁-C₁₀ alkyl, substituted C₂₋₁₀-alkenyl, substituted C₁₋₁₀-alkoxy, substituted C₃-C₁₀ cycloalkyl,
wherein if X¹ is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'} , -NO₂, -NR⁵C(O)R^{5'} , -NR⁵C(O)OR^{5'} and halogen up to per-halo substitution;
wherein R⁵ and R^{5'} are independently selected from H, C₁-C₁₀ alkyl, C₂₋₁₀-alkenyl, C₃-C₁₀ cycloalkyl, up to per-halosubstituted C₁-C₁₀ alkyl, up to per-halosubstituted C₂₋₁₀-alkenyl, and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m =1-3, and X^{a} is halogen; and
Ar is a 5-10 member aromatic structure containing 0-4 members of the group consisting of nitrogen, oxygen and sulfur which is unsubstituted or substituted by halogen up to per-halosubstitution and optionally substituted by Zₙ₁, wherein n1 is 0 to 3 and each Z is independently selected from the group consisting of -CN, -CO₂R⁵, =O, -C(O)R⁵, -C(O)NR⁵R^{5'}, -C(O)- NR⁵, -NO₂, -OR⁵, - SR⁵, - NR⁵R^{5'}, -NR⁵C(O)OR^{5'}, -NR⁵C(O)R^{5'}, -SO₂R⁵, -SO₂R⁵R^{5'}, C₁-C₁₀ alkyl, C₁-C₁₀ alkoxy, C₃-C₁₀ cycloalkyl, substituted C₁-C₁₀ alkyl, substituted C₃-C₁₀ cycloalkyl, wherein if Z is a substituted group, it is substituted by the one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀ alkyl, C₁-C₁₀ alkoxyl and C₃-C₁₀ cycloalkyl.

62. A compound of one of the formulae 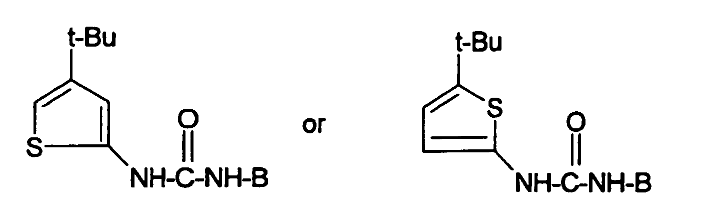 wherein B is as defined in claim 1 .

63. A compound of claim 61, wherein B is 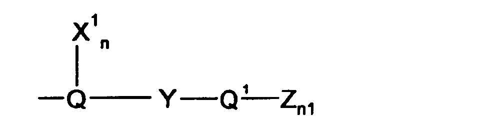 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂-,
X^{a} is halogen,
Q is phenyl or pyridinyl substituted or unsubstituted by halogen, up to per-halosubstitution;
Q¹ is a mono- or bicyclic aromatic structure of 3 to 10 carbon atoms and 0-4 members of the group eonsisting of N, O and S, unsubstituted or substituted by halogen up to per-halosubstitution,
X¹ is C₁-C₄ alkyl or halosubstituted C₁-C₄ alkyl up to per halo,
Z, n and n1 are as defined in claim 61 or 1.

64. A compound of claim 63, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, optionally substituted by halogen, up to per-halo, X¹ is as defined in claim 63 and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl, wherein R⁶ and R⁷ can be substituted by halogen or up to per-halosubstitution.

65. A compound of claim 63,
wherein Q is phenyl optionally substituted by halogen up to per-halosubstitution, Q¹ is phenyl or pyridinyl optionally substituted by halogen up to per-halosubstitution, and Y is -O- or -S-, X¹ is as defined in claim 63, n = 0 or 1, Z is -Cl, -CH₃, -OH or OCH₃, and n1 = 0-2.

66. A compound of the formula 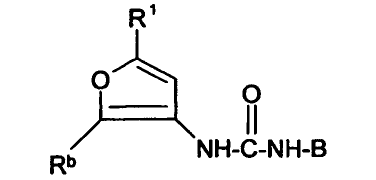 wherein R¹ is selected from the group consisting of C₃-C₁₀ alkyl, C₃-C₁₀ cycloalkyl, up to per-halosubstituted C₃-C₁₀ alkyl and up to per-halosubstituted C₃-C₁₀ cycloalkyl, R^{b} is hydrogen or halogen, and
wherein B is phenyl, pyridinyl, indolinyl, isoquinolinyl, quinolinyl, or napthyl
substituted by phenyl, pyridinyl or -Y-Ar,
wherein the cyclic structures of B are optionally substituted by halogen, up to per halo, and
optionally substituted by X¹ₙ
n = 0-3 and
each X¹ is independently selected from the group consisting of-CN, -CO₂R⁵, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, substituted C₁-C₁₀ alkyl, substituted C₂₋₁₀-alkenyl, substituted C₁₋₁₀-alkoxy, and substituted C₃-C₁₀ cycloalkyl,
wherein if X¹ is a substituted group, it is substituted by one or more substituents independently selected from the group consisting of-CN, -CO₂R⁵,
-C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, up to per-halosubstituted C₁-C₁₀ alkyl, up to per-halosubstituted C₂₋₁₀-alkenyl, and up to per-halosubstituted C₃-C₁₀ cycloalkyl,
wherein Y is - O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-4 members of the group consisting of nitrogen, oxygen and sulfur which is unsubstituted or substituted by halogen up to per-halo and optionally substituted by Zₙ₁, wherein n1 is 0 to 3 and each Z is independently selected from the group consisting of -CN, -CO₂R⁵, -C(O)R⁵, =O, -C(O)NR⁵R^{5'}, -C(O)R⁵, -NO₂, -OR⁵, - SR⁵, - NR⁵R^{5'} ,-NR⁵C(O)OR^{5'}, -NR⁵C(O)R^{5'}, -SO₂R⁵, -SO₂ R⁵R^{5'}, C₁-C₁₀ alkyl, C₃-C₁₀ cycloalkyl, substituted C₁-C₁₀ alkyl, substituted C₃-C₁₀ cycloalkyl, wherein if Z is a substituted group, it is substituted by one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀ alkyl, C₁-C₁₀ alkoxyl, and C₃-C₁₀ cycloalkyl.

67. A compound of claim 66, wherein B is 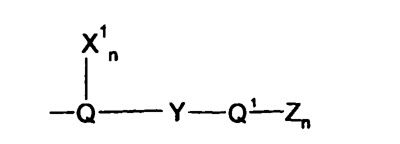 wherein
Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂-,
X^{a} is halogen
Q is phenyl or pyridinyl, substituted or unsubstituted by halogen, up to per-halosubstitution;
Q¹ is a mono- or bicyclic aromatic structure of 3 to 10 carbon atoms and 0-4 members of the group consisting of N, O and S, substituted or unsubstituted by halogen up to per-halosubstitution,
X¹ is C₁-C₄ alkyl or halosubstituted C₁-C₄ alkyl up to per halo, and
Z, n and n1 are as defined in claim 66.

68. A compound of claim 67, wherein
Q is phenyl or pyridinyl, substituted or unsubstituted by halogen, up to per-halosubstitution,
Q¹ is selected from the group consisting of phenyl, pyridinyl, naphthyl, pyrimidinyl, quinoline, isoquinoline, imidazole and benzothiazolyl, optionally substituted by halogen, up to per halo, X¹ is as defined in claim 67 and
Z is selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-alkyl, and C₃-C₆-cycloalkyl, wherein R⁶ and R⁷ can be substituted by halogen or up to per-halosubstitution.

69. A compound of the formula 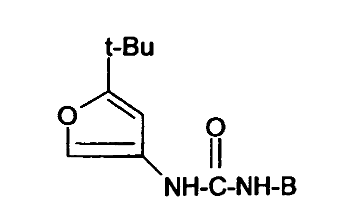 wherein B is as defined in claim 1

70. A compound as in claim 67,
wherein Q is phenyl optionally substituted by halogen up to per-halosubstitution, Q¹ is phenyl or pyridinyl optionally substituted by halogen up to per-halosubstitution, and Y is -O- or -S-, X¹ is as defined in claim 67, Z is -Cl or -OCH₃, n = 0, s = 0 and n1 = 0-2.

71. A pharmaceutical composition comprising a compound according to claim 31 and a physiologically acceptable carrier.

72. A pharmaceutical composition comprising a compound according to claim 37 and a physiologically acceptable carrier.

73. A pharmaceutical composition comprising a compound according to claim 43 and a physiologically acceptable carrier.

74. A pharmaceutical composition comprising a compound according to claim 49 and a physiologically acceptable carrier.

75. A pharmaceutical composition comprising a compound according to claim 55 and a physiologically acceptable carrier.

76. A pharmaceutical composition comprising a compound according to claim 61 and a physiologically acceptable carrier.

77. A pharmaceutical composition comprising a compound according to claim 66 and a physiologically acceptable carrier.

78. The use of claim 1, wherein B is phenyl, pyridinyl, pyrimidinyl, pyrazinyl, pyridazinyl, naphthyl, quinolinyl, isoquinolinyl, phthalimidinyl, furyl, pyrrolyl, imidazolyl, pyrazolyl, oxazolyl, isoxazolyl, thiazolyl, isothiazolyl, benzofuryl, benzothienyl, indolyl, benzopyrazolyl, benzoxazolyl, benzisoxazolyl, benzothiazolyl or benzisothiazolyl substituted by -Y-Ar and optionally substituted by halogen up to per-halosubstitution, C₁-C₁₀ alkyl and up to per-halosubstituted C₁-C₁₀ alkyl, wherein Y and Ar are as defined in claim 1.

79. The use of claim 1, wherein B is
a) phenyl, pyridinyl, naphthyl, quinolinyl or isoquinolinyl, substituted by -Y-Ar and optionally substituted by halogen up to per-halosubstitution, C₁-C₁₀ alkyl and up to per-halosubstituted C₁-C₁₀ alkyl, wherein Y and Ar are as defined in claim 1; or
b) indolyl substituted by C₆-C₁₄ aryl or C₃-C₁₃ heteroaryl.

## Patentansprüche

1. Verwendung einer Verbindung der Formel I 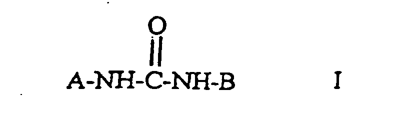 worin B Phenyl, Pyridinyl, Pyrimidinyl, Pyrazinyl, Pyridazinyl, Naphthyl, Chinolinyl, Isochinolinyl, Phthalimidinyl, Furyl, Pyrrolyl, Imidazolyl, Pyrazolyl, Oxazolyl, Isoxazolyl, Thiazolyl, Isothiazolyl, Benzofuryl, Benzothienyl, Indolyl, Benzopyrazolyl, Benzoazolyl, Benzisoxazolyl, Benzothiazolyl oder Benzisothiazolyl ist, substituiert durch einen oder mehrere Substituenten, unabhängig ausgewählt aus der Gruppe, bestehend aus Halogen, bis zur Perhalogensubstitution, und Xₙ, worin n 0-3 ist und jedes X unabhängig ausgewählt ist aus der Gruppe, bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂-C₁₀-Alkenyl, C₁- C₁₀-Alkoxy, C₃-C₁₀-Cycloalkyl, C₆-C₁₄-Aryl, C₃-C₁₃-Heteroaryl, bis zu perhalogensubstituiertem C₁-C₁₀-Alkyl, bis zu perhalogensubstituiertem C₂-C₁₀-Alkenyl, bis zu perhalogensubstituiertem C₁-C₁₀-Alkoxy, bis zu perhalogensubstituiertem C₃-C₁₀-Cycloalkyl und -Y-Ar,
worin R⁵ und R^{5'} unabhängig ausgewählt sind aus H, C₁-C₁₀- Alkyl, C₂-C₁₀-Alkenyl, C₃-C₁₀-Cycloalkyl, bis zu perhalogensubstituiertem C₁-C₁₀-Alkyl, bis zu perhalogensubstituiertem C₂-C₁₀-Alkenyl, bis zu perhalogensubstituiertem C₃-C₁₀-Cycloalkyl,
worin Y -O-, -S-, -N(R⁵)-, -(CH₂)ₘ-, -C(O)-, -CH(OH)-, -(CH₂)ₘO-, -NR⁵C(O)NR⁵NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ- ist,
m = 1 bis 3 und X^{a} Halogen ist; und
Ar eine 5-1 0-gliedrige aromatische Struktur ist, enthaltend 0 bis 4 Mitglieder der Gruppe, bestehend aus Stickstoff, Sauerstoff und Schwefel, optional substituiert durch Halogen, bis zur Perhalogensubstitution, und optional substituiert durch Zₙ₁, worin n1 0 bis 3 ist und jedes Z unabhängig ausgewählt ist aus der Gruppe, bestehend aus -CN, =O, -CO₂R⁵, -C(O)NR⁵R^{5'}, -C(O)-NR⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, SO₂NR⁵R^{5'}, C₁-C₁₀-Alkyl, C₁-C₁₀-Alkoxyl, C₃-C₁₀- Cycloalkyl, bis zu perhalogensubstituiertem C₁-C₁₀-Alkyl und bis zu perhalogensubstituiertem C₃-C₁₀-Cycloalkyl; und worin A ein Heteroarylrest ist, ausgewählt aus der Gruppe, bestehend aus 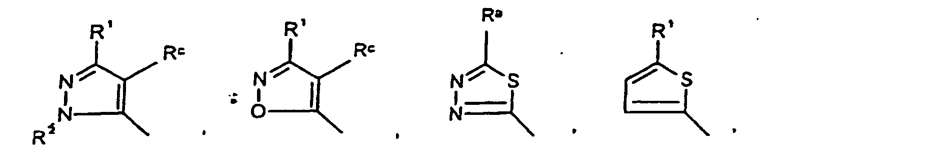 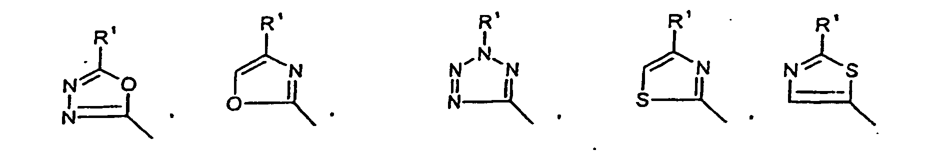 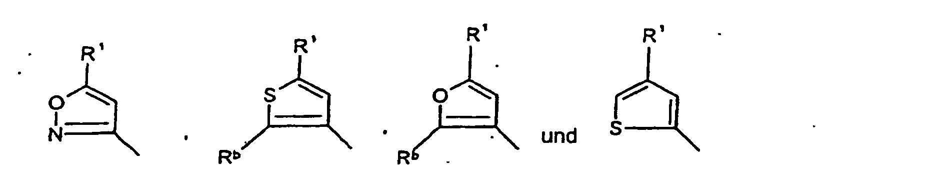 worin
R¹ ausgewählt ist aus der Gruppe, bestehend aus Halogen, C₃-C₁₀-Alkyl, C₃-C₁₀-Cycloalkyl, C₁-C₁₃-Heteroaryl, C₆-C₁₄-Aryl, C₇-C₂₄-Alkaryl, bis zu perhalogensubstituiertem C₁-C₁₀-Alkyl, bis zu perhalogensubstituiertem C₃-C₁₀-Cycloalkyl, bis zu perhalogensubstituiertem C₁-C₁₃-Heteroaryl, bis zu perhalogensubstituiertem C₆-C₁₄-Aryl und bis zu perhalogensubstituiertem C₇-C₂₄-Alkaryl;
R² ausgewählt ist aus der Gruppe, bestehend aus H, -C(O)R⁴, -CO₂R⁴, -C(O)NR³R^{3'}, C₁-C₁₀-Alkyl, C₃-C₁₀-Cycloalkyl, C₇-C₂₄- Alkaryl, C₄-C₂₃-Alkheteroaryl, substituiertem C₁-C₁₀-Alkyl, substituiertem C₃-C₁₀-Cycloalkyl, substituiertem C₇-C₂₄- Alkaryl und substituiertem C₄-C₂₃-Alkheteroaryl,
worin wenn R² eine substituierte Gruppe ist, sie substituiert ist durch ein oder mehrere Substituenten, unabhängig ausgewählt aus der Gruppe, bestehend aus
-CN, -CO₂R⁴, -C(O)-NR³R^{3'}, -NO₂, -OR⁴, -SR⁴ und Halogen, bis zur Perhalogensubstitution,
worin R³ und R^{3'} unabhängig ausgewählt sind aus der Gruppe, bestehend aus H, -OR⁴, -SR⁴, -NR⁴R^{4'}, -C(O)R⁴, -CO₂R⁴, -C(O)NR⁴R^{4'}, C₁-C₁₀-Alkyl, C₃-C₁₀-Cycloalkyl, C₆-C₁₄-Aryl, C₃-C₁₃-Heteroaryl, bis zu perhalogensubstituiertem C₁-C₁₀-Alkyl, bis zu perhalogensubstituiertem C₃-C₁₀-Cycloalkyl und bis zu perhalogensubstituiertem C₆-C₁₄-Aryl oder C₃-C₁₃-Heteroaryl; und
worin R⁴ und R^{4'} unabhängig ausgewählt sind aus der Gruppe, bestehend aus H, C₁-C₁₀-Alkyl, C₃-C₁₀-Cycloalkyl, C₆-C₁₄-Aryl, C₃-C₁₃-Heteroaryl; bis zu perhalogensubstituiertem C₁-C₁₀-Alkyl, bis zu perhalogensubstituiertem C₃-C₁₀-Cycloalkyl und bis zu perhalogensubstituiertem C₆-C₁₄-Aryl oder C₃-C₁₃-Heteroaryl,
R^{a} C₁-C₁₀-Alkyl, C₃-C₁₀-Cycloalkyl, bis zu perhalogensubstituiertem C₁-C₁₀-Alkyl und bis zu perhalogensubstituiertem C₃-C₁₀-Cycloalkyl ist; und
R^{b} Wasserstoff oder Halogen ist,
R^{c} Wasserstoff, Halogen, C₁-C₁₀-Alkyl, bis zu perhalogensubstituiertem C₁-C₁₀-Alkyl ist oder sich mit R¹ und den Ringkohlenstoffatomen, an welche R¹ und R^{c} gebunden sind, vereinigt, um einen 5- oder 6-gliedrigen Cycloalkyl-, Aryl- oder Heteroarylring zu bilden, mit 0 bis 2 Mitgliedern, ausgewählt aus O, N und S;
mit der Maßgabe, daß wenn A 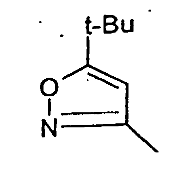 ist, B nicht 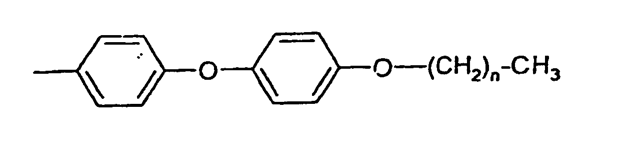 ist,
worin n = 2-4, oder 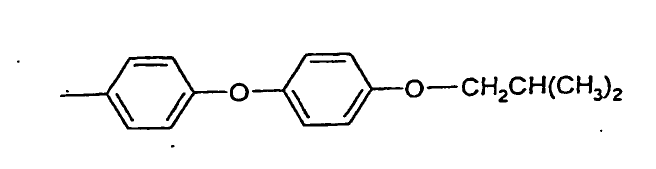 ist, zur Herstellung eines Medikaments zur Behandlung von krebsartigem Zellwachstum, vermittelt durch raf-Kinase.

2. Verwendung nach Anspruch 1, worin B 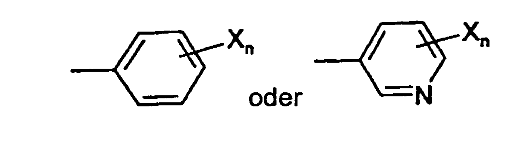 ist, das substituiert oder unsubstituiert ist durch Halogen, bis zur Perhalogensubstitution, und worin
n = 1 bis 3 und
jedes X unabhängig ausgewählt ist aus der Gruppe, bestehend C₁-C₁₀-Alkyl, bis zu perhalogensubstituiertem C₁-C₁₀-Alkyl und -Y-Ar;
worin Y -O-, -S-, -N(R⁵)-, -(CH₂)ₘ-, -C(O)-, -CH(OH)-, -(CH₂)ₘO-, -NR⁵C(O)NR⁵NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ- ist,
m = 1 - 3 und X^{a} Halogen ist; und
Ar eine 5-10-gliedrige aromatische Struktur ist, enthaltend 0 bis 4 Mitglieder der Gruppe, bestehend aus Stickstoff, Sauerstoff und Schwefel, optional substituiert durch Halogen, bis zur Perhalogensubstitution, und optional substituiert durch Zₙ₁, worin n1 0 bis 3 ist und jedes Z unabhängig ausgewählt ist aus der Gruppe, bestehend aus -CN, =O, -CO₂R⁵, -C(O)NR⁵R^{5'}, -C(O)-R⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, SO₂R⁵R^{5'}, C₁-C₁₀-Alkyl, C₁-C₁₀-Alkoxy, C₃-C₁₀- Cycloalkyl, bis zu perhalogensubstituiertem C₁-C₁₀-Alkyl und bis zu perhalogensubstituiertem C₃-C₁₀-Cycloalkyl, worin R⁵ und R^{5'} unabhängig ausgewählt sind aus H, C₁-C₁₀-Alkyl, C₂-C₁₀-Alkenyl, C₃-C₁₀-Cycloalkyl, bis zu perhalogensubstituiertem C₁-C₁₀-Alkyl, bis zu perhalogensubstituiertem C₂-C₁₀-Alkenyl und bis zu perhalogensubstituiertem C₃-C₁₀-Cycloalkyl.

3. Verwendung nach Anspruch 1 worin B 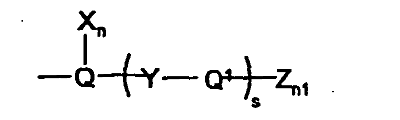 ist, worin
Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution;
Q¹ eine mono- oder bicyclische aromatische Struktur mit 3 bis 10 Kohlenstoffatomen und 0-4 Mitgliedern der Gruppe, bestehend aus N, O und S ist, optional substituiert durch Halogen bis zur Perhalogensubstitution,
X, Z, n und n1 wie in Anspruch 1 definiert sind und s =0 oder 1.

4. Verwendung nach Anspruch 3, worin
Q Phenyl oder Pyridinyl ist, substituiert oder unsubstituiert mit Halogen, bis zur Perhalogensubstitution,
Q¹ ausgewählt ist aus der Gruppe, bestehend aus Phenyl, Pyridinyl, Naphthyl, Pyrimidinyl, Chinolin, Isochinolin, Imidazol und Benzothiazolyl, substituiert oder unsubstituiert durch Halogen, bis zur Perhalogensubstitution, und
jedes X unabhängig ausgewählt ist aus der Gruppe, bestehend aus -R⁶, -OR⁶ und -NHR⁷, worin R⁶ Wasserstoff, C₁- C₁₀-Alkyl oder C₃-C₁₀-Cycloalkyl ist und R⁷ ausgewählt ist aus der Gruppe, bestehend aus Wasserstoff, C₃-C₁₀-Alkyl und C₃-C₆- Cycloalkyl, worin R⁶ und R⁷ durch Halogen oder bis zur Perhalogensubstitution substituiert sein können.

5. Verwendung nach Anspruch 1, worin die Verbindung die Formel 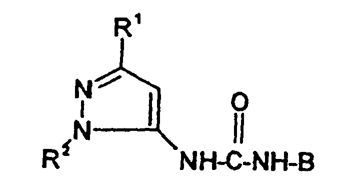 aufweist, worin R¹ und R² und B wie in Anspruch 1 definiert sind.

6. Verwendung nach Anspruch 5, worin B die Formel 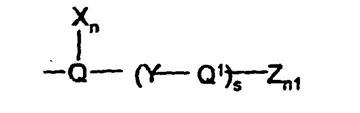 aufweist, worin Q Phenyl oder Pyridinyl ist, optional substituiert durch Halogen, bis zur Perhalogensubstitution, Q¹ Pyridinyl, Phenyl oder Benzothiazolyl ist, optional substituiert durch Halogen, bis zur Perhalogensubstitution, Y -O-, -S-, -CH₂S-, -SCH₂-, -CH₂O-, -OCH₂- oder -CH₂- ist, X C₁-C₄-Alkyl oder bis zu perhalogensubstituiertem C₁-C₄-Alkyl ist und Z wie in Anspruch 1 definiert ist, n = 0 oder 1, s = 1 und n1 = 0-1.

7. Verwendung nach Anspruch 1, worin die Verbindung ausgewählt wird aus der Gruppe, bestehend aus
N-(3-tert-Butyl-5-pyrazolyl)-N'-(4-phenyloxyphenyl)harnstoff;
N-(3-tert-Butyl-5-pyrazolyl)-N'-(3-(3-methylaminocarbonylphenyl)oxyphenyl)harnstoff;
N-(3-tert-Butyl-5-pyrazolyl)-N'-(3-(4-pyridinyl)thiophenyl)harnstoff;
N-(3-tert-Butyl-5-pyrazolyl)-N'-(4-(4-pyridinyl)thiophenyl)harnstoff;
N-(3-tert-Butyl-5-pyrazolyl)-N'-(4-(4-pyridinyl)oxyphenyl)harnstoff;
N-(3-tert-Butyl-5-pyrazolyl)-N'-(4-(4-pyridinyl)methylphenyl)harnstoff;
N-(1-Methyl-3-tert-butyl-5-pyrazolyl)-N'-(4-phenyloxyphenyl)harnstoff;
N-(1-Methyl-3-tert-butyl-5-pyrazolyl)-N'-(3-(4-pyridinyl)thiophenyl)harnstoff;
N-(1-Methyl-3-tert-butyl-5-pyrazolyl)-N'-((4-(4-pyridinyl)thiomethyl)phenyl)-harnstoff;
N-(1-Methyl-3-tert-butyl-5-pyrazolyl)-N'-(4-(4-pyridinyl)thiophenyl)harnstoff;
N-(1-Methyl-3-tert-butyl-5-pyrazolyl)-N'-(4-(4-pyridinyl)oxyphenyl)harnstoff;
N-(1-Methyl-3-tert-butyl-5-pyrazolyl)-N'-((4-(4-pyridinyl)methyloxy)phenyl)harnstoff;
N-(1-Methyl-3-tert-butyl-5-pyrazolyl)-N'-(3-(2-benzothiazolyl)oxyphenyl)harnstoff;
N-(3-tert-Butyl-5-pyrazolyl)-N'-(3-(4-pyridyl)thiophenyl)harnstoff;
N-(3-tert-Butyl-5-pyrazolyl)-N'-(4-(4-pyridyl)thiophenylharnstoff;
N-(3-tert-Butyl-5-pyrazolyl)-N'-(3-(4-pyridyl)oxyphenyl)harnstoff;
N-(3-tert-Butyl-5-pyrazolyl)-N'-(4-(4-pyridyl)oxyphenyl)harnstoff;
N-(1-Methyl-3-tert-butyl-5-pyrazolyl)-N'-(3-(4-pyridyl)thiophenyl)harnstoff;
N-(1-Methyl-3-tert-butyl-5-pyrazolyl)-N'-(4-(4-pyridyl)thiophenyl)harnstoff;
N-(1-Methyl-3-tert-butyl-5-pyrazolyl)-N'-(3-(4-pyridyl)oxyphenyl)harnstoff;
N-(1-Methyl-3-tert-butyl-5-pyrazolyl)-N'-(4-(4-pyridyl)oxyphenyl)harnstoff;
Und pharmazeutisch verträglichen Salze davon.

8. Verwendung nach Anspruch 5, worin R¹ t-Butyl ist.

9. Verwendung nach Anspruch 1, worin die Verbindung die Formel 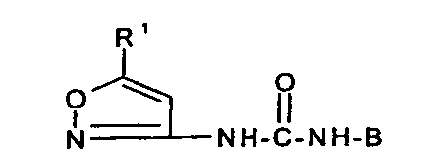 aufweist, worin R¹ und B wie in Anspruch 1 definiert sind.

10. Verwendung nach Anspruch 9, worin B die Formel 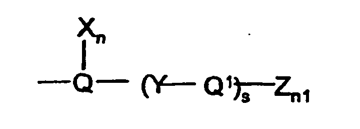 aufweist, worin Q Phenyl oder Pyridinyl ist, optional substituiert durch Halogen bis zur Perhalogensubstitution, Q¹ Pyridinyl, Phenyl oder Benzothiazolyl ist, optional substituiert durch Halogen, bis zur Perhalogensubstitution, Y -O-, -S-, -C(O)- oder -CH₂- ist, X C₁-C₄-Alkyl oder bis zu perhalogensubstituiertem C₁-C₄-Alkyl ist, Z wie in Anspruch 1 definiert ist, n = 0 oder 1, s = 0 oder 1 und n1 = 0 oder 1.

11. Verwendung nach Anspruch 1, worin die Verbindung ausgewählt ist aus der Gruppe, bestehend aus:
N-(5-tert-Butyl-3-isoxazolyl)-N'-(4-(4- hydroxyphenyl)oxyphenyl)harnstoff; N-(5-tert-Butyl-3- isoxazolyl)-N'-(4-(3-hydroxyphenyl)oxyphenyl)harnstoff; N-(5- tert-Butyl-3-isoxazolyl)-N'-(4-(4- acetylphenyl)oxyphenyl)harnstoff; N-(5-tert-Butyl-3-isoxazolyl)-N'-(3-benzoylphenyl)harnstoff; N-(5-tert-Butyl-3-isoxazolyl)-N'-(4-phenyloxyphenyl)harnstoff; N-(5-tert-Butyl-3-isoxazolyl)-N'-(4-(3-Methylaminocarbonylphenyl)thiophenyl)harnstoff; N-(5-tert-Butyl-3-isoxazolyl)-N'-(4-(4-(1,2-methylendioxy)phenyl)oxyphenyl)harnstoff; N-(5-tert-Butyl-3-isoxazolyl)-N'-(4-(3-pyridinyl)oxyphenyl)harnstoff; N-(5-tert-Butyl-3-isoxazolyl)-N'-(4-(4-pyridinyl)oxyphenyl)harnstoff; N-(5-tert-Butyl-3-isoxazolyl)-N'-(4-(4-pyridyl)thiophenyl)harnstoff; N-(5-tert-Butyl-3-isoxazolyl)-N'-(4-(4-pyridinyl)methyl-phenyl)harnstoff; N-(5-tert-Butyl-3-isoxazolyl)-N'-(3-(4-pyridinyl)oxyphenyl)harnstoff; N-(5-tert-Butyl-3-isoxazolyl)-N'-(3-(4-pyridinyl)thiophenyl)harnstoff; N-(5-tert-Butyl-3- isoxazolyl)-N'-(3-(3-methyl-4-pyridinyl)oxyphenyl)harnstoff; N-(5-tert-Butyl-3-isoxazolyl)-N'-(3-(3-methyl-4-pyridinyl)-thiophenyl)harnstoff; N-(5-tert-Butyl-3-isoxazolyl)-N'-(4-(3- methyl-4-pyridinyl)thiophenyl)harnstoff; N-(5-tert-Butyl-3-isoxazolyl)-N'-(3-(4-methyl-3-pyridinyl)oxyphenyl)harnstoff; N-(5-tert-Butyl-3-isoxazolyl)-N'-(4-(3-methyl-4-pyridinyl)-oxyphenyl)harnstoff; N-(5-tert-Butyl-3-isoxazolyl)-N'-(3-(2-benzothiazolyl)oxyphenyl)harnstoff; N-(5-tert-Butyl-3-isoxazolyl)-N'-(3-chlor-4-(4-(2-methylcarbamoyl)pyridyl)oxyphenyl)harnstoff; N-(5-tert-Butyl-3-isoxazolyl)-N'-(4-(4-(2-methylcarbamoyl)-pyridyl)oxyphenyl)harnstoff; N-(5-tert-Butyl-3-isoxazolyl)-N'- (3-(4-(2-methylcarbamoyl)pyridyl)thiophenyl)harnstoff; N-(5-tert-Butyl-3-isoxazolyl)-N'-(2-Methyl-4-(4-(2-methylcarbamoyl)pyridyl)oxyphenyl)harnstoff; N-(5-tert-Butyl-3-isoxazolyl)-N'-(4-(4-(2-carbamoyl)pyridyl)-oxyphenyl)harnstoff; N-(5-tert-Butyl-3-isoxazolyl)-N'-(3-(4-(2-carbamoyl)pyridyl)-oxyphenyl)harnstoff; N-(5-tert-Butyl-3-isoxazolyl)-N'-(3-(4-(2-methylcarbamoyl)-pyridyl)oxyphenyl)harnstoff; N-(5-tert-Butyl-3-isoxazolyl)-N'- (4-(4-(2-methylcarbamoyl)pyridyl)thiophenyl)harnstoff; N-(5-tert-Butyl-3-isoxazolyl)-N'-(3-Chlor-4-(4-(2- methylcarbamoyl)pyridyl)oxyphenyl)harnstoff; N-(5-tert-Butyl-3-isoxazolyl)-N'-(4-(3-methylcarbamoyl)phenyl)-oxyphenyl)harnstoff und pharmazeutisch verträglichen Salzen davon.

12. Verwendung nach Anspruch 10, worin R¹ t-Butyl ist.

13. Verwendung nach Anspruch 1, worin die Verbindung die Formel 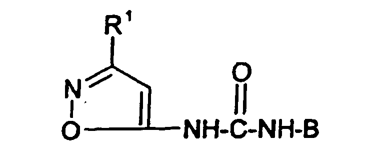 aufweist, worin R¹ und B wie in Anspruch 1 definiert sind.

14. Verwendung nach Anspruch 13, worin B die Formel 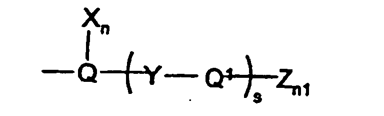 aufweist, worin Q Phenyl oder Pyridinyl ist, optional substituiert durch Halogen bis zur Perhalogensubstitution, Q¹ Phenyl, Benzothiazolyl oder Pyridinyl ist, optional substituiert durch Halogen, bis zur Perhalogensubstitution, Y -O-, -S- oder -CH₂- ist, X C₁-C₄-Alkyl oder bis zu perhalogensubstituiertem C₁-C₄-Alkyl ist Z wie in Anspruch 1 definiert ist, n = 0 oder 1, s = 1 und n1 = 0 oder 1.

15. Verwendung nach Anspruch 1, worin die Verbindung ausgewählt wird aus der Gruppe, bestehend aus
N-(3-lsopropyl-5-isoxazolyl)-N'-(4-(4- pyridinyl)thiophenyl)harnstoff; N-(3-tert-Butyl-5-isoxazolyl)- N'-(4-(4-methoxyphenyl)oxyphenyl)harnstoff; N-(3-tert-Butyl-5-isoxazolyl)-N'-(5-(2-(4-acetylphenyl)oxy)pyridinyl)harnstoff; N-(3-tert-Butyl-5-isoxazolyl)-N'-(3-(4-pyridinyl)thiophenyl)harnstoff; N-(3-tert-Butyl-5-isoxazolyl)-N'-(4-(4-pyridinyl)methylphenyl)harnstoff; N-(3-tert-Butyl-5-isoxazolyl)-N'-(4-(4-pyridinyl)thiophenyl)harnstoff; N-(3-tert-Butyl-5-isoxazolyl)-N'-(4-(4-pyridinyl)oxyphenyl)-harnstoff; N-(3-tert-Butyl-5-isoxazolyl)-N'-(4-(4-methyl-3-pyridinyl)oxyphenyl)harnstoff; N-(3-tert-Butyl-5-isoxazolyl)-N'-(3-(2-benzothiazolyl)oxyphenyl)harnstoff; N-(3-(1,1-Dimethylpropyl)-5-isoxazolyl)N'-(4-(4-methylphenyl)oxyphenyl)harnstoff; N-(3-(1,1-Dimethylpropyl)-5-isoxazolyl)-N'-(3-(4-pyridinyl)thiophenyl)harnstoff; N-(3-(1,1-Dimethylpropyl)-5-isoxazolyl)-N'-(4-(4-pyridinyl)-oxyphenyl)harnstoff; N-(3-(1,1-Dimethylpropyl)-5-isoazolyl)-N'-(4-(4-pyridinyl)-thiophenyl)harnstoff, N-(3-(1,1-Dimethylpropyl)-5-isoxazolyl)- N'-(5-(2-(4-methoxyphenyl)oxy)pyridinyl)harnstoff; N-(3-(1- Methyl-1-ethylpropyl)-5-isoxazolyl)-N'-(4-(4-pyridinyl)oxyphenyl)harnstoff; N-(3-(1-Methyl-1-ethylpropyl)-5-isoxazolyl)-N'-(3-(4-pyridinyl)-thiophenyl)harnstoff; N-(3-Isopropyl-5-isoxazolyl)-N'-(3-(4-(2-methylcarbamoyl)pyridyl)oxyphenyl)harnstoff; N-(3-Isopropyl-5-isoxazolyl)-N'-(4-(4-(2-methylcarbamoyl)pyridyl)-oxyphenyl)harnstoff; N-(3-tert-Butyl-5-isoxazolyl)-N'-(3-(4-(2-methylcarbamoyl)-pyridyl)oxyphenyl)harnstoff; N-(3-tert-Butyl-5-isoxazolyl)-N'- (4-(4-(2-methylcarbamoyl)pyridyl)oxyphenyl)harnstoff; N-(3- tert-Butyl-5-isoxazolyl)-N'-(3-(4-(2- methylcarbamoyl)pyridyl)thiophenyl)-harnstoff; N-(3-(1,1-Dimethylprop-1-yl)-5-isoxazolyl)-N'-(3-(4-(2-methylcarbamoyl)pyridyl)-oxyphenyl)harnstoff; N-(3-(1,1-Dimethylprop-1-yl)-5-isoxazolyl)-N'-(4-(4-(2-methylcarbamoyl)pyridyl)oxyphenyl)harnstoff; N-(3-tert-Butyl-5-isoxazolyl)-N'-(3-Chlor-4-(4-(2-methylcarbamoyl)pyridyl)thiophenyl)harnstoff; und pharmazeutisch verträglichen Salzen davon.

16. Verwendung nach Anspruch 13, worin R¹ t-Butyl ist.

17. Verwendung nach Anspruch 1, worin die Verbindung die Formel 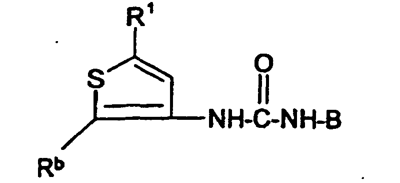 aufweist, worin R¹, R^{b} und B wie in Anspruch 1 definiert sind.

18. Verwendung nach Anspruch 17, worin B die Formel 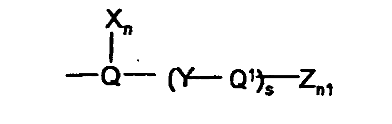 aufweist, worin Q Phenyl ist, optional substituiert durch Halogen bis zur Perhalogensubstitution, Q¹ Phenyl oder Pyridinyl ist, optional substituiert durch Halogen, bis zur Perhalogensubstitution, Y -O- oder -S- ist, X C₁-C₄-Alkyl oder bis zu perhalogensubstituiertem C₁-C₄-Alkyl ist, Z wie in Anspruch 1 definiert ist, n = 0 oder 1, s = 0 oder 1 und n1 = 0-2.

19. Verwendung nach Anspruch 1, worin die Verbindung ausgewählt wird aus der Gruppe, bestehend aus:
N-(5-tert-Butyl-3-thienyl)-N'-(4-(3- methylphenyl)oxyphenyl)harnstoff; N-(5-tert-Butyl-3-thienyl)-N'-(4-(4-hydroxyphenyl)oxyphenyl)harnstoff; N-(5-tert-Butyl-3-thienyl)-N-'(4-(4-methoxyphenyl)oxyphenyl)-harnstoff; N-(5-tert-Butyl-3-thienyl)-N'(4-(4-pyridinyl)thiophenyl)harnstoff; und pharmazeutisch verträglichen Salzen davon.

20. Verwendung nach Anspruch 17, worin R¹ t-Butyl ist.

21. Verwendung nach Anspruch 1, worin die Verbindung die Formel 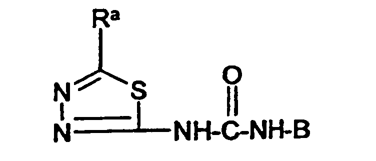 aufweist, worin R^{a} und B wie in Anspruch 1 definiert sind.

22. Verwendung nach Anspruch 21, worin B die Formel 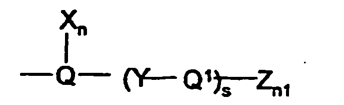 aufweist, worin Q Phenyl ist, optional substituiert durch Halogen bis zur Perhalogensubstitution, Q¹ Phenyl oder Pyridinyl ist, optional substituiert durch Halogen, bis zur Perhalogensubstitution, Y -O- oder -S- ist, X C₁-C₄-Alkyl oder bis zu perhalogensubstituiertem C₁-C₄-Alkyl ist, s = 1; Z wie in Anspruch 1 definiert ist, n = 0 oder 1 und n1 = 0 oder 1.

23. Verwendung nach Anspruch 2, worin die Verbindung ausgewählt wird aus der Gruppe, bestehend aus:
N-(5-tert-Butyl-2-(1-thia-3,4-diazolyl))-N'-(3-(4- pyridinyl)thiophenyl)harnstoff; N-(5-tert-Butyl-2-(1-thia-3,4-diazolyl))-N'-(4-(4-pyridinyl)oxyphenyl)harnstoff; und pharmazeutisch verträglichen Salzen davon.

24. Verwendung nach Anspruch 21, worin R^{a} CF₃- oder t-Butyl ist.

25. Verwendung nach Anspruch 1, worin die Verbindung ausgewählt wird aus einer der Formeln 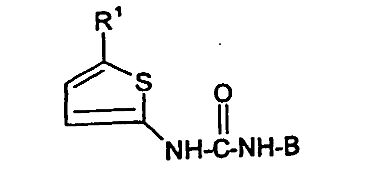 oder 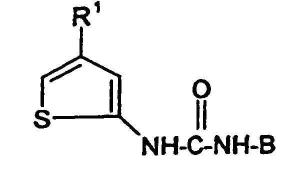 worin R¹ und B wie in Anspruch 1 definiert sind.

26. Verwendung nach Anspruch 25, worin B bis zu perhalogensubstituiertes Phenyl, bis zu perhalogensubstituiertes Pyridinyl ist oder die Formel 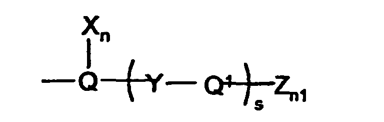 aufweist, worin Q Phenyl ist, optional substituiert durch Halogen bis zur Perhalogensubstitution, Q¹ Phenyl oder Pyridinyl ist, optional substituiert durch Halogen, bis zur Perhalogensubstitution, Y -O- oder -S- ist, X C₁-C₄-Alkyl oder bis zu perhalogensubstituiertem C₁-C₄-Alkyl ist, Z wie in Anspruch 1 definiert ist, n = 0 oder 1, s = 0 oder 1 und n1 = 0 - 2.

27. Verwendung nach Anspruch 25, worin R¹ t-Butyl ist.

28. Verwendung nach Anspruch 1, worin die Verbindung die Formel 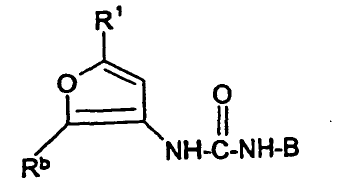 aufweist, worin R¹ und R^{b} und B wie in Anspruch 1 definiert sind.

29. Verwendung nach Anspruch 28, worin B die Formel 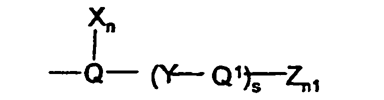 aufweist, worin Q Phenyl ist, optional substituiert durch Halogen bis zur Perhalogensubstitution, Q¹ Phenyl oder Pyridinyl ist, optional substituiert durch Halogen, bis zur Perhalogensubstitution, und Y -O- oder -S- ist, X C₁-C₄-Alkyl oder bis zu perhalogensubstituiertem C₁-C₄-Alkyl ist; Z wie in Anspruch 1 definiert ist, n = 0 oder 1, s = 0 oder 1 und n1 = 0-2.

30. Verwendung nach Anspruch 28, worin R¹ t-Butyl ist.

31. Verbindung der Formel 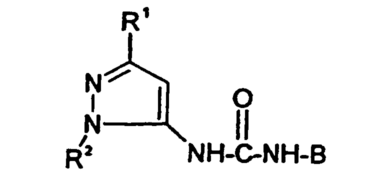 worin R² ausgewählt ist aus der Gruppe, bestehend aus H, -C(O)R⁴, -CO₂R⁴, -C(O)NR³R^{3'}, C₁-C₁₀-Alkyl, C₃-C₁₀-Cycloalkyl, substituiertem C₁-C₁₀- Alkyl, substituiertem C₃-C₁₀-Cycloalkyl, worin, falls R² eine substituierte Gruppe ist, diese substituiert ist durch einen oder mehrere Substituenten, unabhängig ausgewählt aus der Gruppe, bestehend aus -CN, -CO₂R⁴, -C(O)-NR³R^{3'}, -NO₂, -OR⁴, -SR⁴ und Halogen, bis zur Perhalogensubstitution,
worin R³ und R^{3'} unabhängig ausgewählt sind aus der Gruppe, bestehend aus H, C₁-C₁₀-Alkyl, C₃-C₁₀-Cycloalkyl, bis zu perhalogensubstituiertem C₁-C₁₀-Alkyl und bis zu perhalogensubstituiertem C₃-C₁₀-Cycloalkyl und
worin R⁴ und R^{4'} unabhängig ausgewählt sind aus der Gruppe, bestehend aus H, C₁-C₁₀-Alkyl, C₃-C₁₀-Cycloalkyl, bis zu perhalogensubstituiertem C₁-C₁₀-Alkyl und bis zu perhalogensubstituiertem C₃-C₁₀-Cycloalkyl,
worin R¹ ausgewählt ist aus der Gruppe, bestehend aus C₃-C₆-Alkyl, C₃-C₆-Cycloalkyl, bis zu perhalogensubstituiertem C₃-C₆-Alkyl und bis zu perhalogensubstituiertem C₃-C₆-Cycloalkyl,
B Phenyl, Pyridinyl, Indolinyl, Isochinolinyl, Chinolinyl oder Naphtyl ist; substituiert durch C₆-C₁₄-Aryl, C₃-C₁₃-Heteroaryl oder -Y-Ar, worin die cyclischen Strukturen von B optional substituiert sind durch Halogen, bis zu Perhalogen, und optional substituiert durch X¹ₙ,
und worin
n = 0-2; jedes X¹ unabhängig ausgewählt ist aus der Gruppe, bestehend aus -CN, -CO₂R⁵, -C(O)R⁵, -C(O)NR⁵R^{5'}, -OR⁵, -NO₂, -NR⁵R^{5'}, C₁-C₁₀-Alkyl, C₂-C₁₀- Alkenyl, C₁-C₁₀-Alkoxy, C₃-C₁₀-Cycloalkyl, -SR⁵, -NR⁵C(O)OR^{5'}, NR⁵C(O)R^{5'}, substituiertem C₁-C₁₀-Alkyl, substituiertem C₂-C₁₀-Alkenyl, substituiertem C₁-C₁₀-Alkoxy, substituiertem C₃-C₁₀-Cycloalkyl,
worin, falls X¹ eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀- Alkyl, C₂-C₁₀-Alkenyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalogensubstituiertem C₁-C₁₀-Alkyl, bis zu perhalogensubstituiertem C₂₋₁₀-Alkenyl, bis zu perhalogensubstituiertem C₃-C₁₀-Cycloalkyl, worin Y -O-, -S-, -N(R⁵)-, -(CH₂)ₘ, -C(O)-, -CH(OH)-, -(CH₂)ₘO-, -NR⁵C(O)NR⁵R^{5'}, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ- ist,
m = 1-3 und X^{a} Halogen ist; und
Ar eine 5-10-gliedrige aromatische Struktur ist, enthaltend 0-4 Mitglieder der Gruppe, bestehend aus Stickstoff, Sauerstoff und Schwefel, die optional substituiert ist durch Halogen, bis zur Perhalogensubstitution, und optional substituiert ist durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl und substituiertem C₃- C₁₀-Cycloalkyl;
worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxyl und C₃-C₁₀-Cycloalkyl.

32. Verbindung nach Anspruch 31, worin B 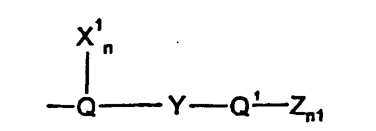 ist, worin
Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution;
Q¹ eine mono- oder bicyclische aromatische Struktur mit 3 bis 10 Kohlenstoffatomen ist und 0-4 Mitgliedern der Gruppe, bestehend aus N, O und S, optional substituiert durch Halogen, bis zur Perhalogensubstitution, X¹ C₁-C₄-Alkyl oder halogensubstituiertes C₁-C₄-Alkyl, bis zu Perhalogen ist,
Z, n und n1 wie in Anspruch 31 definiert sind.

33. Verbindung nach Anspruch 32, worin
Q Phenyl oder Pyridinyl ist, substituiert oder unsubstituiert durch Halogen, bis zur Perhalogensubstitution,
Q¹ ausgewählt ist aus der Gruppe, bestehend aus Phenyl, Pyridinyl, Naphthyl, Pyrimidinyl, Chinolin, lsochinolin, Imidazol und Benzothiazolyl, optional substituiert durch Halogen, bis zu Perhalogen, und X¹ wie in Anspruch 32 ist, und
Z ausgewählt ist aus der Gruppe, bestehend aus -R⁶, -OR⁶ und -NHR⁷, worin R⁶ Wasserstoff, C₁-C₁₀-Alkyl oder C₃-C₁₀-Cycloalkyl ist und R⁷ ausgewählt ist aus der Gruppe, bestehend aus Wasserstoff, C₃-C₁₀-Alkyl und C₃-C₆-Cycloalkyl, worin R⁶ und R⁷ durch Halogen oder bis zur Perhalogensubstitution substituiert sein können.

34. Verbindung nach Anspruch 32, worin Q Phenyl oder Pyridinyl ist, optional substituiert durch Halogen, bis zur Perhalogensubstitution, Q¹ Pyridinyl, Phenyl oder Benzothiazolyl ist, optional substituiert durch Halogen, bis zur Perhalogensubstitution, Y -O-, -S-, -CH₂S-, -SCH₂-, -CH₂O-, -OCH₂- oder -CH₂- ist, X¹ wie in Anspruch 32 definiert ist und Z -SCH₃ oder -NH-C(O)-CₚH₂ₚ₊₁ ist, worin p 1-4 ist, n = 0 oder 1 und n1 = 0-1.

35. Verbindung der Formel 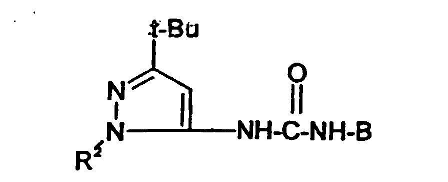 worin R² wie in Anspruch 31 definiert ist, und worin B Phenyl, Pyridinyl, Pyrimidinyl, Pyrazinyl, Pyridazinyl, Naphthyl, Chinolinyl, Isochinolinyl, Phthalimidinyl, Furyl, Thienyl, Pyrrolyl, Imidazolyl, Pyrazolyl, Oxazolyl, Isoxazolyl, Thiazolyl, Isothiazolyl, Benzofuryl, Benzothienyl, Indolyl, Benzopyrazolyl, Benzoazolyl, Benzisoxazolyl, Benzothiazolyl oder Benzisothiazolyl ist, substituiert durch einen oder mehrere Substituenten, unabhängig ausgewählt aus der Gruppe, bestehend aus Halogen, bis zur Perhalogensubstitution, und Xₙ, worin n 0 - 3 ist und jedes X unabhängig ausgewählt ist aus der Gruppe, bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂-C₁₀-Alkenyl, C₁-C₁₀-Alkoxy, C₃-C₁₀-Cycloalkyl, C₆-C₁₄-Aryl, C₃-C₁₃-Heteroaryl, bis zu perhalogensubstituiertem C₁-C₁₀-Alkyl, bis zu perhalogensubstituiertem C₂-C₁₀-Alkenyl, bis zu perhalogensubstituiertem C₁-C₁₀-Alkoxy, bis zu perhalogensubstituiertem C₃-C₁₀-Cycloalkyl und -Y-Ar;
worin R⁵ und R^{5'} unabhängig ausgewählt sind aus H, C₁-C₁₀- Alkyl, C₂-C₁₀-Alkenyl, C₃-C₁₀-Cycloalkyl, C₆-C₁₄-Aryl, C₃-C₁₃-Heteroaryl, bis zu perhalogensubstituiertem C₁-C₁₀-Alkyl, bis zu perhalogensubstituiertem C₂-C₁₀-Alkenyl, bis zu perhalogensubstituiertem C₃-C₁₀-Cycloalkyl und bis zu perhalogensubstituiertem C₆-C₁₄-Aryl und C₃-C₁₃-Heteroaryl,
worin Y -O-, -S-, -N(R⁵)-, -(CH₂)ₘ-, -C(O)-, -CH(OH)-, -(CH₂)ₘO-, -NR⁵C(O)NR⁵NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ- ist,
m = 1 bis 3 und X^{a} Halogen ist; und
Ar eine 5-10-gliedrige aromatische Struktur ist, enthaltend 0 bis 4 Mitglieder der Gruppe, bestehend aus Stickstoff, Sauerstoff und Schwefel, optional substituiert durch Zₙ₁, worin n1 0 bis 3 ist und jedes Z unabhängig ausgewählt ist aus der Gruppe, bestehend aus -CN, =O, -CO₂R⁵, -C(O)NR⁵R^{5'}, -C(O)-NR⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, SO₂NR⁵R^{5'}, C₁-C₁₀-Alkyl, C₁-C₁₀-Alkoxyl, C₃-C₁₀- Cycloalkyl, bis zu perhalogensubstituiertem C₁-C₁₀-Alkyl, und bis zu perhalogensubstituiertem C₃-C₁₀-Cycloalkyl; mit der Maßgabe, dass wenn R^{a} Methyl ist, B nicht 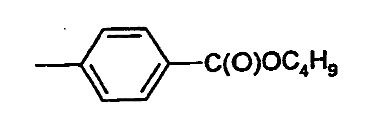 ist.

36. Verbindung nach Anspruch 31, ausgewählt aus der Gruppe, bestehend aus N-(3-tert-Butyl-5-pyrazolyl)-N'-(4- phenyloxyphenyl)harnstoff; N-(3-tert-Butyl-5-pyrazolyl)-N'-(3-(3-methylaminocarbonylphenyl)oxyphenyl)harnstoff; N-(3-tert-Butyl-5-pyrazolyl)-N'-(3-(4-pyridinyl)thiophenyl)harnstoff; N- (3-tert-Butyl-5-pyrazolyl)-N'-(4-(4- pyridinyl)thiophenyl)harnstoff; N-(3-tert-Butyl-5-pyrazolyl)-N'-(4-(4-pyridinyl)oxyphenyl)harnstoff; N-(3-tert-Butyl-5 pyrazolyl)-N'-(4-(4-pyridinyl)methylphenyl)harnstoff; N-(1-Methyl-3-tert-butyl-5-pyrazolyl)-N'-(4-phenyloxyphenyl)harnstoff; N-(1-Methyl-3-tert-butyl-5- pyrazolyl)-N'-(3-(4-pyridinyl)thiophenyl)harnstoff; N-(1-Methyl-3-tert-butyl-5-pyrazolyl)-N'-((4-(4-pyridinyl)thiomethyl)phenyl)harnstoff; N-(1-Methyl-3-tert- butyl-5-pyrazolyl)-N'-(4-(4-pyridinyl)thiophenyl)harnstoff; N-(1-Methyl-3-tert-butyl-5-pyrazolyl)-N'-(4-(4-pyridinyl)oxyphenyl)harnstoff; N-(1-Methyl-3-tert-butyl-5-pyrazolyl)-N'-((4-(4-pyridinyl)methyloxy)phenyl)harnstoff; N-(1-Methyl-3-tert-butyl-5-pyrazolyl)-N'-(3-(2-benzothiazolyl)oxyphenyl)harnstoff; N-(3-tert-Butyl-5-pyrazolyl)-N'-(3-(4-pyridyl)thiophenyl)harnstoff; N-(3-tert- Butyl-5-pyrazolyl)-N'-(4-(4-pyridyl)thiophenyl)harnstoff; N-(3-tert-Butyl-5-pyrazolyl)-N'-(3-(4-pyridyl)oxyphenyl)harnstoff; N-(3-tert-Butyl-5-pyrazolyl)-N'-(4-(4-pyridyl)oxyphenyl)harnstoff; N-(1-Methyl-3-tert-butyl-5- pyrazolyl)-N'-(3-(4-pyridyl)thiophenyl)harnstoff; N-(1-Methyl- 3-tert-butyl-5-pyrazolyl)-N'-(4-(4-pyridyl)thiophenyl)harnstoff; N-(1-Methyl-3-tert-butyl-5- pyrazolyl)-N'-(3-(4-pyridyl)oxyphenyl)harnstoff; N-(1-Methyl-3-tert-butyl-5-pyrazolyl)-N'-(4-(4-pyridyl)oxyphenyl)harnstoff; und pharmazeutisch verträgliche Salze davon.

37. Verbindung der Formel 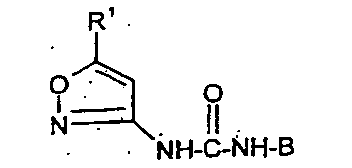 worin R¹ ausgewählt ist aus der Gruppe, bestehend aus C₃-C₁₀-Alkyl, C₃-C₁₀-Cycloalkyl, bis zu perhalogensubstituiertem C₃-C₁₀-Alkyl und bis zu perhalogensubstituiertem C₃-C₁₀-Cycloalkyl;
B Phenyl, Pyridinyl, Indolinyl, Isochinolinyl, Chinolinyl oder Naphthyl ist, das
substituiert ist durch Phenyl, Pyridinyl oder Y-R,
worin die cyclischen Strukturen von B optional substituiert sind durch Halogen, bis zur Perhalogensubstitution, und
optional substituiert durch X¹ₙ, worin n = 0-2;
jedes X¹ unabhängig ausgewählt ist aus der Gruppe, bestehend aus -CN, -CO₂R⁵, -C(O)R⁵, -C(O)NR⁵R^{5'}, -OR⁵, -NO₂, -NR⁵R^{5'}, C₁-C₁₀-Alkyl, C₂₋₁₀- Alkenyl, C₁₋₁₀-Alkoxy, C₃-C₁₀-Cycloalkyl und C₆-C₁₄-Aryl, -SR⁵, -NR⁵C(O)OR^{5'}, NR⁵C(O)R^{5'}, C₃-C₁₃-Heteroaryl, substituiertem C₁-C₁₀-Alkyl, substituiertem C₂-₁₀-Alkenyl, substituiertem C₁-₁₀-Alkoxy, substituiertem C₃-C₁₀-Cycloalkyl, substituiertem C₆-C₁₄-Aryl, substituiertem C₃-C₁₃-Heteroaryl, und
worin, falls X¹ eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀-Alkyl, C₂-₁₀-Alkenyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalogensubstituiertem C₁-C₁₀-Alkyl, bis zu perhalogensubstituiertem C₂-₁₀-Alkenyl und bis zu perhalogensubstituiertem C₃-C₁₀-Cycloalkyl,
worin Y -O-, -S-, -N(R⁵)-, -(CH₂)ₘ, -C(O)-, -CH(OH)-, -(CH₂)ₘO-, -NR⁵C(O)NR⁵R^{5'}, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ-, ist
m = 1-3 und X^{a} Halogen ist; und
Ar eine 5-10-gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zu Perhalogen, und optional substituiert durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl und substituiertem C₃-C₁₀-Cycloalkyl; worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxyl, C₃-C₁₀-Cycloalkyl, mit der Maßgabe, dass wenn R¹ t-Butyl ist, B nicht 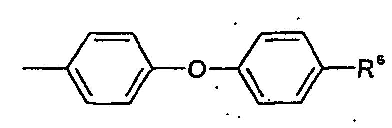 ist, worin R⁶-NHC(O)-O-t-butyl, -O-n-Pentyl, -O-n-Butyl, -O-n-Propyl, -C(O)NH-(CH₃)₂, -OCH₂CH(CH₃)₂ oder 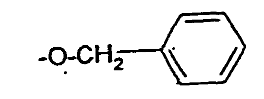 ist.

38. Verbindung nach Anspruch 37, worin B 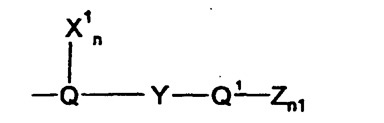 ist,
Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution,
Q¹ eine mono- oder bicyclische aromatische Struktur mit 3 bis 10 Kohlenstoffatomen ist und mit 0-4 Mitgliedern aus der Gruppe, bestehend aus N, O und S, substituiert oder unsubstituiert durch Halogen, bis zur Perhalogensubstitution, X¹ C₁-C₄-Alkyl oder halogensubstituiertes C₁-C₄-Alkyl, bis zu Perhalogen, ist und
Z, n und n1 wie in Anspruch 37 definiert sind.

39. Verbindung nach Anspruch 38, worin
Q Phenyl oder Pyridinyl ist, substituiert oder unsubstituiert durch Halogen, bis zur Perhalogensubstitution,
Q¹ ausgewählt ist aus der Gruppe, bestehend aus Phenyl, Pyridinyl, Naphthyl, Pyrimidinyl, Chinolin, Isochinolin, Imidazol und Benzothiazolyl, optional substituiert durch Halogen, bis zu Perhalogen, X¹ wie in Anspruch 38 definiert ist und
Z ausgewählt ist aus der Gruppe, bestehend aus -R⁶, -OR⁶ und -NHR⁷, worin R⁶ Wasserstoff, C₁-C₁₀-Alkyl oder C₃-C₁₀-Cycloalkyl ist und R⁷ ausgewählt ist aus der Gruppe, bestehend aus Wasserstoff, C₃-C₁₀-Alkyl und C₃-C₆-Cycloalkyl, worin R⁶ und R⁷ substituiert sein können durch Halogen oder bis zur Perhalogensubstitution.

40. Verbindung nach Anspruch 38, worin Q Phenyl oder Pyridinyl ist, optional substituiert durch Halogen, bis zur Perhalogensubstitution, Q¹ Pyridinyl, Phenyl oder Benzothiazolyl, optional substituiert durch Halogen bis zur Perhalogensubstitution, ist, Y = -O-, -S-, -C(O)- oder -CH₂-, X¹ wie in Anspruch 38 definiert ist und Z -NH-C(O)-CₚH₂ₚ₊₁, worin p 1-4 ist, -CH₃, -OH, -OCH₃, -OC₂H₅, -CN oder -C(O)CH₃ ist, n = 0 oder 1 und n1 = 0 oder 1.

41. Verbindung nach Anspruch 37, ausgewählt aus der Gruppe, bestehend aus:
N-(5-tert-Butyl-3-isoxazolyl)-N'-(4-(4- hydroxyphenyl)oxyphenyl)harnstoff; N-(5-tert-Butyl-3-isoxazolyl)-N'-(4-(3-hydroxyphenyl)oxyphenyl)harnstoff; N-(5-tert-Butyl-3-isoxazolyl)-N'-(4-(4-acetylphenyl)oxyphenyl)harnstoff; N-(5-tert-Butyl-3-isoxazolyl)-N'-(3-benzoylphenyl)harnstoff; N-(5-tert-Butyl-3-isoxazolyl)-N'-(4-phenyloxyphenyl)harnstoff; N-(5-tert-Butyl-3-isoxazolyl)-N'-(4-(3-methylaminocarbonyl- phenyl)thiophenyl)harnstoff; N-(5-tert-Butyl-3-isoxazolyl)-N'- (4-(4-(1,2-methylendioxy)phenyl)oxyphenyl)harnstoff; N-(5-tert-Butyl-3-isoxazolyl)-N'-(4-(3-pyridinyl)oxyphenyl)-harnstoff; N-(5-tert-Butyl-3-isoxazolyl)-N'-(4-(4-pyridinyl)oxyphenyl)harnstoff; N-(5-tert-Butyl-3-isoxazolyl)-N'-(4-(4-pyridyl)thiophenyl)harnstoff; N-(5-tert-Butyl-3-isoxazolyl)-N'-(4-(4-pyridinyl)methylphenyl)harnstoff; N-(5-tert-Butyl-3-isoxazolyl)-N'-(3-(4-pyridinyl)oxyphenyl)harnstoff; N-(5-tert-Butyl-3-isoxazolyl)- N'-(3-(4-pyridinyl)thiophenyl)harnstoff; N-(5-tert-Butyl-3-isoxazolyl)-N'-(3-(3-methyl-4-pyridinyl)oxyphenyl)harnstoff; N-(5-tert-Butyl-3-isoxazolyl)-N'-(3-(3-methyl-4-pyridinyl)thiophenyl)harnstoff; N-(5-tert-Butyl-3-isoxazolyl)-N'-(4-(3-methyl-4-pyridinyl)thiophenyl)harnstoff; N-(5-tert-Butyl-3-isoxazolyl)-N'-(3-(4-methyl-3-pyridinyl)oxyphenyl)harnstoff; N-(5-tert-Butyl-3-isoxazolyl)-N'-(4-(3-methyl-4-pyridinyl)oxyphenyl)harnstoff; N-(5-tert-Butyl-3-isoxazolyl)-N'-(3-(2-benzothiazolyl)oxyphenyl)harnstoff; N-(5-tert-Butyl-3-isoxazolyl)-N'-(3-chlor-4-(4-(2-methylcarbamoyl)-pyridyl)oxyphenyl)harnstoff; N-(5-tert-Butyl-3-isoxazolyl)-N'-(4-(4-(2-methylcarbamoyl)pyridyl)oxyphenyl)harnstoff; N-(5-tert-Butyl-3-isoxazolyl)-N'-(3-(4-(2-methylcarbamoyl)-pyridyl)thiophenyl)harnstoff; N-(5-tert-Butyl-3-isoxazolyl)-N'-(2-methyl-4-(4-(2-methylcarbamoyl)pyridyl)oxyphenyl)-harnstoff; N-(5-tert-Butyl-3-isoxazolyl)-N'-(-4(4-(2-carbamoyl)pyridyl)oxyphenyl)harnstoff; N-(5-tert-Butyl-3-isoxazolyl)-N'-(3-(4-(2-carbamoyl)pyridyl)oxyphenyl)harnstoff; N-(5-tert-Butyl-3-isoxazolyl)-N'-(3-(4-(2-methylcarbamoyl)pyridyl)-oxyphenyl)harnstoff; N-(5-tert-Butyl-3-isoxazolyl)-N'-(4-(4-(2-methylcarbamoyl)pyridyl)-thiophenyl)harnstoff; N-(5-tert-Butyl-3-isoxazolyl)-N'-(3-chlor-4-(4-(2-methylcarbamoyl)pyridyl)oxyphenyl)harnstoff; N-(5-tert-Butyl-3-isoxazolyl)-N'-(4-(3-methylcarbamoyl)phenyl)-oxyphenyl)harnstoff, und pharmazeutisch verträgliche Salze davon.

42. Verbindung der Formel 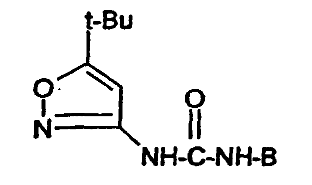 worin B 5-Methyl-2-thienyl ist oder
ausgewählt ist aus der Gruppe, bestehend aus Phenyl, Pyridinyl, Pyrimidinyl, Pyrazinyl, Pyridazinyl, Naphthyl, Chinolinyl, Isochinolinyl, Phthalimidinyl, Furyl, Thienyl, Pyrrolyl, Imidazolyl, Pyrazolyl, Oxazolyl, Isoxazolyl, Thiazolyl, Isothiazolyl, Benzofuryl, Benzothienyl, Indolyl, Benzopyrazolyl, Benzoxazolyl, Benzisoxazolyl, Benzothiazolyl oder Benzisothiazolyl, substituiert durch einen oder mehrere Substituenten, unabhängig ausgewählt aus der Gruppe, bestehend aus Halogen, bis zur Perhalogensubstitution, und Xₙ, worin n 0-3 ist und jedes X unabhängig ausgewählt ist aus der Gruppe, bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂-C₁₀-Alkenyl, C₁-C₁₀-Alkoxy, C₃-C₁₀-Cycloalkyl, C₆-C₁₄-Aryl, C₃-C₁₃-Heteroaryl, bis zu perhalogensubstituiertem C₁-C₁₀-Alkyl, bis zu perhalogensubstituiertem C₂-C₁₀-Alkenyl, bis zu perhalogensubstituiertem C₁-C₁₀-Alkoxy und bis zu perhalogensubstituiertem C₃-C₁₀-Cycloalkyl;
worin R⁵ und R^{5'} unabhängig ausgewählt sind aus H, C₁-C₁₀- Alkyl, C₂-C₁₀-Alkenyl, C₃-C₁₀-Cycloalkyl, C₆-C₁₄-Aryl, C₃-C₁₃-Heteroaryl, bis zu perhalogensubstituiertem C₁-C₁₀-Alkyl, bis zu perhalogensubstituiertem C₂-C₁₀-Alkenyl und bis zu perhalogensubstituiertem C₃-C₁₀-Cycloalkyl,
worin Y -O-, -S-, -N(R⁵)-, -(CH₂)ₘ-, -C(O)-, -CH(OH)-, -(CH₂)ₘO-, -NR⁵C(O)NR⁵NR^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ- ist,
m = 1 bis 3 und X^{a} Halogen ist; und
Ar eine 5-10-gliedrige aromatische Struktur ist, enthaltend 0 bis 4 Mitglieder der Gruppe, bestehend aus Stickstoff, Sauerstoff und Schwefel, optional substituiert durch Halogen, bis zur Perhalogensubstitution, und optional substituiert durch Zₙ₁, worin n1 0 bis 3 ist und jedes Z unabhängig ausgewählt ist aus der Gruppe, bestehend aus -CN, =O, -CO₂R⁵, -C(O)NR⁵R^{5'}, -C(O)-NR⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, SO₂NR⁵R^{5'}, C₁-C₁₀-Alkyl, C₁-C₁₀-Alkoxyl, C₃-C₁₀-Cycloalkyl, bis zu perhalogensubstituiertem C₁-C₁₀-Alkyl, und bis zu perhalogensubstituiertem C₃-C₁₀-Cycloalkyl, mit der Maßgabe, dass B nicht 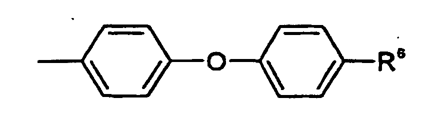 ist,
worin R⁶ -NHC(O)-O-t-butyl, -O-n-Pentyl, -O-n-Butyl, -O-n-Propyl, -C(O)NH-(CH₃)₂, -OCH₂CH(CH₃)₂ oder 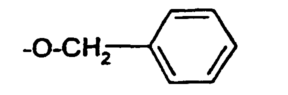 ist.

43. Verbindung der Formel 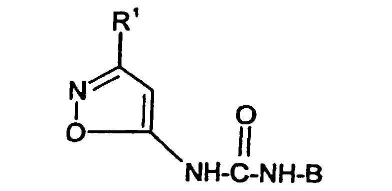 worin R¹ ausgewählt ist aus der Gruppe, bestehend aus C₃-C₁₀-Alkyl, C₃-C₁₀-Cycloalkyl, bis zu perhalogensubstituiertem C₃-C₁₀-Alkyl und bis zu perhalogensubstituiertem C₃-C₁₀-Cycloalkyl und
worin B Phenyl, Pyridinyl, Indolinyl, Isochinolinyl, Chinolinyl oder Naphthyl ist,
das substituiert ist durch X, optional substituiert durch Halogen, bis zur Perhalogensubstitution, und optional substituiert ist durch X¹ₙ, worin n = 0-2;
jedes X¹ unabhängig ausgewählt ist aus der Gruppe von X oder aus der Gruppe, bestehend aus -CN, -CO₂R⁵, -C(O)R⁵, -C(O)NR⁵R^{5'}, -OR⁵, -NO₂, -NR⁵R^{5'}, C₁-C₁₀-Alkyl, C₂-₁₀-Alkenyl, C₁-₁₀-Alkoxy, C₃-C₁₀-Cycloalkyl, C₆-C₁₄-Aryl und C₇-C₂₄-Alkaryl und
X ausgewählt ist aus der Gruppe, bestehend aus -SR⁵, -NR⁵C(O)OR^{5'}, NR⁵C(O)R^{5'}, C₃-C₁₃-Heteroaryl, C₁-C₁₀-Alkyl, substituiertem C₂-₁₀-Alkenyl, substituiertem C₁-₁₀-Alkoxy, substituiertem C₃-C₁₀-Cycloalkyl, substituiertem C₆-C₁₄-Aryl, substituiertem C₃-C₁₃-Heteroaryl und -Y-Ar, und
worin, falls X eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀- Alkyl, C₂-₁₀-Alkenyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalogensubstituiertem C₁-C₁₀-Alkyl, bis zu perhalogensubstituiertem C₂-₁₀-Alkenyl, bis zu perhalogensubstituiertem C₃-C₁₀-Cycloalkyl,
worin Y -O-, -S-, -N(R⁵)-, -(CH₂)ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ-, ist
m = 1-3 und X^{a} Halogen ist, und
Ar eine 5- oder 10-gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zu Perhalogen und gegebenenfalls substituiert durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl und substituiertem C₃-C₁₀-Cycloalkyl,
worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'} und -NR⁵C(O)OR^{5'}, C₁-C₁₀-Alkyl, C₁-C₁₀-Alkoxyl und C₃-C₁₀-Cycloalkyl, und worin wenn R¹ CH₂-t-Butyl ist, B nicht 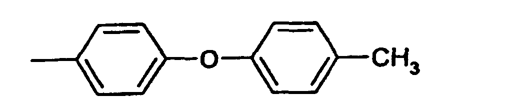 ist.

44. Verbindung nach Anspruch 43, worin B 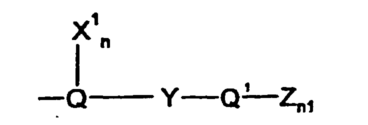 ist,
worin Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution,
Q¹ eine mono- oder bicyclische aromatische Struktur mit 3 bis 10 Kohlenstoffatomen ist und 0-4 Mitgliedern der Gruppe, bestehend aus N, O und S, substituiert oder unsubstituiert durch Halogen bis zur Perhalogensubstitution,
Z, n und n1 wie in Anspruch 43 definiert sind und X¹ C₁-C₄-Alkyl oder halogensubstituiertes C₁-C₄-Alkyl, bis zu Perhalogen, ist.

45. Verbindung nach Anspruch 44, worin
Q Phenyl oder Pyridinyl ist, substituiert oder unsubstituiert durch Halogen, bis zur Perhalogensubstitution
Q¹ ausgewählt ist aus der Gruppe, bestehend aus Phenyl, Pyridinyl, Naphthyl, Pyrimidinyl, Chinolin, Isochinolin, Imidazol und Benzothiazolyl, optional substituiert durch Halogen, bis zu Perhalogen, X¹ wie in Anspruch 44 definiert ist, und
Z ausgewählt ist aus der Gruppe, bestehend aus -R⁶, -OR⁶ und -NHR⁷, worin R⁶ Wasserstoff, C₁-C₁₀-Alkyl oder C₃-C₁₀-Cycloalkyl ist und R⁷ ausgewählt ist aus der Gruppe, bestehend aus Wasserstoff, C₃-C₁₀-Alkyl und C₃-C₆-Cycloalkyl, worin R⁶ und R⁷ durch Halogen bis zur Perhalogensubstitution substituiert sein können.

46. Verbindung der Formel 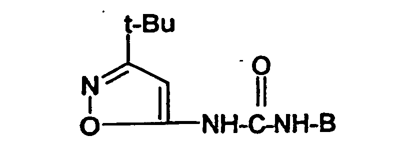 worin B wie in Anspruch 1 definiert ist.

47. Verbindung nach Anspruch 44, worin Q Phenyl oder Pyridinyl ist, optional substituiert durch Halogen, bis zur Perhalogensubstitution, Q¹ Phenyl, Benzothiazolyl oder Pyridinyl ist, optional substituiert durch Halogen, bis zur Perhalogensubstitution, Y -O-, -S- oder CH₂- ist, X¹ wie in Anspruch 44 definiert ist, n = 0 oder 1, Z -CH₃, -Cl, -OC₂H₅ oder -OCH₃ ist, und n1 = 0 oder 1.

48. Verbindung nach Anspruch 43, ausgewählt aus der Gruppe, bestehend aus:
N-(3-Isopropyl-5-isoxazolyl)-N'-(4-(4-pyridinyl)thiophenyl)harnstoff; N-(3-tert-Butyl-5-isoxazolyl)-N'-(4-(4-methoxyphenyl)oxyphenyl)harnstoff; N-(3-tert-Butyl-5-isoxazolyl)-N'-(5-(2-(4-acetylphenyl)oxy)pyridinyl)harnstoff; N-(3-tert-Butyl-5-isoxazolyl)-N'-(3-(4-pyridinyl)thiophenyl)harnstoff; N-(3-tert-Butyl-5-isoxazolyl)- N'-(4-(4-pyridinyl)methylphenyl)harnstoff; N-(3-tert-Butyl-5-isoxazolyl)-N'-(4-(4-pyridinyl)thiophenyl)harnstoff; N-(3-tert-Butyl-5-isoxazolyl)-N'-(4-(4-pyridinyl)oxyphenyl)harnstoff; N-(3-tert-Butyl-5-isoxazolyl)-N'-(4-(4-methyl-3-pyridinyl)oxyphenyl)harnstoff; N-(3-tert-Butyl-5-isoxazolyl)- N'-(3-(2-benzothiazolyl)oxyphenyl)harnstoff; N-(3-(1,1-Dimethylpropyl)-5-isoxazolyl)-N'-(4-(4-methylphenyl)oxyphenyl)harnstoff; N-(3-(1,1-Dimethylpropyl)-5-isoxazolyl)-N'-(3-(4-pyridinyl)thiophenyl)harnstoff; N-(3- (1,1-Dimethylpropyl)-5-isoxazolyl)-N'-(4-(4-pyridinyl)oxyphenyl)harnstoff; N-(3- (1,1-Dimethylpropyl)-5-isoxazolyl)-N'-(4-(4-pyridinyl)thiophenyl)harnstoff; N-(3- (1,1-Dimethylpropyl)-5-isoxazolyl)-N'-(5-(2-(4-methoxyphenyl)oxy)pyridinyl)harnstoff; N-(3-(1-Methyl-1-ethylpropyl)-5-isoxazolyl)-N'-(4-(4-pyridinyl)oxyphenyl)-harnstoff; N-(3-(1-Methyl-1-ethylpropyl)-5-isoxazolyl)-N'-(3-(4-pyridinyl)thiophenyl)harnstoff; N-(3-Isopropyl-5-isoxazolyl)-N'-(3-(4-(2-methylcarbamoyl)pyridyl)-oxyphenyl)harnstoff; N-(3-Isopropyl-5-isoxazolyl)-N'-(4-(4-(2- methylcarbamoyl)pyridyl)oxyphenyl)harnstoff; N-(3-tert-Butyl-5-isoxazolyl)-N'-(3-(4-(2-methylcarbamoyl)-pyridyl)oxyphenyl)harnstoff; N-(3-tert-Butyl-5-isoxazolyl)-N'-(4-(4-(2-methylcarbamoyl)pyridiyl)oxyphenyl)harnstoff; N-(3-tert-Butyl-5-isoxazolyl)-N'-(3-(4-(2-methylcarbamoyl)pyridyl)-thiophenyl)harnstoff; N-(3-(1,1-Dimethylprop-1-yl)-5-isoxazolyl)-N'-(3-(4-(2-methylcarbamoyl)pyridyl)-oxyphenyl)harnstoff; N-(3-(1,1-Dimethylprop-1-yl)-5-isoxazolyl)-N'-(4-(4-(2-methylcarbamoyl)pyridyl)oxyphenyl)-harnstoff; N-(3-tert-Butyl-5-isoxazolyl)-N'-(3-chlor-4-(4-(2- methylcarbamoyl)pyridyl)thiophenyl)-harnstoff; und pharmazeutisch verträgliche Salze davon.

49. Verbindung der Formel 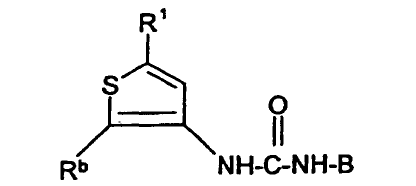 worin R¹ ausgewählt ist aus der Gruppe, bestehend aus C₃-C₁₀-Alkyl, C₃-C₁₀-Cycloalkyl, bis zu perhalogensubstituiertem C₃₋₁₀-Alkyl und bis zu perhalogensubstituiertem C₃-C₁₀-Cycloalkyl,
R^{b} Wasserstoff oder Halogen ist und
B Phenyl, Pyridinyl, Indolinyl, Isochinolinyl, Chinolinyl oder Naphthyl, substituiert durch Phenyl, Pyridinyl oder Y-Ar, ist,
worin die cyclischen Strukturen von B optional substituiert sind durch Halogen, bis zu Perhalogen, und
optional substituiert durch X¹ₙ
und
worin n = 0-2, jedes X¹ unabhängig ausgewählt ist aus der Gruppe, bestehend aus -CN, -OR⁵, -NR⁵R^{5'}, C₁-C₁₀-Alkyl;
-CO₂R⁵, -C(O)NR⁵R^{5'}, C(O)R⁵,
-NO₂, -SR⁵, -NR⁵C(O)OR^{5'}, -NR⁵C(O)R^{5'}, C₃-C₁₀-Cycloalkyl, substituiertem C₁-C₁₀-Alkyl, substituiertem C₂₋₁₀-Alkenyl, substituiertem C₁₋₁₀- Alkoxy und substituiertem C₃-C₁₀-Cycloalkyl,
worin, falls X¹ eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀- Alkyl, C₂-₁₀-Alkenyl, C₃-C₁₀-Cycloalkyl, bis zu perhalogensubstituiertem C₁-C₁₀-Alkyl, bis zu perhalogensubstituiertem C₂₋₁₀-Alkenyl und bis zu perhalogensubstituiertem C₃-C₁₀-Cycloalkyl,
worin Y -O-, -S-, -N(R⁵)-, -(CH₂)ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ-, ist
m = 1-3 und X^{a} Halogen ist, und
Ar eine 5-10-gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zur Perhalogensubstitution, und optional substituiert ist durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl und substituiertem C₃-C₁₀-Cycloalkyl; worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxyl und C₃-C₁₀-Cycloalkyl,
mit der Maßgabe, dass wenn R¹ t-Butyl und R^{b} H ist, B nicht die Formel 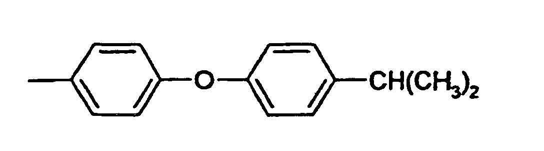 aufweist.

50. Verbindung nach Anspruch 49, worin B 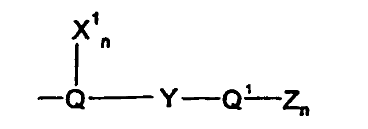 ist, worin
Y ausgewählt ist aus der Gruppe, bestehend aus -O-, -S-, -CH₂-, -SCH₂-, -CH₂S-, -CH(OH)-, -C(O)-, -CX^{a2}, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution,
Q¹ eine mono- oder bicyclische aromatische Struktur mit 3 bis 10 Kohlenstoffatomen und 0-4 Mitgliedern der Gruppe, bestehend aus N, O und S, ist, optional substituiert durch Halogen, bis zur Perhalogensubstitution, X¹ C₁-C₄-Alkyl oder halogensubstituiertes C₁-C₄-Alkyl, bis zu Perhalogen, ist und Z, n und n1 wie in Anspruch 49 definiert sind.

51. Verbindung nach Anspruch 50, worin
Q Phenyl oder Pyridinyl ist, substituiert oder unsubstituiert durch Halogen, bis zur Perhalogensubstitution
Q¹ ausgewählt ist aus der Gruppe, bestehend aus Phenyl, Pyridinyl, Naphthyl, Pyrimidinyl, Chinolin, Isochinolin, Imidazol und Benzothiazolyl, substituiert oder unsubstituiert durch Halogen, bis zu Perhalogen, X¹ wie in Anspruch 50 definiert ist, und
Z ausgewählt ist aus der Gruppe, bestehend aus -R⁶, -OR⁶ und -NHR⁷,
worin R⁶ Wasserstoff, C₁-C₁₀-Alkyl oder C₃-C₁₀-Cycloalkyl ist und R⁷ ausgewählt ist aus der Gruppe, bestehend aus Wasserstoff, C₃-C₁₀-Alkyl und C₃-C₆-Cycloalkyl, worin R⁶ und R⁷ durch Halogen bis zur Perhalogensubstitution substituiert sein können.

52. Verbindung der Formel 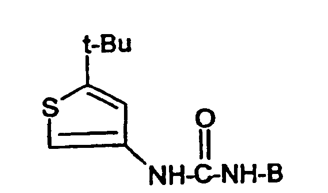 worin B wie ein Anspruch 1 definiert ist, mit der Maßgabe, dass B nicht die Formel 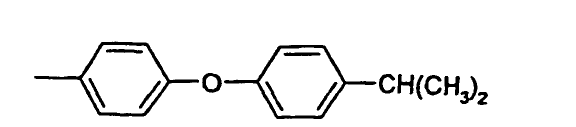 aufweist.

53. Verbindung nach Anspruch 50, worin Q Phenyl ist, optional substituiert durch Halogen bis zur Perhalogensubstitution, Q¹ Phenyl oder Pyridinyl ist, optional substituiert durch Halogen bis zur Perhalogensubstitution, und Y -O- oder -S- ist, Z -Cl, -CH₃, -OH oder -OCH₃ ist, X¹ wie in Anspruch 50 definiert ist, n = 0 oder 1 und n1 = 0-2.

54. Verbindung nach Anspruch 49, ausgewählt aus der Gruppe, bestehend aus : N-(5-tert-Butyl-3-thienyl)-N'-(4-(3- methylphenyl)oxyphenyl)harnstoff; N-(5-tert-Butyl-3-thienyl)-N'-(4-(4-hydroxyphenyl)oxyphenyl)harnstoff; N-(5-tert-Butyl-3-thienyl)-N'-(4-(4-methoxyphenyl)oxyphenyl)harnstoff; N-(5-tert-Butyl-3-thienyl)-N'-(4-(4-pyridinyl)thiophenyl)harnstoff; und pharmazeutisch verträgliche Salze davon.

55. Verbindung der Formel 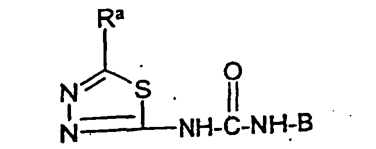 worin R^{a} C₁-C₁₀-Alkyl, C₃-C₁₀-Cycloalkyl, bis zu perhalogensubstituiertem C₁-C₁₀-Alkyl und perhalogensubstituiertem C₃-C₁₀-Cycloalkyl ist; und
B Phenyl, Pyridinyl, Indolinyl, Isochinolinyl, Chinolinyl oder Naphthyl, substituiert durch Phenyl, Pyridinyl oder -Y-Ar, ist
worin die cyclischen Strukturen von B optional substituiert sind durch Halogen, bis zu Perhalogen, und
optional substituiert durch X¹ₙ,
worin n = 0-2,
jedes X¹ unabhängig ausgewählt ist aus der Gruppe, bestehend aus -CN, -NO₂, -OR⁵, und C₁-C₁₀-Alkyl, -SR⁵, -CO₂R⁵, C(O)R⁵,
-C(O)NR⁵R^{5'}, -NR⁵R^{5'}, -NR⁵C(O)OR^{5'}, -NR⁵C(O)R^{5'}, -C₃-C₁₀-Cycloalkyl, substituiertem C₁-C₁₀- Alkyl, substituiertem C₂₋₁₀-Alkenyl, substituiertem C₁₋₁₀-Alkoxy und substituiertem C₃-C₁₀-Cycloalkyl,
worin, falls X¹ eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀- Alkyl, C₂₋₁₀-Alkenyl, C₃-C₁₀-Cycloalkyl, bis zu perhalogensubstituiertem C₁-C₁₀-Alkyl, bis zu perhalogensubstituiertem C₂₋₁₀-Alkenyl und bis zu perhalogensubstituiertem C₃-C₁₀-Cycloalkyl,
worin Y -O-, -S-, -N(R⁵)-, -(CH₂)ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ-, ist
m = 1-3 und X^{a} Halogen ist, und
Ar eine 5-10-gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zur Perhalogensubstitution und optional substituiert durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl, substituiertem C₃-C₁₀-Cycloalkyl; worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'},und -NR⁵C(O)OR^{5'}, C₁-C₁₀-Alkyl, C₁-C₁₀-Alkoxyl und C₃- C₁₀-Cycloalkyl.

56. Verbindung nach Anspruch 55, worin B 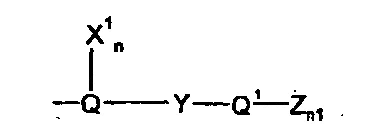 ist, worin
Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H, -CH₂O-, -OCH₂-,
X^{a} Halogen ist,
Q Phenyl oder Pyridinyl ist, substituiert oder unsubstituiert durch Halogen, bis zur Perhalogensubstiution;
Q¹ Phenyl, Pyridinyl, Naphthyl, Pyrimidyl, Chinolin, Isochinolin, Imidazolyl oder Benzothiazolyl ist, optional substituiert durch Halogen bis zur Perhalogensubstitution,
X¹ C₁-C₄-Alkyl oder halogensubstituiertes C₁-C₄-Alkyl ,bis zu Perhalogen, ist,
Z, n und n1 wie in Anspruch 55 definiert sind und s 0 oder 1 ist.

57. Verbindung nach Anspruch 56, worin
Q Phenyl oder Pyridinyl ist, substituiert oder unsubstituiert durch Halogen, bis zur Perhalogensubstitution,
Q¹ ausgewählt ist aus der Gruppe, bestehend aus Phenyl, Pyridinyl, Naphthyl, Pyrimidinyl, Chinolin, Isochinolin, Imidazol und Benzothiazolyl, optional substituiert durch Halogen, bis zu Perhalogen, X¹ definiert ist wie in Anspruch 56, und
Z ausgewählt ist aus der Gruppe, bestehend aus -R⁶, -OR⁶ und -NHR⁷, worin R⁶ Wasserstoff, C₁-C₁₀-Alkyl oder C₃-C₁₀-Cycloalkyl ist und R⁷ ausgewählt ist aus der Gruppe, bestehend aus Wasserstoff, C₃-C₁₀-Alkyl, C₃-C₆-Cycloalkyl und worin R⁶ und R⁷ durch Halogen oder bis zur Perhalogensubstitution substituiert sein können.

58. Verbindung nach Anspruch 56,
worin Q Phenyl ist, optional substituiert durch Halogen bis zur Perhalogensubstitution, Q¹ Phenyl oder Pyridinyl ist, optional substituiert durch Halogen bis zur Perhalogensubstitution, Y -O- oder -S- ist, X¹ wie in Anspruch 56 definiert ist, n = 0 oder 1 und n1 = 0.

59. Verbindung nach Anspruch 55 mit der Formel 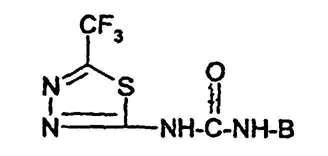 worin B Phenyl, Pyridinyl, Indolinyl, Isochinolinyl, Chinolinyl oder Naphthyl, substituiert durch Phenyl, Pyridinyl oder -Y-Ar, ist, optional substituiert durch Halogen, bis zu Perhalogen, und
worin jede cyclische Struktur von B optional substituiert ist durch X¹ₙ,
worin n = 0-2, jedes X¹ unabhängig ausgewählt ist aus der Gruppe, bestehend aus -CN, -OR⁵, -NR⁵R^{5'}, C₁-C₁₀-Alkyl;
-CO₂R⁵, -C(O)NR⁵R^{5'}, C(O)R⁵,
-NO₂, -SR⁵, -NR⁵C(O)OR^{5'}, -NR⁵C(O)R^{5'}, C₃-C₁₀-Cycloalkyl und substituiertem C₁-C₁₀- Alkyl, substituiertem C₂₋₁₀-Alkenyl, substituiertem C₁₋₁₀- Alkoxy und substituiertem C₃-C₁₀-Cycloalkyl,
worin R⁵ und R^{5'} unabhängig ausgewählt sind aus H, C₁-C₁₀- Alkyl, C₂-₁₀-Alkenyl, C₃-C₁₀-Cycloalkyl, bis zu perhalogensubstituiertem C₁-C₁₀-Alkyl, bis zu perhalogensubstituiertem C₂₋₁₀-Alkenyl, bis zu perhalogensubstituiertem C₃-C₁₀-Cycloalkyl,
worin Y -O-, -S-, -N(R⁵)-, -(CH₂)ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ-, ist
m = 1-3 und X^{a} Halogen ist, und
Ar eine 5-10 gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zur Perhalogensubstitution und optional substituiert durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl, substituiertem C₃-C₁₀-Cycloalkyl; worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxyl und C₃- C₁₀-Cycloalkyl.

60. Verbindung nach Anspruch 59, ausgewählt aus der Gruppe, bestehend aus:
N-(5-tert-Butyl-2-(1-thia-3,4-diazolyl))-N'-(3-(4-pyridinyl)thiophenyl)harnstoff; N-(5-tert-Butyl-2-(1-thia-3,4-diazolyl))-N'-(4-(4-pyridinyl)oxyphenyl)harnstoff; N-(5- tert-Butyl-2-(1-thia-3,4-diazolyl))-N'-(3-(4-(2- methylcarbamoyl)pyridyl)oxyphenyl)-harnstoff; N-(5-tert-Butyl-2-(1-thia-3,4-diazolyl))-N'-(4-(4-(2-methylcarbamoyl)pyridyl)oxyphenyl)harnstoff; N-(5-tert-Butyl-2-(1-thia-3,4-diazolyl))-N'-(3-chlor-4-(4-(2-methylcarbamoyl)-pyridyl)-oxyphenyl)harnstoff; N-(5-tert-Butyl-2-(1-thia-3,4-diazolyl))-N'-(2-chlor-4-(4-(2-methylcarbamoyl)pyridyl)oxyphenyl)harnstoff; N-(5-tert-Butyl-2-(1-thia-3,4-diazolyl))-N'-(3-(4-pyridyl)thiophenyl)harnstoff; N-(5-tert-Butyl-2-(1-thia-3,4-diazolyl))-N'-(2-methyl-4-(4-(2-methylcarbamoyl)- pyridyl)oxyphenyl)harnstoff; N-(5-(1,1-dimethylprop-1-yl)-2- (1-thia-3,4-diazolyl))-N'-(4-(3-carbamoylphenyl)oxyphenyl)-harnstoff; und pharmazeutisch verträgliche Salze davon.

61. Verbindung einer der Formeln 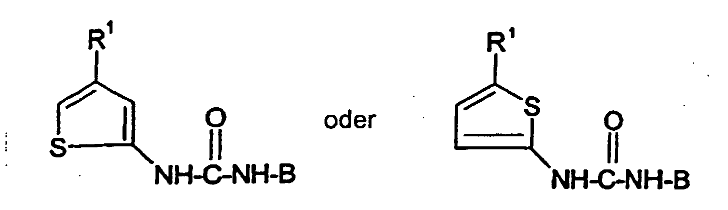 worin R¹ ausgewählt ist aus der Gruppe, bestehend aus Halogen, C₃-C₁₀-Alkyl, C₃₋₁₃-Heteroaryl, C₆₋₁₄-Aryl, C₇₋₂₄- Alkaryl, C₃-C₁₀-Cycloalkyl, bis zu perhalogensubstituiertem C₁-C₁₀-Alkyl, bis zu perhalogensubstituiertem C₃-C₁₀-Cycloalkyl, bis zu perhalogensubstituiertem C₁₋₁₃-Heteroaryl, bis zu perhalogensubstituiertem C₆₋₁₄-Aryl und bis zu perhalogensubstituiertem C₇₋₂₄-Alkaryl;
B Phenyl, Pyridinyl, Indolinyl, Isochinolinyl, Chinolinyl oder Naphthyl ist, substituiert durch Phenyl, Pyridinyl oder -Y-Ar, worin die cyclischen Strukturen von B optional substituiert sind durch Halogen, bis zu Perhalogen, und optional substituiert sind durch X¹ₙ,
worin n = 0-2;
jedes X¹ unabhängig ausgewählt ist aus der Gruppe, bestehend aus -CN, -OR⁵, -NR⁵R^{5'}, C₁-C₁₀-Alkyl;
-CO₂R⁵, -C(O)NR⁵R^{5'}, C(O)R⁵, =O,
-NO₂, -SR⁵, -NR⁵C(O)OR^{5'}, -NR⁵C(O)R^{5'}, C₃-C₁₀-Cycloalkyl, substituiertem C₁-C₁₀- Alkyl, substituiertem C₂₋₁₀-Alkenyl, substituiertem C₁₋₁₀-Alkoxy, substituiertem C₃-C₁₀-Cycloalkyl,
worin, falls X¹ eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀- Alkyl, C₂-₁₀-Alkenyl, C₃-C₁₀-Cycloalkyl, bis zu perhalogensubstituiertem C₁-C₁₀-Alkyl, bis zu perhalogensubstituiertem C₂₋₁₀-Alkenyl und bis zu perhalogensubstituiertem C₃-C₁₀-Cycloalkyl,
worin Y -O-, -S-, -N(R⁵)-, -(CH₂)ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ- ist,
m = 1-3 und X^{a} Halogen ist, und
Ar eine 5-10-gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zur Perhalogensubstitution und optional substituiert durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)NR⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₁-C₁₀-Alkoxy, C₃-C₁₀-Cycloalkyl, substituiertem C₁-C₁₀-Alkyl, substituiertem C₃-C₁₀-Cycloalkyl; worin; falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxyl und C₃- C₁₀-Cycloalkyl.

62. Verbindung einer der Formeln 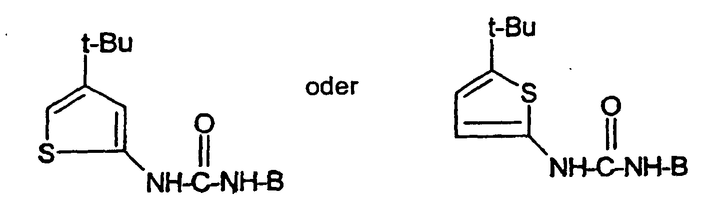 worin B wie in Anspruch 1 definiert ist.

63. Verbindung nach Anspruch 61, worin B 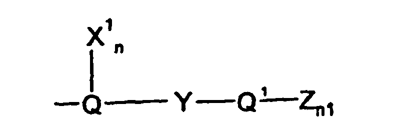 ist, worin
Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution,
Q¹ eine mono- oder bicyclische aromatische Struktur mit 3 bis 10 Kohlenstoffatomen und 0-4 Mitgliedern der Gruppe ist, bestehend aus N, O und S, unsubstituiert oder substituiert durch Halogen, bis zur Perhalogensubstitution,
X¹ C₁-C₄-Alkyl oder halogensubstituiertes C₁-C₄-Alkyl, bis zu Perhalogen, ist,
Z, n und n1 wie in Anspruch 61 oder 1 definiert sind.

64. Verbindung nach Anspruch 63, worin
Q Phenyl oder Pyridinyl ist, substituiert oder unsubstituiert durch Halogen, bis zur Perhalogensubstitution,
Q¹ ausgewählt ist aus der Gruppe, bestehend aus Phenyl, Pyridinyl, Naphthyl, Pyrimidinyl, Chinolin, Isochinolin, Imidazol und Benzothiazolyl, optional substituiert durch Halogen, bis zu Perhalogen, X¹ definiert ist wie in Anspruch 63 und Z ausgewählt ist aus der Gruppe, bestehend aus -R⁶, -OR⁶ und -NHR⁷, worin R⁶ Wasserstoff, C₁-C₁₀-Alkyl oder C₃-C₁₀-Cycloalkyl ist und R⁷ ausgewählt ist aus der Gruppe, bestehend aus Wasserstoff, C₃-C₁₀-Alkyl, C₃-C₆-Cycloalkyl, und worin R⁶ und R⁷ durch Halogen oder bis zur Perhalogensubstitution substituiert sein können.

65. Verbindung nach Anspruch 63
worin Q Phenyl ist, optional substituiert durch Halogen, bis zur Perhalogensubstitution, Q¹ Phenyl oder Pyridinyl ist, optional substituiert durch Halogen, bis zur Perhalogensubstitution, und Y -O- oder -S- ist, X¹ wie in Anspruch 63 definiert ist, n = 0 oder 1, Z -Cl, -CH₃, -OH oder OCH₃ ist und n1 = 0-2.

66. Verbindung der Formel 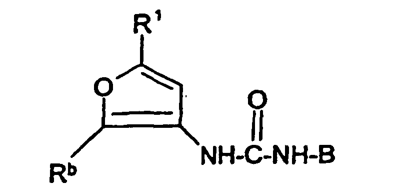 worin R¹ ausgewählt ist aus der Gruppe, bestehend aus C₃-C₁₀-Alkyl, C₃-C₁₀-Cycloalkyl, bis zu perhalogensubstituiertem C₃-C₁₀-Alkyl und bis zu perhalogensubstituiertem C₃-C₁₀-Cycloalkyl, R^{b} Wasserstoff oder Halogen ist, und
worin B Phenyl, Pyridinyl, Indolinyl, Isochinolinyl, Chinolinyl oder Naphthyl ist, substituiert durch Phenyl, Pyridinyl oder -Y-Ar, worin die cyclischen Strukturen von B optional substituiert sind durch Halogen, bis zu Perhalogen, und optional substituiert sind durch X¹ₙ,
worin n = 0-3;
jedes X¹ unabhängig ausgewählt ist aus der Gruppe, bestehend aus -CN, -CO₂R⁵, -C(O)NR⁵R^{5'}, (O)R⁵, -NO₂, -OR⁵, SR⁵, -NR⁵R^{5'}, -NR⁵C(O)OR^{5'}, -NR⁵C(O)R^{5'}, C₁-C₁₀-Alkyl, C₂₋₁₀-Alkenyl, C₁₋₁₀-Alkoxy, C₃-C₁₀-Cycloalkyl, substituiertem C₁-C₁₀- Alkyl, substituiertem C₂₋₁₀-Alkenyl, substituiertem C₁₋₁₀-Alkoxy und substituiertem C₃-C₁₀-Cycloalkyl,
worin, falls X¹ eine substituierte Gruppe ist, sie substituiert ist durch einen oder mehrere Substituenten, unabhängig ausgewählt aus der Gruppe, bestehend aus -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} und Halogen, bis zur Perhalogensubstitution;
worin R⁵ und R^{5'} unabhängig ausgewählt sind aus H, C₁-C₁₀- Alkyl, C₂-₁₀-Alkenyl, C₃-C₁₀-Cycloalkyl, bis zu perhalogensubstituiertem C₁-C₁₀-Alkyl, bis zu perhalogensubstituiertem C₂₋₁₀-Alkenyl und bis zu perhalogensubstituiertem C₃-C₁₀-Cycloalkyl,
worin Y -O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- und -N(R⁵)(CH₂)ₘ-, ist
m = 1-3 und X^{a} Halogen ist, und
Ar eine 5-10-gliedrige aromatische Struktur ist, enthaltend 0-4 Mitglieder der Gruppe, bestehend aus Stickstoff, Sauerstoff und Schwefel, welche unsubstituiert oder substituiert ist durch Halogen, bis zur Perhalogensubstitution und optional substituiert durch Zₙ₁, worin n1 0 bis 3 ist und jedes Z unabhängig ausgewählt ist aus der Gruppe, bestehend aus -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂R⁵R^{5'}, C₁-C₁₀-Alkyl, C₃-C₁₀-Cycloalkyl, substituiertem C₁-C₁₀-Alkyl, substituiertem C₃-C₁₀-Cycloalkyl; worin, falls Z eine substituierte Gruppe ist, sie substituiert ist durch einen oder mehrere Substituenten, unabhängig ausgewählt aus der Gruppe, bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxyl und C₃-C₁₀-Cycloalkyl.

67. Verbindung nach Anspruch 66, worin B 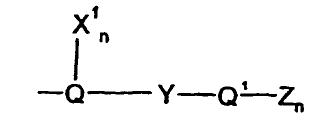 ist, worin
Y ausgewählt ist aus der Gruppe, bestehend aus -O-, -S-, -CH₂-, -SCH₂-, -CH₂S-, -CH(OH)-, -C(O)-, -CX^{a}₂, -CX^{a}H-, -CH₂O- und -OCH₂-,
X^{a} Halogen ist,
Q Phenyl oder Pyridinyl ist, substituiert oder unsubstituiert durch Halogen, bis zur Perhalogensubstitution,
Q¹ eine mono- oder bicyclische aromatische Struktur mit 3 bis 10 Kohlenstoffatomen und 0-4 Mitgliedern der Gruppe, bestehend aus N, O und S, ist, substituiert oder unsubstituiert durch Halogen bis zur Perhalogensubstitution, X¹ C₁-C₄-Alkyl oder halogensubstituiertes C₁-C₄-Alkyl, bis zu Perhalogen, ist, und
Z, n und n1 wie in Anspruch 66 definiert sind.

68. Verbindung nach Anspruch 67, worin
Q Phenyl oder Pyridinyl ist, substituiert oder unsubstituiert durch Halogen, bis zur Perhalogensubstitution,
Q¹ ausgewählt ist aus der Gruppe, bestehend aus Phenyl, Pyridinyl, Naphthyl, Pyrimidinyl, Chinolin, Isochinolin, Imidazol und Benzothiazolyl, unsubstituiert oder substituiert durch Halogen, bis zu Perhalogen, X¹ wie in Anspruch 67 definiert ist, und
Z ausgewählt ist aus der Gruppe, bestehend aus -R⁶, -OR⁶ und -NHR⁷,
worin R⁶ Wasserstoff, C₁-C₁₀-Alkyl oder C₃-C₁₀-Cycloalkyl ist, und R⁷ ausgewählt ist aus der Gruppe, bestehend aus Wasserstoff, C₃-C₁₀-Alkyl und C₃-C₆-Cycloalkyl, worin R⁶ und R⁷ durch Halogen oder bis zur Perhalogensubstitution substituiert sein können.

69. Verbindung der Formel 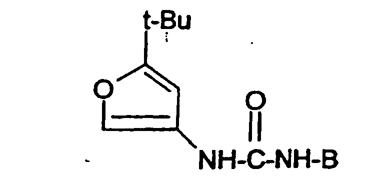 worin B wie in Anspruch 1 definiert ist.

70. Verbindung nach Anspruch 67,
worin Q Phenyl ist, optional substituiert durch Halogen, bis zur Perhalogensubstitution, Q¹ Phenyl oder Pyridinyl ist, optional substituiert durch Halogen, bis zur Perhalogensubstitution, und Y -O- oder -S- ist, X¹ wie in Anspruch 67 definiert ist, Z -Cl oder -OCH₃ ist, n = 0, s = 0 und n1 = 0-2.

71. Pharmazeutische Zusammensetzung, umfassend eine Verbindung nach Anspruch 31 und einen physiologisch verträglichen Träger.

72. Pharmazeutische Zusammensetzung, umfassend eine Verbindung nach Anspruch 37 und einen physiologisch verträglichen Träger.

73. Pharmazeutische Zusammensetzung, umfassend eine Verbindung nach Anspruch 43 und einen physiologisch verträglichen Träger.

74. Pharmazeutische Zusammensetzung, umfassend eine Verbindung nach Anspruch 49 und einen physiologisch verträglichen Träger.

75. Pharmazeutische Zusammensetzung, umfassend eine Verbindung nach Anspruch 55 und einen physiologisch verträglichen Träger.

76. Pharmazeutische Zusammensetzung, umfassend eine Verbindung nach Anspruch 61 und einen physiologisch verträglichen Träger.

77. Pharmazeutische Zusammensetzung, umfassend eine Verbindung nach Anspruch 66 und einen physiologisch verträglichen Träger.

78. Verwendung nach Anspruch 1, worin B Phenyl, Pyridinyl, Pyrimidinyl, Pyrazinyl, Pyridazinyl, Naphthyl, Chinolinyl, Isochinolinyl, Phthalimidinyl, Furyl, Pyrrolyl, Imidazolyl, Pyrazolyl, Oxazolyl, Isoxazolyl, Thiazolyl, Isothiazolyl, Benzofuryl, Benzothienyl, Indolyl, Benzopyrazolyl, Benzoazolyl, Benzisoxazolyl, Benzothiazolyl oder Benzisothiazolyl ist, substituiert durch -Y-Ar, und optional substituiert durch Halogen, bis zur Perhalogensubstitution, C₁-C₁₀-Alkyl und bis zu perhalogeniertem C₁-C₁₀-Alkyl ist, worin Y und Ar wie in Anspruch 1 definiert sind.

79. Verwendung nach Anspruch 1, worin B
a. Phenyl, Pyridinyl, Naphthyl, Chinolinyl oder Isochinolinyl ist, substituiert durch -Y-Ar, und optional substituiert durch Halogen, bis zur Perhalogensubstitution, C₁-C₁₀-Alkyl und bis zu perhalogensubstituiertem C₁-C₁₀-Alkyl, worin Y und Ar wie in Anspruch 1 definiert sind; oder
b. Indolyl, substituiert durch C₁-C₁₄-Aryl oder C₃-C₁₃-Heteroaryl, ist.

## Revendications

1. Utilisation d'un composé de formule I 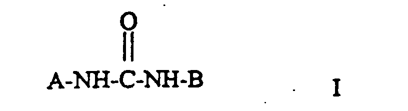 où B est phényle, pyridinyle, pyrimidinyle, pyrazinyle, pyridazinyle, naphtyle, quinolinyle, isoquinolinyle, phtalimidinyle, furyle, pyrrolyle, imidazolyle, pyrazolyle, oxazolyle, isoxazolyle, thiazolyle, isothiazolyle, benzofuryle, benzothiényle, indolyle, benzopyrazolyle, benzoxazolyle, benzisoxazolyle, benzothiazolyle ou benzisothiazolyle, substitué par un ou plusieurs substituants choisis indépendamment dans le groupe consistant en halogène, jusqu'à la perhalogénosubstitution, et Xₙ, où n est 0-3 et chaque X est choisi indépendamment dans le groupe consistant en -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, hétéroaryle en C₃-C₁₃, alkyle en C₁-C₁₀ jusqu'à perhalogéno-substitué, alcényle en C₂-C₁₀ jusqu'à perhalogéno-substitué, alcoxy en C₁-C₁₀ jusqu'à perhalogénosubstitué, cycloalkyle en C₃-C₁₀ jusqu'à perhalogénosubstitué et -Y-Ar ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre éventuellement substituée par halogène jusqu'à la perhalogénosubstitution et éventuellement substituée par Zₙ₁, où n1 est 0 à 3 et chaque Z est choisi indépendamment dans le groupe consistant en -CN, =O, -CO₂R⁵, -C(O)NR⁵R^{5'}, -C(O)-NR⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ jusqu'à perhalogénosubstitué, et cycloalkyle en C₃-C₁₀ jusqu'à perhalogénosubstitué, et
A est une entité hétéroaryle choisie dans le groupe consistant en 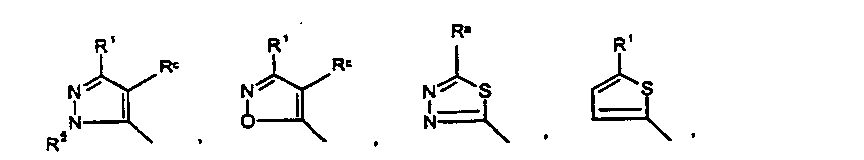 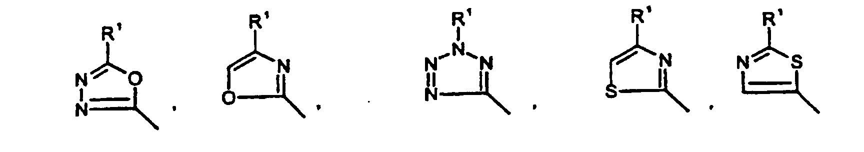 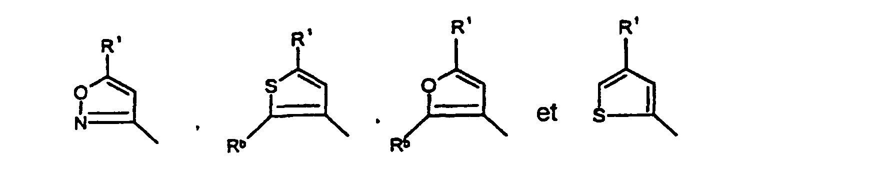 où
R¹ est choisi dans le groupe consistant en halogène, alkyle en C₃-C₁₀, cycloalkyle en C₃-C₁₀, hétéroaryle en C₁-C₁₃, aryle en C₆-C₁₄, alkylaryle en C₇-C₂₄, alkyle en C₁-C₁₀ jusqu'à perhalogénosubstitué, cycloalkyle en C₃-C₁₀ jusqu'à perhalogénosubstitué, hétéroaryle en C₁-C₁₃ jusqu'à perhalogénosubstitué, aryle en C₆-C₁₄ jusqu'à perhalogénosubstitué et alkylaryle en C₇-C₂₄ jusqu'à perhalogénosubstitué ;
R² est choisi dans le groupe consistant en H, -C(O)R⁴, -CO₂R⁴, -C(O)NR³R^{3'}, alkyle en C₁-C₁₀, cycloalkyle en C₃-C₁₀, alkylaryle en C₇-C₂₄, alkylhétéroaryle en C₄-C₂₃, alkyle en C₁-C₁₀ substitué, cycloalkyle en C₃-C₁₀ substitué, alkylaryle en C₇-C₂₄ substitué et alkylhétéroaryle en C₄-C₂₃ substitué ;
quand R² est un groupe substitué, il est substitué par un ou plusieurs substituants choisis indépendamment dans le groupe consistant en -CN, -CO₂R⁴, -C(O)-NR³R^{3'}, -NO₂, -OR⁴, -SR⁴ et halogène jusqu'à la perhalogénosubstitution,
où R³ et R^{3'} sont choisis indépendamment dans le groupe consistant en H, -OR⁴, -SR⁴, -NR⁴R^{4'}, -C(O)R⁴, -CO₂R⁴, -C(O)NR⁴R^{4'}, alkyle en C₁-C₁₀, cycloalkyle en C₃-C₁₀, aryle en C₆-C₁₄, hétéroaryle en C₃-C₁₃, alkyle en C₁-C₁₀ jusqu'à perhalogénosubstitué, cycloalkyle en C₃-C₁₀ jusqu'à perhalogénosubstitué, et aryle en C₆-C₁₄ jusqu'à perhalogénosubstitué ou hétéroaryle en C₃-C₁₃ jusqu'à perhalogénosubstitué ; et
où R⁴ et R^{4'} sont choisis indépendamment dans le groupe consistant en H, alkyle en C₁-C₁₀, cycloalkyle en C₃-C₁₀, aryle en C₆-C₁₄, hétéroaryle en C₃-C₁₃, alkyle en C₁-C₁₀ jusqu'à perhalogénosubstitué, cycloalkyle en C₃-C₁₀ jusqu'à perhalogénosubstitué, et aryle en C₆-C₁₄ jusqu'à perhalogénosubstitué ou hétéroaryle en C₃-C₁₃ jusqu'à perhalogénosubstitué ;
R^{a} est alkyle en C₁-C₁₀, cycloalkyle en C₃-C₁₀, alkyle en C₁-C₁₀ jusqu'à perhalogénosubstitué et cycloalkyle en C₃-C₁₀ jusqu'à perhalogénosubstitué ; et
R^{b} est l'hydrogène ou halogène,
R^{c} est l'hydrogène, halogène, alkyle en C₁-C₁₀, alkyle en C₁-C₁₀ jusqu'à perhalogénosubstitué ou se combine avec R¹ et les atomes de carbone cycliques auxquels R¹ et R^{c} sont liés pour former un cycle cycloalkyle, aryle ou hétéroaryle à 5
ou 6 membres avec 0-2 membres choisis parmi O, N et S ;
soumis à la condition que, quand A est : 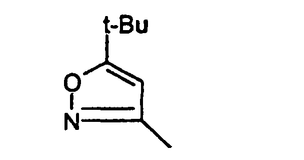 B ne soit pas 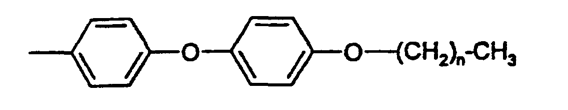 où n = 2-4,
ou 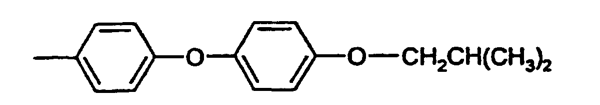 pour la production d'un médicament pour le traitement de la croissance cellulaire cancéreuse à médiation par la raf kinase.

2. Utilisation selon la revendication 1 où B est 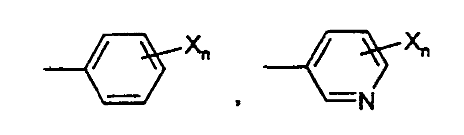 qui est substitué ou non substitué par halogène, jusqu'à la perhalogénosubstitution, et où
n = 0-3 et
chaque X et choisi indépendamment dans le groupe consistant en alkyle en C₁-C₁₀ substitué, alkyle en C₁-C₁₀ jusqu'à perhalogénosubstitué et -Y-Ar ;
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre éventuellement substituée par halogène jusqu'à la perhalogénosubstitution et éventuellement substituée par Zₙ₁, où n1 est 0 à 3 et chaque Z est choisi indépendamment dans le groupe consistant en -CN, =O, -CO₂R⁵, -C (O)NR⁵R^{5'}, -C (O)R⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ jusqu'à perhalogénosubstitué, et cycloalkyle en C₃-C₁₀ jusqu'à perhalogénosubstitué,
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué ;

3. Utilisation selon la revendication 1 où B est 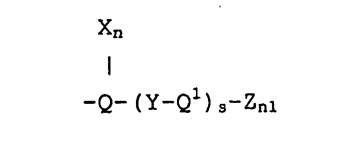 où
Y est choisi dans le groupe consistant en -O-, -S-, -CH₂-, -SCH₂-, -CH₂S-, -CH(OH)-, -C(O)-, -CX^{a}₂, -CX^{a}H-, -CH₂O-et -OCH₂-,
X^{a} est halogène,
Q est phényle ou pyridinyle, substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, éventuellement substituée par halogène jusqu'à la perhalogénosubstitution,
X, Z, n et n1 sont définis comme dans la revendication 1 et s = 0 ou 1.

4. Utilisation selon la revendication 3 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, substitué ou non substitué par halogène, jusqu'à la perhalogénosubstitution, et
chaque X est choisi indépendamment dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀ et cycloalkyle en C₃-C₆, où R⁶ et R⁷ peuvent être substitués par halogène ou jusqu'à la perhalogénosubstitution.

5. Utilisation selon la revendication 1 où le composé a la formule 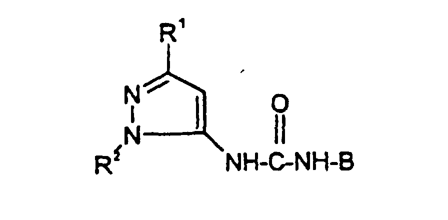 où R¹ et R² et B sont définis comme dans la revendication 1.

6. Utilisation selon la revendication 5 où B est de formule 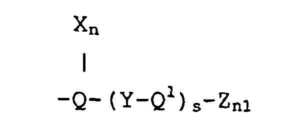 où Q est phényle ou pyridinyle, éventuellement substitué par halogène jusqu'à la perhalogénosubstitution, Q¹ est pyridinyle, phényle ou benzothiazolyle éventuellement substitué par halogène jusqu'à la perhalogénosubstitution, Y est -O-, -S-, -CH₂S-, -SCH₂-, -CH₂O-, -OCH₂- ou -CH₂-, X est alkyle en C₁-C₄ ou alkyle en C₁-C₄ jusqu'à perhalogénosubstitué et Z est défini comme dans la revendication 1, n = 0 ou 1, s = 1 et n1 = 0-1.

7. Utilisation selon la revendication 1 où le composé est choisi dans le groupe consistant en :
*N*-(3-*tert*-butyl-5-pyrazolyl)-*N'*-(4-phényloxyphényl)urée ;
*N*-(3-*tert*-butyl-5-pyrazolyl)-*N'*-(3-(3-méthylaminocarbonylphényl)oxyphényl)urée ;
*N*-(3-*tert*-butyl-5-pyrazolyl)-*N'*-(3-(4-pyridinyl)thiophényl)-urée ;
*N*-(3-*tert*-butyl-5-pyrazolyl)-*N'*-(4-(4-pyridinyl)thiophényl)urée ;
*N*-(3-*tert*-butyl-5-pyrazolyl)-*N'*-(4-(4-pyridinyl)oxyphényl)-urée ;
*N*-(3-*tert*-butyl-5-pyrazolyl)-*N'*-(4-(4-pyridinyl)méthylphényl)urée ;
*N*-(1-méthyl-3-*tert*-butyl-5-pyrazolyl)-*N'*-(4-phényloxyphényl)urée ;
*N*-(1-méthyl-3-*tert*-butyl-5-pyrazolyl)-*N'*-(3-(4-pyridinyl)-thiophényl)urée ;
*N*-(1-méthyl-3-*tert*-butyl-5-pyrazolyl)-*N*'-((4-(4-pyridinyl)-thiométhyl)phényl)urée ;
*N*-(1-méthyl-3-*tert*-butyl-5-pyrazolyl)-*N*'-(4-(4-pyridinyl)-thiophényl)urée ;
*N*-(1-méthyl-3-*tert*-butyl-5-pyrazolyl)-*N*'-(4-(4-pyridinyl)-oxyphényl)urée ;
*N*-(1-méthyl-3-*tert*-butyl-5-pyrazolyl)-*N*'-((4-(4-pyridinyl)-méthyloxy)phényl)urée ;
*N*-(1-méthyl-3-*tert*-butyl-5-pyrazolyl)-*N*'-(3-(2-benzothiazolyl)oxyphényl)urée ;
*N*-(3-*tert*-butyl-5-pyrazolyl)-*N'*-(3-(4-pyridyl)thiophényl)-urée ;
*N*-(3-*tert*-butyl-5-pyrazolyl)-*N'*-(4-(4-pyridyl)thiophényl)urée ;
*N*-(3-*tert*-butyl-5-pyrazolyl)-*N'*-(4-(4-pyridyl)oxyphényl)-urée ;
*N*-(3-*tert*-butyl-5-pyrazolyl)-*N'*-(4-(4-pyridyl)méthylphényl)urée ;
*N*-(1-méthyl-3-*tert*-butyl-5-pyrazolyl)-*N'*-(3-(4-pyridyl)-thiophényl)urée ;
*N*-(1-méthyl-3-*tert*-butyl-5-pyrazolyl)-*N'*-(4-(4-pyridyl)-thiophényl)urée ;
*N*-(1-méthyl-3-*tert*-butyl-5-pyrazolyl)-*N'*-(3-(4-pyridyl)-oxyphényl)urée ;
*N*-(1-méthyl-3-*tert*-butyl-5-pyrazolyl)-*N'*-(4-(4-pyridyl)-oxyphényl)urée ;
et ses sels pharmaceutiquement acceptables.

8. Utilisation selon la revendication 5 où R¹ est t-butyle.

9. Utilisation selon la revendication 1 où le composé a la formule 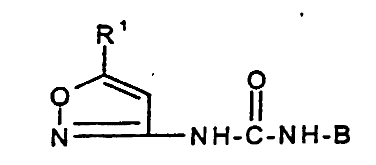 où R¹ et B sont définis comme dans la revendication 1.

10. Utilisation selon la revendication 9 où B est de formule 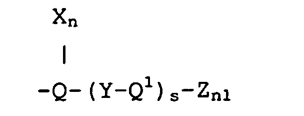 où Q est phényle ou pyridinyle, éventuellement substitué par halogène jusqu'à la perhalogénosubstitution, Q¹ est pyridinyle, phényle ou benzothiazolyle éventuellement substitué par halogène jusqu'à la perhalogénosubstitution, Y est -O-, -S-, -C(O)- ou -CH₂-, X est alkyle en C₁-C₄ ou alkyle en C₁-C₄ jusqu'à perhalogénosubstitué et Z est défini comme dans la revendication 1, n = 0 ou 1, s = 0 ou 1 et n1 = 0 ou 1.

11. Utilisation selon la revendication 1 où le composé est choisi dans le groupe consistant en :
*N*-(5-*tert*-butyl-3-isoxazolyl)-*N*'-(4-(4-hydroxyphényl)-oxyphényl)urée ;
*N*-(5-*tert*-butyl-3-isoxazolyl)-*N*'-(4-(3-hydroxyphényl)-oxyphényl)urée ;
*N*-(5-*tert*-butyl-3-isoxazolyl)-*N*'-(4-(4-acétylphényl)-oxyphényl)urée ;
*N*-(5-*tert*-butyl-3-isoxazolyl)-*N*'-(3-benzoylphényl)urée ;
*N*-(5-*tert*-butyl-3-isoxazolyl)-*N*'-(4-phényloxyphényl)urée ;
*N*-(5-*tert*-butyl-3-isoxazolyl)-*N*'-(4-(3-méthylaminocarbonyl-phényl)thiophényl)urée ;
*N*-(5-*tert*-butyl-3-isoxazolyl)-*N'*-(4-(4-(1,2-méthylènedioxy)-phényl)oxyphényl)urée ;
*N*-(5-*tert*-butyl-3-isoxazolyl)-*N'*-(4-(3-pyridinyl)oxyphényl)urée ;
*N*-(5-*tert*-butyl-3-isoxazolyl)-*N'*-(4-(4-pyridinyl)oxyphényl)urée ;
*N*-(5-*tert*-butyl-3-isoxazolyl)-*N'*-(4-(4-pyridinyl)thiophényl)urée ;
*N*-(5-*tert*-butyl-3-isoxazolyl)-*N'*-(4-(4-pyridinyl)méthylphényl)urée ;
*N*-(5-*tert*-butyl-3-isoxazolyl)-*N'*-(3-(4-pyridinyl)oxyphényl)-urée ;
*N*-(5-*tert*-butyl-3-isoxazolyl)-*N'*-(3-(4-pyridinyl)-thiophényl)urée ;
*N*-(5-*tert*-butyl-3-isoxazolyl)-*N'*-(3-(3-méthyl-4-pyridinyl)-oxyphényl)urée ;
*N*-(5-*tert*-butyl-3-isoxazolyl)-*N'*-(3-(3-méthyl-4-pyridinyl)-thiophényl)urée ;
*N*-(5-*tert*-butyl-3-isoxazolyl)-*N'*-(4-(3-méthyl-4-pyridinyl)-thiophényl)urée ;
*N*-(5-*tert*-butyl-3-isoxazolyl)-*N'*-(3-(4-méthyl-3-pyridinyl)-oxyphényl)urée ;
*N*-(5-*tert*-butyl-3-isoxazolyl)-*N'*-(4-(3-méthyl-4-pyridinyl)-oxyphényl)urée :
*N*-(5-*tert*-butyl-3-isoxazolyl)-*N'*-(3-(2-benzothiazolyl)-oxyphényl)urée ;
*N*-(5-*tert*-butyl-3-isoxazolyl)-*N*'-(3-chloro-4-(4-(2-méthylcarbamoyl)pyridyl)oxyphényl)urée ;
*N*-(5-*tert*-butyl-3-isoxazolyl)-*N*'-(4-(4-(2-méthylcarbamoyl)-pyridyl)oxyphényl)urée ;
*N*-(5-*tert*-butyl-3-isoxazolyl)-*N*'-(3-(4-(2-méthylcarbamoyl)-pyridyl)thiophényl)urée ;
*N*-(5-*tert*-butyl-3-isoxazolyl)-*N*'-(2-méthyl-4-(4-(2-méthylcarbamoyl)pyridyl)oxyphényl)urée ;
*N*-(5-*tert*-butyl-3-isoxazolyl)-*N'*-(4-(4-(2-carbamoyl)-pyridyl)oxyphényl)urée ;
*N*-(5-*tert*-butyl-3-isoxazolyl)-*N*'-(3-(4-(2-carbamoyl)-pyridyl)oxyphényl)urée ;
*N*-(5-*tert*-butyl-3-isoxazolyl)-*N*'-(3-(4-(2-méthylcarbamoyl)-pyridyl)oxyphényl)urée ;
*N*-(5-*tert*-butyl-3-isoxazolyl)-*N'*-(4-(4-(2-méthylcarbamoyl)-pyridyl)thiophényl)urée ;
*N*-(5-*tert*-butyl-3-isoxazolyl)-*N'*-(3-chloro-4-(4-(2-méthylcarbamoyl)pyridyl)oxyphényl)urée ;
*N*-(5-*tert*-butyl-3-isoxazolyl)-*N*'-(4-(3-méthylcarbamoyl)-phényl)oxyphényl)urée ;
et ses sels pharmaceutiquement acceptables.

12. Utilisation selon la revendication 10 où R¹ est t-butyle.

13. Utilisation selon la revendication 1 où le composé a la formule 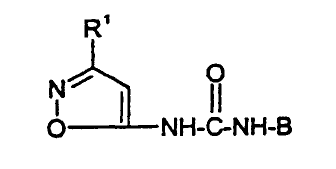 où R¹ et B sont définis comme dans la revendication 1.

14. Utilisation selon la revendication 13 où B est de formule 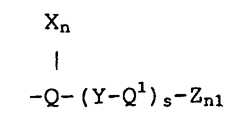 Q est phényle ou pyridinyle éventuellement substitué par halogène jusqu'à la perhalogénosubstitution, Q¹ est phényle, benzothiazolyle ou pyridinyle éventuellement substitué par halogène jusqu'à la perhalogénosubstitution, Y est -O-, -S- ou -CH₂-, X est alkyle en C₁-C₄ ou alkyle en C₁-C₄ jusqu'à perhalogénosubstitué, Z est défini comme dans la revendication 1, n = 0 ou 1, s = 1 et n1 = 0 ou 1.

15. Utilisation selon la revendication 1 où le composé est choisi dans le groupe consistant en :
*N*-(3-isopropyl-5-isoxazolyl)-*N*'-(4-(4-pyridinyl)thiophényl)-urée ;
*N*-(3-*tert*-butyl-5-isoxazolyl)-*N*'-(4-(4-méthoxyphényl)oxyphényl)urée ;
*N*-(3-*tert*-butyl-5-isoxazolyl)-*N'*-(5-(2-(4-acétylphényl)oxy)-pyridinyl)urée ;
*N*-(3-*tert*-butyl-5-isoxazolyl)-*N'*-(3-(4-pyridinyl)thiophényl)urée ;
*N*-(3-*tert*-butyl-5-isoxazolyl)-*N'*-(4-(4-pyridinyl)méthylphényl)urée ;
*N*-(3-*tert*-butyl-5-isoxazolyl)-*N'*-(4-(4-pyridinyl)thiophényl)urée ;
*N*-(3-*tert*-butyl-5-isoxazolyl)-*N'*-(4-(4-pyridinyl)oxyphényl)urée ;
*N*-(3-*tert*-butyl-5-isoxazolyl)-*N*'-(4-(4-méthyl-3-pyridinyl)oxy-phényl)urée ;
*N*-(3-*tert*-butyl-5-isoxazolyl)-*N*'-(3-(2-benzothiazolyl)oxyphényl)urée ;
*N*-(3-(1,1-diméthylpropyl)-5-isoxazolyl)-*N*'-(4-(4-méthylphényl)oxyphényl)urée ;
*N*-(3-(1,1-diméthylpropyl)-5-isoxazolyl)-*N*'-(3-(4-pyridinyl)-thiophényl)urée ;
*N*-(3-(1,1-diméthylpropyl)-5-isoxazolyl)-*N*'-(4-(4-pyridinyl)oxyphényl)urée ;
*N*-(3-(1,1-diméthylpropyl)-5-isoxazolyl)-*N'*-(4-(4-pyridinyl)-thiophényl)urée ;
*N*-(3-(1,1-diméthylpropyl)-5-isoxazolyl)-*N'*-(5-(2-(4-méthoxyphényl)oxy)pyridinyl)urée ;
*N*-(3-(1-méthyl-1-éthylpropyl)-5-isoxazolyl)-*N'*-(4-(4-pyridinyl)oxyphényl)urée ;
*N*-(3-(1-méthyl-1-éthylpropyl)-5-isoxazolyl)-*N'*-(3-(4-pyridinyl)thiophényl)urée ;
*N*-(3-isopropyl-5-isoxazolyl)-*N'*-(3-(4-(2-méthylcarbamoyl)-pyridyl)oxyphényl)urée ;
*N*-(3-isopropyl-5-isoxazolyl)-*N'*-(4-(4-(2-méthylcarbamoyl)-pyridyl)oxyphényl)urée ;
*N*-(3-*tert*-butyl-5-isoxazolyl)-*N'*-(3-(4-(2-méthylcarbamoyl)-pyridyl)oxyphényl)urée ;
*N*-(3-*tert*-butyl-5-isoxazolyl)-*N'*-(4-(4-(2-méthylcarbamoyl)-pyridyl)oxyphényl)urée ;
*N*-(3-*tert*-butyl-5-isoxazolyl)-*N'*-(3-(4-(2-méthylcarbamoyl)-pyridyl)thiophényl)urée ;
*N*-(3-(1,1-diméthylprop-1-yl)-5-isoxazolyl)-*N'*-(3-(4-(2-méthylcarbamoyl)pyridyl)oxyphényl)urée ;
*N*-(3-(1,1-diméthylprop-1-yl)-5-isoxazolyl)-*N*'-(4-(4-(2-méthylcarbamoyl)pyridyl)oxyphényl)urée ;
*N*-(3-*tert*-butyl-5-isoxazolyl)-*N'*-(3-chloro-4-(4-(2-méthylcarbamoyl)pyridyl)thiophényl)urée ;
et ses sels pharmaceutiquement acceptables.

16. Utilisation selon la revendication 13 où R¹ est t-butyle.

17. Utilisation selon la revendication 1 où le composé a la formule 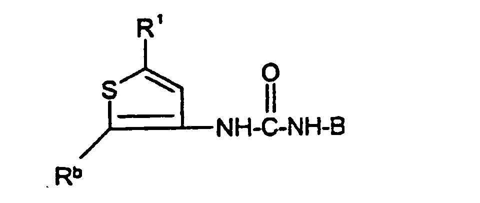 où R¹, R^{b} et B sont définis comme dans la revendication 1.

18. Utilisation selon la revendication 17 où B est de formule 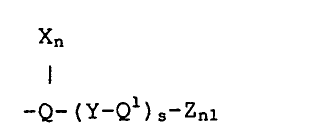 où Q est phényle éventuellement substitué par halogène jusqu'à la perhalogénosubstitution, Q¹ est phényle ou pyridinyle éventuellement substitué par halogène jusqu'à la perhalogénosubstitution, Y est -O- ou -S-, X est alkyle en C₁-C₄ ou alkyle en C₁-C₄ jusqu'à perhalogénosubstitué, Z est défini comme dans la revendication 1, n = 0 ou 1, s = 0 ou 1 et n1 = 0-2.

19. Utilisation selon la revendication 1 où le composé est choisi dans le groupe consistant en :
*N*-(5-*tert*-butyl-3-thiényl)-*N*'-(4-(3-méthylphényl)oxyphényl)-urée ;
*N*-(5-*tert*-butyl-3-thiényl)-*N*'-(4-(4-hydroxyphényl)oxyphényl)urée ;
*N*-(5-*tert*-butyl-3-thiényl)-*N*'-(4-(4-méthoxyphényl)oxyphényl)urée ;
*N*-(5-*tert*-butyl-3-thiényl)-*N*'-(4-(4-pyridinyl)thiophényl)-urée ;
et ses sels pharmaceutiquement acceptables.

20. Utilisation selon la revendication 17 où R¹ est t-butyle.

21. Utilisation selon la revendication 1 où le composé a la formule : 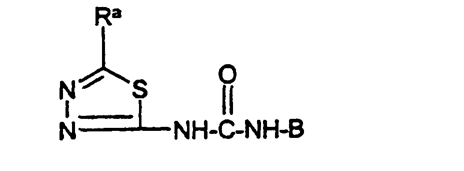 où R^{a} et B sont définis comme dans la revendication 1.

22. Utilisation selon la revendication 21 où B est de formule 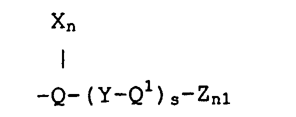 où Q est phényle éventuellement substitué par halogène jusqu'à la perhalogénosubstitution, Q¹ est phényle ou pyridinyle éventuellement substitué par halogène jusqu'à la perhalogénosubstitution,, Y est -O- ou -S-, X est alkyle en C₁-C₄ ou alkyle en C₁-C₄ jusqu'à perhalogénosubstitué, s = 1 ; Z est défini comme dans la revendication 1, n = 0 ou 1 et n1 = 0 ou 1.

23. Utilisation selon la revendication 2 où le composé est choisi dans le groupe consistant en :
*N*-(5-*tert*-butyl-2-(1-thia-3,4-diazolyl))-*N'*-(3-(4-pyridinyl)thiophényl)urée ;
*N*-(5-*tert*-butyl-2-(1-thia-3,4-diazolyl))-*N'*-(4-(4-pyridinyl)oxyphényl)urée ;
et ses sels pharmaceutiquement acceptables.

24. Utilisation selon la revendication 21 où R^{a} est CF₃- ou t-butyle.

25. Utilisation selon la revendication 1 où le composé est de l'une des formules 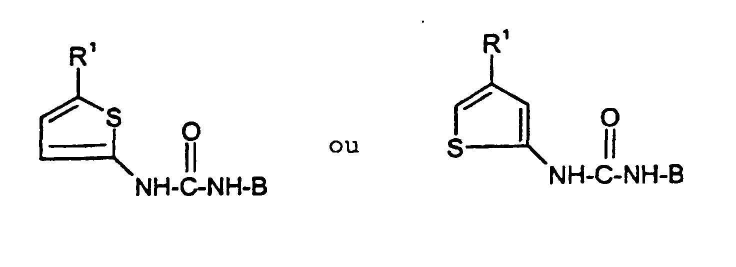 où R¹ et B sont définis comme dans la revendication 1.

26. Utilisation selon la revendication 25 où B est phényle jusqu'à perhalogénosubstitué, pyridinyle jusqu'à perhalogénosubstitué, ou de formule 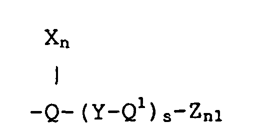 où Q est phényle éventuellement substitué par halogène jusqu'à la perhalogénosubstitution, Q¹ est phényle ou pyridinyle éventuellement substitué par halogène jusqu'à la perhalogénosubstitution, et Y est -O ou -S-, X est alkyle en C₁-C₄ ou alkyle en C₁-C₄ jusqu'à perhalogénosubstitué, Z est défini comme dans la revendication 1, n = 0 ou 1, s = 0 ou 1 et n1 = 0-2.

27. Utilisation selon la revendication 25 où R¹ est t-butyle.

28. Utilisation selon la revendication 1 où le composé est de formule 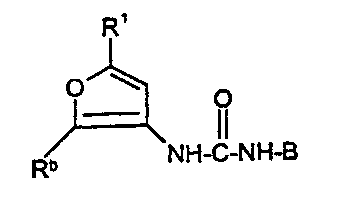 où R¹ et R^{b} et B sont définis comme dans la revendication 1.

29. Utilisation selon la revendication 28 où B est de formule 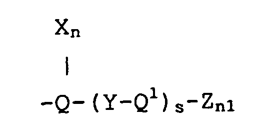 où Q est phényle éventuellement substitué par halogène jusqu'à la perhalogénosubstitution, Q¹ est phényle ou pyridinyle éventuellement substitué par halogène jusqu'à la perhalogénosubstitution, et Y est -O- ou -S-, X est alkyle en C₁-C₄ ou alkyle en C₁-C₄ jusqu'à perhalogénosubstitué, Z est défini comme dans la revendication 1, n = 0 ou 1, s = 0 ou 1 et n1 = 0-2.

30. Utilisation selon la revendication 28 où R¹ est t-butyle.

31. Composé de formule 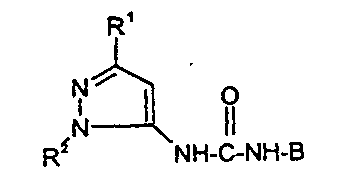 où R² est choisi dans le groupe consistant en H, -C(O)R⁴, -CO₂R⁴, -C(O)NR³R^{3'}, alkyle en C₁-C₁₀, cycloalkyle en C₃-C₁₀, alkyle en C₁-C₁₀ substitué, cycloalkyle en C₃-C₁₀ substitué, où si R² est un groupe substitué, il est substitué par un ou plusieurs substituants choisis indépendamment dans le groupe consistant en -CN, -CO₂R⁴, -C(O)-NR³R^{3'}, -NO₂, -OR⁴, -SR⁴ et halogène jusqu'à la perhalogénosubstitution,
où R³ et R^{3'} sont choisis indépendamment dans le groupe consistant en H, alkyle en C₁-C₁₀, cycloalkyle en C₃-C₁₀, alkyle en C₁-C₁₀ jusqu'à perhalogénosubstitué et cycloalkyle en C₃-C₁₀ jusqu'à perhalogénosubstitué, et
où R⁴ et R^{4'} sont choisis indépendamment dans le groupe consistant en H, alkyle en C₁-C₁₀, cycloalkyle en C₃-C₁₀, alkyle en C₁-C₁₀ jusqu'à perhalogénosubstitué et cycloalkyle en C₃-C₁₀ jusqu'à perhalogénosubstitué,
où R¹ est choisi dans le groupe consistant en alkyle en C₃-C₆, cycloalkyle en C₃-C₆, alkyle en C₃-C₆ jusqu'à perhalogénosubstutitué et cycloalkyle en C₃-C₆ jusqu'à perhalogénosubstitué ;
B est phényle, pyridinyle, indolinyle, isoquinolinyle, quinolinyle ou naphtyle ; substitué par aryle en C₆-C₁₄, hétéroaryle en C₃-C₁₃ ou -Y-Ar,
où les structures cycliques de B sont éventuellement substituées par halogène, jusqu'à perhalogéno, et éventuellement substituées par X¹ₙ
et où
n = 0-2 ; chaque X est choisi indépendamment dans le groupe de -CN, -CO₂R⁵, -C(O)R⁵, -C (O)NR⁵R^{5'}, -OR⁵, -NO₂, -NR⁵R^{5'}, alkyle en C₁-C₁₀, alcényle en C₂-C₁₀, alcoxy en C₁-C₁₀, cycloalkyle en C₃-C₁₀, -SR⁵, -NR⁵C(O)OR^{5'}, -NR⁵C(O)R^{5'}, alkyle en C₁-C₁₀ substitué, alcényle en C₂-C₁₀ substitué, alcoxy en C₁-C₁₀ substitué, cycloalkyle en C₃-C₁₀ substitué, où, si X est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ jusqu'à perhalogénosubstitué, alcényle en C₂-C₁₀ jusqu'à perhalogénosubstitué, et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre qui est éventuellement substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C (O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué, et cycloalkyle en C₃-C₁₀ substitué, si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, alkyle en C₁-C₁₀, alcoxy en C₁-C₁₀ et cycloalkyle en C₃-C₁₀.

32. Composé selon la revendication 31 où B est 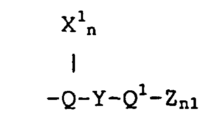 où
Y est choisi dans le groupe consistant en -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est halogène,
Q est phényle ou pyridinyle substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, éventuellement substituée par halogène jusqu'à la perhalogénosubstitution,
X¹ est alkyle en C₁-C₄ ou alkyle en C₁-C₄ halogénosubstitué jusqu'à perhalogéno,
Z, n et n1 sont définis comme dans la revendication 31.

33. Composé selon la revendication 32 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, éventuellement substitué par halogène, jusqu'à la perhalogénosubstitution, et X¹ est défini comme dans la revendication 32, et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀, et cycloalkyle en C₃-C₆, où R⁶ et R⁷ peuvent être substitués par halogène ou jusqu'à la perhalogénosubstitution.

34. Composé selon la revendication 32 où Q est phényle ou pyridinyle éventuellement substitué par halogène jusqu'à la perhalogénosubstitution, Q¹ est pyridinyle, phényle ou benzothiazolyle éventuellement substitué par halogène jusqu'à la perhalogénosubstitution,, Y est -O-, -S-, -CH₂S-, -SCH₂-, -CH₂O-, -OCH₂- ou -CH₂-, X¹ est défini comme dans la revenedication 32 et Z est -SCH₃ ou NH-C(O)-CₚH₂ₚ₊₁, où p = 1-4, n = 0 ou 1 et n1 = 0 - 1.

35. Composé de formule 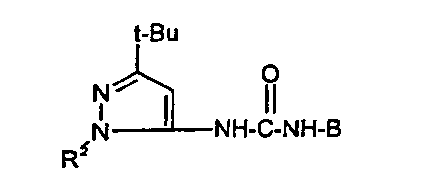 où R² est défini dans la revendication 31 et B est phényle, pyridinyle, pyrimidinyle, pyrazinyle, pyridazinyle, naphtyle, quinolinyle, isoquinolinyle, phtalimidinyle, furyle, pyrrolyle, imidazolyle, pyrazolyle, oxazolyle, isoxazolyle, thiazolyle, isothiazolyle, benzofuryle, benzothiényle, indolyle, benzopyrazolyle, benzoxazolyle, benzisoxazolyle, benzothiazolyle ou benzisothiazolyle, substitué par un ou plusieurs substituants choisis indépendamment dans le groupe consistant en halogène, jusqu'à la perhalogénosubstitution, et Xₙ, où n est 0-3 et chaque X est choisi indépendamment dans le groupe consistant en -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, hétéroaryle en C₃-C₁₃, alkyle en C₁-C₁₀ jusqu'à perhalogéno-substitué, alcényle en C₂-C₁₀ jusqu'à perhalogéno-substitué, alcoxy en C₁-C₁₀ jusqu'à perhalogénosubstitué, cycloalkyle en C₃-C₁₀ jusqu'à perhalogénosubstitué et -Y-Ar ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre éventuellement substituée par halogène jusqu'à la perhalogénosubstitution et éventuellement substituée par Zₙ₁, où n1 est 0 à 3 et chaque Z est choisi indépendamment dans le groupe consistant en -CN, =O, -CO₂R⁵, -C (O)NR⁵R^{5'}, -C(O)-NR⁵, -NO₂, -OR⁵, -SR⁵, -NR⁵R^{5'}, -NR⁵C(O)OR^{5'}, -C (O)R⁵, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ jusqu'à perhalogénosubstitué, et cycloalkyle en C₃-C₁₀ jusqu'à perhalogénosubstitué, à condition que, quand R^{a} est méthyle, B ne soit pas

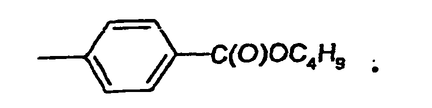

36. Composé selon la revendication 31 choisi dans le groupe consistant en :
*N*-(3-*tert*-butyl-5-pyrazolyl)-*N*'-(4-phényloxyphényl)urée ;
*N*-(3-*tert*-butyl-5-pyrazolyl)-*N*'-(3-(3-méthylaminocarbonylphényl)oxyphényl)urée ;
*N*-(3-*tert*-butyl-5-pyrazolyl)-*N*'-(3-(4-pyridinyl)thiophényl)-urée ;
*N*-(3-*tert*-butyl-5-pyrazolyl)-*N*'-(4-(4-pyridinyl)thiophényl)urée ;
*N*-(3-*tert*-butyl-5-pyrazolyl)-*N*'-(4-(4-pyridinyl)oxyphényl)-urée ;
*N*-(3-*tert*-butyl-5-pyrazolyl)-*N*'-(4-(4-pyridinyl)méthylphényl)urée ;
*N*-(1-méthyl-3-*tert*-butyl-5-pyrazolyl)-*N*'-(4-phényloxyphényl)urée ;
*N*-(1-méthyl-3-*tert*-butyl-5-pyrazolyl)-*N*'-(3-(4-pyridinyl)-thiophényl)urée ;
*N*-(1-méthyl-3-*tert*-butyl-5-pyrazolyl)-*N'*-((4-(4-pyridinyl)-thiométhyl)phényl)urée ;
*N*-(1-méthyl-3-*tert*-butyl-5-pyrazolyl)-*N*'-(4-(4-pyridinyl)-thiophényl)urée ;
*N*-(1-méthyl-3-*tert*-butyl-5-pyrazolyl)-*N'*-(4-(4-pyridinyl)-oxyphényl)urée ;
*N*-(1-méthyl-3-*tert*-butyl-5-pyrazolyl)-*N'*-((4-(4-pyridinyl)-méthyloxy)phényl)urée ;
*N*-(1-méthyl-3-*tert*-butyl-5-pyrazolyl)-*N'*-(3-(2-benzothiazolyl)oxyphényl)urée ;
*N*-(3-*tert*-butyl-5-pyrazolyl)-*N'*-(3-(4-pyridyl)thiophényl)-urée ;
*N*-(3-*tert*-butyl-5-pyrazolyl)-*N'*-(4-(4-pyridyl)thiophényl)urée ;
*N*-(3-*tert*-butyl-5-pyrazolyl)-*N'*-(4-(4-pyridyl)oxyphényl)-urée ;
*N*-(3-*tert*-butyl-5-pyrazolyl)-*N'*-(4-(4-pyridyl)méthylphényl)urée ;
*N*-(1-méthyl-3-*tert*-butyl-5-pyrazolyl)-*N'*-(3-(4-pyridyl)-thiophényl)urée ;
*N*-(1-méthyl-3-*tert*-butyl-5-pyrazolyl)-*N'*-(4-(4-pyridyl)-thiophényl)urée ;
*N*-(1-méthyl-3-*tert*-butyl-5-pyrazolyl)-*N'*-(3-(4-pyridyl)-oxyphényl)urée ;
*N*-(1-méthyl-3-*tert*-butyl-5-pyrazolyl)-*N'*-(4-(4-pyridyl)-oxyphényl)urée ;
et ses sels pharmaceutiquement acceptables.

37. Composé de formule 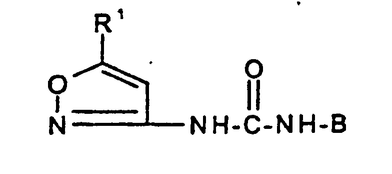 où R¹ est choisi dans le groupe consistant en alkyle en C₃-C₁₀, cycloalkyle en C₃-C₁₀, alkyle en C₃-C₁₀ jusqu'à perhalogénosubstitué et cycloalkyle en C₃-C₁₀ jusqu'à perhalogénosubstitué ;
B est phényle, pyridinyle, indolinyle, isoquinolinyle, quinolinyle ou naphtyle qui est substitué par phényle, pyridinyle ou Y-Ar,
où les structures cycliques de B sont éventuellement substituées par halogène, jusqu'à la perhalogénosubstitution, et éventuellement substituées par X¹ₙ, où n = 0-2 ;
chaque X¹ et choisi indépendamment dans le groupe consistant en -CN, -CO₂R⁵, -C(O)R⁵, -C(O)NR⁵R^{5'}, -OR⁵, -NO₂, -NR⁵R^{5'}, alkyle en C₁-C₁₀, alcényle en C₂-C₁₀, alcoxy en C₁-C₁₀, cycloalkyle en C₃-C₁₀, et aryle en C₆-C₁₄, -SR⁵, -NR⁵C(O)OR^{5'}, -NR⁵C(O)R^{5'}, hétéroaryle en C₃-C₁₃, alkyle en C₁-C₁₀ substitué, alcényle en C₂-C₁₀ substitué, alcoxy en C₁-C₁₀ substitué, cycloalkyle en C₃-C₁₀ substitué, aryle en C₆-C₁₄ substitué, hétéroaryle en C₃-C₁₃ substitué, et
où, si X¹ est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué et cycloalkyle en C₃-C₁₀ substitué ; où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, alkyle en C₁-C₁₀, alcoxy en C₁-C₁₀ et cycloalkyle en C₃-C₁₀,
soumis à la condition que quand R¹ est t-butyle, B n'est pas 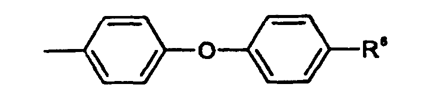 où R⁶ est -NHC(O)-O-t-butyle, -O-n-pentyle, -O-n-butyle, -O-n-propyle, -C(O)NH-(CH₃)₂, -OCH₂CH(CH₃)₂ ou

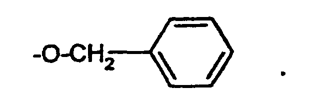

38. Composé selon la revendication 37 où B est 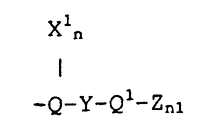 Y est choisi dans le groupe consistant en -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est halogène,
Q est phénylé ou pyridinyle substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, non substituée ou substituée par halogène jusqu'à la perhalogénosubstitution, X¹ est alkyle en C₁-C₄
ou alkyle en C₁-C₄ halogénosubstitué jusqu'à perhalogéno, et
Z, n et n1 sont définis comme dans la revendication 37.

39. Composé selon la revendication 38 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, substitué ou non substitué par halogène, jusqu'à la perhalogénosubstitution, X¹ est défini comme dans la revendication 38 et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀ et cycloalkyle en C₃-C₆, où R⁶ et R⁷ peuvent être substitués par halogène ou jusqu'à la perhalogénosubstitution.

40. Composé selon la revendication 38 où Q est phényle ou pyridinyle éventuellement substitué par halogène jusqu'à la perhalogénosubstitution, Q¹ est pyridinyle, phényle ou benzothiazolyle éventuellement substitué par halogène jusqu'à la perhalogénosubstitution, Y est -O-, -S-, -C(O)-, X¹ est défini comme dans la revendication 38 et Z est -NH-C(O)-CₚH₂ₚ₊₁, où p = 1-4, -CH₃, -OH, -OCH₃, -OC₂CH₅, -CN ou -C(O)CH₃, n = 0 ou 1 et n1 = 0 - 1.

41. Composé selon la revendication 37 choisi dans le groupe consistant en :
*N*-(5-*tert*-butyl-3-isoxazolyl)-*N*'-(4-(4-hydroxyphényl)-oxyphényl)urée ;
*N*-(5-*tert*-butyl-3-isoxazolyl)-*N*'-(4-(3-hydroxyphényl)-oxyphényl)urée ;
*N*-(5-*tert*-butyl-3-isoxazolyl)-*N*'-(4-(4-acétylphényl)-oxyphényl)urée ;
*N*-(5-*tert*-butyl-3-isoxazolyl)-*N*'-(3-benzoylphényl)urée ;
*N*-(5-*tert*-butyl-3-isoxazolyl)-*N*'-(4-phényloxyphényl)urée ;
*N*-(5-*tert*-butyl-3-isoxazolyl)-*N*'-(4-(3-méthylaminocarbonyl-phényl)thiophényl)urée ;
*N*-(5-*tert*-butyl-3-isoxazolyl)-*N*'-(4-(4-(1,2-méthylènedioxy)-phényl)oxyphényl)urée ;
*N*-(5-*tert*-butyl-3-isoxazolyl)-*N*'-(4-(3-pyridinyl)oxyphényl)urée ;
*N*-(5-*tert*-butyl-3-isoxazolyl)-*N*'-(4-(4-pyridinyl)oxyphényl)urée ;
*N-*(5-*tert*-butyl-3-isoxazolyl)-*N*'-(4-(4-pyridinyl)thiophényl)urée ;
*N*-(5-*tert*-butyl-3-isoxazolyl)-*N*'-(4-(4-pyridinyl)méthylphényl)urée ;
*N*-(5-*tert*-butyl-3-isoxazolyl)-*N*'-(3-(4-pyridinyl)oxyphényl)-urée ;
*N*-(5-*tert*-butyl-3-isoxazolyl)-*N*'-(3-(4-pyridinyl)-thiophényl)urée ;
*N*-(5-*tert*-butyl-3-isoxazolyl)-*N'*-(3-(3-méthyl-4-pyridinyl)-oxyphényl)urée ;
*N*-(5-*tert*-butyl-3-isoxazolyl)-*N'*-(3-(3-méthyl-4-pyridinyl)-thiophényl)urée ;
*N*-(5-*tert*-butyl-3-isoxazolyl)-*N'*-(4-(3-méthyl-4-pyridinyl)-thiophényl)urée ;
*N*-(5-*tert*-butyl-3-isoxazolyl)-*N'*-(3-(4-méthyl-3-pyridinyl)-oxyphényl)urée ;
*N*-(5-*tert*-butyl-3-isoxazolyl)-*N'*-(4-(3-méthyl-4-pyridinyl)-oxyphényl)urée ;
*N*-(5-*tert*-butyl-3-isoxazolyl)-*N*'-(3-(2-benzothiazolyl)-oxyphényl)urée ;
*N*-(5-*tert*-butyl-3-isoxazolyl)-*N*'-(3-chloro-4-(4-(2-méthylcarbamoyl)pyridyl)oxyphényl)urée ;
*N*-(5-*tert*-butyl-3-isoxazolyl)-*N*'-(4-(4-(2-méthylcarbamoyl)-pyridyl)oxyphényl)urée ;
*N*-(5-*tert*-butyl-3-isoxazolyl)-*N*'-(3-(4-(2-méthylcarbamoyl)-pyridyl)thiophényl)urée ;
*N*-(5-*tert*-butyl-3-isoxazolyl)-*N*'-(2-méthyl-4-(4-(2-méthylcarbamoyl)pyridyl)oxyphényl)urée ;
*N*-(5-*tert*-butyl-3-isoxazolyl)-*N'*-(4-(4-(2-carbamoyl)-pyridyl)oxyphényl)urée ;
*N*-(5-*tert*-butyl-3-isoxazolyl)-*N'*-(3-(4-(2-carbamoyl)-pyridyl)oxyphényl)urée ;
*N*-(5-*tert*-butyl-3-isoxazolyl)-*N'*-(3-(4-(2-méthylcarbamoyl)-pyridyl)oxyphényl)urée ;
*N*-(5-*tert*-butyl-3-isoxazolyl)-*N'*-(4-(4-(2-méthylcarbamoyl)-pyridyl)thiophényl)urée ;
*N*-(5-*tert*-butyl-3-isoxazolyl)-*N'*-(3-chloro-4-(4-(2-méthylcarbamoyl)pyridyl)oxyphényl)urée ;
*N*-(5-*tert*-butyl-3-isoxazolyl)-*N'*-(4-(3-méthylcarbamoyl)-phényl)oxyphényl)urée ;
et ses sels pharmaceutiquement acceptables.

42. Composé de formule 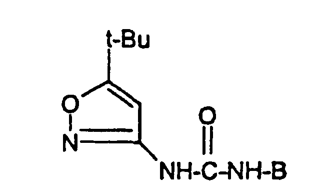 où B est 5-méthyl-2-thiényle ou
choisi dans le groupe consistant en phényle, pyridinyle, pyrimidinyle, pyrazinyle, pyridazinyle, naphtyle, quinolinyle, isoquinolinyle, phtalimidinyle, furyle, pyrrolyle, imidazolyle, pyrazolyle, oxazolyle, isoxazolyle, thiazolyle, isothiazolyle, benzofuryle, benzothiényle, indolyle, benzopyrazolyle, benzoxazolyle, benzisoxazolyle, benzothiazolyle ou benzisothiazolyle, substitué par un ou plusieurs substituants choisis indépendamment dans le groupe consistant en halogène, jusqu'à la perhalogénosubstitution, et Xₙ, où n est 0-3 et chaque X est choisi indépendamment dans le groupe consistant en -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, hétéroaryle en C₃-C₁₃, alkyle en C₁-C₁₀ jusqu'à perhalogéno-substitué, alcényle en C₂-C₁₀ jusqu'à perhalogéno-substitué, alcoxy en C₁-C₁₀ jusqu'à perhalogénosubstitué, et cycloalkyle en C₃-C₁₀ jusqu'à perhalogénosubstitué ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S- (CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre éventuellement substituée par halogène jusqu'à la perhalogénosubstitution et éventuellement substituée par Zₙ₁, où n1 est 0 à 3 et chaque Z est choisi indépendamment dans le groupe consistant en -CN, =O, -CO₂R⁵, -C(O)NR⁵R^{5'}, -C(O)-NR⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ jusqu'à perhalogénosubstitué, et cycloalkyle en C₃-C₁₀ jusqu'à perhalogénosubstitué ; soumis à la condition que B n'est pas 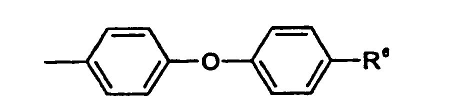 où R⁶ est -NHC(O)-O-t-butyle, -O-n-pentyle, -O-n-butyle, -O-n-propyle, -C(O)NH-(CH₃)₂, -OCH₂CH(CH₃)₂ ou

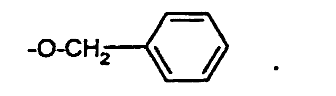

43. Composé de formule 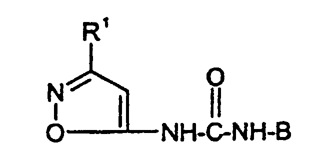 où R¹ est choisi dans le groupe consistant en alkyle en C₃-C₁₀, cycloalkyle en C₃-C₁₀, alkyle en C₃-C₁₀ jusqu'à perhalogénosubstitué et cycloalkyle en C₃-C₁₀ jusqu'à perhalogénosubstitué ; et
B est phényle, pyridinyle, indolinyle, isoquinolinyle, quinolinyle ou naphtyle qui est substitué par X, éventuellement substitué par halogène, jusqu'à la perhalogénosubstitution, et éventuellement substitué par X¹ₙ, où n = 0-2 ;
chaque X¹ et choisi indépendamment dans le groupe de X ou dans le groupe consistant en -CN, -CO₂R⁵, -C(O)R⁵, -C(O)NR⁵R^{5'}, -OR⁵, -NO₂, -NR⁵R^{5'}, alkyle en C₁-C₁₀, alcényle en C₂-C₁₀, alcoxy en C₁-C₁₀, cycloalkyle en C₃-C₁₀, aryle en C₆-C₁₄ et alkylaryle en C₇-C₂₄, et
X est choisi dans le groupe consistant en -SR⁵, -NR⁵C(O)OR^{5'}, -NR⁵C(O)R^{5'}, hétéroaryle en C₃-C₁₃, alkyle en C₁-C₁₀ substitué, alcényle en C₂-C₁₀ substitué, alcoxy en C₁-C₁₀ substitué, cycloalkyle en C₃-C₁₀ substitué, aryle en C₆-C₁₄ substitué, hétéroaryle en C₃-C₁₃ substitué et -Y-Ar, et où, si X est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ jusqu'à perhalogénosubstitué, alcényle en C₂-C₁₀ jusqu'à perhalogénosubstitué, et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué et cycloalkyle en C₃-C₁₀ substitué, où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'} et -NR⁵C(O)OR^{5'}, alkyle en C₁-C₁₀, alcoxy en C₁-C₁₀ et cycloalkyle en C₃-C₁₀,
et quand R¹ est -CH₂-t-butyle,
B n'est pas

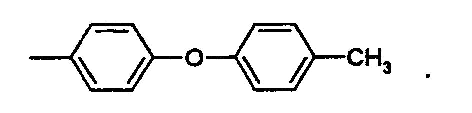

44. Composé selon la revendication 43 où B est 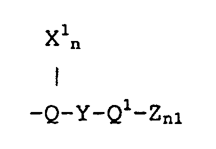 où
Y est choisi dans le groupe consistant en -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est halogène,
Q est phényle ou pyridinyle substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, non substituée ou substituée par halogène jusqu'à la perhalogénosubstitution,
Z, n et n1 sont définis comme dans la revendication 43 et X¹ est alkyle en C₁-C₄ ou alkyle en C₁-C₄ halogénosubstitué jusqu'à perhalogéno.

45. Composé selon la revendication 44 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, éventuellement substitué par halogène, jusqu'à la perhalogénosubstitution, X¹ est défini comme dans la revendication 44, et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀ et cycloalkyle en C₃-C₆ où R⁶ et R⁷ peuvent être substitués par halogène ou jusqu'à la perhalogénosubstitution.

46. Composé de formule 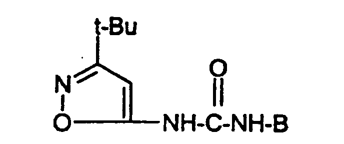 où B est défini comme dans la revendication 1.

47. Composé selon la revendication 44 où Q est phényle ou pyridinyle éventuellement substitué par halogène jusqu'à la perhalogénosubstitution, Q¹ est phényle, benzothiazolyle ou pyridinyle éventuellement substitué par halogène jusqu'à la perhalogénosubstitution,, Y est -O-, -S- ou -CH₂-, X¹ est défini comme dans la revendication 44, n =0 ou 1, Z est -CH₃, Cl-, OC₂H₅ ou -OCH₃ et n1 = 0 ou 1.

48. Composé selon la revendication 43 choisi dans le groupe consistant en :
*N*-(3-isopropyl-5-isoxazolyl)-*N'*-(4-(4-pyridinyl)thiophényl)-urée ;
*N*-(3-*tert*-butyl-5-isoxazolyl)-*N'*-(4-(4-méthoxyphényl)oxyphényl)urée ;
*N*-(3-*tert*-butyl-5-isoxazolyl)-*N'*-(5-(2-(4-acétylphényl)oxy)-pyridinyl)urée ;
*N*-(3-*tert*-butyl-5-isoxazolyl)-*N'*-(3-(4-pyridinyl)thiophényl)urée ;
*N*-(3-*tert*-butyl-5-isoxazolyl)-*N'*-(4-(4-pyridinyl)méthylphényl)urée ;
*N*-(3-*tert*-butyl-5-isoxazolyl)-*N'*-(4-(4-pyridinyl)thiophényl)urée ;
*N*-(3-*tert*-butyl-5-isoxazolyl)-*N*'-(4-(4-pyridinyl)oxyphényl)urée ;
*N*-(3-*tert*-butyl-5-isoxazolyl)-*N*'-(3-(2-benzothiazolyl)oxyphényl)urée ;
*N*-(3-(1,1-diméthylpropyl)-5-isoxazolyl)-*N*'-(4-(4-méthylphényl)oxyphényl)urée ;
*N*-(3-(1,1-diméthylpropyl)-5-isoxazolyl)-*N*'-(3-(4-pyridinyl)-thiophényl)urée ;
*N*-(3-(1,1-diméthylpropyl)-5-isoxazolyl)-*N*'-(4-(4-pyridinyl)oxyphényl)urée ;
*N*-(3-(1,1-diméthylpropyl)-5-isoxazolyl)-*N*'-(4-(4-pyridinyl)-thiophényl)urée ;
*N*-(3-(1,1-diméthylpropyl)-5-isoxazolyl)-*N*'-(5-(2-(4-méthoxyphényl)oxy)pyridinyl)urée ;
*N*-(3-(1-méthyl-1-éthylpropyl)-5-isoxazolyl)-*N'*-(4-(4-pyridinyl)oxyphényl)urée ;
*N*-(3-(1-méthyl-1-éthylpropyl)-5-isoxazolyl)-*N'*-(3-(4-pyridinyl)thiophényl)urée ;
*N*-(3-isopropyl-5-isoxazolyl)-*N'*-(3-(4-(2-méthylcarbamoyl)-pyridyl)oxyphényl)urée ;
*N*-(3-isopropyl-5-isoxazolyl)-*N'*-(4-(4-(2-méthylcarbamoyl)-pyridyl)oxyphényl)urée ;
*N*-(3-*tert*-butyl-5-isoxazolyl)-*N'*-(3-(4-(2-méthylcarbamoyl)-pyridyl)oxyphényl)urée ;
*N*-(3-*tert*-butyl-5-isoxazolyl)-*N'*-(4-(4-(2-méthylcarbamoyl)-pyridyl)oxyphényl)urée ;
*N*-(3-*tert*-butyl-5-isoxazolyl)-*N'*-(3-(4-(2-méthylcarbamoyl)-pyridyl)thiophényl)urée ;
*N*-(3-(1,1-diméthylprop-1-yl)-5-isoxazolyl)-*N'*-(3-(4-(2-méthylcarbamoyl)pyridyl)oxyphényl)urée ;
*N*-(3-(1,1-diméthylprop-1-yl)-5-isoxazolyl)-*N'*-(4-(4-(2-méthylcarbamoyl)pyridyl)oxyphényl)urée ;
*N*-(3-*tert*-butyl-5-isoxazolyl)-*N'*-(3-chloro-4-(4-(2-méthylcarbamoyl)pyridyl)thiophényl)urée ;
et ses sels pharmaceutiquement acceptables.

49. Composé de formule 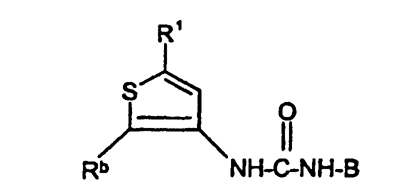 où R¹ est choisi dans le groupe consistant en alkyle en C₃-C₁₀, cycloalkyle en C₃-C₁₀, alkyle en C₁-C₁₀ jusqu'à perhalogénosubstitué et cycloalkyle en C₃-C₁₀ jusqu'à perhalogénosubstitué ;
R^{b} est l'hydrogène ou un halogène et
B est phényle, pyridinyle, indolinyle, isoquinolinyle, quinolinyle ou naphtyle substitué par phényle, pyridinyle ou -Y-Ar,
où les structures cycliques de B sont éventuellement substituées par halogène, jusqu'à perhalogéno, et
éventuellement substitué par X¹ₙ
et où n = 0-2 ; chaque X¹ et choisi indépendamment dans le groupe consistant en -CN, -OR⁵, -NR⁵R^{5'}, alkyle en C₁-C₁₀ ; -CO₂R⁵, -C(O)NR⁵R^{5'}, -C(O)R⁵, -NO₂, -SR⁵, -NR⁵C(O)OR^{5'}, -NR⁵C(O)R^{5'}, cycloalkyle en C₃-C₁₀, alkyle en C₁-C₁₀ substitué, alcényle en C₂-C₁₀ substitué, alcoxy en C₁-C₁₀ substitué et cycloalkyle en C₃-C₁₀ substitué,
où, si X¹ est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-, m = 1-3, et X^{a} est halogène ; et
Ar est une structure aromatique à 5-10 membres contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)-NR⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué et cycloalkyle en C₃-C₁₀ substitué ; où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, alkyle en C₁-C₁₀, alcoxy en C₁-C₁₀ et cycloalkyle en C₃-C₁₀,
soumis à la condition que quand R¹ est t-butyle et R^{b} est H, B n'est pas de formule

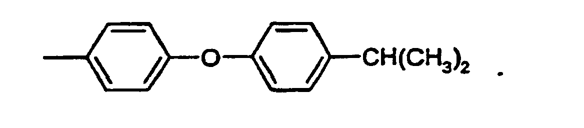

50. Composé selon la revendication 49 où B est 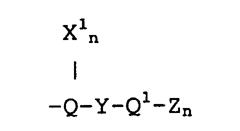 où
Y est choisi dans le groupe consistant en -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est halogène,
Q est phényle ou pyridinyle substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, éventuellement substituée par halogène jusqu'à la perhalogénosubstitution,
X¹ est alkyle en C₁-C₄ ou alkyle en C₁-C₄ halogénosubstitué jusqu'à perhalogéno, et
Z, n et n1 sont définis comme dans la revendication 49.

51. Composé selon la revendication 50 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, substitué ou non substitué par halogène, jusqu'à la perhalogénosubstitution, X¹ est défini comme dans la revendication 50, et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀ et cycloalkyle en C₃-C₆ où R⁶ et R⁷ peuvent être substitués par halogène ou jusqu'à la perhalogénosubstitution.

52. Composé de formule 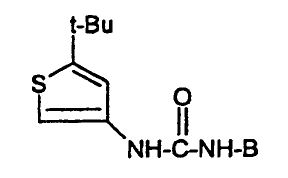 où B est défini comme dans la revendication 1, soumis à la condition que B n'est pas de formule

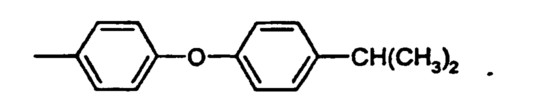

53. Composé selon la revendication 50 où Q est phényle éventuellement substitué par halogène jusqu'à la perhalogénosubstitution, Q¹ est phényle ou pyridinyle éventuellement substitué par halogène jusqu'à la perhalogénosubstitution, et Y est -O- ou -S-, Z est -Cl, -CH₃, -OH ou -OCH₃, X¹ est défini comme dans la revendication 50, n = 0 ou 1 et n1 = 0-2.

54. Composé selon la revendication 49 choisi dans le groupe consistant en :
*N*-(5-*tert*-butyl-3-thiényl)-*N*'-(4-(3-méthylphényl)oxyphényl)-urée ;
*N*-(5-*tert*-butyl-3-thiényl)-*N*'-(4-(4-hydroxyphényl)oxyphényl)urée ;
*N*-(5-*tert*-butyl-3-thiényl)-*N*'-(4-(4-méthoxyphényl)oxyphényl)urée ;
*N*-(5-*tert*-butyl-3-thiényl)-*N*'-(4-(4-pyridinyl)thiophényl)-urée ;
et ses sels pharmaceutiquement acceptables.

55. Composé de formule : 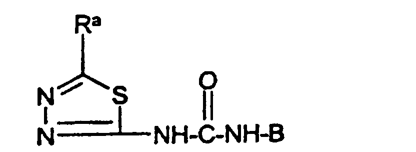 où R^{a} est alkyle en C₁-C₁₀, cycloalkyle en C₃-C₁₀, jusqu'à perhalogénosubstitué, alkyle en C₁-C₁₀ et cycloalkyle en C₃-C₁₀ jusqu'à perhalogénosubstitué ;
et B est phényle, pyridinyle, indolinyle, isoquinolinyle, quinolinyle ou naphtyle ; substitué par phényle, pyridinyle ou -Y-Ar,
où les structures cycliques de B sont éventuellement substituées par halogène, jusqu'à perhalogéno, et
éventuellement substitué par X¹ₙ
où n = 0-2 ;
chaque X¹ et choisi indépendamment dans le groupe consistant en -CN, -NO₂, -OR⁵ et alkyle en C₁-C₁₀, -SR⁵, -CO₂R⁵, -C(O)R⁵, -C(O)NR⁵R^{5'}, -NR⁵R^{5'}, -NR⁵C(O)OR^{5'}, -NR⁵C(O)R^{5'}, cycloalkyle en C₃-C₁₀, alkyle en C₁-C₁₀ substitué, alcényle en C₂-C₁₀ substitué, alcoxy en C₁-C₁₀ substitué et cycloalkyle en C₃-C₁₀ substitué,
où, si X¹ est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ substitué, alcényle en C₂-C₁₀ substitué, alcoxy en C₁-C₁₀ substitué et cycloalkyle en C₃-C₁₀ substitué,
où, si X¹ est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-, m = 1-3, et X^{a} est halogène ; et
Ar est une structure aromatique à 5-10 membres contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)-NR⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué, cycloalkyle en C₃-C₁₀ substitué ; où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, alkyle en C₁-C₁₀, alcoxy en C₁-C₁₀ et cycloalkyle en C₃-C₁₀.

56. Composé selon la revendication 55 où B est 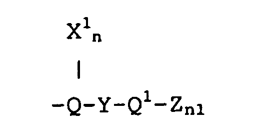 où
Y est choisi dans le groupe consistant en -O-, -S-, -CH₂-, -SCH₂-, -CH₂S-, -CH (OH)-, -C(O)-, -CX^{a}₂, -CX^{a}H-, -CH₂O-, -OCH₂-,
X^{a} est halogène,
Q est phényle ou pyridinyle substitué ou non substitué par halogène, jusqu'à la perhalogénosubstitution ;
Q¹ est phényle, pyridinyle, naphtyle, pyrimidinyle, quinoléine, isoquinoléine, imidazolyle ou benzothiazolyle, éventuellement substitué par halogène jusqu'à la perhalogénosubstitution,
X¹ est alkyle en C₁-C₄ ou alkyle en C₁-C₄ halogénosubstitué jusqu'à perhalogéno,
Z, n et n1 sont définis comme dans la revendication 55 et s est 0 ou 1.

57. Composé selon la revendication 56 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, éventuellement substitué par halogène, jusqu'à la perhalogénosubstitution,
X¹ est défini comme dans la revendication 56, et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀, cycloalkyle en C₃-C₆ et où R⁶ et R⁷ peuvent être substitués par halogène ou jusqu'à la perhalogénosubstitution.

58. Composé selon la revendication 56 où Q est phényle éventuellement substitué par halogène jusqu'à la perhalogénosubstitution, Q¹ est phényle ou pyridinyle éventuellement substitué par halogène jusqu'à la perhalogénosubstitution,, Y est -O- ou -S-, X¹ est défini comme dans la revendication 56, n = 0 ou 1 et n1 = 0.

59. Composé selon la revendication 55, de formule 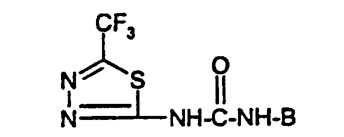 où B est phényle, pyridinyle, indolinyle, isoquinolinyle, quinolinyle ou naphtyle ; substitué par phényle, pyridinyle ou -Y-Ar, éventuellement substitué par halogène, jusqu'à perhalogéno, et
où chaque structure cyclique de B est éventuellement substituée par X¹ₙ
où n = 0-2 ; chaque X¹ et choisi indépendamment dans le groupe consistant en -CN, -OR⁵, alkyle en C₁-C₁₀ ; -CO₂R⁵, -C (O)NR⁵R^{5'}, -C (O)R⁵, -NO₂, -SR⁵, -NR⁵R^{5'}, -NR⁵C(O)OR^{5'}, -NR⁵C(O)R^{5'}, cycloalkyle en C₃-C₁₀ et alkyle en C₁-C₁₀ substitué, alcényle en C₂-C₁₀ substitué, alcoxy en C₁-C₁₀ substitué et cycloalkyle en C₃-C₁₀ substitué,
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué, cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-, m = 1-3, et X^{a} est halogène ; et
Ar est une structure aromatique contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)-NR⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué, cycloalkyle en C₃-C₁₀ substitué ; où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, alkyle en C₁-C₁₀, alcoxy en C₁-C₁₀ et cycloalkyle en C₃-C₁₀.

60. Composé selon la revendication 59 choisi dans le groupe consistant en :
*N*-(5-*tert*-butyl-2-(1-thia-3,4-diazolyl))-*N'*-(3-(4-pyridinyl)thiophényl)urée ;
*N*-(5-*tert*-butyl-2-(1-thia-3,4-diazolyl))-*N'*-(4-(4-pyridinyl)oxyphényl)urée ;
*N*-(5-*tert*-butyl-2-(1-thia-3,4-diazolyl))-*N'*-(3-(4-(2-méthylcarbamoyl)pyridyl)oxyphényl)urée ;
*N*-(5-*tert*-butyl-2-(1-thia-3,4-diazolyl))-*N'*-(4-(4-(2-méthylcarbamoyl)pyridyl)oxyphényl)urée ;
*N*-(5-*tert*-butyl-2-(1-thia-3,4-diazolyl))-*N'*-(3-chloro-4-(4-(2-méthylcarbamoyl)pyridinyl)oxyphényl)urée ;
*N*-(5-*tert*-butyl-2-(1-thia-3,4-diazolyl))-*N'*-(2-chloro-4-(4-(2-méthylcarbamoyl)pyridyl)oxyphényl)urée ;
*N*-(5-*tert*-butyl-2-(1-thia-3,4-diazolyl))-*N'*-(3-(4-pyridyl)thiophényl)urée ;
*N*-(5-*tert*-butyl-2-(1-thia-3,4-diazolyl))-*N'*-(2-méthyl-4-(4-(2-méthylcarbamoyl)pyridyl)oxyphényl)urée ;
*N*-(5-(1,1-diméthylprop-1-yl)-2-(1-thia-3,4-diazolyl))-*N'*-(4-(3-carbamoylphényl)oxyphényl)urée ;
et ses sels pharmaceutiquement acceptables.

61. Composé de l'une des formules : 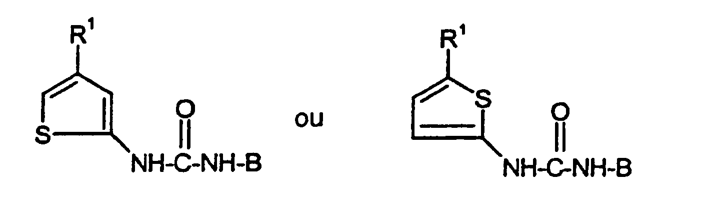 où R¹ est choisi dans le groupe consistant en halogène, alkyle en C₃-C₁₀, hétéroaryle en C₁-C₁₃, aryle en C₆-C₁₄, alkylaryle en C₇-C₂₄, cycloalkyle en C₃-C₁₀, alkyle en C₁-C₁₀ jusqu'à perhalogénosubstitué, cycloalkyle en C₃-C₁₀ jusqu'à perhalogénosubstitué, hétéroaryle en C₁-C₁₃ jusqu'à perhalogénosubstitué, aryle en C₆-C₁₄ jusqu'à perhalogénosubstitué et alkylaryle en C₇-C₂₄ jusqu'à perhalogénosubstitué ;
B est phényle, pyridinyle, indolinyle, isoquinolinyle, quinolinyle ou naphtyle substitué par phényle, pyridinyle ou -Y-Ar,
où les structures cycliques de B sont éventuellement substituées par halogène, jusqu'à perhalogéno, et
éventuellement substituée par X¹ₙ
où n = 0-2 ;
chaque X¹ et choisi indépendamment dans le groupe consistant en -CN, -OR⁵, -NR⁵R^{5'}, alkyle en C₁-C₁₀ ; -CO₂R⁵, -C (O)NR⁵R^{5'}, -C(O)R⁵, =O, -NO₂, -SR⁵, -NR⁵C(O)OR^{5'}, -NR⁵C(O)R^{5'}, cycloalkyle en C₃-C₁₀, alkyle en C₁-C₁₀ substitué, alcényle en C₂-C₁₀ substitué, alcoxy en C₁-C₁₀ substitué et cycloalkyle en C₃-C₁₀ substitué,
où, si X¹ est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ jusqu'à perhalogénosubstitué, alcényle en C₂-C₁₀ jusqu'à perhalogénosubstitué, cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S-(CH₂)ₘ- et -N(R⁵)(CH₂)ₘ-,
m = 1-3, et X^{a} est halogène ; et
Ar est une structure aromatique contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)-NR⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, alcoxy en C₁-C₁₀, cycloalkyle en C₃-C₁₀, alkyle en C₁-C₁₀ substitué, cycloalkyle en C₃-C₁₀ substitué ; où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, alkyle en C₁-C₁₀, alcoxy en C₁-C₁₀ et cycloalkyle en C₃-C₁₀.

62. Composé de l'une des formules 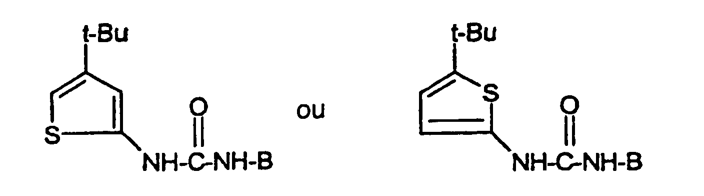 où B est défini comme dans la revendication 1.

63. Composé selon la revendication 61 où B est 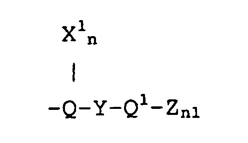 où
Y est choisi dans le groupe consistant en -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est halogène,
Q est phényle ou pyridinyle substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, non substituée ou substituée par halogène jusqu'à la perhalogénosubstitution,
X¹ est alkyle en C₁-C₄ ou alkyle en C₁-C₄ halogénosubstitué jusqu'à perhalogéno,
Z, n et n1 sont définis comme dans la revendication 61 ou 1.

64. Composé selon la revendication 63 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, substitué ou non substitué par halogène, jusqu'à la perhalogénosubstitution, X¹ est défini comme dans la revendication 63 et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀ ou cycloalkyle en C₃-C₆, où R⁶ et R⁷ peuvent être substitués par halogène ou jusqu'à la perhalogénosubstitution.

65. Composé selon la revendication 63 où Q est phényle éventuellement substitué jusqu'à la perhalogénosubstitution, Q¹ est phényle ou pyridinyle éventuellement substitué jusqu'à la perhalogénosubstitution,, et Y est -O- ou -S-, X¹ est défini comme dans la revendication 63, n = 0 ou 1, Z est -Cl, -CH₃, -OH ou OCH₃, et n1 = 0-2.

66. Composé de formule 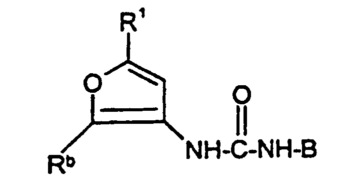 où R¹ est choisi dans le groupe consistant en alkyle en C₃-C₁₀, cycloalkyle en C₃-C₁₀, alkyle en C₁-C₁₀ jusqu' à perhalogénosubstitué et cycloalkyle en C₃-C₁₀ jusqu'à perhalogénosubstitué, R^{b} est l'hydrogène ou halogène et où B est phényle, pyridinyle, indolinyle, isoquinolinyle, quinolinyle ou naphtyle substitué par phényle, pyridinyle ou -Y-Ar,
où les structures cycliques de B sont éventuellement substituées par halogène, jusqu'à perhalogéno, et
éventuellement substitué par X¹ₙ
n = 0-3 et
chaque X¹ est choisi indépendamment dans le groupe consistant en -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, alkyle en C₁-C₁₀ substitué, alcényle en C₂-C₁₀ substitué, alcoxy en C₁-C₁₀ substitué et cycloalkyle en C₃-C₁₀ substitué,
où, si X¹ est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénosubstitution ;
où R⁵ et R^{5'} sont choisis indépendamment parmi H, alkyle en C₁-C₁₀, alcényle en C₂-C₁₀, cycloalkyle en C₃-C₁₀, alkyle en C₁-C₁₀ usqu'à perhalogénosubstitué, alcényle en C₂-C₁₀ jusqu'à perhalogénosubstitué et cycloalkyle en C₃-C₁₀ jusqu'à perhalogénosubstitué,
où Y est -O-, -S-, -N(R⁵)-, -(CH₂)ₘ-, -C(O)-, -CH(OH)-, -(CH₂)ₘO-, NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}, -CX^{a}₂-, -S- (CH₂)ₘ- et -N(R⁵)(CH₂)ₘ-,
m = 1-3, et X^{a} est halogène ; et
Ar est une structure aromatique à 5-10 membres contenant 0-4 membres du groupe consistant en l'azote, l'oxygène et le soufre qui est non substituée ou substituée par halogène jusqu'à la perhalogénosubstitution et éventuellement substituée par Zₙ₁, où n1 est 0 à 3 et chaque Z est choisi indépendamment dans le groupe consistant en -CN, -CO₂R⁵, -C(O)R⁵, =O, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, -SO₂R⁵, -SO₂NR⁵R^{5'}, alkyle en C₁-C₁₀, cycloalkyle en C₃-C₁₀, alkyle en C₁-C₁₀ substitué, cycloalkyle en C₃-C₁₀ substitué, où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O) OR^{5'}, alkyle en C₁-C₁₀, alcoxy en C₁-C₁₀ et cycloalkyle en C₃-C₁₀.

67. Composé selon la revendication 66 où B est 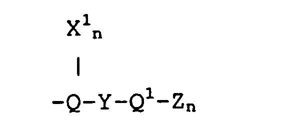 où
Y est choisi dans le groupe consistant en -O-, -S-, -CH₂-, -SCH₂-, -CH₂S-, -CH(OH)-, -C(O)-, -CX^{a}₂, -CX^{a}H-, -CH₂O- et -OCH₂-,
X^{a} est halogène,
Q est phényle ou pyridinyle, substitué ou non substitué par halogène, jusqu'à la perhalogénosubstitution ;
Q¹ est une structure aromatique mono- ou bicyclique de 3 à 10 atomes de carbone et 0-4 membres du groupe consistant en N, O et S, non substituée ou substituée par halogène jusqu'à la perhalogénosubstitution,
X¹ est alkyle en C₁-C₄ ou alkyle en C₁-C₄ halogénosubstitué jusqu'à perhalogéno,et
Z, n et n1 sont définis comme dans la revendication 66.

68. Composé selon la revendication 67 où
Q est phényle ou pyridinyle, substitué ou non substitué par halogène, jusqu'à la perhalogénosubstitution,
Q¹ est choisi dans le groupe consistant en phényle, pyridinyle, naphtyle, pyrimidinyle, quinoléine, isoquinoléine, imidazole et benzothiazolyle, substitué ou non substitué par halogène, jusqu'à la perhalogénosubstitution, X¹ est défini comme dans la revendication 67 et
Z est choisi dans le groupe consistant en -R⁶, -OR⁶ et -NHR⁷, où R⁶ est l'hydrogène, alkyle en C₁-C₁₀ ou cycloalkyle en C₃-C₁₀ et R⁷ est choisi dans le groupe consistant en l'hydrogène, alkyle en C₃-C₁₀ et cycloalkyle en C₃-C₆ où R⁶ et R⁷ peuvent être substitués par halogène ou jusqu'à la perhalogénosubstitution.

69. Composé de formule 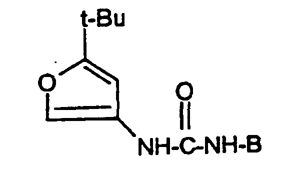 où B est défini comme dans la revendication 1.

70. Composé selon la revendication 67
où Q est phényle éventuellement substitué par halogène jusqu'à la perhalogénosubstitution, Q¹ est phényle ou pyridinyle éventuellement substitué par halogène jusqu'à la perhalogénosubstitution, et Y est -O- ou -S-, X¹ est défini comme dans la revendication 67, Z est -Cl ou -OCH₃, n = 0, s = 0 et n1 = 0-2.

71. Composition pharmaceutique comprenant un composé selon la revendication 31 et un support physiologiquement acceptable.

72. Composition pharmaceutique comprenant un composé selon la revendication 37 et un support physiologiquement acceptable.

73. Composition pharmaceutique comprenant un composé selon la revendication 43 et un support physiologiquement acceptable.

74. Composition pharmaceutique comprenant un composé selon la revendication 49 et un support physiologiquement acceptable.

75. Composition pharmaceutique comprenant un composé selon la revendication 55 et un support physiologiquement acceptable.

76. Composition pharmaceutique comprenant un composé selon la revendication 61 et un support physiologiquement acceptable.

77. Composition pharmaceutique comprenant un composé selon la revendication 66 et un support physiologiquement acceptable.

78. Utilisation selon la revendication 1 où B est phényle, pyridinyle, pyrimidinyle, pyrazinyle, pyridazinyle, naphtyle, quinolinyle, isoquinolinyle, phtalimidinyle, furyle, pyrrolyle, imidazolyle, pyrazolyle, oxazolyle, isoxazolyle, thiazolyle, isothiazolyle, benzofuryle, benzothiényle, indolyle, benzopyrazolyle, benzoxazolyle, benzisoxazolyle, benzothiazolyle ou benzisothiazolyle, substitué par -Y-Ar et éventuellement substitué par halogène jusqu'à la perhalogénosubstitution, alkyle en C₁-C₁₀ et alkyle en C₁-C₁₀ jusqu'à perhalogéno-substitué, où Y et Ar sont définis comme dans la revendication 1.

79. Utilisation selon la revendication 1 où B est
a) phényle, pyridinyle, naphtyle, quinolinyle ou isoquinolinyle, substitué par -Y-Ar et éventuellement substitué par halogène jusqu'à la perhalogénosubstitution, alkyle en C₁-C₁₀ et alkyle en C₁-C₁₀ jusqu'à perhalogéno-substitué, où Y et Ar sont définis comme dans la revendication 1 ; ou
b) indolyle substitué par aryle en C₆-C₁₄ ou hétéroaryle en C₃-C₁₃.
